(19) Europäisches Patentamt
European Patent Office
Office européen des brevets

(11) EP 1 776 350 B1

(12) # EUROPEAN PATENT SPECIFICATION

(45) Date of publication and mention of the grant of the patent:
**23.01.2013 Bulletin 2013/04**

(21) Application number: **05786778.0**

(22) Date of filing: **08.08.2005**

(51) Int Cl.:
***C07D 239/84*** *(2006.01)* ***A61K 31/517*** *(2006.01)*

(86) International application number:
**PCT/US2005/028340**

(87) International publication number:
**WO 2006/017844 (16.02.2006 Gazette 2006/07)**

(54) **NOVEL 2-AMINO-QUINAZOLINE DERIVATIVES USEFUL AS INHIBITORS OF ß-SECRETASE ( BACE )**

NEUE, ALS INHIBITOREN VON ß-SECRETASE (BACE) NÜTZLICHE 2-AMINO-CHINAZOLINDERIVATE

NOUVEAUX DERIVES DE 2-AMINO-QUINAZOLINE UTILES EN TANT QU'INHIBITEURS DE LA ß-SECRETASE (BACE)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.08.2004 US 599317 P**

(43) Date of publication of application:
**25.04.2007 Bulletin 2007/17**

(73) Proprietors:
- **Janssen Pharmaceutica NV
2340 Beerse (BE)**
- **Baxter, Ellen
Glenside PA 19038 (US)**
- **Boyd, Robert
Horsham PA 19044 (US)**
- **Coats, Steve
Quakertown PA 18951 (US)**
- **Jordan, Alfonzo
North Wales PA 19454 (US)**
- **Reitz, Allen
Lansdale PA 19446 (US)**
- **Reynolds, Charles H.
Lansdale PA 19477 (US)**
- **Scott, Malcom
Telford PA 18969 (US)**
- **Schulz, Mark
Skippack PA 19474 (US)**
- **De Winter, Hans Louis Jos
2970 Schilde (BE)**

(72) Inventors:
- **BAXTER, Ellen
Glenside, PA 19038 (US)**
- **BOYD, Robert
Horsham, PA 19044 (US)**
- **COATS, Steve
Quakertown, PA 18951 (US)**
- **JORDAN, Alfonzo
North Wales, PA 19454 (US)**
- **REITZ, Allen
Lansdale, PA 19446 (US)**
- **REYNOLDS, Charles H.
Lansdale, PA 19477 (US)**
- **SCOTT, Malcolm
Telford, PA 18969 (US)**
- **SCHULZ, Mark
Skippack, PA 19474 (US)**

(74) Representative: **Williams, Paul Edwin et al
Ablett & Stebbing
Caparo House
101-103 Baker Street
London W1U 6FQ (GB)**

(56) References cited:
**EP-A- 1 407 774 WO-A-01/38315**

- **KIENZLE, FRANK ET AL: "1,5-Dihydroimidazoquinazolinones as blood platelet aggregation inhibitors" EUROPEAN JOURNAL OF MEDICINAL CHEMISTRY , 17(6), 547-56 CODEN: EJMCA5; ISSN: 0009-4374, 1982, XP009059097**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- **WEBB, THOMAS HAND WILCOX: "Improved Synthesis of Symmetrical and Unsymmterical 5,11-methandibenzo[b,f][1,5]-diazocines. Readily Available Nanoscale Structural Units" JOURNAL OF ORGANIC CHEMISTRY, vol. 55, no. 1, 1990, pages 363-365, XP002359778**

## Description

FIELD OF THE INVENTION

The present invention is directed to novel 2-amino-3,4-dihydroquinazoline derivatives, pharmaceutical compositions containing them and their use in the treatment of Alzheimer's disease (AD), mild cognitive impairment, senility and / or dementia. The compounds of the present invention are inhibitors of β-secretase, also known as β-site amyloid cleaving enzyme, BACE, BACE1, Asp2, or memapsin2.

BACKGROUND OF THE INVENTION

Alzheimer's Disease (AD) is a neurodegenerative disease associated with aging. AD patients suffer from cognition deficits and memory loss as well as behavioral problems such as anxiety. Over 90% of those afflicted with AD have a sporadic form of the disorder while less than 10% of the cases are familial or hereditary. In the United States, about 1 in 10 people at age 65 have AD while at age 85, 1 out of every two individuals are affected with AD. The average life expectancy from the initial diagnosis is 7-10 years, and AD patients require extensive care either in an assisted living facility which is very costly or by family members. With the increasing number of elderly in the population, AD is a growing medical concern. Currently available therapies for AD merely treat the symptoms of the disease and include acetylcholinesterase inhibitors to improve cognitive properties as well as anxiolytics and antipsychotics to control the behavioral problems associated with this ailment.

The hallmark pathological features in the brain of AD patients are neurofibillary tangles which are generated by hyperphosphorylation of tau protein and amyloid plaques which form by aggregation of β-amyloid$_{1-42}$ (A$\beta_{1-42}$) peptide. A$\beta_{1-42}$ forms oligomers and then fibrils, and ultimately amyloid plaques. The fibrils are believed to be especially neurotoxic and may cause most of the neurological damage associated with AD. Agents that prevent the formation of A$\beta_{1-42}$ have the potential to be disease-modifying agents for the treatment of AD. A$\beta_{1-42}$ is generated from the amyloid precursor protein (APP), comprised of 770 amino acids. The N-terminus of A$\beta_{1-42}$ is cleaved by β-secretase (BACE), and then γ-secretase cleaves the C-terminal end. In addition to A$\beta_{1-42}$, γ-secretase also liberates A$\beta_{1-40}$ which is the predominant cleavage product as along with A$\beta_{1-38}$ and A$\beta_{1-43}$. Thus, inhibitors of BACE would be expected to prevent the formation of A$\beta_{1-42}$ and would be potential therapeutic agents in the treatment of AD.

SUMMARY OF THE INVENTION

EP-A-1407774 discloses quinazolines which may be useful in the treatment of Alzheimer's disease.

WO 01/38315A also discloses quinazolines which may be useful in the treatment of Alzheimer's disease, and their use for inhibiting cyclin-dependent kinase enzymes.

Kienzle, Frank et al, European Journal of Medicinal Chemistry, 17(6),547-56 discloses 1,5-Dihydroimidazoquinazolinones as blood platelet aggregation inhibitors.

Webb, Thomas Hand Wilcox, Journal of Organic Chemistry vol. 55, no.1, 1990, pages 363-365, discloses an improved synthesis of symmetrical and unsymmterical 5, 11-methandibenzo[b,f][1 ,5]-diazocines.

The present invention is directed to compound of formula (II).

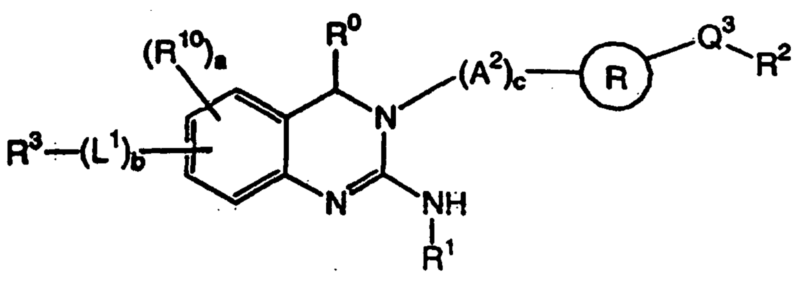

(II)

wherein

$R^0$ is selected from the group consisting of hydrogen, methyl, and $CF_3$;

$R^1$ is selected from the group consisting of hydrogen, hydroxy, methyl, ethyl, trifluoromethyl, 2,2,2-trifluoroethyl, methoxy, ethoxy and methyl-carbonyl;

c is an integer from 0 to 1; .

$A^2$ is selected from the group consisting of $C_{1-4}$alkyl; wherein the $C_{1-4}$alkyl is optionally substituted with one or more

$R^Y$ substituents;
wherein each $R^Y$ is independently selected from the group consisting of hydroxy, oxo, $C_{1-4}$alkyl, $C_{1-4}$alkoxy, hydroxy substituted $C_{1-4}$alkyl, amino substituted $C_{1-6}$alkyl, cycloalkyl, cycloalkyl-$C_{1-4}$alkyl-, biphenyl, aryl, $C_{1-4}$aralkyl, heteroaryl, heteroaryl-$C_{1-4}$alkyl-, heterocycloalkyl, heterocycloalkyl-$C_{1-4}$alkyl- or spiro-heterocyclyl;
wherein the cycloalkyl, aryl, heteroaryl, heterocycloalkyl or spiro-heterocyclyl group, whether alone or as part of a substituent group is optionally substituted with one or more substituents independently selected from the group consisting of fluoro, chloro, hydroxy, oxo, carboxy, $C_{1-4}$alkyl, $C_{1-4}$alkoxy, - C(O)O-($C_{1-4}$alkyl), hydroxy substituted $C_{1-4}$alkyl, fluoro substituted $C_{1-4}$alkyl, fluoro substituted $C_{1-4}$alkoxy, 5-tetrazolyl or 1-(1,4-dihydro-tetrazol-5-one);
® is selected from the group consisting of aryl, aryl-$C_{1-4}$alkyl, cycloalkyl, partially unsaturated carbocyclyl, heteroaryl, heterocycloalkyl and spiro-heterocyclyl;
wherein the aryl, cycloalkyl, partially unsaturated carbocyclyl, heteroaryl, heterocycloalkyl or spiro-heterocyclyl group, whether alone or as part of a substituent group, is optionally substituted with one or more substituents independently selected from the group consisting of $C_{1-4}$alkyl $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino and di ($C_{1-4}$alkyl)amino;
provided that when c is 0, then ® is other than aryl or heteroaryl;
$Q^3$ is selected from the group consisting of -C(O)--C(O)O-, -C(O)-NH-, -C(O)-N($C_{1-8}$alkyl)-, -C(O)-N(cycloalkyl)-, -NH-C(O)- and -NH-C(O)O-; wherein the cycloalkyl is optionally substituted with $C_{1-4}$alkyl;
$R^{2'}$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;
wherein the $C_{1-10}$alkyl, cycloalkyl, aryl, partially unsaturated carbocyclyl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C (O) -$C_{1-4}$alkyl, -C (O) -$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$alkyl-S-$C_1$-4alkyl, -C(O)-N($R^L$-$R^M$), -$C_{1-4}$alkyl-C(O)-N($R^L R^M$), - $NR^L$-C(O)-$C_{1-4}$alkyl, -$SO_2$-N ($R^L R^N$), -$C_{1-4}$alkyl-$SO_2$-N($R^L R^M$), $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, -O-$C_{1-4}$aralkyl, -O-(tetrahydropyranyl),- NH-C(O)O-$CH_2$-(tetrahydmpyranyl), -N($CH_3$)-C(O)O-$CH_2$-(tetrahydropyranyl), nitro, cyano, amino, $C_{1-4}$alkylamino, di($C_{1-4}$alkyl)amino, phenyl, 5-tetrazolyl and 1-(1,4-dihydro-tetrazol-5-one);
wherein the phenyl or tetrahydropyranyl is optionally substituted with one or more substituent independently selected from the group consisting of halogen, hydroxy, oxo, carboxy, C(O)O-$C_{1-4}$alkyl, C(O)-$C_{1-4}$alkyl, OC(O)-$C_{1-4}$alkyl, -$C_{1-4}$alkyl-OC(O)-$C_{1-4}$alkyl, -O-$C_{1-4}$aralkyl, $C_{1-4}$alkyl, fluoro substituted $C_{1-4}$alkyl, hydroxy substituted $C_{1-4}$alkyl, $C_{1-4}$alkoxy, nitro, cyano, amino, $C_{1-4}$alkylamino and di($C_{1-4}$alkyl)amino;
wherein each $R^L$ and $R^M$ is independently selected from the group consisting of hydrogen and $C_{1-4}$alkyl;
b is an integer from 0 to 1;
$L^1$ is selected from the group consisting of -O-, -$S(O)_{0-2}$-, -$NR^N$-, -C(O)-, -C(S)-, -$C_{1-4}$alkyl-, -(hydroxy substituted $C_{1-4}$alkyl)- and -($C_{2-4}$alkenyl)-;
wherein $R^N$ is selected from the group consisting of hydrogen and $C_{1-4}$alkyl;
$R^3$ is selected from the group consisting of $C_{1-6}$alkyl, $C_{2-6}$alkenyl, cycloalkyl, partially unsaturated carbocyclyl, aryl, biphenyl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;
wherein the $C_{1-6}$alkyl, cycloalkyl, partially unsaturated carbocyclyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, hydroxy substituted $C_{1-4}$alkyl, halogen substituted $C_{1-4}$alkyl, cyano substituted $C_{1-4}$alkyl, $C_{2-4}$alkenyl, $C_{2-4}$alkynyl, $C_{1-4}$alkoxy, halogen substituted $C_{1-4}$alkoxy, nitro, cyano, -$R^5$, -O-$R^5$, -S-$R^5$, -$SO_2$-$R^5$, -$SO_2$-$NR^P$-$R^b$, - $NR^P$-$SO_2$-$R^5$, -$NH_2$, -N($R^P$)-$R^5$, -C (O)-$R^5$, -C(O)-$NH_2$, -C(O)-$NR^P$-$R^5$, -$NR^P$-C(O)-$R^5$ and -$NR^P$-C(O)O-$R^5$;
wherein $R^5$ is selected from the group consisting of $C_{1-4}$alkyl, aryl, $C_{1-4}$aralkyl, partially unsaturated carbocyclyl, cycloalkyl, cycloalkyl-$C_{1-4}$alkyl-, partially unsaturated carbocyclyl-$C_{1-4}$alkyl-, heteroaryl, heteroaryl-$C_{1-4}$alkyl-, heterocycloalkyl and heterocyclyl-$C_{1-4}$alkyl-;
wherein the aryl, partially unsaturated carbocyclyl, cycloalkyl, heteroaryl or heterocycloalkyl, whether alone or as part of a substituent group is optionally substituted with one or more substituent independently selected from the group consisting of $C_{1-4}$alkyl, $C_{1-4}$alkoxy, halogen, hydroxy, carboxy, amino, $C_{1-4}$alkylamino, di($C_{1-4}$alkyl)amlno, cyano and nitro;
wherein $R^P$ is selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;
wherein the cycloalkyl, aryl, heteroaryl, heterocycloalkyl or spiro-heterocyclyl, whether alone or as part of a substituent

group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di ($C_{1-4}$alkyl)amino,-$SO_2$-N($R^SR^T$), 5-tetrazolyl and 1-(1,4-dihydro-tetrazol-5-one);
wherein each $R^S$ and $R^T$ is independently selected from the group consisting of hydrogen and $C_{1-4}$alkyl;
a is an integer form 0 to 3;
each $R^{10}$ is independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, -C(O)-$NR^VR^W$, -$SO_2$-$NR^VR^W$, -C(O)-$C_{1-4}$alkyl and -$SO_2$-$C_1$-$_4$alkyl;
wherein each $R^V$ and $R^W$ is Independently selected from the group consisting of hydrogen and $C_{1-4}$alkyl; alternatively $R^V$ and $R^W$ are taken together with the N atom to which they are bound to form a 5 to 6 membered saturated, partially unsaturated or aromatic ring structure;
provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

Preferably,
$R^0$ is selected from the group consisting of hydrogen and methyl;
$R^1$ is selected from the group consisting of hydrogen, methyl, trifluoromethyl, methoxy and methylcarbonyl;
c is an integer from 0 to 1;
$A^2$ is selected from the group consisting of $C_{1-4}$alkyl; wherein the $C_{1-4}$alkyl is optionally substituted with one to two $R^Y$ substituents;
wherein each $R^Y$ is independently selected from the group consisting of hydroxy, $C_{1-4}$alkyl, hydroxy substituted $C_{1-4}$alkyl, amino substituted $C_{1-6}$alkyl, cycloalkyl, cycloalkyl-$C_1$-$_4$alkyl-, aryl, $C_{1-4}$aralkyl, heteroaryl, heteroaryl-$C_{1-4}$alkyl-, heterocycloalkyl and heterocycloalkyl-$C_{1-4}$alkyl-;
wherein the cycloalkyl, aryl, heteroaryl or heterocycloalkyl group, whether alone or as part of a substituent group is optionally substituted with one or more substituents independently selected from the group consisting of fluoro, chloro, hydroxy, oxo, carboxy, $C_{1-4}$alkyl, $C_{1-4}$alkoxy, -C(O)O-($C_{1-4}$alkyl), hydroxy substituted $C_{1-4}$alkyl, fluoro substituted $C_{1-4}$alkyl and fluoro substituted $C_{1-4}$alkoxy;

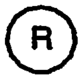

is selected from the group consisting of aryl, aryl-$C_{1-4}$alkyl-, cycloalkyl, heteroaryl and heterocycloalkyl;
wherein the aryl, cycloalkyl, heteroaryl or heterocycloalkyl group, whether alone or as part of a substituent group, is optionally substituted with one to two substituents independently selected from the group consisting of $C_{1-4}$alkyl, $C_{1-4}$alkoxy, halogen substituted $C_{1-4}$alkyl, halogen substituted $C_{1-4}$akoxy, hydroxy substituted $C_{1-4}$alkyl, hydroxy, carboxy, cyano, nitro, amino, $C_{1-4}$alkylamino and di($C_{1-4}$alkyl)amino;
provided that when c is 0, then

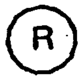

is other than aryl or heteroaryl;
$R^2$ is selected from the group consisting of $C_{1-8}$alkyl, cycloalkyl, aryl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl- and heterocycloalkyl-$C_{1-4}$alkyl-;
wherein the $C_{1-8}$alkyl, cycloalkyl, aryl, heteroaryl, heterocycloalkyl or spiro-heterocyclyl, whether alone or as part of a substituent group is optionally substituted with one or more substituents independently selected from the group consisting of halogen, hydroxy, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -C(O)-N($R^LR^m$), -$NR^L$-C(O)-$C_{1-4}$alkyl, -$SO_2$-N($R^LR^M$) $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, nitro, cyano, amino, $C_{1-4}$alkylamino, di($C_{1-4}$alkyl)amin and phenyl;
wherein the phenyl is optionally substituted with one to two substituent independently selected from the group consisting of halogen, hydroxy, carboxy, -C(O)-$C_{1-4}$alkyl, $C_{1-4}$alkyl, fluoro substituted $C_{1-4}$alkyl, $C_{1-4}$alkoxy, nitro; cyano, amino, $C_{1-4}$alkylamino and di($C_{1-4}$alkyl)amino;
wherein each $R^L$ and $R^M$ is independently selected from the group consisting of hydrogen and $C_{1-4}$alkyl;
b is an integer selected from 0 to 1;
$L^1$ is selected from the group consisting of -0-, -$S(O)_{0-2}$, -$NR^N$-, -$C_{1-4}$alkyl-, -(hydroxy substituted $C_{1-4}$alkyl)- and

-($C_{2-4}$alkenyl)-;
wherein $R^N$ is selected from the group consisting of hydrogen and $C_{1-4}$alkyl;
$R^3$ is selected from the group consisting of $C_{1-6}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl- and heterocycloalkyl-$C_{1-4}$alkyl-;
wherein the $C_{1-6}$alkyl, cycloalkyl, partially unsaturated carbocyclyl, aryl, heteroaryl or heterocycloalkyl, whether alone or as part of a substituent group is optionally substituted with one or more substituents independently selected from the group consisting of halogen, hydroxy, carboxy, $C_{1-4}$alkyl, hydroxy substituted $C_1$-$_4$alkyl, halogen substituted $C_{1-4}$alkyl, cyano substituted $C_{1-4}$alkyl, $C_{2-4}$alkenyl, $C_{2-4}$alkynyl, $C_{1-4}$alkoxy, halogen substituted $C_{1-4}$alkoxy, nitro, cyano, -$R^6$, -O-$R^5$, -S-$R^5$, -$SO_2$-$R^5$, -$SO_2$-$NR^P$-$R^5$, -$NR^P$-$SO_2$-$R^5$, -$NH_2$, -N($R^P$)-$R^5$,-C(O)-$R^6$, -C(O)-$NH_2$, -C(O)-$NR^P$-$R^5$ and -$NR^P$-C(O)-$R^5$;
wherein $R^5$ is selected from the group consisting of $C_{1-4}$alkyl, aryl, $C_{1-4}$aralkyl, cycloalkyl, cycloalkyl-$C_{1-4}$alkyl-, partially unsaturated carbocyclyl-$C_{1-4}$alkyl-, heteroaryl, heteroaryl-$C_{1-4}$alkyl-, heterocycloalkyl and heterocyclyl-$C_{1-4}$alkyl-;
wherein the aryl, $C_{1-4}$aralkyl, partially unsaturated carbocyclyl, cycloalkyl, heteroaryl or heterocycloalkyl, whether alone or as part of a substituent group is optionally substituted with one or more substituent independently selected from the group consisting of $C_{1-4}$alkyl, $C_{1-4}$alkoxy, halogen, hydroxy, carboxy, amino, $C_{1-4}$alkylamino, di($C_{1-4}$alkyl)amino, cyano and nitro;
wherein $R^P$, is selected from the group consisting of hydrogen, $C_{1-8}$alkyl, hydroxy substituted $C_{1-4}$alkyl, cycloalkyl, aryl, $C_{1-4}$aralkyl, heteroaryl and heterocycloalkyl;
wherein the cycloalkyl, aryl, heteroaryl or heterocycloalkyl, whether alone or as part of a substituent group is optionally substituted with one to two substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino and di($C_{1-4}$alkyl)amino;
a is an integer from 0 to 1;
$R^{10}$ is selected from the group consisting of hydroxy, halogen, $C_{1-4}$alkyl, $C_{1-4}$alkoxy, halogen substituted $C_{1-4}$alkyl, halogen substituted $C_{1-4}$alkoxy;
provided that the halogens on the halogen substituted $C_{1-4}$alkyl and the halogen substituted $C_{1-4}$alkoxy are selected from the group consisting of fluoro and chloro;

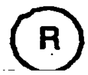

or a pharmaceutically acceptable salt thereof.

More preferably,
$R^0$ is hydrogen;
$R^1$ is selected from the group consisting of hydrogen, methyl and methylcarbonyl;
c is an integer from 0 to 1;
$A^2$ is selected from the group consisting of $C_{1-4}$alkyl; wherein the $C_{1-4}$alkyl is optionally substituted with an $R^Y$ substituent;
wherein $R^Y$ is selected from the group consisting of $C_{1-4}$alkyl, aryl, $C_{1-4}$aralkyl and cycloalkyl-$C_{1-4}$alkyl-;

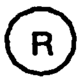

is selected from the group consisting of cycloalkyl, aryl, aryl-$C_{1-4}$alkyl-, heteroaryl, and heterocycloalkyl;
wherein the aryl, heteroaryl or heterocycloalkyl, whether alone or as part of a substituent group, is optionally substituted with one to two substituents independently selected from the group consisting of $C_{1-4}$alkyl and $C_{1-4}$alkoxy;
provided that when c is 0, then

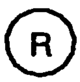

is other than aryl or heteroaryl;
$R^2$ is selected from the group consisting of $C_{1-8}$alkyl, cycloalkyl, cycloalkyl-$C_{1-4}$alkyl-, aryl, $C_{1-4}$aralkyl, heteroaryl, heteroaryl-$C_{1-4}$alkyl-, heterocycloalkyl and heterocycloalkyl-$C_{1-4}$alkyl-,
wherein the $C_{1-8}$alkyl, cycloalkyl, aryl or heterocycloalkyl, whether alone or as part of a substituent group is optionally substituted with one to three substituent independently selected from the group consisting of halogen, $C_{1-4}$alkyl, -$SO_2$-$NH_2$ and phenyl;

b is an integer selected from 0 to 1;
$L^1$ is selected from the group consisting of -O-, -S-, -NH-, -$C_{1-4}$alkyl- and -$C_{2-4}$alkenyl-;
$R^3$ is selected from the group consisting of $C_{1-4}$alkyl, cycloalkyl, cycloalkyl-$C_{1-4}$alkyl-, aryl, heteroaryl and heterocycloalkyl; wherein the cycloalkyl or aryl, whether alone or as art of a substituent group is optionally substituted with one to two substituents independently selected from the group consisting of halogen, hydroxy, carboxy, cyano, nitro, amino, $C_{1-4}$alkylamino, di($C_{1-4}$alkylamino), $C_{1-4}$alkyl, $C_{2-4}$alkenyl, $C_{1-4}$alkoxy, hydroxy substituted $C_{1-4}$alkyl, halogen substituted $C_{1-4}$alkyl, cyano substituted $C_{1-4}$alkyl, halogen substituted $C_{1-4}$alkoxy, -O-aryl, -O-$C_{1-4}$aralkyl, -S-$C_{1-4}$alkyl, -$SO_2$-$C_{1-4}$alkyl, -C(O)-$C_{1-4}$alkyl, -C(O)-$NH_2$, -C(O)-NH($C_{1-4}$alkyl), -C(O)-N($C_{1-4}$alkyl)$_2$, -NH-C(O)-$C_{1-4}$alkyl, -C(O)-NH-$C_{1-4}$alkyl, -NH-$SO_2$-$C_{1-4}$alkyl, -NH-$SO_2$-phenyl, aryl, $C_{1-4}$aralkyl, cycloalkyl, cycloalkyl-$C_{1-4}$alkyl-, heteroaryl, heteroaryl-$C_{1-4}$alkyl-, heterocycloalkyl and heterocycloalkyl-$C_{1-4}$alkyl-;
wherein the aryl, cycloalkyl, heteroaryl or heterocycloalkyl, whether alone or as part of a substituent group, is optionally substituted with one to three substituents independently selected from the group consisting of $C_{1-4}$alkyl and $C_{1-4}$alkoxy;
a is 0;
or a pharmaceutically acceptable salt thereof.

Still more preferably,
$R^0$ is hydrogen;
$R^1$ is selected from the group consisting of hydrogen, methyl and methylcarbonyl;
c is an integer from 0 to 1;
$A^2$ is selected from the group consisting of -$CH_2$-, -CH($CH_2CH_3$)-, - CH(phenyl)-, -CH(benzyl)- and -CH(cyclohexylmethyl)-;

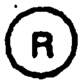

is selected from the group consisting of cyclopentyl, (S)-cyclopentyl, (R)-cyclopentyl, cyclohexyl, (R)-cyclohexyl, (S)-cyclohexyl, transcyclohexyl, phenyl, benzyl, 9-fluorenyl, 3-pyrrolidinyl, 1-indanyl, 1-(5-methoxy-indanyl)-methyl, 3-piperidinyl, 4-piperidinyl, 3-azepinyl, 2-pyridyl, 4-pyridyl, 2-furyl, 2-thienyl and 5-oxazolyl;
provided that when c is 0, then

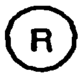

is other than phenyl, 2-pyridyl, 4-pyridyl, 2-furyl, 2-thienyl or 5-oxazolyl;
$Q^3$ is selected from the group consisting of -C(O)-, -C(O)-NH-, -C(O)-N($CH_3$)-, -(R)-O(O)-N($CH_3$), -(S)-C(O)-N($CH_3$), -C(O)-N(isopropyl)-, -C(O)-N(n-propyl)-, -C(O)-N(isobutyl)-, -C(O)-N(2-ethyl-n-hexyn-, -C(O)-N(cyclohexyl)-, - C(O)-N(4-methyl-cyclohexyl), -C(O)O-, -NH-C(O)- and -NH-C(O)O-;
$R^2$ is selected from the group consisting of trifluoromethyl, methyl, ethyl, isobutyl, t-butyl, 3-n-heptyl, 4-n-heptyl, 2-ethyl-n-hexyl, cyclopentyl, cyclohexyl, 4-methyl-cyclohexyl, cyclopropyl-methyl, 4-aminosulfonyl-phenylethyl, benzhydryl, 1-adamantyl, 2-adamantyl, 2-(R)-adamantyl, 2-(S')-adamantyi, 2-docahydro-isoquinolinyl, 2-(1-methyl-pyrrolidinyl)-ethyl, 1-piperidinyl and 4-(1-methyl-piperidinyl);
b is an integer selected from 0 to 1 ;
$L^1$ is selected from the group consisting of -O-, -S-, -NH-, -CH($CH_3$)- and -CH=CH-;
$R^9$ is selected from the group consisting of n-pentyl, isopentyl, isobutyl, isopropyl, cyclopentyl, cyclopentyl-methyl, phenyl, 2-hydroxy-phenyl, 3-carboxy-phenyl, 2-cyano-phenyl, 2-nitro-phenyl, 2-bromo-phenyl, 2-fluorophenyl, 2-chloro-phenyl, 2,6-dichloro-phenyl, 2-methyl-phenyl, 3-methyl-phenyl, 2-ethyl-phenyl, 4-isopropyl-phenyl, 3,4-dimethyl-phenyl, 3,5-dimethyl-phenyl, 2,6-dimethyl-phenyl, 2-methoxy-phenyl, 3-methoxy-phenyl, 4-methoxy-phenyl, 3,4dimethoxy-phenyl, 2,6-dimethoxy-phenyl, 2-ethoxy-phenyl, 3-ethoxyphenyl, 2-isopropoxy-phenyl, 2-methoxy-5-methyl-phenyl, 2-methoxy-6-methylphenol, 2-trifluoromethyl-phenyl, 2-trifluoromethoxy-phenyl, 2-methylthiophenyl, 4-methylthio-phenyl, 2-hydroxymethyl-phenyl, 2-cyanomethyl-phenyl, 2-(aminocarbonyl)-phenyl, 4-(aminocarbonyl)-phenyl, 2-(dimethylaminocarbonyl)-phenyl, 3-(dimethylamino)-phenyl, 4-(dimethylamino)-phenyl, 2-allyl-6-methylphenyl, 2-allyl-6-ethoxy-phenyl, 2-methyl-6-n-propyl-phenyl, 3-(methylcarbonylamino)-phenyl, 2-(methylaminocarbonyl)-phenyl, 2-(methylcarbonyl)-phenyl, 4-(methylcarbony)amino)-phenyl, 2-(aminocarbonylmethyl)-phenyl, 2-(methylsulfonyl)-phenyl, (3-(2-methoxy-4-methyl-phenyl)-sulfonylamino)-phenyl, 3-(2,4,6-trimethylphenylsulfonylamino)-phenyl, 3-(phenylsulfonylamino)-phenyl, 2-(t-butylaminosulfonyl)-phenyl, 2-(t-butylcarbonylamino)-5-methoxy-phenyl, 3-(phenylsulfonylamino)-phenyl, 2-phenoxy-phenyl, 3-phenoxy-phenyl, 2-benzyloxy-phenyl, 2-(2-benzthiazolyl)-5-methoxy-phenyl, 2-(2-benzthiazolyl)-phenyl, 2-(1-pyrrolyl)-phenyl, 3-(2-quinolinyl-phenyl, 2-(1-pyrrolidinyl-methyl)-phenyl; 2-cyclopentyl-phenyl, 4-cyclohexyl-phenyl, 4-(4-morpholinyl)-phenyl, 3-methoxy-benzyl, 1-naphthyl, 2-naphthyl, 2-(5,6,7,8-tetrahydro-naphthyl), 2-biphenyl, 3-biphenyl, 2-biphenylmethyl, 3-pyridyl, 3,4-methylenedioxyphenyl, 4(3,5-dimethyl-isoxazolyl), 4-pyrazolyl, 3-thienyl, 3-pyridyl, 4-pyridyl, 5-indolyl and 3-benzothienyl;
a is 0;
or a pharmaceutically acceptable salt thereof.

Still more preferably,
$R^0$ is hydrogen;
$R^1$ is hydrogen;
c is an integer from 0 to 1;
$A^2$ is selected from the group consisting of $-CH_2-$ and $-CH(CH_2CH_3)-$;

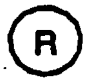

is selected from the group consisting of phenyl, 4-piperidinyl and 4-pyridyl; provided that when c is 0, then

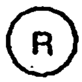

is other than phenyl or 4-pyridyl;
$Q^3$ Is selected from the group consisting of -1-C(O)O-, -3-C(O)O-, -2-C(O)-N($CH_3$)- and -3-C(O)-N($CH_3$)-;
$R^2$ is selected from the group consisting of methyl, ethyl, t-butyl and cyclohexyl;
b is an integer from 0 to 1;
$L^1$ is selected from the group consisting of -O- and -S-;
$R^3$ is selected from the group consisting of phenyl, 2-bromophenyl, 2-chlorophenyl, 2,6-dichlorophenyl, 2-hydroxy-phenyl, 2-hydroxymethyl-phenyl, 2-methoxy-phenyl, 3-methoxy-phenyl, 2-ethoxy-phenyl, 2-methylthio-phenyl, 2-cyanomethyl-phenyl, 3-(phenyl-sutfonyl-amino)-phenyl, 3-(2,4,6-trimethylphenyl-sulfonyl-amino)-phenyl, (3-(2-methoxy-4-methyl-phenyl)-sulfonyl-amino)-phenyl, 2-(t-butyl-carbonyl-amino)-5-methoxy-phenyl, 1-naphthyl, 3-thienyl and 4-(3,5-dimethylisoxazolyl);
a is 0;
or a pharmaceutically acceptable salt thereof.

Still more preferably,
$R^0$ is hydrogen;
$R^1$ is hydrogen;
c is an integer from 0 to 1;
$A^2$ is selected from the group consisting of $-CH_2-$ and $-CH(CH_2CH_3)-$;

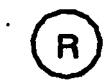

is selected from the group consisting of phenyl and 4-piperidinyl; provided that when c is 0, then

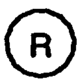

is 4-piperidinyl;
$Q^3$ is selected from the group consisting of -1-C(O)O-, -3-C(O)O- and -3-C(O)-N($CH_3$)-;
$R^2$ is selected from the group consisting of methyl, ethyl, t-butyl and cyclohexyl;
b is an integer from 0 to 1;
$L^1$ is selected from the group consisting of -O- and -S-;
$R^3$ is selected from the group consisting of phenyl, 2-bromophenyl, 2-chlorophenyl, 2,6-dichlorophenyl, 2-hydroxy-phenyl, 2-hydroxymethyl-phenyl, 2-methoxy-phenyl, 3-methoxy-phenyl, 2-ethoxy-phenyl, 2-methylthio-phenyl, 2-cyanomethyl-phenyl, 3-(phenyl-sulfonyl-amino)-phenyl, 1-naphthyl and 3-thienyl;
a is 0;

or a pharmaceutically acceptable salt thereof.

Still more preferably,
$R^0$ is hydrogen;
$R^1$ is hydrogen;
c is 1;
$A^2$ is $-CH_2-$;

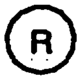

is phenyl;
$Q^3$ is -3-C(O)-N($CH_3$)-;
$R^2$ is selected from the group consisting of methyl, ethyl, t-butyl and cyclohexyl;
b is 0;
$R^3$ is selected from the group consisting of phenyl, 2-methoxy-phenyl, 2-ethoxy-phenyl and 1-naphthyl;
a is 0;
or a pharmaceutically acceptable salt thereof.

Preferably and alternatively,
$R^0$ is hydrogen
$R^1$ is selected from the group consisting of hydrogen and methoxy;
c is 1;
$A^2$ is selected from the group consisting of $-CH_2-$, $-CH(CH_2CH_3)-$,-CH(phenyl)- and -CH(cyclohexyl)-;

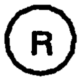

is selected from the group consisting of phenyl, 2-pyridyl, and 2-furyl;
$Q^3$ is selected from the group consisting of -3-C(O)-N($CH_3$)- -3-C(O)-N(isopropyl)-, -3-C(C)-N(isobutyl)-,-3-C(O)-N(cyclohexyl)-, -4-C(O)-N($CH_3$)- and 5-C(O)-N($CH_3$)-;
$R^2$ is selected from the group consisting of isobutyl and cyclohexyl;
b is an integer from 0 to 1;
$L^1$ is selected from the group consisting of -0-, -S- and -NH-;
$R^3$ is selected from the group consisting of phenyl, 2-bromophenyl, 2-fluorophenyl, 2-chlorophenyl, 2,6-dichlorophenyl, 2-hydroxyphenyl, 2-hydroxymethyl-phenyl, 2-methylphenyl, 3-methylphenyl, 2,6-dimethylphenyl, 2-methoxyphenyl, 3-methoxyphenyl, 2,6-dimethoxyphenyl, 2-ethoxyphenyl, 2-trifluoromethylphenyl, 2-trifluoromethoxyphenyl, 2-methylthiophenyl, 2-nitrophenyl, 2-cyanophenyl. 2-cyanomethyl-phenyl, 2-phenoxy-phenyl, 2-(methyl-carbonyl-amino)-phenyl, 2-(amino-carbonyl)-phenyl, 3-(phenyl-sulfonylamino)-phenyl, 2-(t-butyl-amino-sulfonyl)-phenyl, 2-(t-butyl-carbonyl-amino)-5-methoxy-phenyl, 4-(3,5-dimethyl-soxazolyl), 1-naphthyl, 3-thienyl and 3-pyridyl;
a is 0;
or a pharmaceutically acceptable salt thereof

More preferably alternatively
$R^0$ is hydrogen
$R^1$ is hydrogen;
c is 1;
$A^2$ is selected from the group consisting of $-CH_2-$, $-CH(CH_2CH_3)-$ and - CH(cyclohexyl)-;

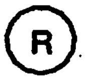

is selected from the group consisting of phenyl and 2-pyridyl;
$Q^3$ is selected from the group consisting of -3-C(O)-N($CH_3$)-, -3-C(O)-N(isopropyl)-,-3-C(O)-N(cyclohexyl)- and -4-C (O)-N($CH_3$)-;
$R^2$ is cyclohexyl;
b is an integer from 0 to 1;
$L^1$ is-O-;

$R^3$ is selected from the group consisting of phenyl, 2-bromophenyl, 2-fluorophenyl, 2-chlorophenyl, 2-hydrpxyphenyl, 2-hydroxymethyl-phenyl, 3-methylphenyl, 2-methoxyphenyl, 2-ethoxyphenyl, 2-cyanomethyl-phenyl, 2-(t-butyl-carbonyl-amino)-5-methoxy-phenyl, 1-naphthyl and 3-thienyl;
a is 0;
or a pharmaceutically acceptable salt thereof

Still more preferably alternatively,
$R^0$ is hydrogen; $R^1$ is hydrogen; c is 1; $A^2$ is $-CH_2-$;

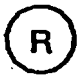

is phenyl.. $Q^3$ is $-3-C(O)-N(CH_3)-$; $R^2$ is cyclohexyl; b is 0; $R^3$ is selected from the group consisting of 2-methoxyphenyl and 2-ethoxyphenyl; a is 0; or a pharmaceutically acceptable salt thereof The present invention is also directed to a compound of formula (CII)

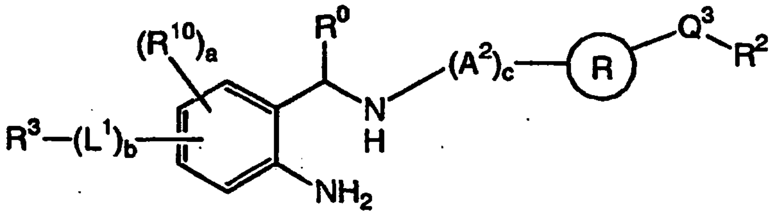

(CII)

wherein

$R^0$ is selected from the group consisting of hydrogen, methyl, and $CF_3$;
c is an integer from 0 to 1;
$A^2$ is selected from the group consisting of $C_{1-4}$alkyl; wherein the $C_{1-4}$alkyl is optionally substituted with one or more $R^Y$ substituents;
wherein each $R^Y$ is independently selected from the group consisting of hydroxy, oxo, $C_{1-4}$alkyl, $C_{1-4}$alkoxy, hydroxy substituted $C_{1-4}$alkyl, amino substituted $C_{1-6}$alkyl, cycloalkyl, cycloalkyl-$C_{1-4}$alkyl-, biphenyl, aryl, $C_{1-4}$aralkyl, heteroaryl, heteroaryl-$C_{1-4}$alkyl-, heterocycloalkyl, heterocycloalkyl-$C_{1-4}$alkyl- and spiro-heterocyclyl;
wherein the cycloalkyl, aryl, heteroaryl, heterocycloalkyl or spiro-heterocyclyl group, whether alone or as part of a substituent group is optionally substituted with one or more substituents independently selected from the group consisting of fluoro, chloro, hydroxy, oxo, carboxy, $C_{1-4}$alkyl, $C_{1-4}$alkoxy, - C(O)O-($C_{1-4}$alkyl), hydroxy substituted $C_{1-4}$alkyl, fluoro substituted $C_{1-4}$alkyl, fluoro substituted $C_{1-4}$alkoxy, 5-tetrazolyl and 1-(1,4-dihydro-tetrazol-5-one);

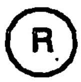

is selected from the group consisting of aryl, aryl-$C_{1-4}$alkyl, cycloalkyl, partially unsaturated carbocyclyl, heteroaryl, heterocycloalkyl and spiro-heterocyclyl;
wherein the aryl, cycloalkyl, partially unsaturated carbocyclyl, heteroaryl, heterocycloalkyl or spiro-heterocyclyl group, whether alone or as part of a substituent group, is optionally substituted with one or more substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino and dl ($C_{1-4}$alkyl)amino;
provided that when c is 0, then

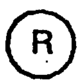

is other than aryl or heteroaryl;
$Q^3$ is selected from the group consisting of -O-, -S-, -C(O)-, -C(S)-, - C(O)O-, -OC(O)-, -C(O)-$NR^A$-, -$NR^A$-C(O)-, -C (S)-$NR^A$, -$SO_2$-$NR^A$-, -SO-$NR^A$, OC(O)-$NR^A$-, -$NR^A$-C(O)O- and -O-$SO_2$-$NR^A$-;

wherein each $R^A$ is independently selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;

wherein the cycloalkyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di ($C_{1-4}$alkyl)amino,-$SO_2$-N($R^CR^D$), 5-tetrazolyl and 1-(1,4-dlhydro-tetrazol-5-one);

wherein each $R^C$ and $R^D$ is independently selected from the group consisting of hydrogen and $C_{1-4}$alkyl;

$R^2$ is selected from the group consisting of $C_{1-8}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;

wherein the $C_{1-8}$alkyl, cycloalkyl, aryl, partially unsaturated carbocyclyl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C(O)$C_{1-4}$alkyl, -C (O)-$C_{1-4}$aralkyl, -C(O)O-$C_{1-4}$alkyl, -C(O)O- $C_{1-4}$aralkyl, -$C_{1-4}$alkyl-C-(O)O-$C_{1-4}$alkyl, -$C_{1-4}$alkyl-S-$C_{1-4}$alkyl, -C(O)-N ($R^LR^M$), -$C_{1-4}$alkyl-C(O)N($R^LR^M$), -$NR^L$-C(O)-$C_{1-4}$alkyl, -$SO_2$-N($R^LR^M$), -$C_{1-4}$alkyl-$SO_2$-N($R^LR^M$), $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, -O-$C_{1-4}$aralkyl, -O-(tetrahydropyranyl), -NH-OC(O)-$CH_2$-(tetrahydropyranyl), -N($CH_3$)-OC(O)-$CH_2$-(tetrahydropyranyl), nitro, cyano, amino, $C_{1-4}$alkylamlno, di($C_{1-4}$alkyl)amino, phenyl, 5-tetrazolyl and 1-(1,4-dihydro-tetrazol-5-one);

wherein the phenyl or tetrahydropyranyl is optionally substituted with one or more substituent independently selected from the group consisting of halogen, hydroxy, oxo, carboxy, C(O)O-$C_{1-4}$alkyl, C(O)-$C_{1-4}$alkyl, OC(O)-$C_{1-4}$alkyl; -$C_{1-4}$alkyl-OC(O)-$C_{1-4}$alkyl, -O-$C_{1-4}$aralkyl, $C_{1-4}$alkyl, fluoro substituted $C_{1-4}$alkyl, hydroxy substituted $C_{1-4}$alkyl, $C_{1-4}$alkoxy, nitro, cyano, amino, $C_{1-4}$alkylamino and di($C_{1-4}$alkyl)amino;

wherein each $R^L$ and $R^M$ is independently selected from the group consisting of hydrogen and $C_{1-4}$alkyl;

b is an integer from 0 to 1;

$L^1$ is selected from the group consisting of -O-, -S(O)$_{0-2}$-, -$NR^N$-, -C(O)-, -C(S)-, -$C_{1-4}$alkyl-, -(hydroxy substituted $C_{1-4}$alkyl)- and -($C_{2-4}$alkenyl)-;

wherein $R^N$ is selected from the group consisting of hydrogen and $C_{1-4}$alkyl;

$R^3$ is selected from the group consisting of $C_{1-6}$alkyl, $C_{2-6}$alkenyl, cycloalkyl, partially unsaturated carbocyclyl, aryl, biphenyl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;

wherein the $C_{1-6}$alkyl, cycloalkyl, partially unsaturated carbocyclyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, hydroxy substituted $C_{1-4}$alkyl, halogen substituted $C_{1-4}$alkyl, cyano substituted $C_{1-4}$alkyl, $C_{2-4}$alkenyl, $C_{2-4}$alkynyl, $C_{1-4}$alkoxy, halogen substituted $C_{1-4}$alkoxy, nitro, cyano, -$R^5$, -O-$R^5$, -S-$R^5$, -$SO_2$-$R^5$, -$SO_2$-$NR^P$-$R^5$, - $NR^P$-$SO_2$-$R^5$, -$NH_2$, -N($R^P$)-$R^5$, -C (O)-$R^5$, -C(O)-$NH_2$, -C(O)-$NR^P$-$R^5$, -$NR^P$-C(O)-$R^5$ and -$NR^P$-C(O)O-$R^5$;

wherein $R^5$ is selected from the group consisting of $C_{1-4}$alkyl, aryl, $C_{1-4}$aralkyl, partially unsaturated carbocyclyl, cycloalkyl, cycloalkyl-$C_{1-4}$alkyl-, partially unsaturated carbocyclyl-$C_{1-4}$alkyl-, heteroaryl, heteroaryl-$C_{1-4}$alkyl-, heterocycloalkyl and heterocyclyl-$C_{1-4}$alkyl-;

wherein the aryl, partially unsaturated carbocyclyl, cycloalkyl, heteroaryl or heterocycloalkyl, whether alone or as part of a substituent group is optionally substituted with one or more substituent independently selected from the group consisting of $C_{1-4}$alkyl, $C_{1-4}$alkoxy, halogen, hydroxy, carboxy, amino, $C_{1-4}$alkylamlno, di($C_{1-4}$alkyl)amino, cyano and nitro;

wherein $R^P$ is selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;

wherein the cycloalkyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di ($C_{1-4}$alkyl)amino,-$SO_2$-N($R^SR^T$), 5-tetrazolyl and 1-(1,4-dihydro-tetrazol-5-one);

wherein each $R^S$ and $R^T$ is independently selected from the group consisting of hydrogen and $C_{1-4}$alkyl;

a is an integer form 0 to 3;

each $R^{10}$ is independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, -C(O)-$NR^VR^W$, -$SO_2$-$NR^VR^W$, -C(O)-$C_{1-4}$alkyl and -$SO_2$-$C_{1-4}$alkyl;

wherein each $R^V$ and $R^W$ is independently selected from the group consisting of hydrogen and $C_{1-4}$alkyl; alternatively $R^V$ and $R^W$ are taken together with the N atom to which they are bound to form a 5 to 6 membered saturated, partially

unsaturated or aromatic ring structure;
provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

In a method of treating a disorder mediated by the β-secretase enzyme, a therapeutically effective amount of any of the compounds or pharmaceutical compositions described above can be administered to a subject in need thereof. For an example, disorder selected from the group consisting of Alzheimer's disease (AD), mild cognitive impairment, senility, dementia, dementia with Lewy bodies, Down's syndrome, dementia associated with Parkinson's disease and dementia associated with beta-amyloid, may be treated by administering to a subject in need thereof, a therapeutically effective amount of any of the compounds or pharmaceutical compositions described above.

Another example of the invention is the use of any of the compounds described above in the preparation of a medicament for treating: (a) Alzheimer's Disease (AD), (b) mild cognitive impairment, (c) senility, (d) dementia, (e) dementia with Lewy bodies, (f) Down's syndrome, (g) dementia associated with Parkinson's disease and (h) dementia associated with beta-amyloid, in a subject in need thereof.

<u>DETAILED DESCRIPTION OF THE INVENTION</u>

The present invention is directed to compounds of formula (II)

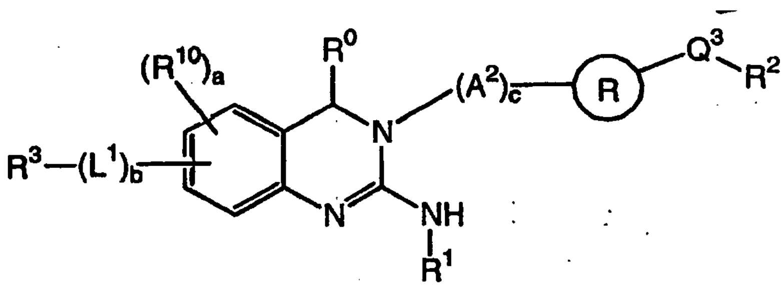

(II)

wherein $R^0$, $R^1$, $R^2$, $R^3$, $R^{10}$, $L^1$, a, b, c, $A^2$, $Q^3$ and

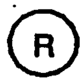

are as herein defined. The compounds of formula (II) are inhibitors of the β-secretase enzyme (also known as β-site cleaving enzyme, BACE, BACE1, Asp2 or memapsin2), and are useful in the treatment of Alzheimer's disease (AD), mild cognitive impairment (MCI), senility, dementia, dementia with Lewy bodies, Down's syndrome, dementia associated , with Parkinson's disease and dementia associated with beta-amyloid, preferably Alzheimer's disease.

In an embodiment, the present invention is directed to compounds of formula (II)

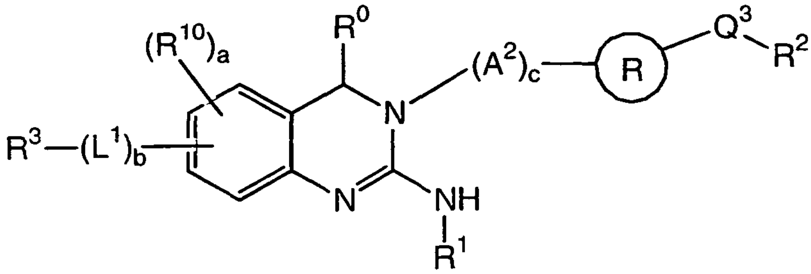

(II)

wherein

$R^0$ is selected from the group consisting of hydrogen, methyl, and $CF_3$;

$R^1$ is selected from the group consisting of hydrogen, hydroxy, methyl, ethyl, trifluoromethyl, 2,2,2-trifluoroethyl, methoxy, ethoxy and methyl-carbonyl;

c is an integer from 0 to 1;

$A^2$ is selected from $C_{1-4}$alkyl; wherein the $C_{1-4}$alkyl is optionally substituted with one or more $R^Y$ substituents; wherein each $R^Y$ is independently selected from the group consisting of hydroxy, oxo, $C_{1-4}$alkyl, $C_{1-4}$alkoxy, hydroxy substituted $C_{1-4}$alkyl, amino substituted $C_{1-6}$alkyl, cycloalkyl, cycloalkyl-$C_{1-4}$alkyl-, biphenyl, aryl, $C_{1-4}$aralkyl, heteroaryl, heteroaryl-$C_{1-4}$alkyl-, heterocycloalkyl, heterocycloalkyl-$C_{1-4}$alkyl- and spiro-heterocyclyl;

wherein the cycloalkyl, aryl, heteroaryl, heterocycloalkyl or spiro-heterocyclyl group, whether alone or as part of a substituent group is optionally substituted with a substituent selected from the group consisting of fluoro, chloro, hydroxy, oxo, carboxy, $C_{1-4}$alkyl, $C_{1-4}$alkoxy, -C(O)-($C_{1-4}$alkoxy), hydroxy substituted $C_{1-4}$alkyl, fluoro substituted $C_{1-4}$alkyl, fluoro substituted $C_{1-4}$alkoxy, 5-tetrazolyl and 1-(1,4-dihydro-tetrazol-5-one);

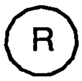

is selected from the group consisting of aryl, aryl-$C_{1-4}$alkyl, cycloalkyl, partially unsaturated carbocyclyl, heteroaryl, heterocycloalkyl and spiro-heterocyclyl;

wherein the aryl, cycloalkyl, partially unsaturated carbocyclyl, heteroaryl, heterocycloalkyl or spiro-heterocyclyl group is optionally substituted with one or more substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino and di($C_{1-4}$alkyl)amino;

provided that when c is 0 (i.e. $A^1$ is absent), then

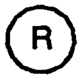

is other than aryl or heteroaryl;

$Q^3$ is as defined above;

wherein each $R^A$ is independently selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;

wherein the cycloalkyl, aryl, heteroaryl, heterocycloalkyl or spiroheterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl-, nitro, cyano, amino, $C_{1-4}$alkylamino, di ($C_{1-4}$alkyl)amino,-$SO_2$-N($R^CR^D$), 5-tetrazolyl and 1-(1,4-dihydro-tetrazol-5-one);

wherein each $R^C$ and $R^D$ is independently selected from the group consisting of hydrogen and $C_{1-4}$alkyl;

$R^2$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;

wherein the $C_{1-10}$alkyl, cycloalkyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$ alkyl-S-$C_{1-4}$alkyl, -C(O)-N($R^LR^M$), -$C_{1-4}$alkyl-C(O)-N($R^LR^M$), -$NR^L$-C (O)-$C_{1-4}$alkyl, -$SO_2$-N($R^LR^M$), -$C_{1-4}$alkyl-$SO_2$-N($R^LR^M$), $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, nitro, cyano, amino, $C_{1-4}$alkylamino, di($C_{1-4}$alkyl)amino, phenyl, 5-tetrazolyl and 1-(1,4-dihydro-tetrazol-5-one);

wherein the phenyl is optionally substituted with one or more substituent independently selected from the group consisting of halogen, hydroxy, oxo, carboxy, C(O)O-$C_{1-4}$alkyl, C(O)-$C_{1-4}$alkyl, $C_{1-4}$alkyl, fluoro substituted $C_{1-4}$alkyl, $C_{1-4}$alkoxy, nitro, cyano, amino, $C_{1-4}$alkylamino and di($C_{1-4}$alkyl)amino;

wherein each $R^L$ and $R^M$ is independently selected from the group consisting of hydrogen and $C_{1-4}$alkyl;

b is an integer from 0 to 1;

$L^1$ is selected from the group consisting of -O-, -$S(O)_{0-2}$-, -$NR^N$-, -C(O)-, -C(S)-, -$C_{1-4}$alkyl-, -(hydroxy substituted $C_{1-4}$alkyl)- and -($C_{2-4}$alkenyl)-;

wherein $R^N$ is selected from the group consisting of hydrogen and $C_{1-4}$alkyl;

$R^3$ is selected from the group consisting of $C_{1-6}$alkyl, $C_{2-6}$alkenyl, cycloalkyl, partially unsaturated carbocyclyl, aryl, biphenyl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;

wherein the $C_{1-6}$alkyl, cycloalkyl, partially unsaturated carbocyclyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, hydroxy substituted $C_{1-4}$alkyl, halogen substituted $C_{1-4}$alkyl, cyano substituted $C_{1-4}$alkyl, $C_{2-4}$alkenyl, $C_{2-4}$alkynyl, $C_{1-4}$alkoxy, halogen substituted $C_{1-4}$alkoxy, nitro, cyano, $-R^5$, $-O-R^5$, $-S-R^5$, $-SO_2-R^5$, $-SO_2-NR^P-R^5$, $-NR^A-SO_2-R^5$, $-NH_2$, $-N(R^P)-R^5$, $-C(O)-R^5$, $-C(O)-NH_2$, $-C(O)-NR^P-R^5$, $-NR^P-C(O)-R^5$ and $-NR^P-C(O)O-R^5$;

wherein $R^5$ is selected from the group consisting of $C_{1-4}$alkyl, aryl, $C_{1-4}$aralkyl, partially unsaturated carbocyclyl, cycloalkyl, cycloalkyl-$C_{1-4}$alkyl-, partially unsaturated carbocyclyl-$C_{1-4}$alkyl-, heteroaryl, heteroaryl-$C_{1-4}$alkyl-, heterocycloalkyl and heterocyclyl-$C_{1-4}$alkyl-;

wherein the aryl, $C_{1-4}$aralkyl, partially unsaturated carbocyclyl, cycloalkyl, heteroaryl or heterocycloalkyl, whether alone or as part of a substituent group may be optionally substituted with one or more substituent independently selected from the group consisting of $C_{1-4}$alkyl, $C_{1-4}$alkoxy, halogen, hydroxy, carboxy, amino, $C_{1-4}$alkylamino, di($C_{1-4}$alkyl)amino, cyano and nitro;

wherein $R^P$ is selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;

wherein the cycloalkyl, aryl, heteroaryl, heterocycloalkyl or spiroheterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di($C_{1-4}$alkyl)amino, $-SO_2-N(R^SR^T)$, 5-tetrazolyl and 1-(1,4-dihydro-tetrazol-5-one);

wherein each $R^S$ and $R^T$ is independently selected from the group consisting of hydrogen and $C_{1-4}$alkyl;

a is an integer form 0 to 3;

each $R^{10}$ is independently selected from the group consisting of hydroxy, halogen, $C_{1-4}$alkyl, $C_{1-4}$alkoxy, halogenated $C_{1-4}$alkyl, halogenated $C_{1-4}$alkoxy, $-C(O)-NR^VR^W$, $-SO_2-NR^VR^W$, $-C(O)-C_{1-4}$alkyl and $-SO_2-C_{1-4}$alkyl;

wherein each $R^V$ and $R^W$ is independently selected from the group consisting of hydrogen and $C_{1-4}$alkyl; alternatively $R^V$ and $R^W$ are taken together with the N atom to which they are bound to form a 5 to 6 membered saturated, partially unsaturated or aromatic ring structure;

provided that the halogens on the halogenated $C_{1-4}$alkyl and the halogenated $C_{1-4}$alkoxy are selected from chloro or fluoro;

and pharmaceutically acceptable salts thereof.

In an embodiment, the present invention is directed to compounds of formula (II-AA)

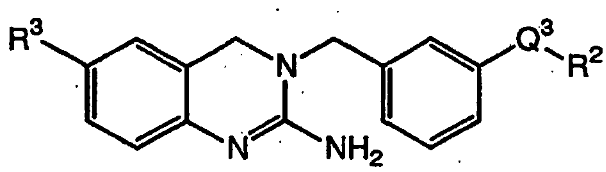

**(II-AA)**

wherein $R^3$, $Q^3$ and $R^2$ are as herein defined.

In an embodiment of the present invention are compounds of formula (II) wherein the $-(L^1)_b-R^3$ substituent group is bound at the 6 or 7 position, preferably at the 6-position. In another embodiment of the present invention, are compounds of formula (II) wherein the $-Q^3-R^2$ substituent group is bound at the 2-, 3-, 4- or 5- position of the

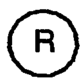

substituent, preferably at the 3- position of the

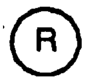

substituent.

In an embodiment of the present invention, $R^0$ is selected from the group consisting of hydrogen and methyl. In another embodiment of the present invention, $R^0$ is hydrogen.

In an embodiment of the present invention, $R^1$ is selected from the group consisting of hydrogen, hydroxy, methyl, trifluoromethyl, methoxy and methylcarbonyl. In another embodiment of the present invention $R^1$ is selected from the group consisting of hydrogen, hydroxy and methoxy. In yet another embodiment of the present invention, $R^1$ is hydrogen.

In an embodiment of the present invention, $R^2$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;
wherein the $C_{1-10}$alkyl, cycloalkyl, aryl, heteroaryl, heterocycloalkyl or splro-heterocyclyl, whether alone or as part of a substituent group is optionally substituted with one or more substituents independently selected from the group consisting of halogen, hydroxy, oxo, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$alkyl-S-$C_{1-4}$alkyl, -C(O)-N($R^L$-$R^M$), -$C_{1-4}$alkyl-C(O)-N($R^LR^M$), -N$R^L$-C(O)-$C_{1-4}$alkyl, -$SO_2$-N($R^L$-$R^M$), -$C_{1-4}$alkyl-$SO_2$-N($R^LR^M$), $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, nitro, cyano, amino, $C_{1-4}$alkylamino, di($C_{1-4}$alkyl)amino, phenyl, 5-tetrazolyl and 1-(1,4-dihydro-tetrazol-5-one);
wherein the phenyl is optionally substituted with one or more substituent independently selected from the group consisting of halogen, hydroxy, oxo, carboxy, C(O)O-$C_{1-4}$alkyl, C(O)-$C_{1-4}$alkyl, $C_{1-4}$alkyl, fluoro substituted $C_{1-4}$alkyl, $C_{1-4}$alkoxy, nitro, cyano, amino, $C_{1-4}$alkylamino and di($C_{1-4}$alkyl)amino;
wherein each $R^L$ and $R^M$ is independently selected from the group consisting of hydrogen and $C_{1-4}$alkyl;

In another embodiment of the present invention, $R^2$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;
wherein the $C_{1-10}$alkyl, cycloalkyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -C(O)-N ($R^LR^M$), -$C_{1-4}$alkyl-C(O)O-$C_{1-4}$alkyl, -$C_{1-4}$alkyl-C(O)-N($R^LR^M$), -N$R^L$-C(O)-$C_{1-4}$alkyl, $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, -O-$C_{1-4}$aralkyl, -O-(tetrahydropyranyl), -NH-C(O)-O-$CH_2$-(tetrahydropyranyl), nitro, cyano, amino, $C_{1-4}$alkylamino, di($C_{1-4}$alkyl)amino phenyl and 5-tetrazolyl;
wherein the phenyl or tetrahydropyranyl is optionally substituted with one to two substituent independently selected from the group consisting of halogen, hydroxy, oxo, carboxy, -OC(O)-$C_{1-4}$alkyl, -$C_{1-4}$alkyl-OC(O)-$C_{1-4}$alkyl, $C_{1-4}$alkyl, fluoro substituted $C_{1-4}$alkyl, hydroxy substituted $C_{1-4}$alkyl, $C_{1-4}$alkoxy, nitro, cyano, amino, $C_{1-4}$alkylamino and di($C_{1-4}$alkyl) amino;
wherein each $R^L$ and $R^M$ is independently selected from the group consisting of hydrogen and $C_{1-4}$alkyl.

In another embodiment of the present invention, $R^2$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl; wherein the $C_{1-10}$alkyl, cycloalkyl, aryl, heteroaryl, heterocycloalkyl or spiro-heterocyclyl, whether alone or as part of a substituent group is optionally substituted with one or more substituents independently selected from halogen, hydroxy, oxo, carboxy, - C(O)-$C_{1-4}$alkyl, -C(O)-$C_{1-4}$aralkyl, -C(O)O-$C_{1-4}$alkyl, -C(O)O-$C_{1-4}$aralkyl, -C(O)-N($R^LR^M$), -$C_{1-4}$alkyl-C(O)-N($R^LR^M$), -N$R^L$-C(O)-$C_{1-4}$alkyl, $C_{1-6}$alkyl, fluoro substituted $C_{1-4}$alkyl, hydroxy substituted $C_{1-4}$alkyl, carboxy substituted $C_{1- 4}$alkyl, $C_{1-4}$alkoxy, nitro, cyano, amino, $C_{1-4}$alkylamino, di($C_{1-4}$alkyl)amino or phenyl; wherein the phenyl is optionally substituted with one to two substituent independently selected from halogen, hydroxy, oxo, carboxy, $C_{1-4}$alkyl, fluoro substituted $C_{1-4}$alkyl, $C_{1-4}$alkoxy, nitro, cyano, amino, $C_{1-4}$alkylamino or di($C_{1-4}$alkyl)amino; and wherein each $R^L$ and $R^M$ is independently selected from the group consisting of hydrogen and $C_{1-4}$alkyl.

In another embodiment of the present invention, $R^2$ is selected from the group consisting of $C_{1-10}$alkyl, cycloalkyl, cycloalkyl-alkyl, aryl, $C_{1-4}$aralkyl, heteroaryl, heterocycloalkyl, spiro-heterocyclyl and heteroaryl-$C_{1-4}$alkyl-;
wherein the $C_{1-10}$alkyl, aryl, cycloalkyl, heteroaryl or heterocycloalkyl, whether alone or as part of a substituent group is optionally substituted with one to two substituent independently selected from the group consisting of $C_{1-5}$alkyl, $C_{1-4}$alkoxy, -O-$C_{1-2}$aralkyl, -O-(tetrahydropyranyl), -NH-C(O)-O-$CH_2$-(tetrahydropyranyl), halogen, trifluoromethyl, amino, cyano, hydroxy, oxo, carboxy, phenyl, -C(O)-$C_{1-4}$alkyl, -C(O)O-$C_{1-2}$aralkyl, -C(O)-N($R^LR^M$), -C(O)-N($C_{1-4}$alkyl)(cycloalkyl), -NH-C(O)-$C_{1-4}$alkyl, -C(O)O-$C_{1-4}$alkyl, -$C_{1-2}$alkyl-C(O)O-$C_{1-4}$alkyl, carboxy substituted $C_{1-2}$alkyl and 5-tetrazolyl;
wherein the phenyl or tetrahydropyranyl is optionally substituted with one to two substituents independently selected from the group consisting of hydroxy, halogen, cyano, $C_{1-4}$alkyl, $C_{1-4}$alkoxy, hydroxy substituted $C_{1-2}$alkyl, - OC (O)-$C_{1-2}$alkyl, -$C_{1-2}$alkyl-OC(O)-$C_{1-2}$alkyl, amino, $C_{1-4}$alkylamino and di($C_{1-4}$alkyl)amino;
wherein $R^L$ and $R^M$ are each independently selected from the group consisting of hydrogen, methyl and ethyl.

In another embodiment of the present invention, $R^2$ is selected from the group consisting of $C_{1-10}$alkyl, cycloalkyl, cycloalkyl-alkyl, aryl, $C_{1-4}$aralkyl, heteroaryl, heterocycloalkyl and spiro-heterocyclyl; wherein the $C_{1-10}$alkyl, aryl, cycloalkyl, heteroaryl or heterocycloalkyl, whether alone or as part of a substituent group is optionally substituted with one to two substituent independently selected from $C_{1-5}$alkyl, $C_{1-4}$alkoxy, halogen, trifluoromethyl, amino, cyano, hydroxy, oxo, carboxy, phenyl, -C(O)-$C_{1-4}$alkyl, -C(O)-$NH_2$, - C(O)-N($C_{1-4}$alkyl)(cycloalkyl), -NH-C(O)-$C_{1-4}$alkyl or -C(O)O-$C_{1-4}$alkyl; wherein the phenyl is optionally substituted with one to two substituents independently selected from the group consisting of halogen, cyano, $C_{1-4}$alkyl, $C_{1-4}$alkoxy, amino, $C_{1-4}$alkylamino and di($C_{1-4}$alkyl)amino.

In another embodiment of the present invention, $R^2$ is selected from the group consisting of methyl, ethyl, n-propyl, isopropyl, t-butyl, isobutyl, isopentyl, 3-n-heptyl, n-nonyl, amino-methyl, carboxy-methyl-, 2-amino-ethyl, 2-cyano-ethyl, 4-carboxy-n-butyl, 3-n-heptyl, 4-n-heptyl, 3-amino-n-propyl, 3,3,3-trifluoro-n-propyl, 3,3,3-trifluoro-isobutyl, 1-(1-carboxy-2-hydroxy-ethyl), 1-(1-(S)-carboxy-2-hydroxy-ethyl), 1-(1-(R)-carboxy-2-hydroxy-ethyl), 1-(1-carboxy-2-t-butoxy-ethyl), 1-(1-(R)-carboxy-2-t-butoxy-ethyl), 1-(1-carboxy-2-benzyloxyethyl), 1-(1-(S)-carboxy-2-benzyloxy-ethyl), 1-(1-(R)-carboxy-2-benzyloxyethyl), 1-(1-methoxy-carbonyl-2-benzyloxy-ethyl), 1-(1-(S)-methoxy-carbonyl-2-benzyloxy-ethyl), 1-(1-(R)-methoxy-carbonyl-2-benzyloxy-ethyl), cyclopropyl, cyclobutyl, cyclopentyl, cyclohexyl, cycloheptyl, 2-phenyl-cyclopropyl, cyclopentyl-methyl, cyclopentyl-ethyl, 1-(1-aminocarbonyl-cyclopropyl), 4-hydroxy-cyclohexyl, 4-carboxy-cyclohexyl, cis-(4-carboxy)-cyclohexyl, trans-(4-carboxy)-cyclohexyl, 3-carboxy-cyclohexyl, cis-(3-carboxy)-cyclohexyl, 4-cyano-cyclohexyl, 4-methoxy-carbonyl-cyclohexyl, 3-methoxy-carbonyl-cyclohexyl, cis-(3-methoxy-carbonyl)-cyclohexyl, 4-ethoxy-carbonyl-cyclohexyl, (1)-(S)-((4R)-methoxy-carbonyl-cyclohexyl, (1)-(R)-((45)-methoxy-carbonyl-cyclohexyl, 2-methyl-cyclohexyl, 4-methyl-cyclohexyl, 4-n-pentyl-cyclohexyl, 4-t-butyl-cyclohexyl, (1)-(S)-2-(R)-methyl-cyclopentyl, 3-methoxy-cyclohexyl, 1-(1-(4-chlorophenyl)-cyclopentyl), 4-trifluoromethyl-cyclohexyl, 4-oxo-cyclohexyl, 1-(4-benzyloxy-carbonyl-cyclohexyl), 1-(S)-(4-(S)-benzyloxy-carbonyl-cyclohexyl), 1-(4-amino-carbonyl-cyclohexyl), 1-(S)-(4-(S)-amino-carbonyl-cyclohexyl), 1-(4-methylamino-carbonyl-cyclohexyl), 1-(S)-(4-(S)-methylamino-carbonyl-cyclohexyl), 1-(4-(5-tetrazolyl)-cyclohexyl), phenyl, benzyl, phenyl-ethyl, 3-carboxy-methyl-benzyl, 3-methoxy-carbonyl-methyl-benzyl, 4-carboxy-phenyl, 3-cyano-phenyl, 4-methyl-phenyl, 4-t-butyl-phenyl, 4-n-butyl-phenyl, 2-methoxy-phenyl, 3-methoxy-phenyl, 4-dimethylamino-phenyl, 4-(methylcarbonylamino)-phenyl, 1-naphthyl-methyl, 1-(1,2,3,4-tetrahydro-naphthyl), 4-biphenyl, benzhydryl, 1-adamantyl, 2-adamantyl, 2-(*R*)-adamantyl, 2-(*S*)-adamantyl, N-piperidinyl, 1-(2-carboxy-piperidinyl), 1-(S)-2-carboxypiperidinyl), 1-(2-methoxy-carbonyl-piperidinyl), 1-(S)-2-methoxy-carbonyl-piperidinyl), 1-(2-methyl-piperidinyl), 1-(4-methyl-piperidinyl), 1-(4-isoproyl-piperidinyl), 4-(1-methylcarbonyl)-piperidinyl), 3-(2,5-dimethyl-furyl), 4-tetrahydropyranyl, 4-(2-phenyl-thiazolyl)-methyl, 4-(1-phenyl-pyrazolyl)-methyl, 5-(3-methyl-isoxazolyl)-methyl, 3-(5-phenyl-isoxazolyl)-methyl, 1-(2-carboxy-pyrrolidinyl), 1-(S)-(2-carboxy-pyrrolidinyl), 1-(2-(N-methyl-N-cyclohexylaminocarbonyl)-pyrrolidinyl), 1,4-dioxaspiro[4.5]dec-8-yl, 2-(bicyclo[2.2.1]heptyl), 1-(3-n-pentyl-bicyclo[2.2.2]-octyl, 2-bicyclo[2.2.2]octyl, 2-(R)-bicyclo[2.2.2]octyl, 2-(S)-bicyclo[2.2.2]octyl, 5-tetrazolyl-methyl, 2-imidazolyl-methyl, 5-imidazolyl-methyl, 4-pyridyl-methyl, 3-(1,2,4-triazolyl)-methyl, 1-(2-carboxy-octahydroindolyl), 1-(S)-(2-carboxy-octahydroindolyl), 1-(2-methoxy-carbonyl-octahydroindolyl), 1-(S)-2-methoxy-carbonyl-octahydroindolyl), 2R-(3R,4S,5R-tri(methyl-carbonyloxy)-6R-(methyl-carbonyloxy-methyl)-tetrahydropyranyl)oxy-ethyl, 2R-(3S,4S,5R-trihydroxy-6R-(hydroxy-methyl)-tetrahydropyranyl)oxy-ethyl and 3-(2R-(3S,4S,5R,6R-tetrahydroxy-tetrahydropyrantl)-methoxy-carbonyl-amino)-n-propyl.

In another embodiment of the present invention, $R^2$ is selected from the group consisting of methyl, ethyl, n-propyl, isopropyl, t-butyl, isobutyl, isopentyl, 3-n-heptyl, n-nonyl, amino-methyl, 2-amino-ethyl, 2-cyano-ethyl, 4-carboxy-n-butyl, 3-n-heptyl, 4-n-heptyl, 3-amino-n-propyl, 3,3,3-trifluoro-n-propyl, 3,3,3-trifluoro-isobutyl, cyclopropyl, cyclobutyl, cyclopentyl, cyclohexyl, cycloheptyl, 2-phenyl-cyclopropyl, cyclopentyl-methyl, cyclopentyl-ethyl, 1-(1-aminocarbonyl-cyclopropyl), 4-hydroxy-cyclohexyl, 4-carboxy-cyclohexyl, cis-(4-carboxy)-cyclohexyl, trans-(4--carboxy)-cyclohexyl, 4-methoxy-carbonyl-cyclohexyl, 4-ethoxy-carbonyl-cyclohexyl, (1)-(S)-((4R)-methoxy-carbonyl-cyclohexyl, (1)-(R)-((4S)-methoxy-carbonyl-cyclohexyl, 2-methyl-cyclohexyl, 4-methyl-cyclohexyl, 4-n-pentyl-cyclohexyl, 4-t-butyl-cyclohexyl, (1)-(S)-2-(R)-methyl-cyclopentyl, 3-methoxy-cyclohexyl, 1-(1-(4-chlorophenyl)-cyclopentyl), 4-trifluoromethyl-cyclohexyl, 4-oxo-cyclohexyl, phenyl, benzyl, phenyl-ethyl, 3-cyano-phenyl, 4-methyl-phenyl, 4-t-butyl-phenyl, 4-n-butyl-phenyl, 2-methoxy-phenyl, 3-methoxy-phenyl, 4-dimethylamino-phenyl, 4-(methylcarbonylamino)-phenyl, 1-naphthyl-methyl, 1-(1,2,3,4-tetrahydro-naphthyl), 4-biphenyl, benzhydryl, 1-adamantyl, 2-adamantyl, 2-(*R*)-adamantyl, 2-(*S*)-adamantyl, N-piperidinyl, 1-(2-methyl-piperidinyl), 1-(4-methyl-piperidinyl), 1-(4-isoproyl-piperidinyl), 4-(1-methylcarbonyl)-piperidinyl), 3-(2,5-dimethyl-furyl), 4-tetrahydropyranyl, 4-(2-phenyl-thiazolyl)-methyl, 4-(1-phenyl-pyrazolyl)-methyl, 5-(3-methyl-isoxazolyl)-methyl, 3-(5-phenyl-isoxazolyl)-methyl, 1-(2-(N-methyl-N-cyclohexylaminocarbonyl)-pyrrolidinyl), 1,4-dioxaspiro[4.5]dec-8-yl, 2-(bicyclo[2.2.1]heptyl), 1-(3-n-pentyl-bicyclo[2.2.2]-octyl, 2-bicyclo[2.2.2]octyl, 2-(R)-bicyclo [2.2.2]octyl and 2-(S)-bicyclo[2.2.2]octyl.

In an embodiment of the present invention, $R^2$ is selected from the group consisting of isopropyl, n-butyl, t-butyl, 1-ethyl-n-pentyl, isopentyl, 3-n-heptyl, 4-n-heptyl, cyclobutyl, cyclopentyl, cyclohexyl, cycloheptyl, cyclopentyl-methyl-, 2-methyl-cyclohexyl, 3-methoxy-cyclohexyl, phenyl, phenylethyl-, 4-(1-methyl-piperidinyl), 1-(1-(4-chlorophenyl)-cyclopentyl), 1-adamantyl and 2-adamantyl.

In another embodiment of the present invention, $R^2$ is selected from the group consisting of isopropyl, t-butyl,

1-ethyl-n-pentyl, isopentyl, 3-n-heptyl, 4-n-heptyl, cyclopentyl, cyclohexyl, cycloheptyl, cyclopentyl-methyl-, 2-methyl-cyclohexyl, phenyl, 4-(1-methyl-piperidinyl), 1-(1-(4-chlorophenyl)-cyclopentyl), 1-adamantyl and 2-adamantyl.

In another embodiment of the present invention, $R^2$ is selected from the group consisting of isopentyl, 4-n-heptyl, cyclopentyl, cyclohexyl, cyclopentyl-methyl-, 1-(1-(4-chlorophenyl)-cyclopentyl), 1-adamantyl and 2-adamantyl.

In another embodiment of the present invention, $R^2$ is selected from the group consisting of carboxy-methyl, 2-cyanoethyl-, n-propyl, isopropyl, n-butyl, isobutyl, t-butyl, isopentyl, 3-n-heptyl, 4-n-heptyl, 4-carboxy-n-butyl, cyclopropyl, cyclobutyl, cyclopentyl, cyclohexyl, cycloheptyl, cyclopentyl-methyl-, cyclopentyl-ethyl-, 1-(S)-(1-carboxy-2-hydroxy-ethyl), 1-(R)-(1-carboxy-2-hydroxy-ethyl), 1-(R)-(1-carboxy-2-t-butoxy-ethyl), 1-(S)-(1-methoxy-carbonyl-2-benzyloxy-ethyl), 1-(R)-(1-methoxy-carbonyl-2-benzyloxy-ethyl), 1-(S)-(1-carboxy-2-benzyloxy-ethyl), 1-(R)-(-carboxy-2-benzyloxy-ethyl), trans-2-methyl-cyclohexyl-, 1-(1-(4-chlorophenyl)-cyclopentyl), 3-methoxy-cyclohexyl, 4-hydroxy-cyclohexyl, 1-cis-(3-carboxy-cyclohexyl), 4-carboxy-cyclohexyl, (1)-(S)-((4R)-carboxy-cyclohexyl), (1)-(R)-((4S)-carboxy-cyclohexyl), 4-(ethoxy-carbonyl)-cyclohexyl, cis-(4-methoxy-carbonyl)-cyclohexyl, trans-(4-methoxy-carbonyl)-cyclohexyl, 1-4-oxo-cyclohexyl, 1-cis-(4-amino-carbonyl-cyclohexyl), phenyl, 2-methoxyphenyl, 2-methylphenyl, benzyl, phenylethyl-, benzhydryl, 4-(1-isopropyl)-piperidinyl, 4-(1-methyl-piperidinyl), 1-adamantyl, 2-adamantyl, 4-(tetrahydropyranyl), 5-(3-methyl-isoxazolyl)-methyl, 1,4-oxaspiro[4.5]dec-8-yl and 5-tetrazolyl-methyl.

In an embodiment of the present invention, $R^2$ is selected from the group consisting of 2-cyanoethyl-, n-propyl, isopropyl, n-butyl, isobutyl, t-butyl, isopentyl, 3-n-heptyl, 4-n-heptyl, 4-carboxy-n-butyl, cyclopropyl, cyclobutyl, cyclopentyl, cyclohexyl, cycloheptyl, cyclopentyl-methyl-, cyclopentyl-ethyl-, trans-2-methyl-cyclohexyl-, 1-(1-(4-chlorophenyl)-cyclopentyl), 3-methoxy-cyclohexyl, 4-hydroxy-cyclohexyl, 4-carboxy-cyclohexyl, (1)-(S)-((4R)-carboxy-cyclohexyl), (1)-(R)-((4S)-carboxy-cyclohexyl), 4-(ethoxy-carbonyl)-cyclohexyl, cis-(4--methoxy-carbonyl)-cyclohexyl, trans-(4--methoxy-carbonyl)-cyclohexyl, 1-4-oxo-cyclohexyl, phenyl, 2-methoxyphenyl, 2-methylphenyl, benzyl, phenylethyl-, benzhydryl, 4-(1-isopropyl)-piperidinyl, 4-(1-methyl-piperidinyl), 1-adamantyl, 2-adamantyl, 4-(tetrahydropyranyl), 5-(3-methyl-isoxazolyl)-methyl and 1,4-oxaspiro[4.5]dec-8-yl.

In another embodiment of the present invention, $R^2$ is selected from the group consisting of carboxy-methyl, isopropyl, isobutyl, t-butyl, isopentyl, 3-n-heptyl, 4-n-heptyl, 4-carboxy-n-butyl, cyclobutyl, cyclopentyl, cyclohexyl, cycloheptyl, cyclopentyl-methyl-, cyclopentyl-ethyl-, 1-(S)-(1-carboxy-2-hydroxy-ethyl), 1-(R)-(1-carboxy-2-hydroxy-ethyl), 1-(R)-(1-carboxy-2-t-butoxyethyl), 1-(S)-(1-methoxy-carbonyl-2-benzyloxy-ethyl), 1-(R)-(1-methoxy-carbonyl-2-benzyloxy-ethyl), 1-(S)-(1-carboxy-2-benzyloxy-ethyl), trans-2-methyl-cyclohexyl-, 1-(1-(4-chlorophenyl)-cyclopentyl), 3-methoxy-cyclohexyl, 4-hydroxy-cyclohexyl, 1-cis-(3-carboxy-cyclohexyl), 4-carboxy-cyclohexyl, (1)-(S)-((4R)-carboxy-cyclohexyl), (1)-(R)-((45)-carboxy-cyclohexyl), cis-(4-methoxy-carbonyl)-cyclohexyl, trans-(4--methoxy-carbonyl)-cyclohexyl, 1-cis-(4-amino-carbonyl-cyclohexyl), phenyl, 2-methylphenyl, phenylethyl-, 4-(1-methyl-piperidinyl), 1-adamantyl, 2-adamantyl, 4-(tetrahydropyranyl), 5-(3-methyl-isoxazolyl)-methyl and 5-tetrazolyl-methyl.

In another embodiment of the present invention, $R^2$ is selected from the group consisting of isopropyl, isobutyl, t-butyl, isopentyl, 3-n-heptyl, 4-n-heptyl, 4-carboxy-n-butyl, cyclobutyl, cyclopentyl, cyclohexyl, cycloheptyl, cyclopentyl-methyl-, cyclopentyl-ethyl-, trans-2-methyl-cyclohexyl-, 1-(1-(4-chlorophenyl)-cyclopentyl), 3-methoxy-cyclohexyl, 4-hydroxy-cyclohexyl, 4-carboxy-cyclohexyl, (1)-(S)-((4R)-carboxy-cyclohexyl), (1)-(R)-((4S)-carboxy-cyclohexyl), cis-(4--methoxy-carbonyl)-cyclohexyl, trans-(4--methoxy-carbonyl)-cyclohexyl, phenyl, 2-methylphenyl, phenylethyl-, 4-(1-methyl-piperidinyl), 1-adamantyl, 2-adamantyl, 4-(tetrahydropyranyl) and 5-(3-methyl-isoxazolyi)-methyl.

In another embodiment of the present invention, $R^2$ is selected from the group consisting of carboxy-methyl, isobutyl, isopentyl, 1-(1-(R)-carboxy-2-hydroxy-ethyl), 1-(1-(R)-carboxy-2-t-butoxy-ethyl), 1-(1-(S)-carboxy-2-benzyloxy-ethyl), cyclopentyl, cyclohexyl, 4-carboxy-cyclohexyl, (1)-(S)-((4R)-carboxy-cyclohexyl), 1-adamantyl and 2-adamantyl.

In another embodiment of the present invention, $R^2$ is selected from the group consisting of isobutyl, isopentyl, cyclopentyl, cyclohexyl, 4-carboxy-cyclohexyl, (1)-(S)-((4R)-carboxy-cyclohexyl), 1-adamantyl and 2-adamantyl.

In an embodiment of the present invention, $L^1$ is selected from the group consisting of -O-, $-S(O)_{0-2}-$, -C(O)-, -C(S)-, $-C_{1-4}alkyl-$ and (hydroxy substituted $C_{1-4}alkyl$)-. In another embodiment of the present invention, $L^1$ is selected from the group consisting of -O-, -C(O)-, $-CH_2-$ and =C(OH)-. In another embodiment of the present invention, $L^1$ is selected from the group consisting of -O-, -C(O)-, $-CH_2-$ and -C(OH)-.

In an embodiment of the present invention, $L^1$ is selected from the group consisting of -C(O)- and -O-. In another embodiment of the present invention, $L^1$ is -O-.

In an embodiment of the present invention, $L^1$ is selected from the group consisting of -C(O)- and -(O)-. In another embodiment of the present invention, $L^1$ is -(O)-;

In an embodiment of the present invention, $R^3$ is selected from the group consisting of $C_{1-6}alkyl$, $C_{2-6}alkenyl$, cycloalkyl, partially unsaturated carbocyclyl, aryl, $C_{1-4}aralkyl$, biphenyl, heteroaryl and heterocycloalkyl; wherein the cycloalkyl, partially unsaturated carbocyclyl, aryl, heteroaryl or heterocycloalkyl, whether alone or as part of a substituent group is optionally substituted with one to two substituents independently selected from halogen, hydroxy, oxo, carboxy, $C_{1-4}alkyl$, hydroxy substituted $C_{1-4}alkyl$, halogen substituted $C_{1-4}alkyl$, cyano substituted $C_{1-4}alkyl$, $C_{2-4}alkenyl$, $C_{2-4}alkynyl$, $C_{1-4}alkoxy$, halogen substituted $C_{1-4}alkoxy$, nitro, cyano, $-R^5$, $-O-R^5$, $-S-R^5$, $-NH_2$, $-N(R^P)-R^5$, $-C(O)-R^5$, $-C(O)-NH_2$, $-C(O)-NR^P-R^5$, $-NR^P-C(O)-R^5$, $-NR^P-C(O)O-R^5$ and $-SO_2-NR^P-R^5$.

In another embodiment of the present invention, $R^3$ is selected from the group consisting of $C_{2-6}$alkenyl, aryl, biphenyl, partially unsaturated carbocyclyl and heteroaryl; wherein the aryl or heteroaryl group is optionally substituted with one to two substituents independently selected from hydroxy, halogen, $C_{1-4}$alkyl, $C_{1-4}$alkoxy, -S-$C_{1-4}$alkyl, hydroxy substituted $C_{1-4}$alkyl, halogen substituted $C_{1-4}$alkyl, -C(O)-$NH_2$, -C(O)-$C_{1-4}$alkyl, -NH-$C_{1-4}$alkyl-cycloalkyl, -NH-C(O)-$C_{1-4}$alkyl, -NH-C(O)-O-$C_{1-4}$aralkyl, -$SO_2$-NH-$C_{1-4}$alkyl or -$SO_2$-NH-phenyl.

In another embodiment of the present invention, $R^3$ is selected from the group consisting of n-penten-1-yl, phenyl, 2-hydroxy-phenyl, 2-chloro-phenyl, 3-chloro-phenyl, 4-chloro-phenyl, 2-fluoro-phenyl, 4-fluorophenyl, 2,6-difluorophenyl, 2-methyl-phenyl, 3-methyl-phenyl, 4-methyl-phenyl, 2-methoxy-phenyl, 4-methoxy-phenyl, 3,5-dimethoxy-phenyl, 2,6-dimethoxy-phenyl, 2-isopropylphenyl, 2-methylthio-phenyl, 2-fluoro-6-methoxy-phenyl, 2-methoxy-5methylphenyl, 2-methoxy-5-fluoro-phenyl, 3-(hydroxymethyl)-phenyl, 3-trifluoromethylphenyl, 2-(methylcarbonylamino)-phenyl, 2-(t-butylaminosulfonyl)-phenyl, 2-(aminocarbonyl)-phenyl, 2-(methylsulfonylamino)-phenyl, 3-(methylcarbonyl)-phenyl, 3-(benzyloxycarbonylamino)-phenyl, 3-(N-(cyclohexylmethyl)-amino)-phenyl, 3-(phenylsulfonylamino)-phenyl, 2-naphthyl, 1-cyclohexenyl, 1-cyclopentenyl, 2-biphenyl, 5-pyrimidinyl, 4-pyridyl, 3-quinolinyl and 3-(6-fluorobenzo[d]isoxazolyl).

In an embodiment of the present invention, $R^3$ is selected from the group consisting of phenyl, 2-methoxyphenyl, 3-(benzyloxy-carbonyl-amino)-phenyl, 3-(N-(cyclohexyl-methyl)-amino)-phenyl and 3-(phenyl-sulfonyl-amino)-phenyl. In another embodiment of the present invention, $R^3$ is selected from the group consisting of phenyl, 2-methoxyphenyl, 3-(benzyloxy-carbonyl-amino)-phenyl and 3-(phenyl-sulfonyl-amino)-phenyl. In yet another embodiment of the present invention, $R^3$ is selected from the group consisting of phenyl and 2-methoxyphenyl.

In an embodiment of the present invention, $R^3$ is selected from the group consisting of phenyl, 2-hydroxyphenyl, 2-fluorophenyl, 2,6-difluorophenyl, 2-chlorophenyl, 3-chlorophenyl, 2-methylphenyl, 3-methylphenyl, 2-methoxy-phenyl, 2-fluoro-6-methoxyphenyl, 3-hydroxymethyl-phenyl and 3-(phenylsulfonyl-amino)-phenyl. In another embodiment of the present invention, $R^3$ is selected from the group consisting of phenyl, 2-fluorophenyl, 2,6-difluorophenyl, 2-chlorophenyl, 2-methylphenyl, 3-methylphenyl, 2-methoxyphenyl and 2-fluoro-6-methoxyphenyl. In another embodiment of the present invention, $R^3$ is selected from the group consisting of phenyl, 2-fluorophenyl and 2-methoxy-phenyl.

In an embodiment of the present invention, $R^5$ is selected from the group consisting of $C_{1-4}$alkyl, aryl, $C_{1-4}$aralkyl, cycloalkyl and cycloalkyl-$C_{1-4}$alkyl-; wherein the aryl, whether alone or as part of a substituent group is optionally substituted with one to two substituent independently selected from $C_{1-4}$alkyl, $C_{1-4}$alkoxy, halogen, hydroxy, carboxy, amino, $C_{1-4}$alkylamino, di($C_{1-4}$alkyl)amino, cyano or nitro.

In an embodiment of the present invention, $R^P$ is selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl and $C_{1-4}$aralkyl; wherein the cycloalkyl, aryl, heteroaryl, heterocycloalkyl or spiro-, heterocyclyl, whether alone or as part of a substituent group is optionally substituted with one to two substituents independently selected from halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di($C_{1-4}$alkyl)amino,-$SO_2$-N($R^SR^T$), 5-tetrazolyl or 1-(1,4-dihydro-tetrazol-5-one); and wherein each $R^S$ and $R^T$ is independently selected from hydrogen or $C_{1-4}$alkyl.

In an embodiment of the present invention, $R^{10}$ is selected from the group consisting of hydroxy, halogen, $C_{1-4}$alkyl, $C_{1-4}$alkoxy, halogen substituted $C_{1-4}$alkyl, halogen substituted $C_{1-4}$alkoxy; provided that the halogens on the halogen substituted $C_{1-4}$alkyl and the halogen substituted $C_{1-4}$alkoxy are selected from fluoro or chloro. In another embodiment of the present invention, $R^{10}$ is selected from the group consisting of halogen. In another embodiment of the present invention, $R^{10}$ is fluoro.

In an embodiment of the present invention, $R^1$ is selected from the group consisting of hydrogen, methyl and methylcarbonyl. In another embodiment of the present invention, $R^1$ is hydrogen. In another embodiment of the present invention, $R^1$ is selected from the group consisting of hydrogen and methoxy.

In an embodiment of the present invention, c is an integer from 0 to 1. In another embodiment of the present invention, c is 1.

In an embodiment of the present invention, $A^2$ is selected from the group consisting of $C_{1-4}$alkyl; wherein the $C_{1-4}$alkyl is optionally substituted with an $R^Y$ substituent; wherein $R^Y$ is selected from the group consisting of $C_{1-4}$alkyl, aryl, $C_{1-4}$aralkyl and cycloalkyl-$C_{1-4}$alkyl-. In another embodiment of the present invention, $A^2$ is selected from the group consisting of -$CH_2$-, -CH($CH_2CH_3$)-, - CH(phenyl)-, -CH(benzyl)- and -CH(cyclohexylmethyl)-.

In another embodiment of the present invention, $A^2$ is selected from the group consisting of -$CH_2$- and -CH($CH_2CH_3$)-. In another embodiment of the present invention, $A^2$ is -$CH_2$-.

In another embodiment of the present invention, $A^2$ is selected from the group consisting of -$CH_2$-, -CH($CH_2CH_3$)-,-CH(phenyl)- and -CH(cyclohexyl)-. In yet another embodiment of the present invention, $A^2$ is selected from the group consisting of -$CH_2$-, -CH($CH_2CH_3$)- and -CH(cyclohexyl)-. In another embodiment of the present invention, $A^2$ is -$CH_2$-.

In an embodiment of the present invention,

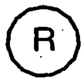

is selected from the group consisting of aryl, aryl-$C_{1-4}$alkyl-, cycloalkyl, heteroaryl and heterocycloalkyl; wherein the aryl, cycloalkyl, heteroaryl or heterocycloalkyl group, whether alone or as part of a substituet group, is optionally substituted with one to two substituents independently selected from $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino or di ($C_{1-4}$alkyl)amino; provided that when c is 0, then

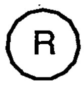

is other than aryl or heteroaryl.

In an embodiment of the present invention,

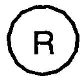

is selected from the group consisting of cycloalkyl, aryl, aryl-$C_{1-4}$alkyl-, heteroaryl, and heterocycloalkyl; wherein the aryl, heteroaryl or heterocycloalkyl, whether alone or as part of a substituent group, is optionally substituted with one to two substituents independently selected from $C_{1-4}$alkyl or $C_{1-4}$alkoxy; provided that when c is 0, then

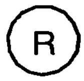

is other than aryl or heteroaryl.

In another embodiment of the present invention,

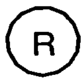

is selected from the group consisting of cyclopentyl, (S)-cyclopentyl, (R)-cyclopentyl, cyclohexyl, (R)-cyclohexyl, (S)-cyclohexyl, trans-cyclohexyl, phenyl, benzyl, 9-fluorenyl, 3-pyrrolidinyl, 1-indanyl, 1-(5-methoxy-indanyl)-methyl, 3-piperidinyl, 4-piperidinyl, 3-azepinyl, 2-pyridyl, 4-pyridyl, 2-furyl, 2-thienyl and 5-oxazolyl; provided that when c is 0, then

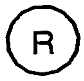

is other than phenyl.

In an embodiment of the present invention,

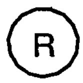

is selected from the group consisting of phenyl, 4-piperidinyl and 4-pyridyl; provided that when c is 0, then

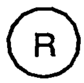

is other than phenyl. In another embodiment of the present invention,

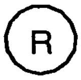

is selected from the group consisting of phenyl and 4-piperidinyl; provided that when c is 0, then

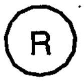

is 4-piperidinyl. In yet another embodiment of the present invention, c is 1 and

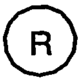

is phenyl.

In an embodiment of the present invention,

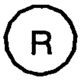

is selected from the group consisting of phenyl, 2-pyridyl and 2-furyl. In another embodiment of the present invention,

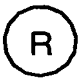

is selected from the group consisting of phenyl and 2-pyridyl.

In an embodiment of the present invention, $Q^3$ is selected from the group consisting of -C(O)-, -C(O)O-, -C (O)-NH-, -C(O)-N($C_{1-8}$alkyl)-, -C(O)-N(cycloalkyl)-, -NH-C(O)- and -NH-C(O)O-; wherein the cycloalkyl is optionally substituted with $C_{1-4}$alkyl.

In another embodiment of the present invention, $Q^3$ is selected from the group consisting of -C(O)-, -C(O)-NH-, -C(O)-N($CH_3$)-, -(R)-C(O)-N($CH_3$), -(S)-C(O)-N($CH_3$), -C(O)-N(isopropyl)-, -C(O)-N(n-propyl)-, -C(O)-N(isobutyl)-, - C (O)-N(2-ethyl-n-hexyl)-, -C(O)-N(cyclohexyl)-, -C(O)-N(4-methyl-cyclohexyl)-, -C(O)O-, -NH-C(O)- and -NH-C(O)O-.

In an embodiment of the present invention, $Q^3$ is selected from the group consisting of -1-C(O)O-, -3-C(O)O-, -2-C(O)-N($CH_3$)- and -3-C(O)-N($CH_3$)-. In another embodiment of the present invention, $Q^3$ is selected from the group consisting of -1-C(O)O-, -3-C(O)O- and -3-C(O)-N($CH_3$)-. In another embodiment of the present invention, $Q^3$ is -3-C (O)-N($CH_3$)-.

In an embodiment of the present invention, $Q^3$ is selected from the group consisting of -3-C(O)-N($CH_3$)-, -3-C (O)-N(isopropyl)-, -3-C(O)-N(isobutyl)-,-3-C(O)-N(cyclohexyl)-, -4-C(O)-N(CH3)- and 5-C(O)-N($CH_3$)-. In another embodiment of the present invention, $Q^3$ is selected from the group consisting of -3-C(O)-N($CH_3$)-, -3-C(O)-N(isopropyl)-,-3-C(O)-N(cyclohexyl)- and -4-C(O)-N($CH_3$)-. In another embodiment of the present invention, $Q^3$ is -3-C(O)-N($CH_3$)-.

In an embodiment of the present invention, $R^2$ is selected from the group consisting of $C_{1-8}$alkyl, cycloalkyl, cycloalkyl-$C_{1-4}$alkyl-, aryl, $C_{1-4}$aralkyl, heteroaryl, heteroaryl-$C_{1-4}$alkyl-, heterocycloalkyl and heterocycloalkyl-$C_{1-4}$alkyl-; wherein the $C_{1-8}$alkyl, cycloalkyl, aryl or heterocycloalkyl, whether alone or as part of a substituent group is optionally substituted with one to three halogen, $C_{1-4}$alkyl, -$SO_2$-$NH_2$ or phenyl.

In another embodiment of the present invention, $R^2$ is selected from the group consisting of trifluoromethyl, methyl, ethyl, isobutyl, t-butyl, 3-n-heptyl, 4-n-heptyl, 2-ethyl-n-hexyl, cyclopentyl, cyclohexyl, 4-methyl-cyclohexyl, cyclopropyl-methyl, 4-aminosulfonyl-phenylethyl, benzhydryl, 1-adamantyl, 2-adamantyl, 2-(*R*)-adamantyl, 2-(*S*)-adamantyl, 2-decahydro-isoquinolinyl, 2-(1-methyl-pyrrolidinyl)-ethyl, 1-piperidinyl and 4-(1-methyl-piperidinyl).

In another embodiment of the present invention, $R^2$ is selected from the group consisting of methyl, ethyl, t-butyl and cyclohexyl. In an embodiment of the present invention, $R^2$ is selected from the group consisting of isobutyl and cyclohexyl. In another embodiment of the present invention, $R^2$ is cyclohexyl.

In an embodiment of the present invention, $L^1$ is selected from the group consisting of -O-, -S-, -NH-, -$C_{1-4}$alkyl- and -$C_{2-4}$alkenyl-. In another embodiment of the present invention, $L^1$ is selected from the group consisting of -O-, -S-, -NH-, -CH($CH_3$)- and -CH=CH-. In another embodiment of the present invention, $L^1$ is selected from the group consisting

of -O-, -S- and -NH-In another embodiment of the present invention, $L^1$ is selected from the group consisting of -O- and -S-.

In an embodiment of the present invention, $R^3$ is selected from the group consisting of $C_{1-4}$alkyl, cycloalkyl, cycloalkyl-$C_{1-4}$alkyl-, aryl, heteroaryl and heterocycloalkyl; wherein the cycloalkyl or aryl, whether alone or as art of a substituent group is optionally substituted with one to two substituents independently selected from halogen, hydroxy, carboxy, cyano, nitro, amino, $C_{1-4}$alkylamino, di($C_{1-4}$alkylamino), $C_{1-4}$alkyl, $C_{2-4}$alkenyl, $C_{1-4}$alkoxy, hydroxy substituted $C_{1-4}$alkyl, halogen substituted $C_{1-4}$alkyl, cyano substituted $C_{1-4}$alkyl, halogen substituted $C_{1-4}$alkoxy, -O-aryl, -O-$C_{1-4}$aralkyl, -S-$C_{1-4}$alkyl, -$SO_2$-$C_{1-4}$alkyl, -C(O)-$C_{1-4}$alkyl, -C(O)-$NH_2$, -C(O)-NH($C_{1-4}$alkyl), -C(O)-N($C_{1-4}$alkyl)$_2$, - NH-C (O)-$C_{1-4}$alkyl, -C(O)-NH-$C_{1-4}$alkyl, -NH-$SO_2$-$C_{1-4}$alkyl, -NH-$SO_2$-phenyl, aryl, $C_{1-4}$aralkyl, cycloalkyl, cycloalkyl-$C_{1-4}$alkyl-, heteroaryl, heteroaryl-$C_{1-4}$alkyl-, heterocycloalkyl or heterocycloalkyl-$C_{1-4}$alkyl-; and wherein the aryl, cycloalkyl, heteroaryl or heterocycloalkyl substituent, whether alone or as part of a substituent group, is optionally substituted with one to three substituents independently selected from $C_{1-4}$alkyl or $C_{1-4}$alkoxy.

In another embodiment of the present invention, $R^3$ is selected from the group consisting of n-pentyl, isopentyl, isobutyl, isopropyl, cyclopentyl, cyclopentyl-methyl, phenyl, 2-hydroxy-phenyl, 3-carboxy-phenyl, 2-cyanophenyl, 2-nitrophenyl, 2-bromo-phenyl, 2-fluoro-phenyl, 2-chloro-phenyl, 2,6-dichloro-phenyl, 2-methyl-phenyl, 3-methyl-phenyl, 2-ethyl-phenyl, 4-isopropylphenyl, 3,4-dimethyl-phenyl, 3,5-dimethyl-phenyl, 2,6-dimethyl-phenyl, 2-methoxy-phenyl, 3-methoxy-phenyl, 4-methoxy-phenyl, 3,4-dimethoxy-phenyl, 2,6-dimethoxy-phenyl, 2-ethoxy-phenyl, 3-ethoxy-phenyl, 2-isopropoxy-phenyl, 2-methoxy-5-methyl-phenyl, 2-methoxy-6-methyl-phenyl, 2-trifluoromethylphenyl, 2-trifluoromethoxy-phenyl, 2-methylthio-phenyl, 4-methylthio-phenyl, 2-hydroxymethyl-phenyl, 2-cyanomethyl-phenyl, 2-(aminocarbonyl)-phenyl, 4-(aminocarbonyl)-phenyl, 2-(dimethylaminocarbonyl)-phenyl, 3-(dimethylamino)-phenyl, 4-(dimethylamino)-phenyl, 2-allyl-6-methyl-phenyl, 2-allyl-6-ethoxyphenyl, 2-methyl-6-n-propyl-phenyl, 3-(methylcarbonylamino)-phenyl, 2-(methylaminocarbonyl)-phenyl, 2-(methylcarbonyl)-phenyl, 4-(methylcarbonylamino)-phenyl, 2-(aminocarbonylmethyl)-phenyl, 2-(methylsulfonyl)-phenyl, (3-(2-methoxy-4-methyl-phenyl)-sulfonylamino)-phenyl, 3-(2,4,6-trimethylphenylsulfonylamino)-phenyl, 3-(phenylsulfonylamino)-phenyl, 2-(t-butylaminosulfonyl)-phenyl, 2-(t-butylcarbonylamino)-5-methoxy-phenyl, 3-(phenylsulfonylamino)-phenyl, 2-phenoxy-phenyl, 3-phenoxy-phenyl, 2-benzyloxy-phenyl, 2-(2-benzthiazolyl)-5-methoxy-phenyl, 2-(2-benzthiazolyl)-phenyl, 2-(1-pyrrolyl)-pheriyl, 3-(2-quinolinyl)-phenyl, 2-(1-pyrrolidinyl-methyl)-phenyl, 2-cyclopentyl-phenyl, 4-cyclohexyl-phenyl, 4-(4-morpholinyl)-phenyl, 3-methoxy-benzyl, 1-naphthyl, 2-naphthyl, 2-(5,6,7,8-tetrahydro-naphthyl), 2-biphenyl, 3-biphenyl, 2-biphenyl-methyl, 3-pyridyl, 3,4-methylenedioxyphenyl, 4(3,5-dimethyl-isoxazolyl), 4-pyrazolyl, 3-thienyl, 3-pyridyl, 4-pyridyl, 5-indolyl and 3-benzothienyl.

In an embodiment of the present invention, $R^3$ is selected from the group consisting of phenyl, 2-bromophenyl, 2-chlorophenyl, 2,6-dichlorophenyl, 2-hydroxy-phenyl, 2-hydroxymethyl-phenyl, 2-methoxy-phenyl, 3-methoxyphenyl, 2-ethoxy-phenyl, 2-methylthio-phenyl, 2-cyanomethyl-phenyl, 3-(phenyl-sulfonyl-amino)-phenyl, 3-(2,4,6-trimethylphenyl-sulfonyl-amino)-phenyl, (3-(2-methoxy-4-methyl-phenyl)-sulfonyl-amino)-phenyl, 2-(t-butylcarbonyl-amino)-5-methoxy-phenyl, 1-naphthyl, 3-thienyl and 4-(3,5-dimethylisoxazolyl).

In another embodiment of the present invention, $R^3$ is selected from the group consisting of phenyl, 2-bromophenyl, 2-chlorophenyl, 2,6-dichlorophenyl, 2-hydroxy-phenyl, 2-hydroxymethyl-phenyl, 2-methoxy-phenyl, 3-methoxyphenyl, 2-ethoxy-phenyl, 2-methylthio-phenyl, 2-cyanomethyl-phenyl, 3-(phenyl-sulfonyl-amino)-phenyl, 1-naphthyl and 3-thienyl. In another embodiment of the present invention, $R^3$ is selected from the group consisting of phenyl, 2-methoxy-phenyl, 2-ethoxy-phenyl and 1-naphthyl.

In an embodiment of the present invention, $R^3$ is selected from the group consisting of phenyl, 2-bromophenyl, 2-fluorophenyl, 2-chlorophenyl, 2,6-dichlorophenyl, 2-hydroxyphenyl, 2-hydroxymethyl-phenyl, 2-methylphenyl, 3-methylphenyl, 2,6-dimethylphenyl, 2-methoxyphenyl, 3-methoxyphenyl, 2,6-dimethoxyphenyl, 2-ethoxyphenyl, 2-trifluoromethylphenyl, 2-trifluoromethoxyphenyl, 2-methylthio-phenyl, 2-nitrophenyl, 2-cyanophenyl, 2-cyanomethyl-phenyl, 2-phenoxy-phenyl, 2-(methyl-carbonyl-amino)-phenyl, 2-(amino-carbonyl)-phenyl, 3-(phenyl-sulfonyl-amino)-phenyl, 2-(t-butyl-aminosulfonyl)-phenyl, 2-(t-butyl-carbonyl-amino)-5-methoxy-phenyl, 4-(3,5-dimethylsoxazolyl), 1-naphthyl, 3-thienyl and 3-pyridyl.

In another embodiment of the present invention, $R^3$ is selected from the group consisting of phenyl, 2-bromophenyl, 2-fluorophenyl, 2-chlorophenyl, 2-hydroxyphenyl, 2-hydroxymethyl-phenyl, 3-methylphenyl, 2-methoxyphenyl, 2-ethoxyphenyl, 2-cyanomethyl-phenyl, 2-(t-butyl-carbonyl-amino)-5-methoxy-phenyl, 1-naphthyl and 3-thienyl.

In another embodiment of the present invention, $R^3$ is selected from the group consisting of 2-methoxyphenyl and 2-ethoxyphenyl.

In an embodiment of the present Invention, $R^A$ is selected from the group consisting of hydrogen, $C_{1-4}$alkyl, hydroxy substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl- and heterocycloalkyl-$C_{1-4}$alkyl-; wherein the cycloalkyl, aryl, heteroaryl or heterocycloalkyl, whether alone or as part of a substituent group is optionally substituted with one to two substituents independently selected from halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino or di($C_{1-4}$alkyl)amino.

In an embodiment of the present invention, $R^{10}$ is selected from the group consisting of hydroxy, halogen, $C_{1-4}$alkyl, $C_{1-4}$alkoxy, halogen substituted $C_{1-4}$alkyl and halogen substituted $C_{1-4}$alkoxy; provided that the halogens on

the halogen substituted $C_{1-4}$alkyl or the halogen substituted $C_{1-4}$alkoxy are selected from the group consisting of chloro and fluoro.

In another embodiment of the present invention are compounds of formula (II) whose Ki, as measured according to the procedure described In Example 159, is less than or equal to about 1μM, preferably, less than or equal to about 250 nM, more preferably, less than or equal to about 100nM, more preferably still, less than or equal to about 50nM.

In another embodiment of the present invention are compounds of formula (II), whose $IC_{50}$, as measured according to the procedure described in Example 158, is less than or equal to about 1μM, preferably, less than or equal to about 250nM, more preferably, less than or equal to about 50nM.

Additional embodiments of the present invention, include those wherein the substituents selected for one or more of the variables defined herein (e. g. $R^0$, $R^1$, $R^3$, $R^{10}$, $L^1$, a, b, c, $A^2$, $Q^3$,

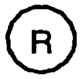,

etc.) are independently selected to be any individual substituent or any subset of substituents selected from the complete list as defined herein.

Representative compounds of the present invention are as listed in Tables 1 through 13, below. Unless otherwise noted, all compounds were prepared as mixtures of stereo-isomers. Representative compounds of formula (II) are as listed in Tables 9-13 below.

For substituent groups bound through two points within the structures in the Tables below, for example $A^2$, $Q^3$, $L^1$, etc., the substituent group is Identified as it would be incorporated into the structure heading the table. Further, unless otherwise noted, any terminal substituent group is bound at the 1-position. Thus for example, 4-fluorophenyl corresponds to a phenyl group bound at the 1-position and substituted with a fluoro group at the 4-position, and could alternatively be defined as 1-(4-fluorophenyl). Further, for compounds of formula (II) listed in Tables 9-13, unless otherwise noted, the

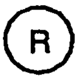

substituent shall be assumed to be bound at the 1-position, unless otherwise noted, with the $Q^3$ substituent bound as indicated by the numbering listed in the $Q^3$ substituent column.

Representative compounds of formula (II) are as listed in Tables 9-13, below.

**Table 9: Compounds of Formula (II)**

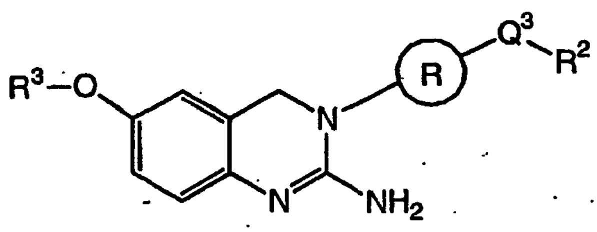

| ID No. | 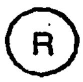 | $Q^3$ | $R^2$ | $R^3$ |
|---|---|---|---|---|
| 72 | -(S)-cyclopentyl- | -2-(R)-C(O)-N(CH$_3$) | cyclohexyl | phenyl |
| 77 | -(S)-cyclopentyl- | -3-(R)-C(O)-N(CH$_3$)- | cyclohexyl | phenyl |
| 81 | -(R)-cyclohexyl- | -2-(S)-C(O)-N(CH$_3$)- | cyclohexyl | phenyl |
| 87 | -(S)-cyclohexyl- | -2-(R)-C(O)-N(CH$_3$)- | cyclohexyl | phenyl |
| 93 | -3-pyrrolidinyl- | -1-C(O)O- | t-butyl | phenyl |
| 94 | -phenyl- | -3-C(O)-N(CH$_3$)- | cyclohexyl | phenyl |

(continued)

| ID No. | 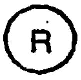 | $Q^3$ | $R^2$ | $R^3$ |
|---|---|---|---|---|
| 118 | -9-fluorenyl- | $-2-C(O)-N(CH_3)-$ | cyclohexyl | phenyl |
| 119 | -3-pyrrolidinyl- | -1-C(O)- | cyclohexyl | phenyl |
| 120 | -3-pyrrolidinyl- | -1-C(O)- | cyclopentyl | phenyl |
| 124 | -9-fluorenyl- | -1-C(O)- N(cyclohexyl)- | cyclohexyl | phenyl |
| 125 | -9-fluorenyl- | $-4-C(O)-N(CH_3)-$ | cyclohexyl | phenyl |
| 145 | -1-indanyl- | $-6-C(O)-N(CH_3)-$ | cyclohexyl | phenyl |
| 146 | -1-(5-methoxy-indanyl)- | $-3-CH_2-C(O)-N(CH_3)-$ | cyclohexyl | phenyl |
| 147 | -9-fluorenyl- | -4-C(O)-N(cyclohexyl)- | cyclohexyl | phenyl |
| 353 | -4-piperidinyl- | -1-C(O)O | ethyl | (3-(2-methoxy-4-methyl-phenyl)-sulfonyl-amino)-phenyl |
| 362 | -4-piperidinyl- | -1-C(O)O- | ethyl | 3-(2,4,6-trimethyl-phenyl-sulfony(amino)-phenyl |
| 376 | -4-piperidinyl- | -1-C(O)O- | ethyl | 3-(phenyl-sulfonyl-amino-phenyl) |
| 499 | -4-piperidinyl- | -1-C(O)O- | t-butyl | phenyl |
| 510 | -3-azepinyl- | -1-C(O)O- | t-butyl | phenyl |
| 522 | -4-piperidinyl- | -1-C(O)- | cyclohexyl | phenyl |
| 523 | -4-piperidinyl- | -1-C(O)- | 4-n-heptyl | phenyl |
| 524 | -4-piperidinyl- | -1-C(O)- | 1-adamantyl | phenyl |
| 550 | -3-azepinyl- | -1-C(O)- | 1-adamantyl | phenyl |
| 551 | -3-azepinyl- | -1-C(O)- | cyclohexyl | phenyl |
| 552 | -3-azepinyl- | -1-C(O)- | 4-n-heptyl | phenyl |
| 611 | -3-piperidinyl- | -1-C(O)- | cyclohexyl | phenyl |
| 612 | -3-piperidinyl- | -1-C(O)- | 4-n-heptyl | phenyl |
| 613 | -3-piperidinyl- | -1-C(O)- | isopentyl | phenyl |

**Table 10: Compounds of Formula (II)**

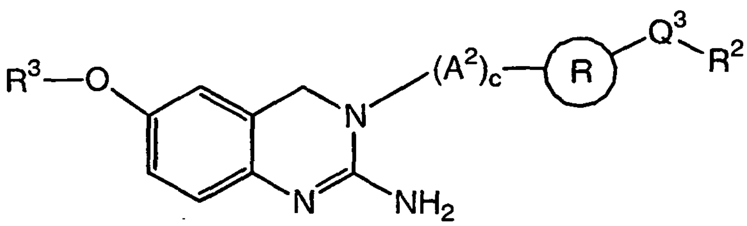

| ID No | $(A^2)_c$ | 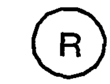 | $Q^3$ | $R^2$ | $R^3$ |
|---|---|---|---|---|---|
| 62 | -methyl- | -phenyl- | $-3-C(O)-N(CH_3)-$ | cyclohexyl | phenyl |

(continued)

| ID No | $(A^2)_c$ | R | $Q^3$ | $R^2$ | $R^3$ |
|---|---|---|---|---|---|
| 89 | -methyl- | -4-pyridyl- | -2-C(O)-N $(CH_3)$- | cyclohexyl | phenyl |
| 97 | -methyl- | -phenyl- | -4-C(O)-N $(CH_3)$- | cyclohexyl | phenyl |
| 98 | -methyl- | -phenyl- | -3-C(O)-N (cyclohexyl)- | cyclohexyl | phenyl |
| 100 | -methyl- | -trans-cyclohexyl- | -4-C(O)-N $(CH_3)$- | cyclohexyl- | phenyl |
| 110 | -methyl- | -phenyl- | -3-C(O)-N $(CH_3)$- | cyclohexyl | 3-(phenyl-sulfonyl-amino)-phenyl, |
| 132 | -CH(phenyl)- | -phenyl- | -3-C(O)-N (cyclohexyl)- | cyclohexyl- | phenyl |
| 136 | -CH(phenyl)- | -phenyl- | -3-C(O)-N $(CH_3)$- | cyclohexyl | phenyl |
| 187 | -methyl- | -phenyl- | -3-C(O)-N(4-methyl-cyclohexyl)- | 4-methyl-cyclohexyl | phenyl |
| 188 | -methyl- | -phenyl- | -3-C(O)-NH- | 4-(amino-sulfonyl)-phenyl-ethyl- | phenyl |
| 192 | -methyl- | -phenyl- | -3-C(O)-N (isopropyl)- | cyclohexyl | phenyl |
| 193 | -methyl- | -phenyl- | -3-C(O)- | 2-deca- hydro-iso-quinolinyl | phenyl |
| 197 | -methyl- | -phenyl- | -3-C(O)-N(n-propyl)- | cyclopropyl- methyl- | phenyl |
| 198 | -methyl- | -phenyl- | -3-C(O)-N(2-ethyl-n-hexyl)- | 2-ethyl-n-hexyl | phenyl |
| 204 | -1-ethyl-methyl- | -phenyl- | -3-C(O)-N $(CH_3)$- | cyclohexyl | phenyl |
| 209 | -methyl- | -phenyl- | -3-C(O)-N $(CH_3)$- | cyclohexyl | 3-methyl-phenyl |
| 211 | -methyl- | -phenyl- | -3-C(O)-N (isobutyl)- | isobutyl | phenyl |
| 212 | -methyl- | -phenyl- | -3-C(O)- | 1-deca-hydro-quinolinyl | phenyl |
| 213 | -methyl- | -phenyl- | -3-C(O)-NH- | 4-methyl-cyclohexyl | phenyl |
| 222 | -methyl- | -phenyl- | -3-C(O)-NH- | 2-(1-methyl-pyrrolidinyl)-ethyl- | phenyl |
| 223 | -methyl- | -phenyl- | -3-C(O)-N $(CH_3)$- | 4-(1-methyl-piperidinyl) | phenyl |
| 224 | -methyl- | -phenyl- | -3-NH-C(O)O- | t-butyl | phenyl |
| 228 | -methyl- | -phenyl- | -3-C(O)-N $(CH_3)$- | cyclohexyl | 3,5-dimethyl-phenyl |

(continued)

| ID No | $(A^2)_c$ | 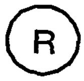 | $Q^3$ | $R^2$ | $R^3$ |
|---|---|---|---|---|---|
| 229 | -methyl- | -phenyl- | -3-C(O)-N $(CH_3)$- | cyclohexyl | 3-(methyl-carbonyl-amino)-phenyl |
| 230 | -methyl- | -phenyl- | -3-C(O)-N $(CH_3)$- | cyclohexyl | 3-pyridyl |
| 231 | -methyl- | -phenyl- | -3-C(O)-N $(CH_3)$- | cyclohexyl | 3-biphenyl |
| 232 | -methyl- | -phenyl- | -3-C(O)-N $(CH_3)$- | cyclohexyl | 2-methoxy-phenyl |
| 233 | -methyl- | -phenyl- | -3-C(O)-N $(CH_3)$- | cyclohexyl | 2-(5,6,7,8-tetrahydro-naphthyl) |
| 234 | -methyl- | -phenyl- | -3-C(O)-N $(CH_3)$- | cyclohexyl | 2-biphenyl |
| 235 | -methyl- | -phenyl- | -3-C(O)-N $(CH_3)$- | cyclohexyl | 2-(amino-carbonyl-methyl)-phenyl |
| 236 | -methyl- | -phenyl- | -3-C(O)-N $(CH_3)$- | cyclohexyl | 3-(phenyloxy)-phenyl |
| 286 | -methyl | -phenyl- | -3-NH-C(O)- | cyclohexyl | phenyl |
| 290 | -methyl- | -phenyl- | -3-NH-C(O)- | t-butyl | phenyl |
| 292 | -methyl- | -phenyl- | -3-C(O)-N $(CH_3)$- | cyclohexyl | 3-methoxy-phenyl |
| 293 | -methyl- | -phenyl- | -3-C(O)-N $(CH_3)$- | cyclohexyl | 2-methyl-phenyl |
| 306 | -methyl- | -phenyl-3-$CH_2$- | -NH-C(O)- | cyclohexyl | phenyl |
| 307 | -methyl- | -phenyl-3-$CH_2$- | -NH-C(O)- | trifluoro-methyl | phenyl |
| 310 | -CH(cyclo-hexyl-methyl)- | -phenyl- | -C(O)-N$(CH_3)$- | cyclohexyl | phenyl |
| 315 | -methyl- | -phenyl-3-$CH_2$- | -NH-C(O)O- | t-butyl | phenyl |
| 316 | -1-phenyl-methyl- | -phenyl- | -3-C(O)-N $(CH_3)$- | 4-(1-methyl-piperidinyl) | phenyl |
| 336 | -methyl- | -phenyl-3-$CH_2$- | -NH-C(O)- | 1-adamantyl | phenyl |
| 337 | -methyl- | -phenyl-3-$CH_2$- | -NH-C(O)- | benzhydryl | phenyl |
| 338 | -methyl- | -phenyl-3-$CH_2$- | -NH-C(O)- | 4-n-heptyl | phenyl |
| 339 | -methyl- | -phenyl-3-$CH_2$- | -NH-C(O)- | 3-n-heptyl | phenyl |
| 384 | -methyl- | -4-piperidinyl- | -1-C(O)O- | t-butyl | 3-(phenyl-sulfonyl-amino)-phenyl |

(continued)

| ID No | $(A^2)_c$ | R | $Q^3$ | $R^2$ | $R^3$ |
|---|---|---|---|---|---|
| 396 | -methyl- | -phenyl- | -3-C(O)O- | methyl | 3-(phenyl-sulfonyl-amino)-phenyl |
| 419 | -1-ethyl-methyl- | -phenyl- | -3-C(O)-N (cyclohexyl)- | cyclohexyl | phenyl |
| 424 | -methyl- | -2-furyl- | -5-C(O)-N $(CH_3)$- | cyclohexyl | phenyl |
| 443 | -methyl- | -phenyl- | -3-C(O)- | 1-piperidinyl | phenyl |
| 451 | -methyl- | -phenyl- | -3-C(O)-NH- | cyclohexyl | phenyl |
| 463 | -methyl- | -phenyl- | -2-C(O)-N $(CH_3)$- | cyclohexyl | phenyl |
| 467 | -methyl- | -phenyl- | -3-C(O)-N $(CH_3)$- | cyclohexyl | 2-methoxy-5-methyl-phenyl |
| 468 | -methyl- | -phenyl- | -3-C(O)-N $(CH_3)$- | cyclohexyl | 2-methoxy-6-methyl-phenyl |
| 469 | -methyl- | -phenyl- | -3-C(O)-N $(CH_3)$- | cyclohexyl | 2-(2-benz-thiazolyl)-5-methoxy-phenyl |
| 470 | -methyl- | -phenyl- | -3-C(O)-N $(CH_3)$- | cyclohexyl | 2-(2-benzo-thiazolyl)-phenyl |
| 471 | -methyl- | -phenyl- | -3-C(O)-N $(CH_3)$- | cyclohexyl | 2-(1-pyrrolyl)-phenyl |
| 472 | -methyl- | -phenyl- | -3-C(O)-N $(CH_3)$- | cyclohexyl | 2-(methyl-sulfonyl)-phenyl |
| 473 | -methyl- | -phenyl- | -3-C(O)-N $(CH_3)$- | cyclohexyl | 3-(2-quinolinyl)-phenyl |
| 474 | -methyl- | -phenyl- | -3-C(O)-N $(CH_3)$- | cyclohexyl | 2-methylthio-phenyl |
| 475 | -methyl- | -phenyl- | -3-C(O)-N $(CH_3)$- | cyclohexyl | 2,6-dimethyl-phenyl |
| 477 | -methyl- | -phenyl- | -3-C(O)-N $(CH_3)$- | cyclohexyl | 2,6-dimethoxy-phenyl |
| 478 | -methyl- | -phenyl- | -3-C(O)-N $(CH_3)$- | cyclohexyl | 2-isopropoxy-phenyl |
| 479 | -methyl- | -phenyl- | -3-C(O)-N $(CH_3)$- | cyclohexyl | 3-(dimethyl-amino)-phenyl |
| 480 | -methyl- | -phenyl- | -3-C(O)-N $(CH_3)$- | cyclohexyl | 2-allyl-6-methyl-phenyl |
| 481 | -methyl- | -phenyl- | -3-C(O)-N $(CH_3)$- | cyclohexyl | 2-allyl-6-methoxy-phenyl |
| 482 | -methyl- | -phenyl- | -3-C(O)-N $(CH_3)$- | cyclohexyl | 2-methyl-6-n-propyl-phenyl |
| 483 | -methyl- | -phenyl- | -3-C(O)-N $(CH_3)$- | cyclohexyl | 2-(1-pyrrolidinyl-carbonyl)-phenyl |

(continued)

| ID No | $(A^2)_c$ | 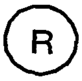 | $Q^3$ | $R^2$ | $R^3$ |
|---|---|---|---|---|---|
| 503 | -methyl- | -2-thienyl- | -5-C(O)-N $(CH_3)$- | cyclohexyl | phenyl |
| 508 | -methyl- | -2-pyridyl- | -4-C(O)-N $(CH_3)$- | cyclohexyl | phenyl |
| 509 | -methyl- | -4-piperidinyl- | -1-C(O)O- | t-butyl | phenyl |
| 512 | -methyl- | -phenyl- | -3-C(O)-N $(CH_3)$- | cyclohexyl | 2-cyclopentyl-phenyl |
| 513 | -methyl- | -phenyl- | -3-C(O)-N $(CH_3)$- | cyclohexyl | 2-biphenyl-methyl- |
| 514 | -methyl- | -phenyl- | -3-C(O)-N $(CH_3)$- | cyclohexyl | 3-methoxy-benzyl |
| 516 | -methyl- | -phenyl- | -3-C(O)-N $(CH_3)$- | cyclohexyl | n-pentyl |
| 517 | -methyl- | -phenyl- | -3-C(O)-N $(CH_3)$- | cyclohexyl | isopentyl |
| 518 | -methyl- | -phenyl- | -3-C(O)-N $(CH_3)$- | cyclohexyl | isobutyl |
| 519 | -methyl- | -phenyl- | -3-C(O)-N $(CH_3)$- | cyclohexyl | isopropyl |
| 520 | -methyl- | -phenyl- | -3-C(O)-N $(CH_3)$- | cyclohexyl | cyclopentyl |
| 521 | -methyl- | -phenyl- | -3-C(O)-N $(CH_3)$- | cyclohexyl | cyclopentyl-methyl- |
| 525 | -methyl- | -4-piperidinyl- | -1-C(O)- | cyclohexyl | phenyl |
| 526 | -methyl- | -4-piperidinyl- | -1-C(O)- | 4-n-heptyl | phenyl |
| 527 | -methyl- | -4-piperidinyl- | -1-C(O)- | 1-adamantyl | phenyl |
| 534 | -1-cyclohexyl-methyl- | -phenyl- | -3-C(O)-N $(CH_3)$- | cyclohexyl | phenyl |
| 559 | -1-benzyl-methyl- | -5-oxazolyl- | -4-C(O)-N $(CH_3)$- | cyclohexyl | phenyl |
| 606 | -1-benzyl-methyl- | -5-oxazolyl- | -4-C(O)-NH- | cyclohexyl | phenyl |
| 640 | -methyl- | -5-oxazolyl- | -4-C(O)-NH- | cyclohexyl | phenyl |
| 641 | -methyl- | -5-oxazolyl- | -4-C(O)-N $(CH_3)$- | cyclohexyl | phenyl |

**Table 11: Compounds of formula (II)**

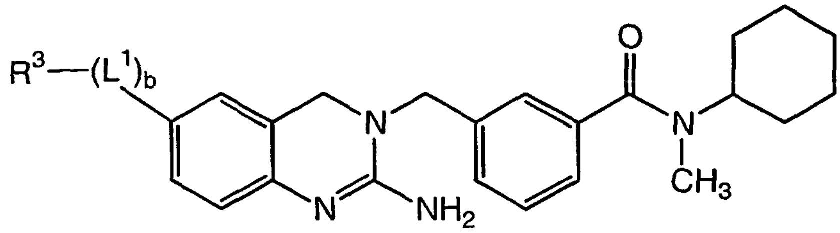

| ID No | $(L^1)_b$ | $R^3$ |
|---|---|---|
| 266 | -CH=CH- | phenyl |
| 332 | $-CH(CH_3)-$ | phenyl |
| 404 | -S- | 2-methyl-phenyl |
| 405 | -S- | 3-methyl-phenyl |
| 406 | -S- | 2-ethyl-phenyl |
| 407 | -S- | 4-isopropyl-phenyl |
| 408 | -S- | 3,4-dimethyl-phenyl |
| 409 | -S- | 3,5-dimethyl-phenyl |
| 410 | -S- | 4-methoxy-phenyl |
| 411 | -S- | 3,4-dimethoxy-phenyl |
| 412 | -S- | 2-methoxy-phenyl |
| 413 | -S- | 4-methylthio-phenyl |
| 414 | -S- | 1-naphthyl |
| 415 | -S- | 2-naphthyl |
| 416 | -S- | 2-(methylaminocarbonyl)-phenyl |
| 417 | -S- | 4-(methylcarbonylamino)-phenyl |
| 432 | -NH- | phenyl |
| 433 | -NH- | 3-methoxy-phenyl |
| 434 | -NH- | 4-methoxy-phenyl |
| 435 | -NH- | 3-methyl-phenyl |
| 436 | -NH- | 2-naphthyl |
| 437 | -NH- | 4-cyclohexyl-phenyl |
| 438 | -NH- | 4-(dimethylamino)-phenyl |
| 439 | -NH- | 4-(4-morpholinyl)-phenyl |
| 440 | -NH- | 3-ethoxy-phenyl |
| 441 | -NH- | 3,4-methylenedioxy-phenyl |
| 442 | -NH- | 4-methylthio-phenyl |
| 476 | -S- | phenyl |
| 549 | -SO- | phenyl |
| 735 | $-SO_2-$ | phenyl |

**Table 12: Compounds of formula (II)**

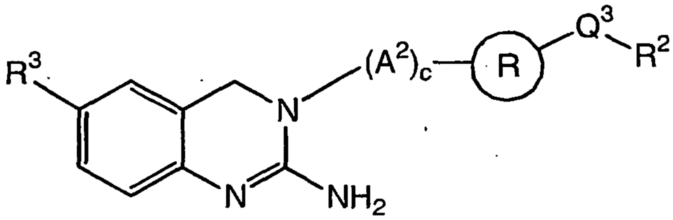

| ID No | $(A^2)_c$ | 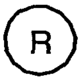 | $Q^3$ | $R^2$ | $R^3$ |
|---|---|---|---|---|---|
| 138 | -methyl- | -phenyl- | $-3-C(O)-N(CH_3)-$ | cyclohexyl | phenyl |
| 158 | -methyl- | -phenyl- | $-3-C(O)-N(CH_3)-$ | cyclohexyl | 2-(t-butyl-amino-sulfonyl)-phenyl |
| 159 | -methyl- | -phenyl- | $-3-C(O)-N(CH_3)-$ | cyclohexyl | 4-(3,5-dimethyl-isoxazolyl) |
| 160 | -methyl- | -phenyl- | $-3-C(O)-N(CH_3)-$ | cyclohexyl | 2-(t-butyl-carbonyl-amino)-5-methoxy-phenyl |
| 161 | -methyl- | -phenyl- | $-3-C(O)-N(CH_3)-$ | cyclohexyl | 4-pyrazolyl |
| 162 | -methyl- | -phenyl- | $-3-C(O)-N(CH_3)-$ | cyclohexyl | 4-(amino-carbonyl)-phenyl |
| 163 | -methyl- | -phenyl- | $-3-C(O)-N(CH_3)-$ | cyclohexyl | 3-carboxy-phenyl |
| 164 | -methyl- | -phenyl- | $-3-C(O)-N(CH_3)-$ | cyclohexyl | 3-thienyl |
| 165 | -methyl- | -phenyl- | $-3-C(O)-N(CH_3)-$ | cyclohexyl | 3-methoxy-phenyl |
| 166 | -methyl- | -phenyl- | $-3-C(O)-N(CH_3)-$ | cyclohexyl | 3-(methyl-carbonyl-amino)-phenyl |
| 167 | -methyl- | -phenyl- | $-3-C(O)-N(CH_3)-$ | cyclohexyl | 2-methoxy-phenyl |
| 168 | -methyl- | -phenyl- | $-3-C(O)-N(CH_3)-$ | cyclohexyl | 4-pyridyl |
| 169 | -methyl- | -phenyl- | $-3-C(O)-N(CH_3)-$ | cyclohexyl | 2-hydroxy-phenyl |
| 170 | -methyl- | -phenyl- | $-3-C(O)-N(CH_3)-$ | cyclohexyl | 3-pyridyl |
| 171 | -methyl- | -phenyl- | $-3-C(O)-N(CH_3)-$ | cyclohexyl | 3-biphenyl |
| 172 | -methyl- | -phenyl- | $-3-C(O)-N(CH_3)-$ | cyclohexyl | 2-(dimethylamino-methyl)-phenyl |
| 173 | -methyl- | -phenyl- | $-3-C(O)-N(CH_3)-$ | cyclohexyl | 2-(amino-carbonyl)-phenyl |
| 174 | -methyl- | -phenyl- | $-3-C(O)-N(CH_3)-$ | cyclohexyl | 2-hydroxymethyl-phenyl |
| 175 | -methyl- | -phenyl- | $-3-C(O)-N(CH_3)-$ | cyclohexyl | 5-indolyl |
| 176 | -methyl- | -phenyl- | $-3-C(O)-N(CH_3)-$ | cyclohexyl | 3-methyl-phenyl |
| 259 | -methyl- | -phenyl- | $-3-C(O)-N(CH_3)-$ | cyclohexyl | 2,6-dichloro-phenyl |
| 260 | -methyl- | -phenyl- | $-3-C(O)-N(CH_3)-$ | cyclohexyl | 2-chloro-phenyl |
| 261 | -methyl- | -phenyl- | $-3-C(O)-N(CH_3)-$ | cyclohexyl | 2-methyl-phenyl |
| 262 | -methyl- | -phenyl- | $-3-C(O)-N(CH_3)-$ | cyclohexyl | 2,6-dimethyl-phenyl |
| 263 | -methyl- | -phenyl- | $-3-C(O)-N(CH_3)-$ | cyclohexyl | 2-cyano-phenyl |
| 264 | -methyl- | -phenyl- | $-3-C(O)-N(CH_3)-$ | cyclohexyl | 2,6-dimethoxy-phenyl |
| 265 | -methyl- | -phenyl- | $-3-C(O)-N(CH_3)-$ | cyclohexyl | 3-benzothienyl |
| 267 | -methyl- | -phenyl- | $-3-C(O)-N(CH_3)-$ | cyclohexyl | 4-cyclohexyl-phenyl |

(continued)

| ID No | $(A^2)_c$ | (R) | $Q^3$ | $R^2$ | $R^3$ |
|---|---|---|---|---|---|
| 268 | -methyl- | -phenyl- | -3-C(O)-N($CH_3$)- | cyclohexyl | 2-trifluoro-methyl-phenyl |
| 320 | -methyl- | -phenyl- | -3-C(O)-N($CH_3$)- | cyclohexyl | 2-ethyl-phenyl- |
| 321 | -methyl- | -phenyl- | -3-C(O)-N($CH_3$)- | cyclohexyl | 2-biphenyl |
| 322 | -methyl- | -phenyl- | -3-C(O)-N($CH_3$)- | cyclohexyl | 2-methylthio-phenyl |
| 323 | -methyl- | -phenyl- | -3-C(O)-N($CH_3$)- | cyclohexyl | 2-(methyl-carbonyl)-phenyl |
| 324 | -methyl- | -phenyl- | -3-C(O)-N($CH_3$)- | cyclohexyl | 2-trifluoro-methoxy-phenyl |
| 325 | -methyl- | -phenyl- | -3-C(O)-N($CH_3$)- | cyclohexyl | 2-cyanomethyl-phenyl |
| 326 | -methyl- | -phenyl- | -3-C(O)-N($CH_3$)- | cyclohexyl | 2-(phenyloxy)-phenyl |
| 327 | -methyl- | -phenyl- | -3-C(O)-N($CH_3$)- | cyclohexyl | 2-benzyloxy-phenyl |
| 328 | -methyl- | -phenyl- | -3-C(O)-N($CH_3$)- | cyclohexyl | 2-(methyl-carbonyl-amino)-phenyl |
| 329 | -methyl- | -phenyl- | -3-C(O)-N($CH_3$)- | cyclohexyl | 2-ethoxy-phenyl |
| 330 | -methyl- | -phenyl- | -3-C(O)-N($CH_3$)- | cyclohexyl | 1-naphthyl |
| 331 | -methyl- | -phenyl- | -3-C(O)-N($CH_3$)- | cyclohexyl | 2-bromo-phenyl |
| 333 | -methyl- | -phenyl- | -3-C(O)-N($CH_3$)- | cyclohexyl | 2-nitro-phenyl |
| 334 | -methyl- | -phenyl- | -3-C(O)-N($CH_3$)- | cyclohexyl | 2-fluoro-phenyl |
| 395 | -1-ethyl-methyl- | -phenyl- | -3-C(O)-N($CH_3$)- | cyclohexyl | 2-methoxy-phenyl |
| 396 | -methyl- | -phenyl- | -3-C(O)O- | methyl | 3-(phenyl-sulfonyl-amino)-phenyl |
| 418 | -1-ethyl-methyl- | -phenyl- | -3-C(O)-N(cyclo-hexyl)- | cyclohexyl | 2-methoxy-phenyl |

**Table 13: Compounds of Formula (II)**

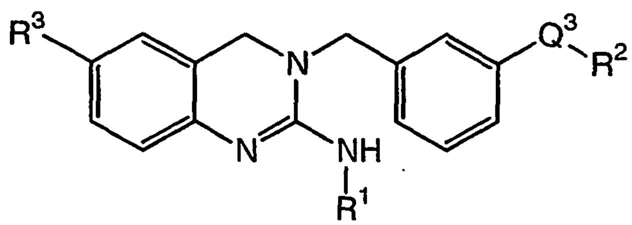

| ID No | $R^1$ | $Q^3$ | $R^2$ | $R^3$ |
|---|---|---|---|---|
| 605 | methoxy | -3-C(O)-N($CH_3$)- | cyclohexyl | 2-methoxy-phenyl |
| 607 | methyl-carbonyl | -3-C(O)-N($CH_3$)- | cyclohexyl | 2-methoxy-phenyl |

Table 14 below lists representative intermediates and by-products in the preparation of the compounds formula (II) of the present invention.

**Table 14: Intermediates or By-Products**

| | |
|---|---|
| 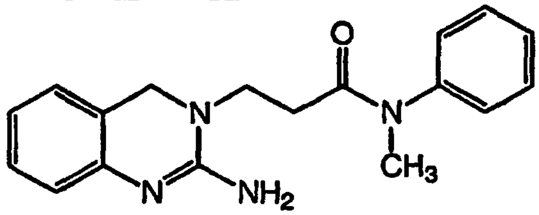 Cmpd #800 | 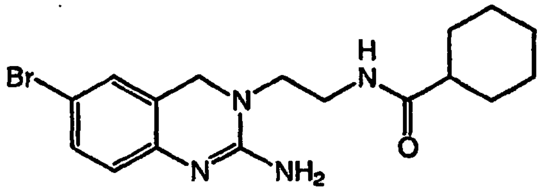 Cmpd #806 |
| 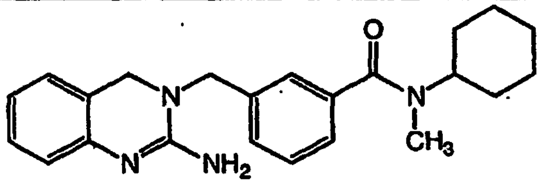 Cmpd #801 | 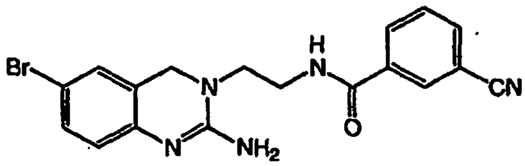 Cmpd #807 |
| 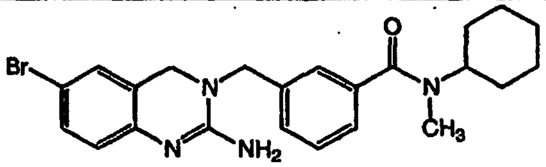 Cmpd #802 | 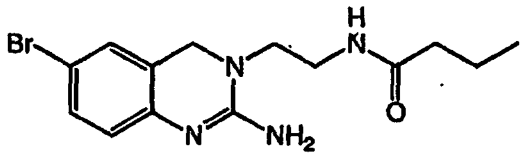 Cmpd #808 |
| 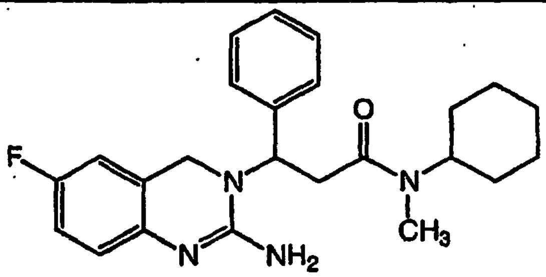 Cmpd #803 | 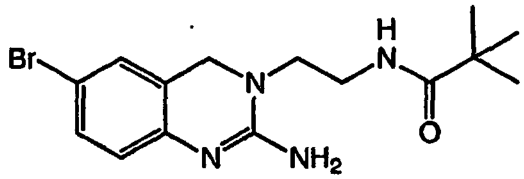 Cmpd #809 |
| 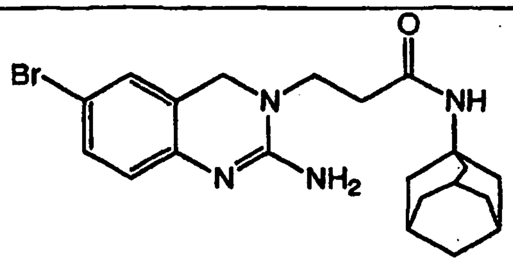 Cmpd #804 | 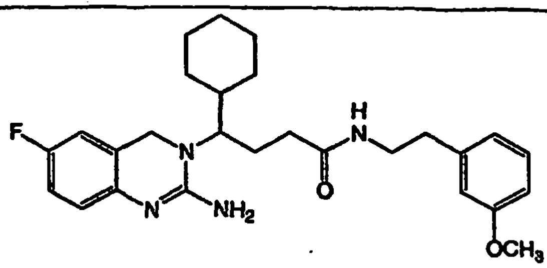 Cmpd #810 |
| 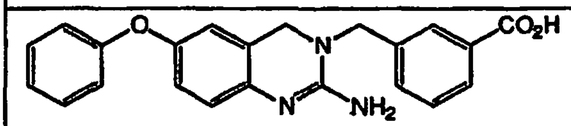 Cmpd #805 | 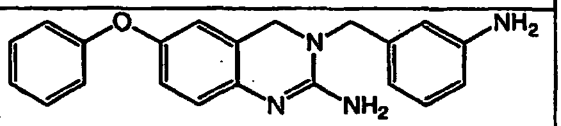 Cmpd #811 |

The present invention is further directed to compounds of formula (CI), compounds of formula (CII) and com-

pounds of formula (CIII)

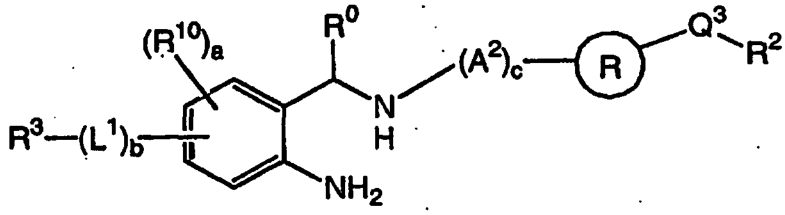

(CII)

wherein $R^0$, $R^1$, $R^2$, $R^3$, $R^{10}$, $L^1$, a, b, c, $A^2$, $Q^3$ and

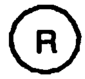

are as herein defined. The compounds of formula (CII) are useful as intermediates in the preparation of the compounds of formula (II) of the present invention.

As used herein, unless otherwise noted, the term "halogen" shall mean chlorine, bromine, fluorine and iodine.

As used herein, unless otherwise noted, the term "**alkyl**" whether used alone or as part of a substituent group, includes straight and branched chains. For example, alkyl radicals include methyl, ethyl, n-propyl, isopropyl, n-butyl, isobutyl, sec-butyl, t-butyl, n-pentyl, isopentyl, hexyl, heptyl, octyl, nonyl, decyl, and the like. Similarly, the term "$C_{1-8}$alkyl" shall include straight and branched chains comprising one to eight carbon atoms. The term "**alkyl**" may also encompass multivalent radicals where indicated in the specification and claims (e.g. $A^2$ is described as as an $C_{1-4}$alkyl group, as shown in the formulas and would be understood by those of ordinary skill the art to be divalent linking groups that may be further substituted, for example $-CH_2-$, $-CH_2CH_2-$, $-CH_2-CH(CH_3)-$, and the like).

As used herein, unless otherwise noted, "**alkoxy**" shall denote an oxygen ether radical of the above described straight or branched chain alkyl groups. For example, methoxy, ethoxy, n-propoxy, sec-butoxy, t-butoxy, n-hexyloxy and the like.

As used herein, unless otherwise noted, the terms "**halogen substituted $C_{1-4}$alkyl**" and "**halogenated $C_{1-4}$alkyl**", shall mean a straight or branched chain alkyl group comprising one to four carbon atoms, wherein the alkyl is substituted with one or more, preferably one to five, more preferably one to three halogen atoms. Preferably, the halogen is selected from the group consisting of chloro and fluoro.

Similarly, the terms "**halogen substituted $C_{1-4}$alkoxy**" and "**halogenated $C_{1-4}$alkoxy**" shall mean a straight or branched chain alkoxy group comprising one to four carbon atoms, wherein the alkoxy is substituted with one or more, preferably one to five, more preferably one to three halogen atoms. Preferably, the halogen is selected from the group consisting of chloro and fluoro.

As used herein, unless otherwise noted, the term "**hydroxy substituted $C_{1-4}$alkyl**" shall mean a straight or branched chain $C_{1-4}$alkyl, wherein the $C_{1-4}$alkyl is substituted with one or more, preferably one to three hydroxy groups, more preferably one to two hydroxy groups. Most preferably, the $C_{1-4}$alkyl group is substituted with one hydroxy group. Preferably, wherein the $C_{1-4}$alkyl group has a terminal carbon atom, the hydroxy group is bound at said terminal carbon atom.

As used herein, unless otherwise noted, the term "**carboxy substituted $C_{1-4}$alkyl**" shall mean a straight or branched chain $C_{1-4}$alkyl, wherein the $C_{1-4}$alkyl is substituted with one or more, preferably one to three carboxy groups, more preferably one to two carboxy groups. Most preferably, the $C_{1-4}$alkyl group is substituted with one carboxy group. Preferably, wherein the $C_{1-4}$alkyl group has a terminal carbon atom, the carboxy group is bound at said terminal carbon atom.

As used herein, unless otherwise noted, "**aryl**" shall refer to fully conjugated aromatic ring structures such as phenyl, naphthyl, and the like.

As used herein, unless otherwise noted, "**$C_{1-4}$aralkyl**" shall mean any $C_{1-4}$alkyl group substituted with an aryl group such as phenyl, naphthyl and the like. For example, benzyl, phenylethyl, phenylpropyl, naphthylmethyl, and the like. Unless otherwise noted, the "**$C_{1-4}$aralkyl**" group is bound through the alkyl portion. For example, phenylethyl- is bound through the terminal carbon atom of the ethyl group (i.e. phenyl-$CH_2$-$CH_2$-).

As used herein, unless otherwise noted, the term "**cycloalkyl**" shall mean any stable monocyclic, bicyclic, polycyclic, bridged or spiro-bound, saturated ring system. Suitable examples include, but are not limited to, cyclopropyl, cyclobutyl, cyclopentyl, cyclohexyl, cycloheptyl, cyclooctyl, norboranyl, adamantyl, spiropentane, 2,2,2-bicyclooctyl, and the like. Unless otherwise noted, "**cycloalkyl**" groups do not contain N, O or S heteroatoms.

As used herein, unless otherwise noted, the term "**partially unsaturated carbocyclyl**" shall mean any stable monocyclic, bicyclic, polycyclic, bridge or spiro-bound ring system containing at least one carbon atom which is not part of an unsaturated bond (i.e. a double or triple bond) or any bicyclic, polycyclic, bridged or spiro-bound, partially aromatic (e.g. benzo-fused) rings system. Suitable examples include, but are not limited to 1,2,3,4-tetrahydro-naphthyl, fluorenyl, 9,10-dihydroanthracenyl, indanyl, and the like. Unless otherwise noted, "**partially unsaturated carbocyclyl**" groups do not contain N, O or S heteroatoms.

As used herein, unless otherwise noted, "**heteroaryl**" shall denote any five or six membered monocyclic aromatic ring structure containing at least one heteroatom selected from the group consisting of O, N and S, optionally containing one to three additional heteroatoms independently selected from the group consisting of O, N and S; or a nine or ten membered bicyclic aromatic ring structure containing at least one heteroatom selected from the group consisting of O, N and S, optionally containing one to four additional heteroatoms independently selected from the group consisting of O, N and S. The heteroaryl group may be attached at any heteroatom or carbon atom of the ring such that the result is a stable structure.

Examples of suitable heteroaryl groups include, but are not limited to, pyrrolyl, furyl, thienyl, oxazolyl, imidazolyl, pyrazolyl, isoxazolyl, isothiazolyl, triazolyl, thiadiazolyl, pyridyl, pyridazinyl, pyrimidinyl, pyrazinyl, pyranyl, furazanyl, indolizinyl, indolyl, isoindolinyl, indazolyl, benzofuryl, benzothienyl, benzimidazolyl, benzthiazolyl, purinyl, quinolizinyl, quinolinyl, isoquinolinyl, isothiazolyl, cinnolinyl, phthalazinyl, quinazolinyl, quinoxalinyl, naphthyridinyl, pteridinyl, tetrazolyl, triazolyl, and the like. Preferred heteroaryl groups include furyl, thienyl, imidazolyl, pyridyl, triazolyl, benzimidazolyl and tetrazolyl.

As used herein, the term "**heterocycloalkyl**" shall denote any five to seven membered monocyclic, saturated or partially unsaturated ring structure containing at least one heteroatom selected from the group consisting of O, N and S, optionally containing one to three additional heteroatoms independently selected from the group consisting of O, N and S; or a nine to ten membered saturated, partially unsaturated or partially aromatic (e.g. benzo-fused) bicyclic ring system containing at least one heteroatom selected from the group consisting of O, N and S, optionally containing one to four additional heteroatoms independently selected from the group consisting of O, N and S. The heterocycloalkyl group may be attached at any heteroatom or carbon atom of the ring such that the result is a stable structure.

Examples of suitable heterocycloalkyl groups include, but are not limited to, pyrrolinyl, pyrrolidinyl, dioxalanyl, imidazolinyl, imidazolidinyl, pyrazolinyl, pyrazolidinyl, piperidinyl, dioxanyl, morpholinyl, dithianyl, thiomorpholinyl, piperazinyl, trithianyl, indolinyl, chromenyl, 3,4-methylenedioxyphenyl, 2,3-dihydrobenzofuryl, tetrahydropyranyl, azepinyl, 2,3-dihydro-1,4-benzodioxanyl, and the like. Preferred heterocycloalkyl groups include piperidinyl, morpholinyl, tetrahydropyranyl (preferably tetrahydropyran-2-yl or tetrahydropyran-6-yl) and azepinyl.

As used herein, unless otherwise noted, the term "**spiro-heterocyclyl**" shall mean any spiro-bound ring structure wherein the spiro-bound ring structure contains at least one heteroartom selected from O, S or N. Suitable examples include, but are not limited to 1,4-dioxaspiro[4.5]decyl, 1-oxa-4-azaspiro[4.5]decyl, 1-thia-4azaspiro[4.5]decyl, 1,4-diazaspiro[4.5]decyl, 1,3-diazaspiro[4.5]dec-2-2nyl and 1-oxa-azaspiro[4.5]dec-2-enyl. Preferred spiro-heterocyclyl groups include

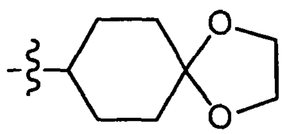

(i.e. 1,4-dioxaspiro[4.5]decyl).

As used herein, the notation "*" shall denote the presence of a stereogenic center.

When a particular group is "**substituted**" (e.g., cycloalkyl, aryl, heterocycloalkyl, heteroaryl, etc.), that group may have one or more substituents, preferably from one to five substituents, more preferably from one to three substituents, most preferably from one to two substituents, independently selected from the list of substituents.

With reference to substituents, the term "**independently**" means that when more than one of such substituents is possible, such substituents may be the same or different from each other.

To provide a more concise description, some of the quantitative expressions given herein are not qualified with the term "**about**". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

As used herein, unless otherwise noted, the term "**aprotic solvent**" shall mean any solvent that does not yield a proton. Suitable examples include, but are not limited to DMF, dioxane, THF, acetonitrile, pyridine, dichloroethane, dichloromethane, MTBE, toluene, and the like.

As used herein, unless otherwise noted, the term "**leaving group**" shall mean a charged or uncharged atom or group which departs during a substitution or displacement reaction. Suitable examples include, but are not limited to, Br, Cl, I, mesylate, tosylate, and the like.

As used herein, unless otherwise noted, the term "**nitrogen protecting group**" shall mean a group which may be attached to a nitrogen atom to protect said nitrogen atom from participating in a reaction and which may be readily removed following the reaction. Suitable nitrogen protecting groups include, but are not limited to carbamates - groups of the formula -C(O)O-R wherein R is for example methyl, ethyl, t-butyl, benzyl, phenylethyl, $CH_2$=CH-$CH_2$-, and the like; amides - groups of the formula -C(O)-R' wherein R' is for example methyl, phenyl, trifluoromethyl, and the like; N-sulfonyl derivatives - groups of the formula -$SI_2$-R" wherein R" is for example tolyl, phenyl, trifluoromethyl, 2,2,5,7,8-pentamethylchroman-6-yl-, 2,3,6-trimethyl-4-methoxybenzene, and the like. Other suitable nitrogen protecting groups may be found in texts such as T.W. Greene & P.G.M. Wuts, Protective Groups in Organic Synthesis, John Wiley & Sons, 1999.

As used herein, unless otherwise noted, the term "**oxygen protecting group**" shall mean a group which may be attached to an oxygen atom to protect said oxygen atom from participating in a reaction and which may be readily removed following the reaction. Suitable examples include, but are not limited to methyl, benzyl, trimethylsilyl, *tert*-butyldimethylsilyl, acetate, 1-ethoxyethyl, and the like. Other suitable nitrogen protecting groups may be found in texts such as T.W. Greene & P.G.M. Wuts, Protective Groups in Organic Synthesis, John Wiley & Sons, 1991.

Under standard nomenclature used throughout this disclosure, the terminal portion of the designated side chain is described first, followed by the adjacent functionality toward the point of attachment. Thus, for example, a "phenyl-($C_1$-$C_6$alkyl)-aminocarbonyl-($C_1$-$C_6$alkyl)-" substituent refers to a group of the formula

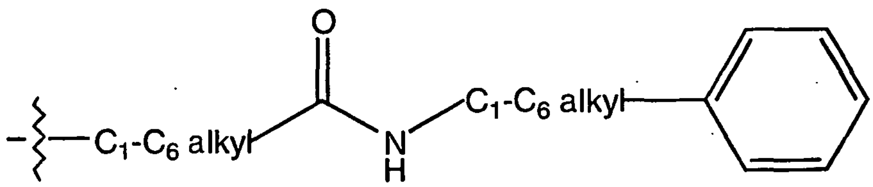

Unless otherwise noted, the position at which substituent groups on the compounds of formula (I), formula (II) and formula (III) are bound to the 2-amino-quinazoline core shall be denoted as follows:

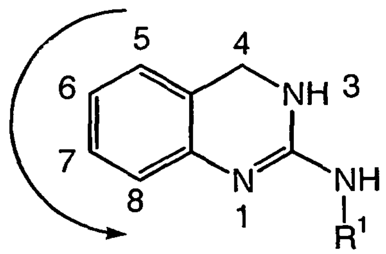

Abbreviations used in the specification, particularly the Schemes and Examples, are as follows:

| | |
|---|---|
| Ac | = Acetyl (i.e. -C(O)-$CH_3$) |
| ACN | = Acetonitrile |
| AD | = Alzheimer's Disease |
| AgOAc | = Silver Acetate |
| BACE | = β-secretase |
| $BH(OAc)_3$ | = Triacetoxy Borohydride |
| BOC or Boc | = *t*-Butoxycarbonyl |
| $(Boc)_2O$ | = Boc Anhydride |
| Cbz | = Carbobenzyloxy |
| DBU | = 1,8-iazabicyclo[5.4.0]undec-7-ene |
| DCC | = N,N'-Dicyclohexylcarbodiimide |
| DCE | = 1,2-Dichloroethane |
| DCM | = Dichloromethane |
| DEA | = diethylamine |
| DEAD | = Diethylazodicarboxylate |

| | |
|---|---|
| DIAD | = Diisopropylazodicarboxylate |
| DIPE | = Diisopropyl Ether |
| DIPCDI | = 1,3-Diisopropylcarbodiimide |
| DIPEA or DIEA | = Diisopropylethylamine |
| DMA | = N,N-Dimethylacetamide |
| DMAP | = 4-N,N-Dimethylaminopyridine |
| DME | = Dimethoxyethane |
| DMF | = N,N-Dimethylformamide |
| DMSO | = Dimethylsulfoxide |
| dppf | = 1,1'-Bis(diphenylphosphino)ferrocene |
| EDC | = 1-(3-Dimethylaminoproyl)-3-ethylcarbodiimide hydrochoride |
| EDCI | = 1-(3-Dimethylaminopropyl)3-ethylcarbodiimide hydrochloride |
| Et | = Ethyl ($-CH_2CH_3$) |
| $Et_3N$ | = Triethylamine |
| $Et_2O$ | = Diethyl Ether |
| EtOAc | = Ethyl acetate |
| EtOH | = Ethanol |
| HOAc | = Acetic acid |
| HATU | = O-(7-Azabenzotriazol-1-yl)-N,N,N",N"-Tetramethyl Uronium Hexafluorophosphate |
| HBTU | = *O*-Benzotriazol-1-yl-*N,N,N',N'*-tetramethyluronium hexafluorophosphate |
| HEPES | = 4-(2-Hydroxyethyl)-1-Piperizine Ethane Sulfonic Acid |
| HOBT or HOBt | = 1-Hydroxybenzotriazole |
| HPLC | = High Pressure Liquid Chromatography |
| LAH | = Lithium Aluminum Hydride |
| μwave | = Microwave |
| MCPBA | = 2-(4-Chloro-2-methylphenoxy)acetic acid |
| Me | = Methyl |
| MeCN | = Acetonitrile |
| MeOH | = Methanol |
| $MeONH_2$ HCl | = O-methylhydroxylamine hydrochloride |
| MTBE | = Methyl-*tert*-Butyl Ether |
| $Na(OAc)_3BH$ | = Sodium triacetoxyborohydride |
| $NH_4OAc$ | = Ammonium Acetate |
| NMR | = Nuclear Magnetic Resonance |
| OXONE® | = Potassium Monopersulfaphate Triple Salt |
| Pd-C or Pd/C | = Palladium on Carbon Catalyst |
| Pt-C or Pt/C | = Platinum on Carbon Catalyst |
| $Pd_2(OAc)_2$ | = Palladium(II)acetate |
| $Pd_2(dba)_3$ | = Tris(dibenzylidene acetone)dipalladium(0) |
| $Pd(dppf)Cl_2$ | = Dichloro[1,1'-bis(diphenylphosphino)ferrocene]palladium |
| $Pd(PPh_3)_4$ | = tetrakistriphenylphosphine palladium (0) |
| $Pd(PCy_3)_2Cl_2$ | = Dichlorobis(tricyclohexylphosphine)palladium |
| PTSA or *p*-TsOH | = *p*-Toluenesulfonic acid Monohydride |
| q. s. | = Quantum Sufficiat (Quantity Sufficient) |
| RP-HPLC | = Reverse Phase High Pressure Liquid Chromatography |
| RT or rt | = Room temperature |
| SPE | = Solid phase extraction |
| t-BOC or Boc | = Tert-Butoxycarbonyl |
| TDA-1 | = Tris(3,6-Dioxaheptyl)amine |
| TEA | = Triethylamine |
| TEMPO | = 2,2,6,6-Tetramethyl-1-piperidinyloxy Free Radical |
| TFA | = Trifluoroacetic Acid |
| THF | = Tetrahydrofuran |
| TLC | = Thin Layer Chromatography |

The term "**subject**" as used herein, refers to an animal, preferably a mammal, most preferably a human, who is or has been the object of treatment, observation or experiment.

The term "**therapeutically effective amount**" as used herein, means that amount of active compound or

pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "**composition**" is intended to encompass a product comprising the specified ingredients in the specified amounts, as well as any product which results, directly or indirectly, from combinations of the specified ingredients in the specified amounts.

Where the compounds according to this invention have at least one **chiral center**, they may accordingly exist as enantiomers. Where the compounds possess two or more chiral centers, they may additionally exist as **diastereomers**. It is to be understood that all such isomers and mixtures thereof are encompassed within the scope of the present invention. Preferably, wherein a compound of the present invention is present an enantiomer, the enantiomer is present at an enantiomeric excess of greater than or equal to about 80%, more preferably, at an enantiomeric excess of greater than or equal to about 90%, more preferably still, at an enantiomeric excess of greater than or equal to about 95%, more preferably still, at an enantiomeric excess of greater than or equal to about 98%, most preferably, at an enantiomeric excess of greater than or equal to about 99%. Similarly, wherein a compound of the present invention is a diastereomer, the diastereomer is present at an diastereomeric excess of greater than or equal to about 80%, more preferably, at an diastereomeric excess of greater than or equal to about 90%, more preferably still, at an diastereomeric excess of greater than or equal to about 95%, more preferably still, at an diastereomeric excess of greater than or equal to about 98%, most preferably, at an diastereomeric excess of greater than or equal to about 99%.

Some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography or recrystallization. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1999. The protecting groups may be removed at a convenient subsequent stage using methods known from the art.

For use in medicine, the salts of the compounds of this invention refer to non-toxic "**pharmaceutically acceptable salts.**" Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:

acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts

include the following:

acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucoronic acid, L-glutamic acid, $\alpha$-oxo-glutaric acid, glycolic acid, hipuric acid, hydrobromic acid, hydrochloric acid, (+)-L-lactic acid, ($\pm$)-DL-lactic acid, lactobionic acid, maleic acid, (-)-L-malic acid, malonic acid, ($\pm$)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and

bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The present Invention further comprises pharmaceutical compositions containing one or more compounds of formula (II) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.1-1000 mg and may be given at a dosage of from about 0.1-500 mg/kg/day. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 1000 mg, preferably, from about 0.1 to about 500 mg, of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The method of treating central nervous system disorders described in the present invention may also be carried out using a pharmaceutical composition comprising any of the compounds as defined herein and a pharmaceutically acceptable carrier. The pharmaceutical composition may contain between about 0.1 mg and 1000 mg, preferably about 50 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

Advantageously, one or more of the compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

The compounds of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phophatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxy-ethylaspartamidephenol, or polyethyl eneoxidepolylysine substituted with palmitoyl residue. Furthermore, the compounds of the present invention may be coupled to a class of biodegradable polymers useful in achieving controlled release of a drug, for example, polylactic acid, polyepsilon caprolactone, polyhydroxy butyeric acid, polyorthoesters, polyacetals, polydihydropyrans, polycyanoacrylates and cross-linked or amphipathic block copolymers of hydrogels.

Compounds of this invention may be administered in any of the foregoing compositions and according to dosage regimens established in the art whenever treatment of disorders of the central nervous system is required.

The daily dosage of the products may be varied over a wide range from 0.01 to 1,000 mg per adult human per

day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.01 mg/kg to about 1000 mg/kg of body weight per day. Preferably, the range is from about 0.5 to about 500 mg/kg of body weight per day, most preferably, from about 1.0 to about 250 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

Compounds of formula (II) may be prepared according to the processes described below for formula (I) by selecting and substituting suitably substituted reagents and intermediates for those disclosed within the schemes.

For example, compounds of formula (II) may be prepared according to the process outlined in Scheme 1 below, by selecting and substituting, a suitably substituted compound of formula (XXI)

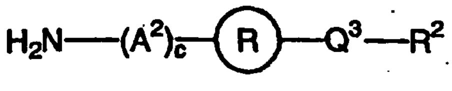

(XXI)

a known compound or compound prepared by known methods, for the compound of (XI).

Compounds of formula (I) may be prepared according to the general process outlined in Scheme 1.

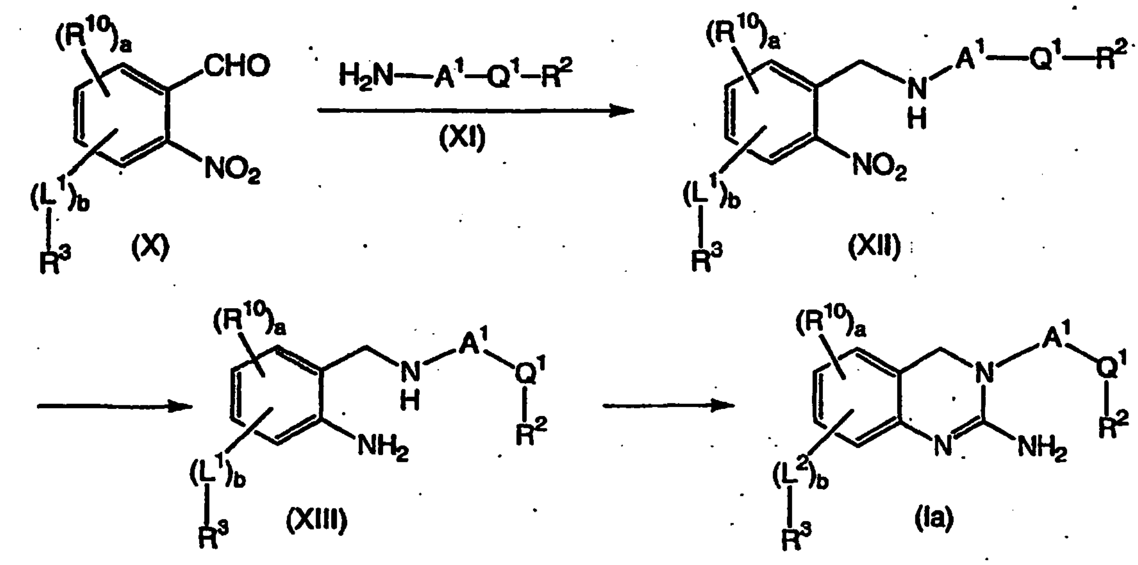


Scheme 1

Accordingly, a suitably substituted compound of formula (X), a known compound or compound prepared by known methods, is reacted with a suitably substituted compound of formula (XI), a known compound or compound prepared by known methods, in the presence of a reducing agent such as $NaBH(OAc)_3$, and the like, in an organic solvent such as dichloromethane, 1,2-dichloroethane, THF, acetonitrile, and the like; or in the presence of a reducing agent such as $NaBH_3CN$, $NaBH_4$, and the like, in an organic solvent such as methanol, acetonitrile, and the like; to yield the corresponding compound of formula (XII).

The compound of formula (XII) is reacted with hydrogen gas, in the presence of a catalyst such as Pd on carbon (Pd/C), and the like, in a protic solvent such as methanol, ethanol, and the like, to yield the corresponding compound of formula (XIII). Alternatively, the compound of formula (XII) is reacted with a reducing agent such as stannous chloride, and the like, in an organic solvent such as methanol, ethanol, ethyl acetate, THF and the like, or in acid such as concentrated HCl, and the like,; or with a reducing agent such as zinc, in the presence of an acid source such as ammonium chloride, calcium chloride, HBr, and the like, in an organic solvent such as methanol, ethanol, ethyl acetate, and the like, or in a mixture of an organic solvent and water as a co-solvent, or in aqueous acid such as acetic acid, and the like, to yield the corresponding compound of formula (XIII).

The compound of formula (XIII) is reacted with cyanogen bromide, and the like, in an organic solvent such as methanol, ethanol, toluene, and the like, to yield the corresponding compound of formula (Ia). Alternatively, the compound of formula (XIII) is reacted with 2-methyl-2-thiopseudourea, in the presence of an acid such as hydrochloric acid, sulfuric acid, and the like, in an organic solvents such as butanol, and the like, to yield the corresponding compound of formula (Ia).

One skilled in the art will recognize that compounds of formula (I) wherein the amine bound at the 2-position of the core structure is substituted with methyl-carbonyl-may be prepared from the corresponding compound of formula (Ia) above by reacting with a suitably substituted anhydride or chloroformate, in the presence of a base such as TEA, DIPEA, pyridine, DMAP, and the like, in an organic solvent such as DCM, chloroform, THF, and the like.

One skilled in the art will further recognize that compounds of formula (I) wherein the amine bound at the 2-position of the core structure is substituted with hydroxy or methoxy may be prepared from the corresponding compound of formula (Ia) by reacting the compound of formula (XIII) with a reagent such as carbon disulfide, 1,1'-thiocarbonyldiimidazole, thiophosgene, and the like, in the presence of a base such as NaOH, KOH, DIPEA, and the like, in an organic solvent such as methanol, ethanol, acetonitrile, DMF, and the like, or in a mixture of an organic solvent and water, to convert the 2-position amine group on the compound of formula (XIII) to the corresponding thiourea. The thiourea is then reacted with a methylating agent such as methyl iodide, dimethyl sulfide, and the like, in the presence of a base such as NaOH, NaH, DMAP, and the like, in an organic solvent such as DMF, acetone, THF, diethyl ether, and the like, to convert the thiourea to the corresponding thiomethyl compound. The thiomethyl compound is then reacted with N-hydroxylamine or N-methoxyamine, in the presence of a base such as TEA, DIPEA, pyridine, and the like, in an organic solvent such as DMF, acetonitrile, THF, methanol, and the like, optionally in the presence of a thiophilic reagent such as $HgCl_2$, AgOAc, and the like, to yield the corresponding compound of formula (I) wherein $R^1$ is hydroxy or methoxy..

Compounds of formula (I) wherein $R^0$ is methyl or trifluoromethyl may be prepared according to the process outlined in Scheme 1 above by substituting a suitably substituted compound of formula (XIV)

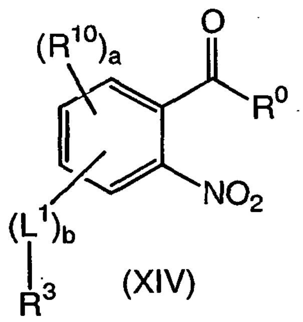

(XIV)

for the compound of formula (X).

Compounds of formula (I) wherein $Q^1$ is -NH-C(O)- may alternatively be prepared according to the process outlined in Scheme 2.

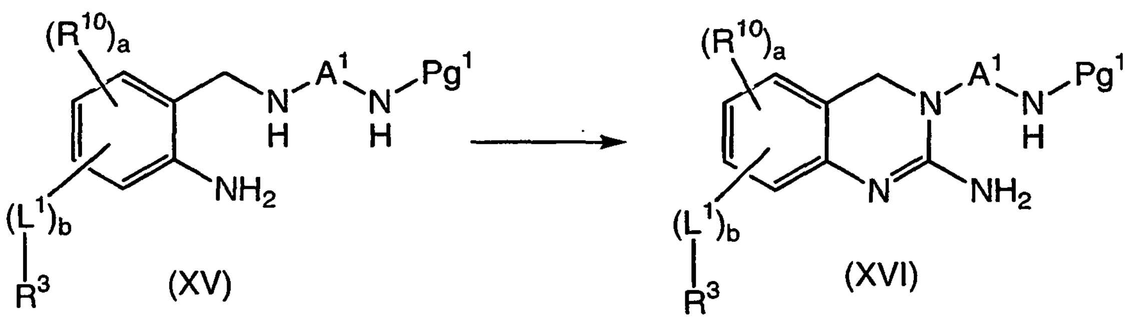

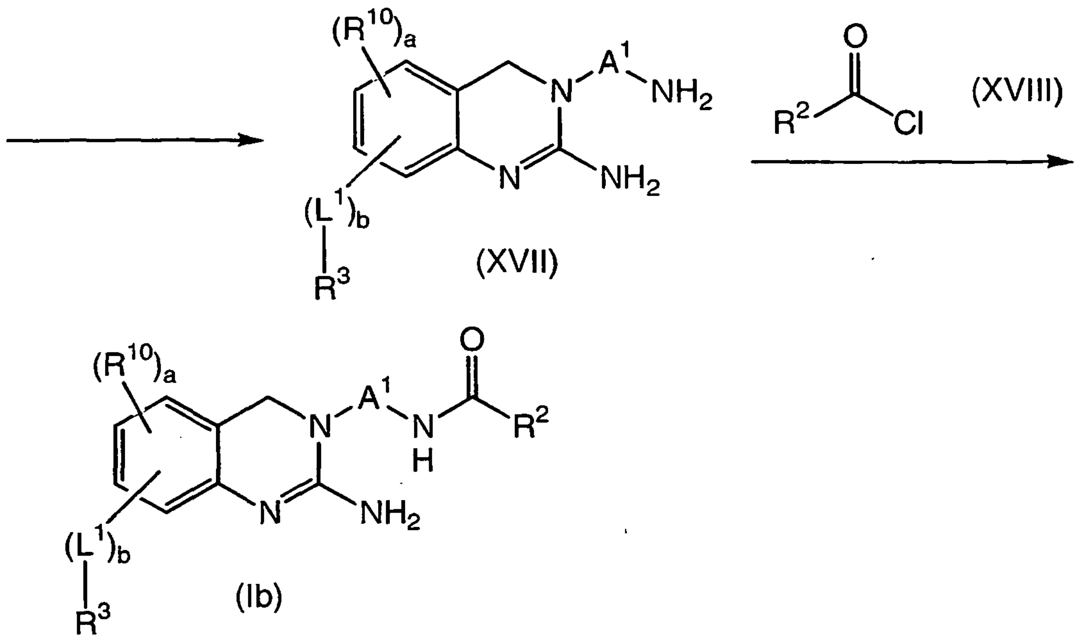

(XVII)

(XVIII)

(Ib)

Scheme 2

Accordingly, a suitably substituted compound of formula (XV), wherein $Pg^1$ is a suitable nitrogen protecting group such as Cbz, BOC, and the like, preferably BOC, a known compound or compound prepared by known methods, (for example by reacting the compound of formula (X), as defined above, with a compound of the formula $NH_2$-$A^1$-$NHPg^1$ and then reducing the nitro group to the corresponding amine), is reacted with cyanogen bromide, in an organic solvent such as methanol, ethanol, toluene, and the like, to yield the corresponding compound of formula (XVI). Alternatively, the compound of formula (XV) is reacted with 2-methyl-2-thiopsuedourea, in the presence of an acid such as hydrochloric acid, sulfuric acid, and the like, in an organic solvent such as ethanol, butanol, xylene, or dioxane, or in an aqueous solvent such as water to yield the corresponding compound of formula (XVI).

The compound of formula (XVI) is de-protected according to known methods, to yield the corresponding compound of formula (XVII). For example, wherein $Pg^1$ is BOC, the compound of formula (XVI) is de-protected by reacting with an acid such as TFA, HCl, formic acid, and the like; wherein $Pg^1$ is Cbz, the compound of formula (XVI) is de-protected by reacting with a hydrogen source such as $H_{2(g)}$ in the presence of a catalyst. (See for example, Protective Groups in Organic Chemistry, ed. J.F.W. McOmie, Plenum Press, 1973; or T.W. Greene & P.G.M. Wuts, Protective Groups in Organic Synthesis, John Wiley & Sons, 1999)

The compound of formula (XVII) is reacted with a suitably substituted acid chloride, a compound of formula (XVIII), a known compound or compound prepared by known methods, in the presence of a base such as TEA, DIPEA, pyridine, and the like, in an organic solvent such as dioxane, DCM, chloroform, and the like; to yield the corresponding compound of formula (Ib).

One skilled in the art will recognize that compounds of formula (I) wherein Q is -NH-C(S)-NH- may be similarly prepared according to the process outlined in Scheme 2 above, by reacting the compound of formula (XVII) with a suitably substituted isothiocyanate, a compound of the formula $R^2$-NCS, a known compound or compound prepared by known methods, in an organic solvent such as dioxane, dichloromethane, chloroform, and the like.

One skilled in the art will further recognize that compounds of formula (I) wherein Q is -NH-C(O)-NH- may be similarly prepared according to the process outlined in Scheme 2 above, by reacting the compound of formula (XVII) with a suitably substituted isocyanate, a compound of the formula $R^2$-NCO, a known compound or compound prepared by known methods, in an organic solvent such as dioxane, DCM, chloroform, and the like.

One skilled in the art will further recognize that compounds of formula (I) wherein Q is -NH-C(O)-O- may be similarly prepared according to the process outlined in Scheme 2 above, by reacting the compound of formula (XVII) with a suitably substituted chloroformate, a compound of the formula $R^2$-O-C(O)-Cl, a known compound or compound prepared by known methods, in the presence of a base such as TEA, DIPEA, pyridine, and the like, in an organic solvent such as dioxane, DCM, chloroform, and the like.

One skilled in the art will further recognize that compounds of formula (I) wherein Q is -NH-$SO_2$- may be similarly prepared according to the process outlined in Scheme 2 above, by reacting the compound of formula (XVII) with a suitably substituted sulfonyl chloride, a compound of the formula $R^2$-$SO_2$-Cl, a known compound or compound prepared

by known methods, in the presence of a base such as TEA, DIPEA, pyridine, and the like, in an organic solvent such as dioxane, DCM, chloroform, and the like.

Compounds of formula (I) wherein $Q^1$ is -NH- and wherein $R^2$ is selected from the group consisting of $C_{1-8}$alkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl- and heterocycloalkyl-$C_{1-4}$alkyl- may alternatively be prepared according to the process outlined in Scheme 3.

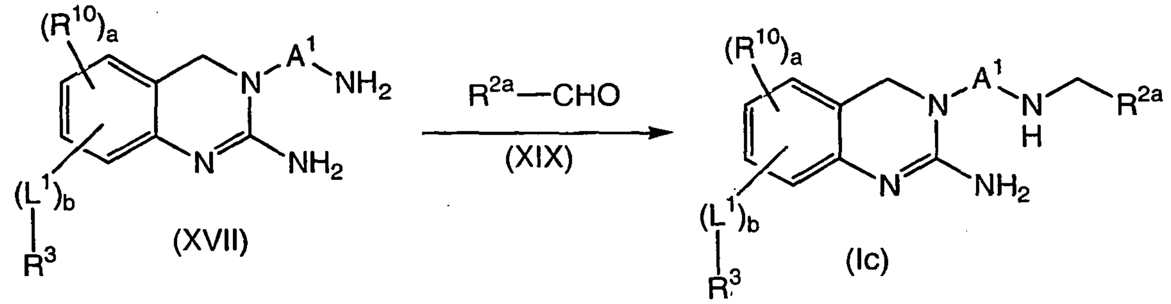


Scheme 3

Accordingly, a suitably substituted compound of formula (XVII) is reacted with a suitably substituted aldehyde, a compound of formula (XIX), wherein $R^{2a}$ is $C_{1-7}$alkyl, cycloalkyl-$C_{0-3}$alkyl-, $C_{0-3}$aralkyl, partially unsaturated carbocyclyl-$C_{0-3}$alkyl-, heteroaryl-$C_{0-3}$alkyl- or heterocycloalkyl-$C_{0-3}$alkyl-, a known compound or compound prepared by known methods, in the presence of a reducing agent such as $NaBH(OAc)_3$, and the like, in an aprotic solvent such as DCM, DCE, THF, and the like; or in the presence of a reducing agent such as $NaCNBH_3$, and the like, in an organic solvent such as methanol, ethanol, THF, acetonitrile, and the like, to yield the corresponding compound of formula (Ic).

One skilled in the art will recognize that wherein $R^2$ is selected from the group consisting of -CH($C_{1-4}$alkyl)-($C_{1-4}$alkyl), cycloalkyl-CH($C_{1-4}$alkyl)-, aryl-CH($C_{1-4}$alkyl)-, partially unsaturated carbocyclyl-CH($C_{1-3}$alkyl)-, heteroaryl-CH($C_{1-4}$alkyl)- and heterocycloalkyl-CH($C_{1-4}$alkyl)-, the compound of formula (I) may be prepared according to the process outlined in Scheme 4.

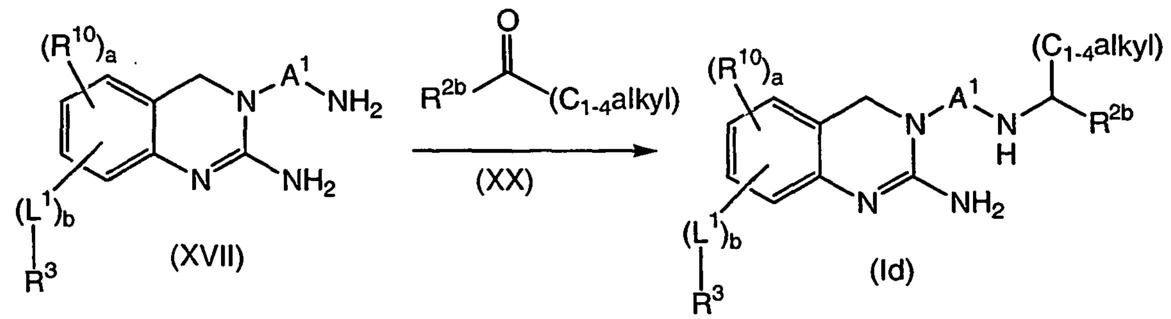


Scheme 4

Accordingly, a suitably substituted compound of formula (XVII) is reacted with a suitably substituted ketone, a compound of formula (XX), wherein $R^{2b}$ is selected from ($C_{1-3}$alkyl), cycloalkyl, aryl, partially unsaturated carbocyclyl, heteroaryl or heterocycloalkyl, a known compound or compound prepared by known methods, In the presence of a reducing agent such as $NaBH(OAc)_3$, and the like, in an aprotic solvent such as DCM, DCE, THF, and the like; or in the presence of a reducing agent such as $NaCNBH_3$, and the like, in an organic solvent such as methanol, ethanol, THF, acetonitrile, and the like, to yield the corresponding compound of formula (Id).

One skilled in the art will further recognize that compounds of formula (I) wherein $Q^1$ is NH and $R^2$ is selected from the group consisting of cycloalkyl, partially unsaturated carbocyclyl and heterocycloalkyl may be similarly prepared according to the process outlined in Scheme 4 above, by substituting a suitably substituted ketone (i.e. a cycloalkyl ketone such as cyclohexone; a partially unsaturated carbocyclyl ketone; or a heterocycloalkyl ketone) for the compound of formula (XX).

Compounds of formula (X) are known compounds or compounds which may be prepared according to known methods. Schemes 8-12 below outline processes for the preparation of representative compounds of formula (X).

For example, compounds of formula (X) wherein $(L^1)_b$ is -O- and $R^3$ is for example, aryl or heteroaryl, may be prepared according to any of the processes outlined in Scheme 8.

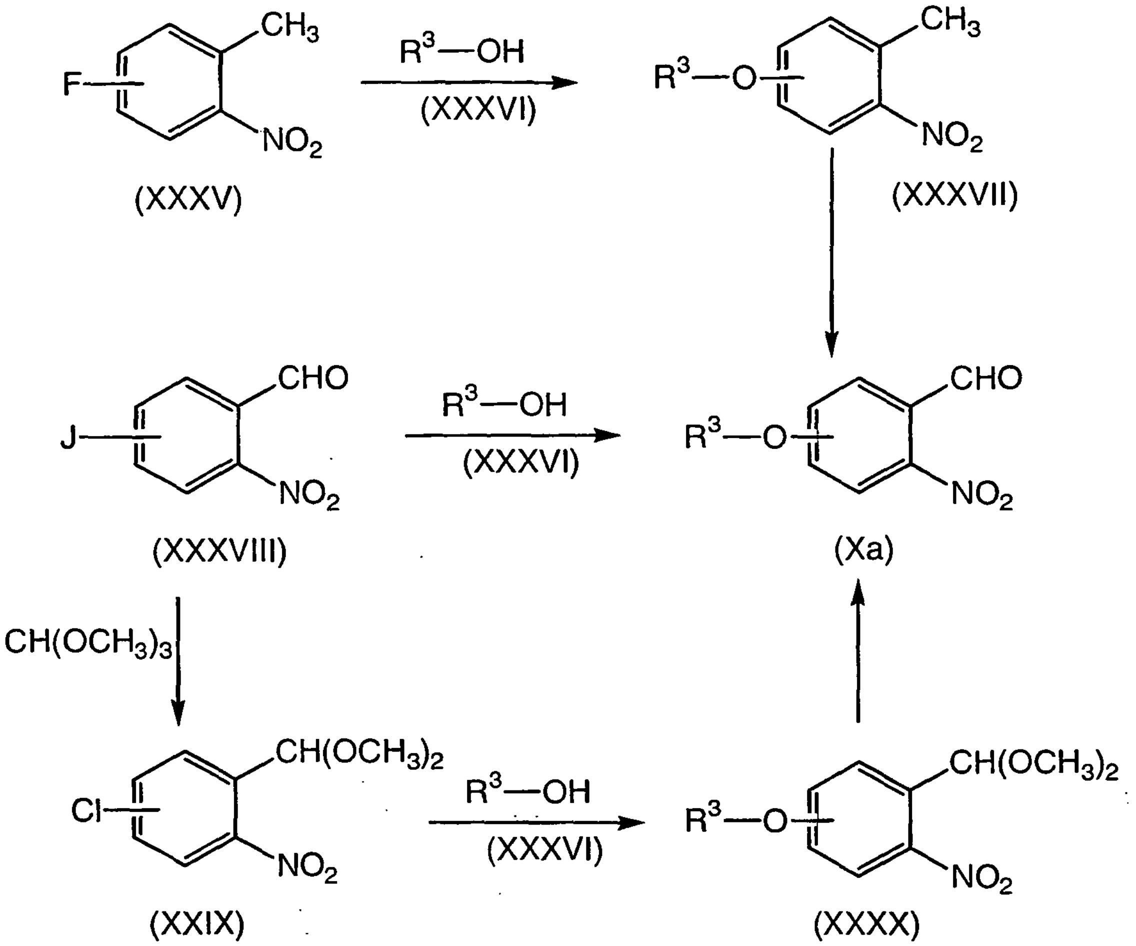

(XXXV)
(XXXVII)
(XXXVIII)
(Xa)
(XXIX)
(XXXX)

Scheme 8

Accordingly, a suitably substituted compound of formula (XXXV), a known compound or compound prepared by known methods, wherein the phenyl ring may be further optionally substituted with one to three $R^{10}$ groups and wherein the F is bound at the carbon of the phenyl ring to which the -$(L^1)_b$-$R^3$ group in the desired compound of formula (I) is to be bound; is reacted with a suitably substituted compound of formula (XXXVI), a known compound or compound prepared by known methods, in the presence of a base such as $K_2CO_3$, $Cs_2CO_3$, and the like, in an organic solvent such as DMF, DMA, and the like, preferably DMF, at an elevated temperature in the range of from about 25°C to about 150°C, preferably, at an elevated temperature in the range of from about 100°C to about 120°C, optionally in the presence of microwave irradiation, to yield the corresponding compound of formula (XXXVII).

The compound of formula (XXXVII) is reacted with an electrophilic formyl source such as DMF dimethyl acetal, DMF diethyl acetal, and the like, in an organic solvent such as DMF, DMA, and the like, preferably DMF; or with neat tripiperidinomethane, preferably under vacuum; or with neat tert-butoxy-bis(dimethyl)aminomethane, at an elevated temperature in the range of from about 100°C to about 150°C, preferably, at an elevated temperature in the range of from about 130°C to about 140°C, followed by reaction with $NaIO_4$, and the like, in an organic solvent such as THF, DME, and the like, in the presence of water as a co-solvent, to yield the corresponding compound of formula (Xa).

Alternatively, a suitably substituted compound of formula (XXXVIII) wherein J is F, a known compound or compound prepared by known methods, wherein the phenyl ring may be further optionally substituted with one to three $R^{10}$ groups and wherein the F is bound at the carbon of the phenyl ring to which the -$(L^1)_b$-$R^3$ group in the desired compound of formula (I) is to be bound; is reacted with a suitably substituted compound of formula (XXXVI), a known compound or compound prepared by known methods, in the presence of a base such as $K_2CO_3$, $Cs_2CO_3$, and the like, in an organic solvent such as DMF, DMA, and the like, at an elevated temperature in the range of from about 100°C to about 170°C, preferably, at an elevated temperature in the range of from about 140°C to about 160°C, to yield the corresponding compound of formula (Xa).

Alternatively, a suitably substituted compound of formula (XXXVIII) wherein J is OH, a known compound or compound prepared by known methods, wherein the phenyl ring may be further optionally substituted with one to three $R^{10}$ groups and wherein the OH is bound at the carbon of the phenyl ring to which the -$(L^1)_b$-$R^3$ group in the desired

compound of formula (I) is to be bound; is reacted with a suitably substituted compound of formula (XXXVI), under Mitsonobu conditions, for example, in the presence of a phosphine reagent such as triphenylphosphine, tributylphosphine, and the like and in the presence of an acetylene dicarboxylate such as DEAD, DIAD, and the like; in an organic solvent such as THF, DMF, and the like, to yield the corresponding compound of formula (Xa). Preferably, the Mitsonobu conditions are applied to the preparation of compounds of formula (Xa) wherein $R^3$ is $C_{1-4}$alkyl.

Alternatively still, a suitably substituted compound of formula (XXXVIII) wherein J is Cl or F, a known compound or compound prepared by known methods, wherein the phenyl ring may be further optionally substituted with one to three $R^{10}$ groups and wherein the Cl or F is bound at the carbon of the phenyl ring to which the $-(L^1)_b-R^3$ group in the desired compound of formula (I) is to be bound; is reacted with $CH(OCH_3)_3$ in the presence of an acid catalyst such as p-TsOH, $NH_4Cl$, $AlCl_3$, and the like, in an organic solvent such as methanol, THF, and the like, at an elevated temperature, preferably, at about reflux temperature, to yield the corresponding compound of formula (XXXIX).

The compound of formula (XXXIX) is reacted with a suitably substituted compound of formula (XXVI), a known compound or compound prepared by known methods, in the presence of a base such as $K_2CO_3$, $Cs_2CO_3$, and the like, in an organic solvent such as DMF, DMA, and the like, at an elevated temperature in the range of from about 25°C to about 150°C, preferably, at an elevated temperature in the range of from about 100°C to about 120°C, to yield the corresponding compound of formula (XXXX).

The compound of formula (XXXX) is hydrolyzed by reacting with an acid such as HCl, $H_2SO_4$, trifluoroacetic acid, and the like, in an organic solvent such as THF, DCM, diethyl ether, and the like, in the presence of water, to yield the corresponding compound of formula (Xa).

Compounds of formula (X) wherein $(L^1)_b$ is -S- may be similarly prepared according to the process described above by reacting a compound of formula (XXXVIII) wherein J is F, with a substituting a suitably substituted compound of the formula $R^3$-SH (i.e. substituting the compound of formula $R^3$-SH for the compound of formula (XXXVI)). The resulting compound may then be optionally, selectively oxidized to yield the corresponding compound of formula (X) wherein the -S- is oxidized to -SO- and / or $-SO_2-$.

Compounds of formula (X) wherein $(L^1)_b$ is $-NR^N-$, may be similarly prepared according to the process outlined in Scheme 7 above, by reacting a compound of formula (XXXVIII) wherein J is Br with a suitably substituted compound of the formula $R^3-NHR^N$, in the presence of a catalyst or mixture thereof, such as a 1:3 mixture of $Pd_2(dba)_3$ and dppf, and the like, in an organic solvent such as DMF, DME, toluene, and the like; or a catalyst such as $Pd_2(dba)_3$ or Pd(dppf) $Cl_2$ in the presence or a base such as $Cs_2CO_3$, $NaOC(CH_3)_3$, and the like, in an organic solvent such as toluene, and the like, to yield the corresponding compound of formula (X) wherein $(L^1)_b$ is $-NR^N-$Compounds of formula (X) wherein $(L^1)_b$ is -C(O)- may be prepared according to the processes disclosed in European Patent Number EP 0 371 564 B1; Katritzky, A.R., Chassaing, C., Toader, D. and Gill, K., J. Chem. Research, (S), 1999, pp 504-505; Katritzky, A.R., Lang, H., Wang, Z., Zhang, Z. and Song, H., J. Org. Chem., 60, 1990, pp 7619-7624; and / or Vetelino, M.G. and Coe, J.W., Tetrahedron Lett., 35(2), 1994, pp 219-22; with suitable modification, as would be clear to one of ordinary skill in art.

Compounds of formula (X) wherein $(L^1)_b$ is -C(S)- may be prepared by reacting the corresponding compound of formula (X) wherein $(L^1)_b$ is -C(O)-with a suitably selected thionating reagent such as $P_2S_5$, Lawesson's reagent, and the like, in an organic solvent such as toluene, benzene, xylene, and the like, at an elevated temperature in the range of from about 70°C to about 150°C, preferably, at an elevated temperature in the range of from about 80°C to about 110°C.

Compounds of formula (X) wherein $(L^1)_b$ is $-C_{2-4}$alkenyl- may be prepared by reacting the compound of formula (XXXVIII) wherein J is Br or I with a suitably substituted stannane, or a suitably substituted boronate, in the presence of a catalyst such as $Pd(PPh_3)_4$, and the like, according to known methods (for example, as disclosed in Miyaura, N.; Suzuki, A. Chem. Rev. 1995, 95, 2457; Suzuki, A. J. Organomet. Chem. 1999, 576, 147).

Alternatively, compounds of formula (X) wherein $(L^1)_b$ is $-C_{2-4}$alkenyl-may be prepared according to the process outlined in Scheme 9.

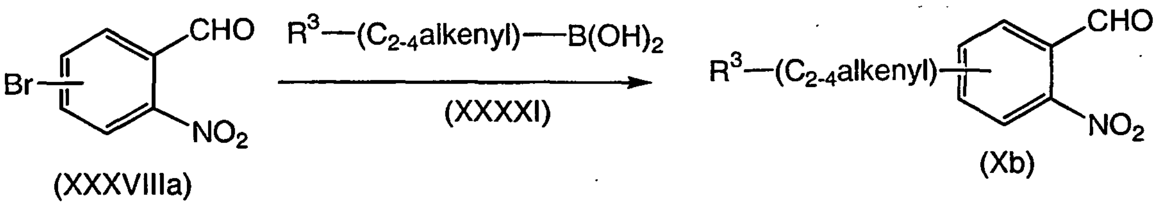


(XXXVIIIa)

(Xb)

Scheme 9

Accordingly, a suitably substituted compound of formula (XXXVIIIa), a compound of formula (XXXVIII) wherein **J** is Br, a known compound or compound prepared by known methods, is reacted with a suitably substituted compound of formula (XXXXI), wherein $R^3$ is as defined above, preferably, wherein $R^3$ is aryl or alkyl, a known compound or

compound prepared by known methods (for example as disclosed in Miyaura, N.; Suzuki, A. Chem. Rev. 1995, 95, 2457; Suzuki, A. J. Organomet. Chem. 1999, 576, 147), in the presence of a catalyst such $Pd(PPh_3)_4$, $Pd(dppf)Cl_2$, and the like, in the presence of a base such as $Na_2CO_3$, $K_2CO_3$, $NaHCO_3$, and the like, in a mixture of an organic solvent such as toluene, DME, THF, MeOH, and the like, and a protic solvent such as water, and the like, at an elevated temperature in the range of from about 60°C to about 150°C, preferably, at an elevated temperature in the range of from about 100°C to about 120°C, optionally in the presence of microwave irradiation, to yield the corresponding compound of formula (Xb).

Compounds of formula (X) wherein $(L^1)_b$ is -$C_{2-4}$alkyl- may be prepared by hydrogenating the corresponding compound of formula (X) wherein $(L^1)_b$ is - ($C_{2-4}$alkenyl)-.

Compounds of formula (X) wherein $(L^1)_b$ is -$CH_2$- or -CH(OH)- may be prepared according to known methods, for example, by reducing the corresponding compound of formula (X) wherein $(L_1)_b$ is -C(O)- with a suitably selected reducing agent, according to known methods.

Compounds of formula (X) wherein $(L^1)_b$ is -CH(OH) may alternatively be prepared according to the process outlined in Scheme 10.

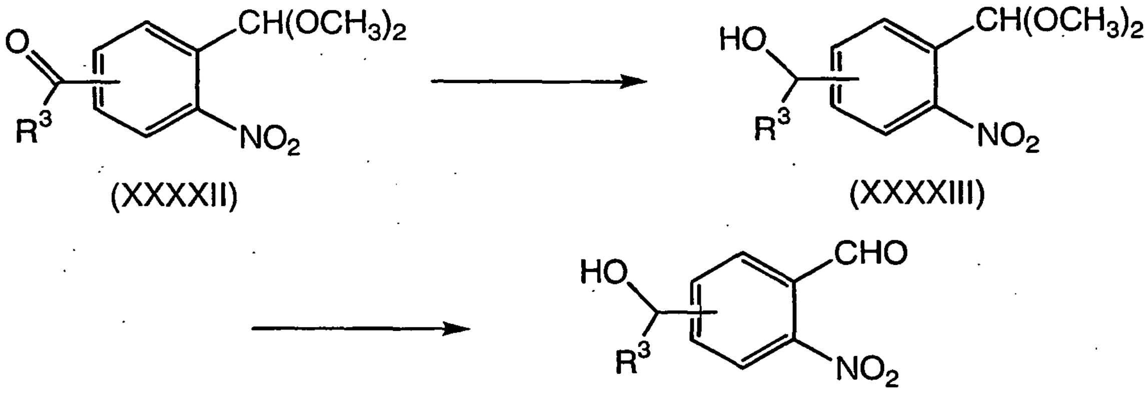

(XXXXII)

(XXXXIII)

(Xc)

Scheme 10

Accordingly, a suitably substituted compound of formula (XXXXII), a known compound or compound prepared by known methods, is reacted with a suitably selected reducing agent, such as sodium borohydride, and the like, in an organic solvent such as methanol, isopropanol, and the like, at a temperature in the range of from about room temperature to about 100°C, preferably, at about room temperature, to yield the corresponding compound of formula (XXXXIII).

The compound of formula (XXXXIII) is hydrolyzed by reacting with a suitably selected acid such as HCl, $H_2SO_4$, trifluoroacetic acid, and the like, in an organic solvent such as THF, DCM, diethyl ether, and the like, in the presence of water, to yield the corresponding compound of formula (Xc).

Compounds of formula (X) wherein $(L^1)_b$ is -(hydroxy substituted $C_{2-4}$alkyl)- may be prepared according to the process outlined in Scheme 11.

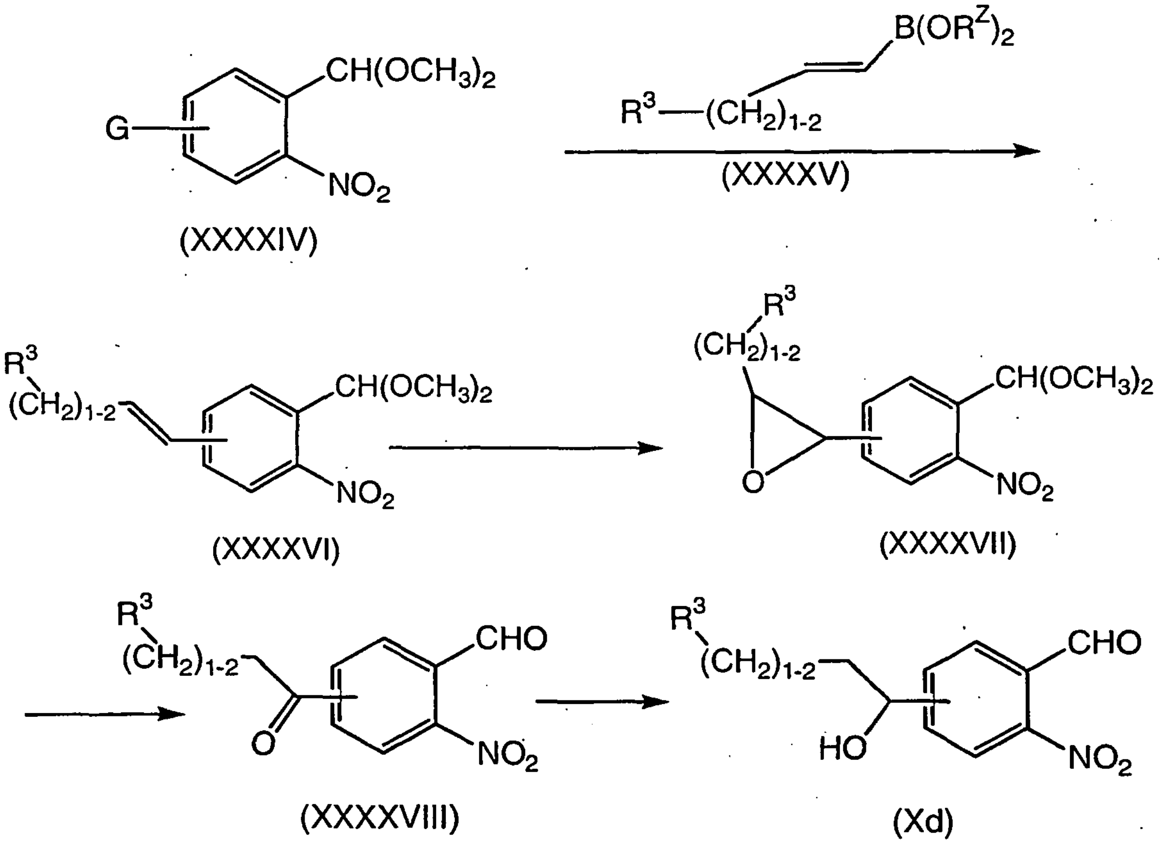

(XXXXIV) (XXXXV)

(XXXXVI) (XXXXVII)

(XXXXVIII) (Xd)

Scheme 11

Accordingly, a suitably substituted compound of formula (XXXXIV), wherein G is selected from chloro, bromo or iodo, a known compound or compound prepared by known methods, is reacted with a suitably substituted vinyl boronate ester, a compound of formula (XXXXV) wherein $R^z$ is selected from aryl or heteroaryl, a known compound or compound prepared by known methods (for example as disclosed in Lhermitte, F.; Carboni, B. SYNLETT 1996, 377; Matsubara, S.; Otake, Y.; Hashimoto, Y.; Utimoto, K. Chem. Lett. 1999, 747; Takai, K.; Shinomiya, N.; Kaihara, H.; Yoshida, N.; Moriwake, T. SYNLETT 1995, 963; Deloux, L.; Srebnik, M. J. Org. Chem. 1994, 59, 6871), in the presence of a catalyst such as $Pd(PPh_3)_4$, $Pd(dppf)Cl_2$, and the like, (Miyaura, N.; Suzuki, A. Chem. Rev. 1995, 95, 2457; Suzuki, A. J. Organomet. Chem. 1999, 576, 147), in the presence of a base such as $Na_2CO_3$, $K_2CO_3$, $NaHCO_3$, and the like, in a mixture of an organic solvent such as toluene, DME, THF, MeOH, and the like, and a protic solvent such as water, at an elevated temperature in the range of from about 60°C to about 150°C, preferably, at an elevated temperature in the range of from about 100°C to about 120°C, optionally in the presence of microwave irradiation, to yield the corresponding compound of formula (XXXXVI).

The compound of formula (XXXXVI) is reacted with a suitably selected oxidizing agent such as OXONE®, and the like, (Yang, D.; Yip, Y.-C.; Jiao, G.-S.; Wong, M.-K. Org. Synth. 2000, 78, 225) in the presence of a catalyst such as tetrahydrothiopyran-4-one, and the like, in the presence of a base such as $Na_2CO_3$, $K_2CO_3$, $NaHCO_3$, and the like, in a mixture of an organic solvent such as MeCN, THF, and the like, and a protic solvent such as water, to yield the corresponding compound of formula (XXXXVII).

Alternatively, the compound of formula (XXXXVI) is reacted with an oxidizing agent, such as MCPBA, (Jung, M. E.; Lam, P. Y.-S.; Mansuri, M. M.; Speltz, L. M. J. Org. Chem. 1985, 50, 1087), hydrogen peroxide, and the like, in an in the presence of a base such as $Na_2CO_3$, $K_2CO_3$, $NaHCO_3$, and the like, in an organic solvent such as DCM, DCE, and the like, at a temperature in the range of from about room temperature to about 100°C, preferably, at about room temperature, to yield the corresponding compound of formula (XXXXVII).

The compound of formula (XXXXVII) is reacted with a protic acid such as *p*-toluenesulfonic acid, methanesulfonic acid, and the like, (Bakke, J. M.; Lorentzen, G. B. Acta Chem. Scand. B 1974, 28, 650) in an organic solvent such as benzene, toluene, and the like, at an elevated temperature in the range of from about 80°C to about 150°C, preferably, at an elevated temperature in the range of from about 80°C to about 100°C, to yield the corresponding compound of formula (XXXXVIII)

Alternatively, the compound of formula (XXXXVII) is reacted with a Lewis acid such as $BF_3{\bullet}Et_2O$, and the like,

(Baumgarth, M.; Beier, N.; Gericke, R. J. Med. Chem. 1998, 41, 3736) in an organic solvent such as DCM, diethyl ether, and the like, at an elevated temperature in the range of from about 40°C to about 100°C, preferably, at an elevated temperature in the range of from about 40°C to about 60°C, to yield the corresponding compound of formula (XXXXVIII).

The compound of formula (XXXXVIII) is reacted with a suitably selected reducing agent, such as sodium borohydride, and the like, in an organic solvent such as MeOH, isopropanol, and the like, at a temperature in the range of from about 0°C to about 100°C, preferably, at about 10°C to abour 40°C, to yield the corresponding compound of formula (Xd). One skilled in the art will recognize that in reacting the compound of formula (XXXXVIII) with a suitably selected reducing agent, the aldehyde on the compound of formula (XXXXVIII) is preferably protected to avoid reduction to the corresponding alcohol.

Compounds of formula (X) wherein $(L^1)_b$ is absent (i.e. b is 0) may be prepared according to the process outlined in Scheme 12.

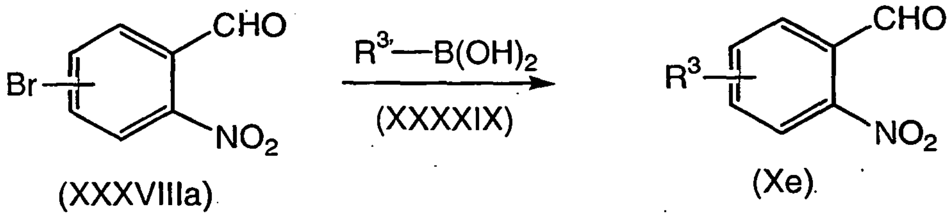


(XXXVIIIa)

(Xe).

Scheme 12

Accordingly, a suitably substituted compound of formula (XXXVIIIa), a compound of formula (XXXVIII) wherein J is Br, a known compound or compound prepared by known methods (For example, 4-bromo-2-nitrobenzaldehyde which may be prepared as disclosed in Jung, M.E. and Dansereau, S.M.K., Heterocycles, Vol. 39, 1994, p. 767; or 5-bromo-2-nitrobenzaldehyde, which may be prepared as disclosed in Hu, Y.-Z., Zhang, G., and Thummel, R.P., Org. Lett., Vol. 5, 2003, p. 2251) is reacted with a suitably substituted compound of formula (XXXXIX), wherein $R^3$ is aryl or heteroaryl, a' known compound or compound prepared by known methods, in the presence of a catalyst such $Pd(PPh_3)_4$, $Pd(dppf)Cl_2$, and the like, in the presence of a base such as $Na_2CO_3$, $K_2CO_3$, $NaHCO_3$, and the like, in a mixture of an organic solvent such as toluene, DME, THF, MeOH, and the like, and a protic solvent such as water, and the like, at an elevated temperature in the range of from about 60°C to about 150°C, preferably, at an elevated temperature in the range of from about 100°C to about 120°C, optionally in the presence of microwave irradiation, to yield the corresponding compound of formula (Xe).

Compounds of formula (XI) are known compounds or compounds which may be prepared by known methods. Schemes 13-17 below, outline representative processes for the preparation of representative compounds of formula (XI).

For example, compounds of formula (XI) wherein $Q^1$ is $-C(O)-NR^A-$ may be prepared according to the processes outlined in Scheme 13.

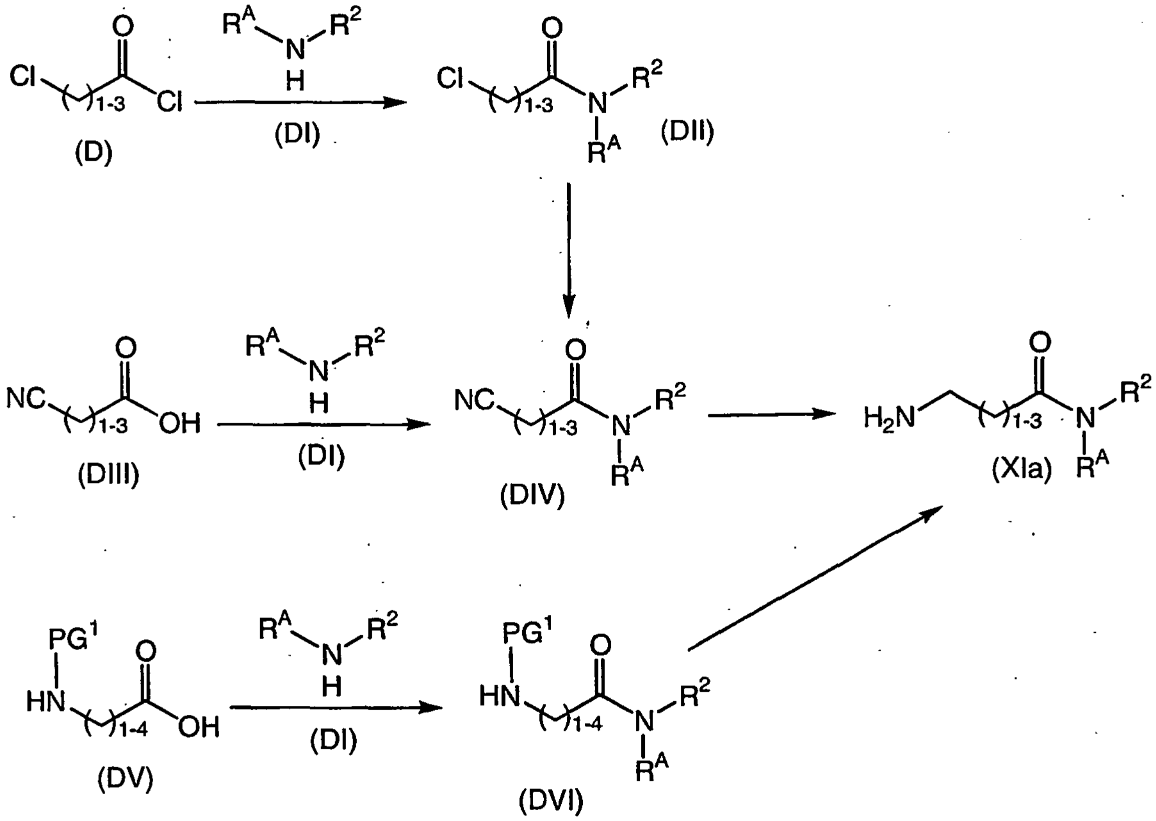

Scheme 13

Accordingly, a suitably substituted compound of formula (D), a known compound or compound prepared by known methods, is reacted with a suitably substituted compound of formula (DI), a known compound or compound prepared by known methods, in the presence of a base such as TEA, DIPEA, pyridine, and the like, in an organic solvent such as THF, DCM, chloroform, and the like, to yield the corresponding compound of formula (DII).

The compound of formula (DII) is reacted with NaCN, in an organic solvent such as DMSO, DMF, NMP, and the like, at an elevated temperature in the range of from about 50 to about 160°C, preferably, at an elevated temperature in the range of from about 80 to about 125°C, to yield the corresponding compound of formula (DIV).

The compound of formula (DIV) is reduced according to known methods, for example by reacting with hydrogen gas in the presence of Raney nickel or rhodium, in an organic solvent such as methanol, ethanol, water, and the like, to yield the corresponding compound of formula (XIa).

Alternatively, a suitably substituted compound of formula (DIII), a known compound or compound prepared by known methods, is reacted with a suitably substituted compound of formula (DI), a known compound or compound prepared by known methods, in the presence of a peptide coupling reagent such as HATU, HOBT, DIPCDI, HBTU, and the like, in the presence of a base such as TEA, DIPEA, pyridine, and the like, in an organic solvent such as THF, DMF, and the like, to yield the corresponding compound of formula (DIV).

The compound of formula (DIV) is reduced according to known methods, for example by reacting with $H_2$ gas, in the presence of Raney nickel or rhodium, in an organic solvent such as methanol, ethanol, water, and the like, to yield the corresponding compound of formula (XIa).

Alternatively still, a suitably substituted compound of formula. (DV), wherein $Pg^1$ is a suitable nitrogen protecting group, such as Cbz, BOC, and the like, a known compound or compound prepared by known methods, is reacted with a suitably substituted compound of formula (DI), a known compound or compound prepared by known methods, in the presence of a peptide coupling reagent such as HATU, HOBT, DIPCDI, HBTU, and the like, in the presence of a base such as TEA, DIPEA, pyridine, and the like, in an organic solvent such as THF, DMF, and the like, to yield the corresponding compound of formula (DVI).

The compound of formula (DVI) is de-protected according to known methods, for example by hydrogenolysis (wherein $PG^1$ is Cbz) or acid cleavage (wherein $PG^1$ is BOC), to yield the corresponding compound of formula (XIa).

One skilled in the art will recognize that for compounds of formula (XI) wherein $Q^1$ is $-C(O)-NR^A-$ and wherein the $A^1$ group (i.e. the $-(C_{1-4}alkyl)-$ group) is substituted as herein defined, may be similarly prepared according to the processes outlined in Scheme 12.

For example, compounds of formula (XI) wherein the -($C_{1-4}$alkyl)- (the $A^1$ group) is substituted at carbon atom bound directly to the core of the desired compound of formula (I) may be prepared by substituting a suitably substituted compound of formula (DVII)

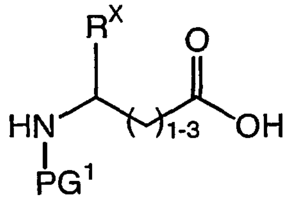

(DVII)

wherein $R^X$ is the substituent on the $A^1$ group as defined herein, for the compound of formula (DV) above.

Similarly, compounds of formula (XI) wherein alternate carbon atoms of the $A^1$ group are substituted may be similarly prepared by selecting and substituting suitably substituted starting reagents for the compound of formula (D), (DIII) or (DV).

Alternatively, compound of formula (XI) wherein $Q^1$ is -C(O)-$NR^A$- and the $A^1$ group (i.e. the -($C_{1-4}$alkyl)- group) is substituted at carbon atom bound directly to the nitrogen atom on the core of the desired compound of formula (I) may be prepared according to the process outlined in Scheme 14.

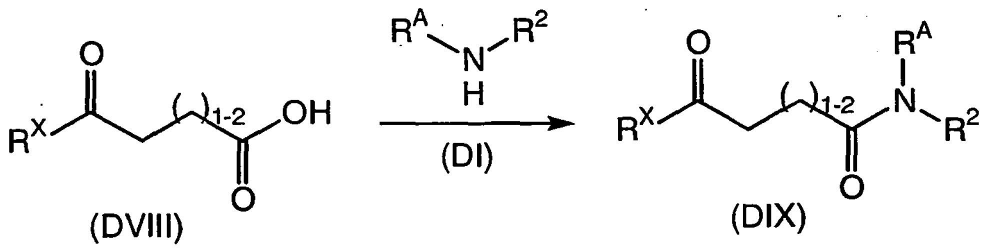

Scheme 14

Accordingly, a suitably substituted compound of formula (DVIII), a known compound or compound prepared by known methods (for example by reacting dihydro-furan-2,5-dione or dihydro-pyran-2,6-dione with a compound of the formula $R^X$-MgCl or $R^X$-MgBr, optionally in the presence of a catalyst such as CuI, in an organic solvent such as THF) is reacted with a suitably substituted compound of formula (DI), a known compound or compound prepared by known methods, in the presence of a peptide coupling reagent such as HATU, HOBT, DIPCDI, HBTU, and the like, in the presence of a base such as TEA, DIPEA, pyridine, and the like, in an organic solvent such as THF, DMF, and the like, to yield the corresponding compound of formula (DIX).

The compound of formula (DIX) is then substituted for the compound of formula (XI) and further reacted according to the processes described above, to yield the corresponding compound of formula (I).

Compounds of formula (XI) wherein $Q^1$ is -$NR^A$- may be prepared by known methods, for example, according to the process outlined in Scheme 15.

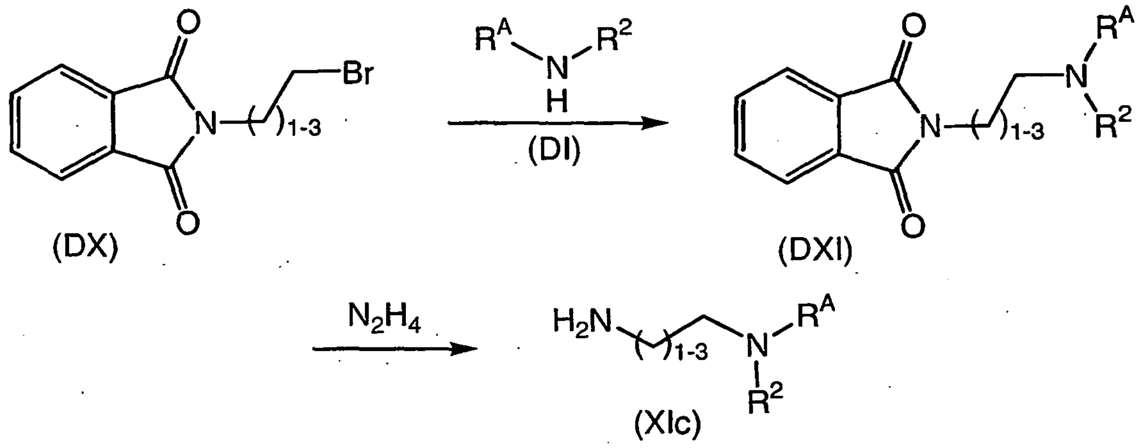


(DX)

(DXI)

(XIc)

Scheme 15

Accordingly, a suitably substituted compound of formula (DX), a known compound or compound prepared by known methods, is reacted with a suitably substituted compound of formula (DI), a known compound or compound prepared by known methods, in the presence of a base such as TEA, DIPEA, pyridine, and the like, in an organic solvent such as THF, DMF, and the like, to yield the corresponding compound of formula (DXI).

The compound of formula (DXI) is reacted with $N_2H_4$, and the like, in an organic solvent such a ethanol, DMF, and the like, to yield the corresponding compound of formula (XIc).

One skilled in the art will recognize that compounds of formula (XI) wherein $Q^1$ is $-NR^A-C(O)-$ may be prepared from the corresponding compound of formula (DXI) wherein $-A^1-O^1-H$ is $-(C_{1-4}alkyl)-NR^A-H$, by reacting with a suitably substituted acid chloride of the formula $R^2-C(O)Cl$, in the presence of a base such as TEA, DIPEA, pyridine, and the like, preferably, the base is present in amount equal to about one equivalent, in an organic solvent such as dioxane, DCM, THF, and the like.

One skilled in the art will further recognize that compounds of formula (XI) wherein $-Q^1-R^2$ is selected from $-NR^A-R^2$, $-NR^A-C(O)-R^2$ or $-NR^A-C(O)-R^2$ and wherein the $A^1$ group (i.e. the ($C_{1-4}$alkyl) group)) is optionally substituted, may be similarly prepared according to the processes outlined in Scheme 15 above, by selecting and substituting suitably substituted starting reagents.

Alternatively, compounds of formula (XI) wherein $Q^1-R^2$ is $-NR^A-R^2$ and wherein the $A^1$ group (i.e. the ($C_{1-4}$alkyl) group)) is optionally substituted, may be similarly prepared according to the processes outlined in Scheme 16.

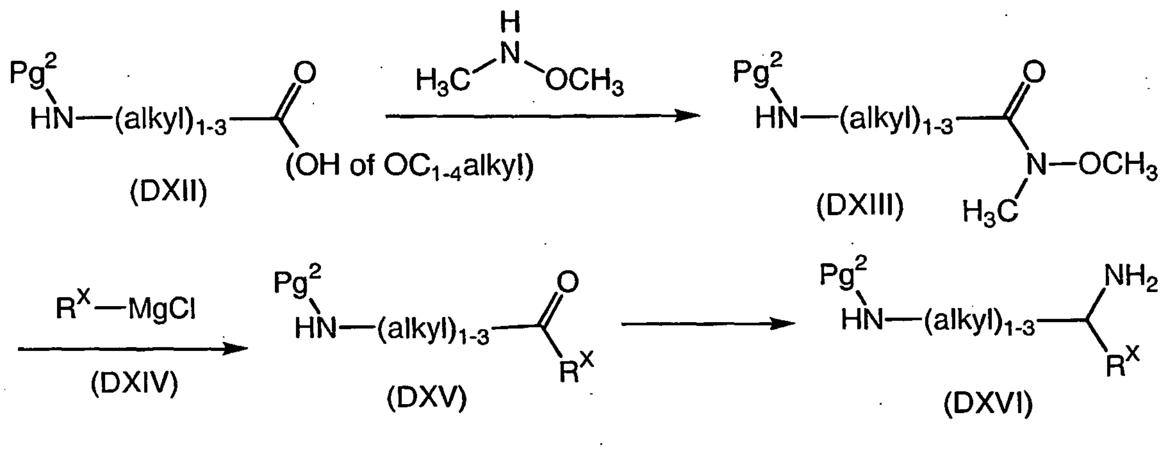


(DXII)

(DXIII)

(DXV)

(DXVI)

Scheme 16

Accordingly, a suitably substituted compound of formula (DXII), a known compound or compound prepared by known methods, is reacted with $NH(CH_3)(OCH_3)$, in the presence of a coupling agent such as $(CH_3)_3Al$ (for compounds of formula (DXII) wherein W is $C_{1-4}$alkyl), or a coupling agent such as DCC, EDC, and the like, in an organic solvent such as DCM, DMF, and the like, to yield the corresponding compound of formula (DXIII).

The compound of formula (DXIII) is reacted with a suitably substituted compound of formula (DXIV), a known

compound or compound prepared by known methods, in an organic solvent such as THF, diethyl ether, and the like, to yield the corresponding compound of formula (DXV).

The compound of formula (DXV) is reacted $NH_4OAc$, $NH_4Cl$, and the like, in the presence of a reducing agent such as $NaBH_3CN$, and the like, in a organic solvent such as methanol, ethanol, and the like; or reacted with $NH_4O_2CCF_3$, $NH_4OAc$, and the like, in the presence of a reducing agent such as $NaBH(OAc)_3$, and the like, in an organic solvent such as DCE, THF, acetonitrile, and the like; to yield the corresponding compound of formula (DXVI).

The compound of formula (DXVI) is then reacted according to the processes disclosed herein (for Example as in Scheme 1) and de-protected to yield the corresponding, desired compound.

One skilled in the art will recognize that the compound of formula (XId) may be further, optionally reacted according to known methods (for example by reacting with a suitably substituted compound of the formula) to further substitute the de-protected amine as desired.

One skilled in the art will recognize that compounds of formula (XI) wherein $-Q^1-R^2$ is $-NH-C(O)-R^2$ and wherein the $A^1$ group (i.e. the ($C_{1-4}$alkyl) group)) is optionally substituted may be prepared from the corresponding compound of formula (DXV) according to the process outlined in Scheme 17.

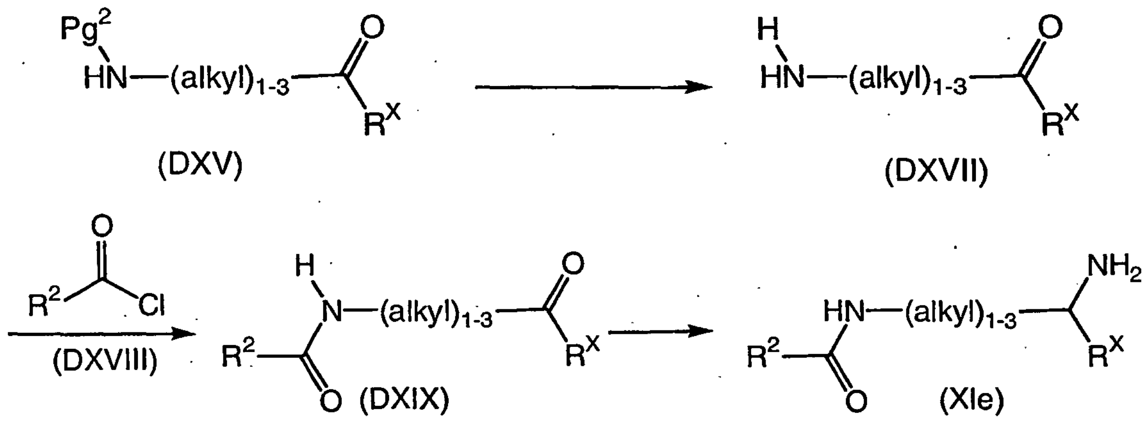

Scheme 17

Accordingly, a suitably substituted compound of formula (DXV) is is de-protected according to known methods, to yield the corresponding compound of formula (DXVII).

The compound of formula (DXVII) is reacted with a suitably substituted acid chloride, a compound of the formula (DXVIII), a known compound or compound prepared by known methods, in the presence of a base such as TEA, DIPEA, pyridine, and the like, in an organic solvent such as dioxane, DCM, THF, and the like, to yield the corresponding compound of formula (DXIX).

The compound of formula (DXIX) is reacted with $NH_4OAc$, $NH_4Cl$, and the like, in the presence of a reducing agent such as $NaBH_3CN$, and the like, in a organic solvent such as methanol, ethanol, and the like; or reacted with $NH_4O_2CCF_3$, $NH_4OAc$, and the like, in the presence of a reducing agent such as $NaBH(OAc)_3$, and the like, in an organic solvent such as DCE, THF, acetonitrile, and the like; to yield the corresponding compound of formula

One skilled in the art will further recognize that compounds of formula (XI) wherein $-A^1-Q^1-R^2$ is $-(C_{1-4}$alkyl$)-NH-C(O)-OR^2$, compounds of formula (XI) wherein $-A^1-Q^1-R^2$ is $-(C_{1-4}$alkyl$)-NH-C(S)-NH-R$ and compounds of formula (XI) wherein $-A^1-Q^1-R^2$ is $-(C_{1-4}$alkyl$)-NH-C(O)-NH-R^2$ may be similarly prepared according to known methods, by modifying the process described in Scheme 2.

One skilled in the art will further recognize that compounds of formula (XI) wherein $Q^1$ is other than one of the substituent groups specifically exemplified above, for example wherein $Q^1$ is selected from -O-, -S-, -OC(O)-, $-NR^A-SO-$, $-NR^A-SO_2-$, $-SO-NR^A-$, $-SO_2-NR^A-$, $-OC(O)-NR^A-$, $-NRA-SO_2-O-$, $-O-SO_2-NR^A-$ or $-NR^A-SO_2-NR^B-$, may be similarly prepared according to known methods.

Compounds of formula (XI) wherein the $A^1$ group (i.e. the $-(C_{1-4}$alkyl$)$-group) is substituted at the carbon atom closest to the core structure is substituted and wherein a single enantiomer is desired (or wherein an enantiomeric excess of a single enantiomers is desired) may alternatively be prepared by chiral separation of the corresponding racemic mixture.

Compounds of formula (XI) wherein the $A^1$ group (i.e. the $-(C_{1-4}$alkyl$)$-group) is substituted at the carbon atom closest to the core structure is substituted and wherein a single enantiomer is desired (or wherein an enantiomeric excess of a single enantiomers is desired) may alternatively be prepared according to any of the process as disclosed in Burk, M. J.; Gross, M. F.; Martinez, J. P. J. Am. Chem. Soc. 1995, 117, 9375; Smrcina, M.; Majer, P.; Majerová, E.;

Guerassina, T. A.; Eissenstat, M. A. Tetrahedron 1997, 53, 12867; and / or Hintermann, T.; Gademann, K.; Jaun, B. Seebach, D. Helv. Chim. Acta 1998, 81, 983

One skilled in the art will, further recognize that the processes described In Schemes 8-17 may be modified, for example, by selecting and substituting suitable starting materials and / or reagents, to yield corresponding compounds for the preparation of compounds of formula (II) and / or compounds of formula (III).

The following Examples are set forth to aid in the understanding of the invention, and are not intended and should not be construed to limit in any way the invention set forth in the claims which follow thereafter.

In the Examples which follow, some synthesis products are listed as having been isolated as a residue. It will be understood by one of ordinary skill In the art that the term "residue" does not limit the physical state in which the product was isolated and may include, for example, a solid, an oil, a foam, a gum, a syrup, and the like.

## Example 1

**3-[2-Amino-6-(2-methoxy-phenyl)-4H-quinazolin-3-ylmethyl]-*N*-cyclohexyl-*N*-methyl-benzamide (Compound # 67)**

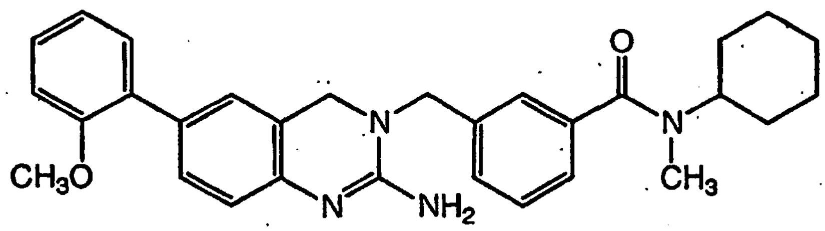

STEP A

To a flask containing 3-cyanobenzoic acid (10 g, 0.068 mol) in DCM (300 mL) was added DIEA (47 mL, 0.27 mol) and *N*-methylcyclohexylamine (13.3 mL, 0.1 mol) with stirring. The resulting solution was cooled to approximately 0°C, and then 2-chloro-1,3-dimethylimidazolium chloride (23 g, 0.14 mol) was added with stirring. After 5 minutes, the cooling bath was removed and the solution was warmed to room temperature over 4 h. The reaction mixture was quenched with 100 mL of water, transferred to a separatory funnel, shaken, and the layers were separated. The aqueous layer was extracted with DCM (2x100 mL) and the combined organics were dried over $Na_2SO_4$, filtered, concentrated to a residue, and placed under vacuum (~1 mmHg) for 1 h.

STEP B

The residue prepared in Step A above was transferred to a Parr bottle with EtOH (250 mL) and then was degassed and blanked with nitrogen before 12*N* aqueous HCl (28 mL, 0.34 mol) and 10% Pd/C (1.6 g) slurried in water were added. The resulting slurry was exposed to 50 psi of $H_2$ for 14 h at room temperature using standard Parr techniques and then degassed with nitrogen for 5 minutes and filtered through a pad of Celite®. The resulting solution was concentrated to the aqueous layer and transferred to a separatory funnel with water (100 mL) and DCM (50 mL). The layers were separated and the aqueous layer was extracted with DCM (2x25 mL). The combined organic layers were extracted with 0.5*N* HCl (25 mL), and the layers were separated. The combined aqueous layers were adjusted to pH >9 with 3*N* NaOH and extracted with DCM (5x50 mL). The combined organic layers were dried over $Na_2SO_4$, filtered, concentrated to a residue, and placed under vacuum (~1 mmHg) for 1 day.
MS *m/z* ($MH^+$) calcd 247.2, found 247.3

STEP C

To a round-bottomed flask containing the residue prepared in Step B above (6.7 g, 0.032 mol) in DCE (175 mL) was added 5-bromo-2-nitrobenzaldehyde (7.4 g, 0.032 mol). After stirring at room temperature for 30 min, NaBH $(OAc)_3$ (13.7 g, 0.065 mol) was added, and the reaction mixture was stirred at room temperature for 18 h. The reaction mixture was quenched with 1*N* NaOH (100 mL). The layers were separated, and the aqueous layer was extracted with DCM (3x25 mL). The combined organic layers were dried over $MgSO_4$, filtered, and concentrated *in vacuo* to constant weight to yield a residue which was used in the following step without further purification.

MS *m/z* ($MH^+$) calcd 460.1, found 460.4

STEP D

To a solution of the residue prepared in Step C above (27 mg, 0.059 mmol) in EtOH (0.5 mL) was added $K_2CO_3$ (12.3 mg, 0.089 mmol) in water (0.025 mL), 2-methoxyphenylboronic acid (13.5 mg, 0.089 mmol) and bis(diphenylphosphino)ferrocene dichloropalladium (4.9 mg, 0.006 mmol). The reaction mixture was irradiated (μw) at 100°C for 10 min. After cooling to room temperature, the solution was used directly in the following step.
MS *m/z* (MH) calcd 488.3, found 488.6

STEP E

To the solution prepared in Step D above was added $SnCl_2$ (56 mg, 0.30 mmol) in EtOH (0.2 mL), and the resulting solution was stirred at room temperature for 36 h. The reaction mixture was quenched with water (0.1 mL) and TEA (0.2 mL), stirred for 5 minutes, and concentrated to a residue. The residue was suspended in 1% TEA in EtOAc and loaded on a 2 g silica SPE cartridge and then eluted with 15 ml of 1% TEA in EtOAc. The eluent was concentrated *in vacuo* to yield a residue which was used directly in the next step without further purification.
MS *m/z* ($MH^+$) calcd 458.3, found 458.7

STEP F

The residue prepared in Step E above was dissolved in EtOH (0.75 mL), and cyanogen bromide (0.047 mL, 0.24 mmol, 5*M* in MeCN) was added. The resulting solution was stirred for 18 h at room temperature. The reaction mixture was quenched with 3*M* NaOH (0.1 mL), stirred for 5 minutes, and concentrated *in vacuo.* The residue was suspended in DCM:water (1 mL:0.1 mL), and the inorganic materials were removed by SLE (solid liquid extraction) cartridge. After concentration, the resulting residue was purified by preparative RP-HPLC to yield the title compound, 3-(2-amino-6-phenoxy-4H-quinazolin-3-ylmethyl)-*N*-cyclohexyl, *N*-methyl-benzamide as an oil.
MS *m/z* ($MH^+$) calcd 483.3, found 483.6

**Example 2**

**3-(2-Amino-6-phenoxy-4H-quinazolin-3-ylmethyl)-*N*-cyclohexyl-*N*-methyl-benzamide (Compound #62)**

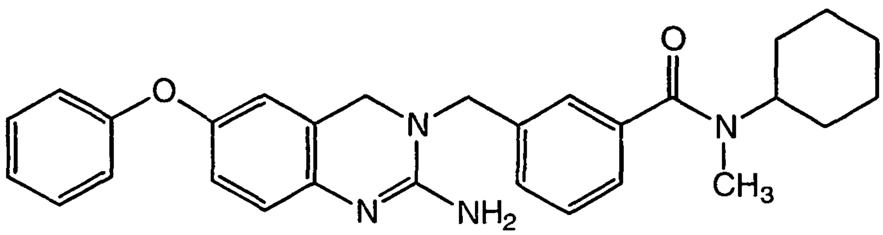

STEP A

To a solution of 3-(*tert*-butoxycarbonylamino-methyl)-benzoic acid (1.00 g, 3.98 mmol) in 21 mL of DMF was added 1,1'-carbonyldiimidazole (0.645 g, 3.98 mmol). After 1.5 h of stirring at room temperature, *N*-methylcyclohexylamine (0.98 mL, 7.50 mmol) was added. After another 2 h of stirring, 50 mL of water was added to the reaction mixture. The reaction mixture was then extracted with diethyl ether. The diethyl ether extracts were combined, washed with water (2x) and brine (2x), dried over $Na_2SO_4$. After filtration, the diethyl ether solution was concentrated *in vacuo* to yield an oil.
MS *m/z* ($MH^+$) = 347

STEP B

To a solution of the oil prepared in Step A above (0.402 g, 1.17 mmol) in $CH_2Cl_2$ (40 mL), was added TFA (4.0 mL). The reaction mixture was stirred overnight, and then 3*N* NaOH solution (35-40 mL) was added with vigorous stirring. The organic layer was separated, dried over $Na_2SO_4$, filtered, and evaporated to yield an oil.

MS *m*/*z* ($MH^+$) =247

STEP C

A solution of the oil prepared in Step B above (0.051 g, 0.21 mmol) and 2-nitro-5-phenoxybenzaldehyde (0.051 g, 0.21 mmol) in 1 mL of DCE was stirred for 10 minutes. Then $NaBH(OAc)_3$ (0.062 g, 0.29 mmol) was added. The reaction mixture was stirred overnight at room temperature, and then 3*N* NaOH solution was added with vigorous stirring. The organic layer was separated, dried over $Na_2SO_4$, filtered, and evaporated to yield a thick oil.
MS *m*/*z* ($MH^+$) = 474

STEP D

A mixture of the oil prepared in Step C above (0.049 g, 0.10 mmol) and tin(II) chloride dihydrate (0.124 g, 0.55 mmol) in 0.4 mL of EtOH was refluxed for 1 hour. The reaction mixture was cooled and saturated $NaHCO_3$ solution and EtOAc were added with thorough mixing. The organic layer was separated, dried over $Na_2SO_4$, filtered, and evaporated to yield a glassy solid.
MS *m*/*z* ($MH^+$) = 444

STEP E

A solution of the glassy solid prepared in Step D above (0.037 g, 0.084 mmol) and cyanogen bromide (0.013 g, 0.12 mmol) in 2 mL of EtOH was refluxed for 2 h. The reaction mixture was cooled and evaporated. The residue was triturated with diethyl ether and filtered to yield the title compound as a brown solid.
MS *m*/*z* ($MH^+$) = 468

**Example 3**

**3-[2-Amino-6-(3-methyl-butoxy)-4H-quinazolin-3-ylmethyl]-N-cyclohexyl-N-methyl-benzemide (Compound #517)**

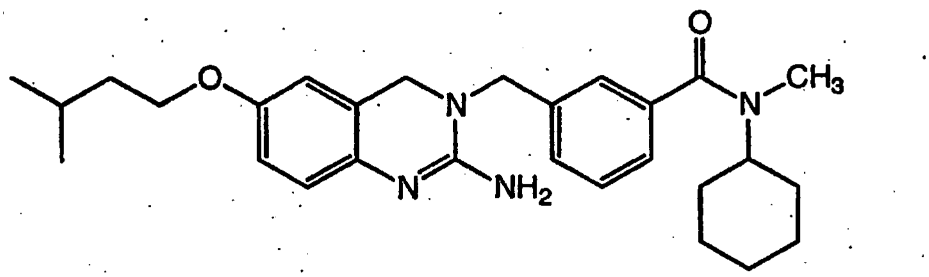

STEP A

To a reaction vessel containing isoamyl alcohol (44 mg, 0.5 mmol), triphenylphosphine (197 mg, 0.75 mmol) in THF (1 mL), and 5-hydroxy-2-nitrobenzaldehyde (125 mg, 0.75 mmol) in THF (1 mL) was added diisopropylazodicarboxylate (148 µL, 0.75 mmol) at 0°C. The recaton mixture was allowed to warm to room temperature and then was stirred at 25°C for 3 h. The solvent was removed *In vacuo,* and the resulting residue was purified by reverse-phase chromatography to yield an oil.

STEP B

To a solution of the oil isolated in Step A (62 mg, 0.26 mmol) in DCE (0.75 mL) was added 3-aminomethyl-*N*-cyclohexyl-*N*-methyl-benzamide (79 mg, 0.32 mmol) in DCE (0.75 mL) followed by addition of HOAc (15 µL). The reaction mixture was stirred at 25°C for 30 min, and then $NaBH(OAc)_3$ (93 mg, 0.44 mmol) in DMF (0.3 mL) was added. The reaction mixture was stirred at 25°C for 20 h. After quenching with $H_2O$, the solvent was removed *in vacuo* to yield crude product.

STEP C

To a solution of the crude product isolated in Step B in ethanol (2 mL) was added zinc dust (588 mg, 9 mmol) and $NH_4Cl$ (120 mg, 2.25 mmol). The reaction mixture was Irradiated (μwave) at 85°C for 10 min. The insolubles were removed by filtration and washed with ethanol (1 mL).

STEP D

To the filtrate isolated in Step C was added cyanogen bromide (0.36 mL, 1.8 mmol, 5*M* solution in $CH_3CN$). The reaction mixture was stirred at 25°C for 18 h. After quenching with 3*M* NaOH (0.4 mL), the reaction mixture was concentrated. The resulting residue was taken up in 1% TEA:$CH_2Cl_2$ (0.8 mL) and $H_2O$ (0.35 mL). The solution was absorbed onto diatomaceous earth and eluted with 1% trlethylamine/ethyl acetate. The eluate was concentrated to a residue and purified by reverse-phase chromatography to yield the title compound as an oil, as its corresponding trifluoroacetate salt.
MS *m/z* ($MH^+$) calcd 463.3, found 463.5

**Example 4**

**N-Cyclohexyl-3-[2-methoxyamino-6-(2-methoxy-phenyl)-4H-quinazolin-3-ylmethyl]-N-methyl-benzamide (Compound #605)**

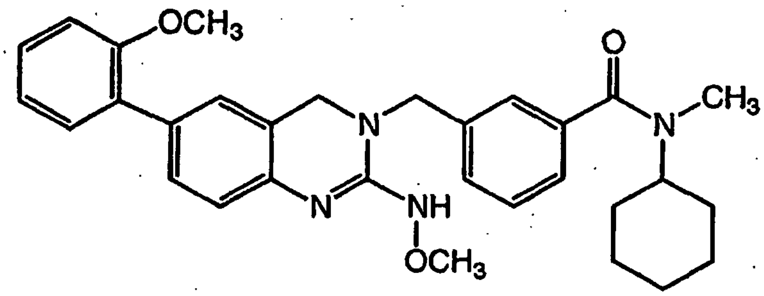

STEP A

To a round-bottomed flask containing $KNO_3$ (14.3 g, 0.14 mol) in $H_2SO_4$ (109 mL) was added 3-bromobenzaldehyde (25 g, 0.14 mol), over 15 min, at 0°C. The reaction mixture was vigorously stirred at 0°C for 20 min, poured onto crushed ice (600 mL), and extracted with DCM (3x). The combined organic layers were dried, filtered, and concentrated *in vacuo.* The residue was recrystallized from EtOAc/hexane to yield a yellow solid.

STEP B

To a flask containing 3-cyanobenzoic acid (10 g, 0.068 mol) in DCM (300 mL) was added diisopropyl ethylamine (47 mL, 0.27 mol) and *N*-methylcyclohexyl amine (13.3 mL, 0.1 mol) with stirring. The resulting solution was cooled to approximately 0°C, and then 2-chloro-1,3-dimethylimidazolium chloride (23 g, 0.14 mol) was added with stirring. After 5 minutes the cooling bath was removed and the reaction mixture was stirred toward room temperature for 4 h. The reaction was quenched with 100 mL of water, transferred to a separatory funnel, mixed, and the layers were separated. The aqueous layer was extracted with DCM (2x100 mL), and the combined organic layers were dried over $Na_2SO_4$, filtered, concentrated to a residue, and placed under vacuum (~1 mm Hg) for 1 h to yield a residue.

STEP C

The residue prepared in Step B was transferred to a Parr bottle with EtOH (250 mL) and then degassed and blanked with nitrogen before 12*N* $HCl_{(aq)}$ (28 mL, 0.34 mol) and 10% Pd/C (1.6 g) slurried in water were added. The slurry was exposed to 50 psi of $H_2$ for 14 h at room temperature using standard Parr techniques and then degassed with nitrogen for 5 minutes and filtered through a pad of Celite®. The reaction mixture was then concentrated to the aqueous layer and transferred to a separatory funnel with water (100 mL) and DCM (50 mL). The layers were separated

and the aqueous layer was extracted with DCM (2x25 mL). The combined organic layers were extracted with 0.5*N* HCl (25 mL). The layers were separated, and the combined aqueous layers were adjusted to pH >9 with 3*N* NaOH and extracted with DCM (5x50 mL). The combined organics were dried over $Na_2SO_4$, filtered, concentrated to a residue, and placed under vacuum (~1 mm Hg) for 1 day. This two-step procedure yielded a residue contaminated by diisopropyl ethyl amine but of sufficient purity for subsequent chemical transformations.
MS *m*/*z* ($MH^+$) calcd 247.2, found 247.3

STEP D

To a round-bottomed flask containing the residue isolated in Step C (6.7 g, 0.032 mol) in DCE (175 mL) was added the material isolated in Step A (7.4 g, 0.032 mol). After stirring at room temperature for 30 min, $NaBH(OAc)_3$ (13.7 g, 0.065 mol) was added, and the reaction mixture was stirred at room temperature for 18 h. The reaction was quenched with 1*N* NaOH (100 mL). The layers were separated and the aqueous layer was extracted with DCM (3x25 mL). The combined organic layers were dried over $MgSO_4$, filtered, and concentrated *in vacuo* to constant weight. The resulting residue (14.7 g) was greater than 95% pure by LC/MS analysis and used in subsequent reactions without further purification.
MS *m*/*z* ($MH^+$) calcd 460.1, found 460.4

STEP E

To a solution of the residue isolated in Step D (543 mg, 1.18 mmol) in ethyl alcohol (5 mL) was added potassium carbonate (245 mg, 1.77 mmol) in water (0.25 mL), 2-methoxyphenylboronic acid (269 mg, 1.77 mmol), and bis(diphenylphosphino)ferrocene dichloropalladium (96 mg, 0.118 mmol). The reaction mixture was irradiated ($\mu$wave) at 100°C for 10 min. After cooling to room temperature, the resulting solution was used directly in the following step.
MS *m*/*z* ($MH^+$) calcd 488.3, found 488.6

STEP F

To the above filtered solution from Step E was added $NH_4Cl$ (0.63 g, 11.8 mmol) and Zn (1.5 g, 23.6 mmol). The reaction slurry was irradiated ($\mu$wave) at 80°C for 15 min. After cooling to room temperature, the reaction mixture was filtered and concentrated. The resulting residue was purified by column chromatography on silica gel (10% EtOAc/heptane containing 1 % $Et_3N$) to 60% EtOAc/heptane containing 1% $Et_3N$) to yield a white solid.
MS *m*/*z* ($MH^+$) calcd 458.3, found 458.7

STEP G

To a round bottom flask fitted with a reflux condenser was added the solid isolated in Step F (0.122 g, 0.267 mmol) dissolved in ethanol (4 mL) and carbon disulfide (6 mL). The resulting solution was heated at reflux for 4 h, cooled to room temperature, and concentrated. The resulting residue was purified by column chromatography on silica gel (2% EtOAc/heptane to 30% EtOAc/heptane) to yield a white solid.
HRMS (ES-TOF) calcd. for $C_{30}H_{34}N_3O_2S$ *m/z* 500.2372 (M+H), found: 500.2

STEP H

To a solution of the solid isolated in Step G (42.7 mg, 0.09 mmol) in $CH_3CN$ (0.2 mL) was added $CH_3I$ (0.021 mL, 0.34 mmol). The resulting solution was stirred at room temperature for 3 h, concentrated to a residue, and dried for 1 h at <1 mmHg to yield a residue.

STEP I

To the residue isolated in Step H dissolved in MeOH (0.2 mL) was added $MeONH_2{\cdot}HCl$ (0.016 g, 0.19 mmol), $NaHCO_3$ (0.016 g, 0.19 mmol), and AgOAc (0.016 mg, 0.10 mmol). The resulting solution was stirred at 70°C for 2 h, cooled to room temperature and concentrated. The resulting residue was suspended in water (1 mL) and extracted with EtOAc (4x0.5 mL). The combined organic layers were dried ($Na_2SO_4$) and concentrated. The resulting residue was purified by preparative RP-HPLC to yield an oil, which was determined to be pure product, as well as an additional residue, which was determined to be 70% pure.
HRMS (ES-TOF) calcd. for $C_{31}H_{37}N_4O_3$ *m/z* 513.2866 (M+H), found: 513.3

**Example 5**

**4-(2-Amino-6-phenoxy-4H-quinazolin-3-yl)-piperidine-1-carboxylic acid *tert*-butyl ester (Compound #499)**

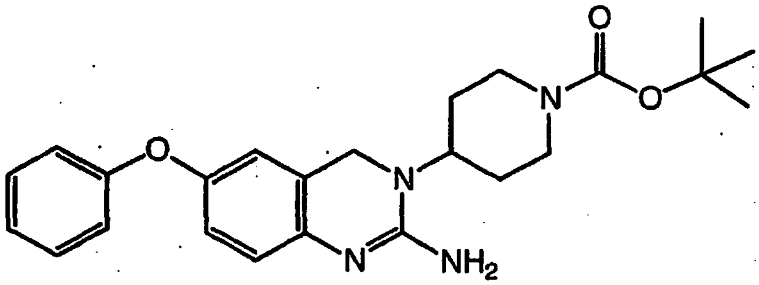

STEP A

A solution of 4-amino-1 -Boc-piperidine (0.60 g, 3.0 mmol) and 2-nitro-5-phenoxybenzaldehyde (0.48 g, 2.0 mmol) in DCE was stirred for 30 minutes. Then $NaBH(OAc)_3$ (0.64 g, 3.0 mmol) was added, and the reaction mixture was stirred overnight. The reaction mixture was quenched with 1*N* NaOH (10 mL), and the resulting solution was extracted with $CH_2Cl_2$ (2x20 mL). The $CH_2Cl_2$ extracts were combined, washed with brine, dried ($MSO_4$), and concentrated to a residue. Purification by flash chromatography (10% to 40% EtOAc-hexanes) yielded an oil.

STEP B

A solution of the oil isolated in Step A (0.67 g) and palladium on carbon (130 mg) in EtOH was hydrogenated at 40 psi in a Parr shaker for 3 h. The reaction mixture was filtered, and the filtrate was concentrated under reduced pressure to yield a residue.

STEP C

To a solution of the residue isolated in Step B (0.97 mmol) in EtOH was added cyanogen bromide (0.183 g, 1.73 mmol). The reaction mixture was stirred at room temperature overnight and then refluxed for 1 h. The reaction mixture was cooled and concentrated to yield a residue. Recrystallization of the residue from EtOH yielded the title compound as an off-white solid.
$MH^+$ 423.2
$^1H$ NMR (300 MHz, DMSO-$d_6$): $\delta$1.41 (s, 9H), 1.69 (m, 4H), 2.81 (m, 2H), 4.09 (m, 3H), 4.48 (s, 2H), 6.95-7.42 (m, 8H), 7.88 (s, 2H)

**Example 6**

**[[4-(2-Amino-6-phenoxy-4H-quinazolin-3-yl)-piperidin-1-yl]-cyclohexyl-methanone (Compound #522)**

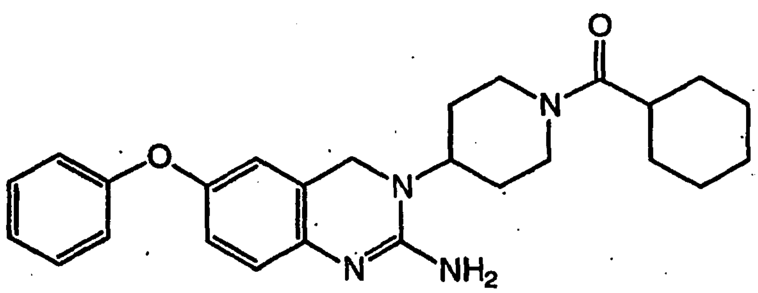

STEP A

A solution of the solid prepared as in Example 44, Step C (0.350 g) in TFA (20 mL) was stirred at room temperature for 5 h. The resulting solution was concentrated *in vacuo* to yield a residue.

STEP B

To a solution of the residue isolated in Step A (0.23 mmol) and TEA (70 mg) in dioxane (5 mL) was added cyclohexanecarbonyl chloride (34 mg). The reaction mixture was stirred overnight at room temperature. The resulting solution was concentrated *in vacuo* to yield the title compound as a yellow solid.
$MH^+$ 433.2
$^1H$ NMR (300 MHz, DMSO-$d_6$): δ1-00-2.01 (m, 15H), 3.4 (m, 2H), 4.1 (m, 2H), 4.5 (m, 3H), 6.9-7.4 (m, 8H), 7.9 (br s, 2H)

**Example 7**

**3-[1-(2-Amino-6-phenoxy-4H-quinazolin-3-yl)-propyl]-N-cyclohexyl-N-methyl-benzamide (Compound #204)**

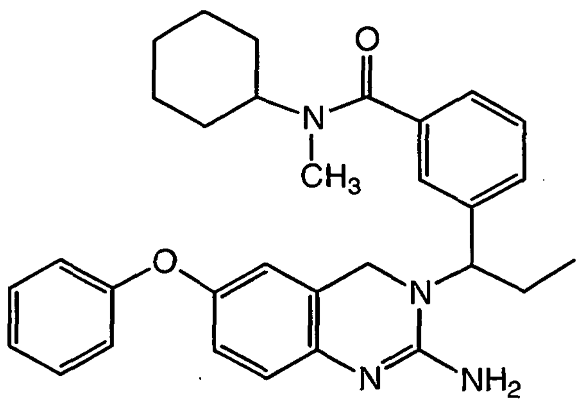

STEP A

A mixture of 3-propionylbenzoic acid (0.194 g, 1.09 mmol), HBTU (0.413 g, 1.09 mmol), and *N*-ethylmorpholine (0.38 g, 3.38 mmol) in DMF (20 mL) was stirred for 15 minutes. Then *N*-methylcyclohexylamine (0.351 g, 3.11 mmol) was added, and the reaction mixture was stirred over the weekend. With thorough mixing, 75 mL of 1*N* HCl and EtOAc were added. The layers were separated, and the organic layer was washed three times with saturated aqueous NaCl solution. The organic layer was dried ($Na_2SO_4$), filtered and yield a a thick oil.
$MH^+$ 274.2

STEP B

A solution of the oil isolated in Step A (0.34 g, 1.25 mmol) and hydroxylamine (0.062 g, 1.88 mmol, 50% solution in water) in EtOH (10 mL) was refluxed for 2.5 h. The reaction mixture was then cooled and diluted with water (75 mL). The resulting mixture was extracted with EtOAc. The organic layer was separated, dried ($Na_2SO_4$), filtered, and evaporated to yield a residue.

STEP C

A solution of the residue isolated in Step B (0.388 g, 1.35 mmol) and zinc (4.39 g) in HOAc (35 mL) was stirred overnight. After overnight stirring, the reaction mixture was filtered through Dicalite. The filtrate was evaporated to yield a residue. The residue was treated with excess 3*N* NaOH, and the resulting solution was extracted with diethyl ether. The diethyl ether extract was dried ($Na_2SO_4$), filtered, and evaporated to yield an oil.
$MH^+$ 275.2

STEP D

A solution of the oil isolated in Step C (0.187 g, 0.68 mmol) and 2-nitro-5-phenoxybenzaldehyde (0.165 g, 0.68 mmol) in DCE (3.5 mL) was stirred for 15 minutes. Then $NaBH(OAc)_3$ (0.200 g, 0.94 mmol) was added, and the reaction mixture was stirred overnight. To the reaction mixture was added 3*N* NaOH until the pH was basic. The resulting solution was mixed thoroughly with $CH_2Cl_2$. The layers were separated, and the organic layer was dried ($Na_2SO_4$), filtered, and evaporated to yield a thick residue.
$MH^+$ 502.5

STEP E

A solution of the residue isolated in Step D (0.337 g, 0.67 mmol) and stannous chloride monohydrate (0.833 g, 3.69 mmol) in EtOH (4 mL) was refluxed for 2 h. The solution was cooled, and aqueous sodium bicarbonate solution was added. EtOAc was added to this mixture, and the mixture was filtered through Dicalite. The organic layer was separated, dried ($Na_2SO_4$), filtered, and evaporated to yield a thick brown oil.
$MH^+$ 427.5

STEP F

To a solution of the oil (0.068 g, 0.14 mmol) prepared in Step E in EtOH (4 mL) was added cyanogen bromide (0.025 g, 0.24 mmol). The reaction mixture was refluxed for 2 h. After cooling, the reaction mixture was evaporated to yield a clear brown glass. The glass was triturated three times with diethyl ether. Filtration yielded the title compound as a tan solid.

**Example 8**

**2-(2-Amino-6-phenoxy-4H-quinazolin-3-ylmethyl)-N-cyclohexyl-N-methyl-isonicotinamide (Compound #508)**

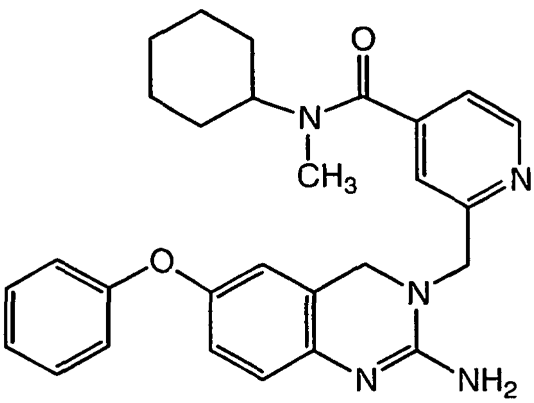

STEP A

A mixture of 2-cyano-4-pyridinecarboxylic acid (0.500 g, 3.38 mmol), HBTU (1.28 g, 3.38 mmol), and *N*-ethylmorpholine (1.21 g, 10.48 mmol) in DMF (35 mL) was stirred for 15 minutes. Then *N*-methylcyclohexylamine (1.089 g, 9.64 mmol) was added, and the reaction mixture was stirred overnight. Water (300 mL) was added to the reaction mixture. The resulting solution was extracted with EtOAc. The layers were separated, and the organic layer was washed three times with saturated aqueous NaCl solution. The organic layer was dried ($Na_2SO_4$), filtered and evaporated to yield a dark oil.
$MH^+$ 244.1

STEP B

Following the procedure described in J. Med. Chem. 1985, 28, 164, a solution of the oil isolated in Step A (0.77 g), 10% palladium on carbon (0.260 g), and concentrated HCl (0.680 mL) in EtOH (30 mL) was hydrogenated at 35 psi

on a Parr shaker. After 2 h, hydrogen uptake ceased, and the reaction was stopped. The reaction mixture was filtered and evaporated to yield a tan solid. The tan solid was slurried in $CH_2Cl_2$, and then 3*N* NaOH was added with thorough mixing. The layers were separated, and the organic layer was dried ($K_2CO_3$); filtered, and evaporated to yield a light brown oil.
$MH^+$ 248.2

STEP C

A solution of the oil isolated in Step B (0.242 g, 1.0 mmol) and 2-nitro-5-phenoxybenzaldehyde (0.238 g, 1.0 mmol) in DCE (10 mL) was stirred for 15 minutes. Then $NaBH(OAc)_3$ (0.317 g, 1.5 mmol) was added, and the reaction mixture was stirred overnight. To the reaction mixture was added 3*N* NaOH with thorough mixing. The $CH_2Cl_2$ layer was separated, and the organic layer was dried ($Na_2SO_4$), filtered, and evaporated to yield a thick orange oil.
$MH^+$ 475.2

STEP D

A solution of the oil isolated in Step C (0.484 g, 1.02 mmol) and 10% palladium on carbon (0.081 g) in EtOH (60 mL) was hydrogenated at 35 psi on a Parr shaker. After 2.5 h, the reaction mixture was filtered and evaporated to yield a thick brown oil.
$MH^+$ 445.2

STEP E

To a solution of the oil (0.404 g, 0.91 mmol) prepared in Step D in EtOH (13 mL) was added cyanogen bromide (0.551 mL, 2.76 mmol) as a 5*M* solution in MeCN. The reaction mixture was stirred at room temperature overnight and then evaporated to yield a residue. The residue was triturated several times with diethyl ether to yield a tan solid. Of this material, 100 mg was purified on flash silica gel using 90:10 $CH_2Cl_2$:(0.5*M* $NH_3$ in MeOH) to yield a residue. This residue was dissolved in $CH_2Cl_2$, and the resulting solution was washed with 3N NaOH solution. The organic layer was dried ($Na_2SO_4$) and filtered. To the filtrate was added TFA (1 mL), and the solution was evaporated to yield the title compound as a dark residue.
$MH^+$ 470.0

**Example 9**

**5-(2-Amino-6-phenoxy-4H-quinazolin-3-ylmethyl)-furan-2-carboxylic acid cyclohexyl-methyl-amide (Compound #424)**

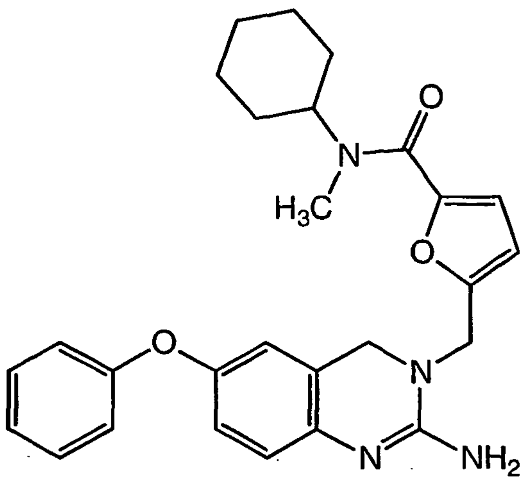

STEP A

A mixture of 5-formyl-2-furancarboxylic acid (0.153 g, 1.09 mmol), HBTU (0.413 g, 1.09 mmol), and *N*-ethylmorpholine (0.38 g, 3.38 mmol) in DMF (20 mL) was stirred for 15 minutes. Then *N*-methylcyclohexylamine (0.351 g, 3.11 mmol) was added, and the reaction mixture was stirred overnight. With thorough mixing, 75 mL of 1*N* HCl and EtOAc were added. The layers were separated, and the organic layer was washed three times with saturated aqueous NaCl solution. The organic layer was dried ($Na_2SO_4$), filtered, and evaporated to yield a thick oil.
$MH^+$ 236.1

STEP B

A solution of the oil isolated in Step A (0.257 g, 1.25 mmol) and hydroxylamine (0.062 g, 1.88 mmol, 50% solution in water) in EtOH (10 mL) was refluxed for 2 h. The reaction mixture was cooled and diluted with water (75 mL). The resulting mixture was extracted with EtOAc. The organic layer was separated, dried ($Na_2SO_4$), filtered, and evaporated to yield a dark oil.
$MH^+$ 251.1

STEP C

A solution of the oil isolated in Step B (0.235 g) and zinc (3.06 g) in HOAc (25 mL) was stirred overnight. After overnight stirring, the reaction mixture was filtered through Dicalite. The filtrate was evaporated to yield a residue. The residue was treated with 3*N* NaOH and $CH_2Cl_2$ with thorough mixing. The organic layer was separated, dried ($Na_2SO_4$), filtered, and evaporated to yield a thick residue.
$MH^+$ 237.1

STEP D

A solution of the residue: isolated in Step C (0.240 g, 1.02 mmol) and 2-nftro-5-phenoxybenzaldehyde (0.248 g, 1.02 mmol) in DCE (5.5 mL) was stirred for 15 minutes. Then $NaBH(OAc)_3$ (0.300 g, 1.42 mmol) was added, and the reaction mixture was stirred overnight. To the reaction mixture was added 3N NaOH and $CH_2Cl_2$ with thorough mixing. The layers were separated, and the organic layer was dried ($Na_2SO_4$), filtered, and evaporated to yield a thick oil.
$MH^+$ 464.2

STEP E

A solution of the oil isolated in Step D (0.500 g, 1.08 mmol) and 10% palladium on carbon (0.086 g) in EtOH (50 mL) was hydrogenated at 34 psi on a Parr shaker. After 2 h, the reaction mixture was filtered and evaporated to yield a semisolid.
$MH^+$ 434.2

STEP F

To a solution of the semisolid (0.175 g, 0.4 mmol) prepared in Step E in EtOH (5 mL) was added cyanogen bromide (0.242 mL, 1.21 mmol) as a 5*M* solution in MeCN. The reaction mixture was stirred over the weekend and then evaporated to yield a residue. The residue was triturated several times with diethyl ether and then filtered to yield a white solid. Of this material, 90 mg was purified on flash silica gel using 98:2 to 90:10 $CH_2Cl_2$:(0.5*M* $NH_3$ in MeOH) to yield a residue. This residue was dissolved in $CH_2Cl_2$, and TFA (0.5 mL) was added. The resulting solution was evaporated and triturated several times with diethyl ether to yield the title compound as a cream-colored solid.
$MH^+$ 459.4

**Example 10**

**9-(2-Amino-6-phenoxy-4H-quinazolin-3-yl)-9H-fluorene-2-carboxylic acid cyclohexyl-methyl-amide (Compound #125).**

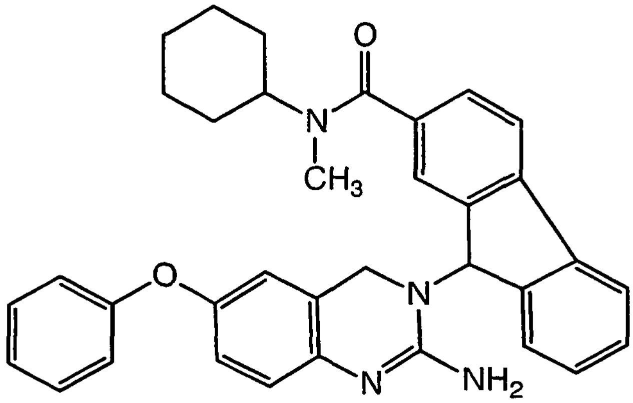

STEP A

A mixture of 9-oxo-9H-fluorene-2-carboxylic acid (0.448 g, 2.18 mmol), HBTU (0.826 g, 2.18 mmol), and *N*-ethylmorpholine (0.780 g, 6.76 mmol) in DMF (22 mL) was stirred for 30 minutes. Then *N*-methylcyclohexylamine (0.702 g, 6.22 mmol) was added, and the reaction mixture was stirred for 3 h. With thorough mixing, 50 mL of 1*N* HCl and EtOAc were added. The layers were separated, and the organic layer was washed with water and with saturated aqueous NaCl solution and then again with water and aqueous NaCl solution. The organic layer was dried ($Na_2SO_4$), filtered and evaporated to yield a solid.
$MH^+$ 319.9

STEP B

A solution of the solid isolated in Step A (0.682 g, 3.13 mmol) and hydroxylamine (0.155 g, 4.70 mmol, 50% solution in water) in EtOH (24 mL) was refluxed for 4 h. The reaction mixture was cooled and diluted with water (130 mL). The resulting mixture was extracted with EtOAc. The organic layer was separated, dried ($Na_2SO_4$), filtered, and evaporated to yield a solid.
$MH^+$ 335.13

STEP C

A solution of the solid isolated in Step B (0.562 g, 1.68 mmol) and zinc (5.47 g) in HOAc (30 mL) was stirred overnight. After overnight stirring, the reaction mixture was filtered through Filter Aid. The filtrate was evaporated to yield a residue. To the residue was added 3*N* NaOH (30 mL), and the resulting solution was extracted with diethyl ether. The diethyl ether extract was dried ($Na_2SO_4$), filtered, and evaporated to yield a thick light green residue.
$MH^+$ 321.2

STEP D

A solution of the residue isolated in Step C (0.378 g, 1.18 mmol) and 2-nitro-5-phenoxybenzaldehyde (0.287 g, 1.18 mmol) in DCE (5 mL) was stirred for 15 minutes. Then $NaBH(OAc)_a$ (0.380 g, 1.79 mmol) was added, and the reaction mixture was stirred overnight. To the reaction mixture was added 3*N* NaOH and $CH_2Cl_2$ with thorough mixing. The layers were separated, and the organic layer was washed with saturated aqueous NaCl solution, dried ($Na_2SO_4$), filtered, and evaporated to yield a clear green oil.
$MH^+$ 548.5

STEP E

A solution of the oil isolated in Step D (0.707 g, 1.29 mmol) and stannous chloride monohydrate (1.60 g, 7.10 mmol) in EtOH (6 mL) was refluxed for 3 h. The resulting solution was cooled, and excess aqueous sodium bicarbonate solution and EtOAc were added, and the resulting mixture was filtered through Filter Ald. The organic layer was separated, dried ($Na_2SO_4$) filtered, and evaporated to yield a thick clear residue.
$MH^+$ 518.3

STEP F

To a solution of the residue (0.450 g, 0.87 mmol) prepared in Step E in EtOH (4 mL) was added cyanogen bromide (0.136 g, 1.29 mmol). The reaction mixture was refluxed for 2.5 h. After cooling, the reaction mixture was evaporated to yield a thick dark residue. This residue was triturated five times with diethyl ether. Filtration yielded a tan solid. Of this material, 0.200 g was purified on a Gilson HPLC to yield the title compound as a white solid.
$MH^+$ 543.6

**Example 11**

**5-[1-(2-Amino-6-phenoxy-4H-quinazolin-3-yl)-2-phenyl-ethyl]-oxazole-4-carboxylic acid cyclohexylamide (Compound #606)**

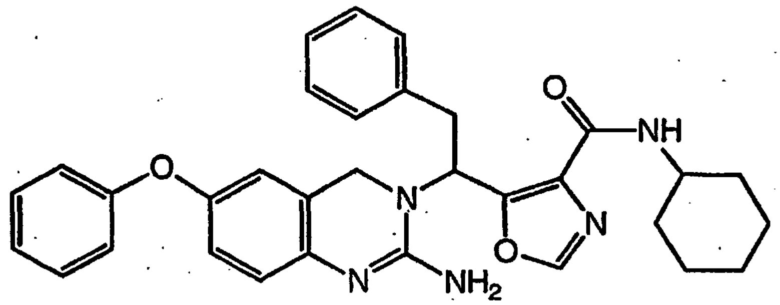

STEP A

To a cold (0°C) mixture of DL-N-Cbz-phenylalanine (12.02 g, 40.0 mmol) and potassium carbonate sesquihydrate (26.50 g, 160 mmol) in anhydrous DMF was added diphenylphosphorylazide (13.5 mL, 62.6 mmol) and methyl isocyanoacetate (7.5 mL, 82.5 mmol). The resulting mixture was stirred at room temperature for 96 h and then was diluted with $H_2O$ (600 mL). The precipitated solid was collected by filtration. The crude solid was dissolved in $CHCl_3$ (200 mL) and washed with aqueous NaCl. The organic solution was dried ($Na_2SO_4$), filtered, and evaporated to yield a solid. The solid was carried on to the next step without further manipulation.
$MH^+ = 381$

STEP B

To a cold (0°C) solution of the solid isolated in Step A (7.60 g, 20.0 mmol) in 2:1 THF:$H_2O$ (300 mL) was added LiOH (526.3 mg, 21.9 mmol). The mixture was stirred for 18 h, acidified to about pH 3 with aqueous citric acid, and extracted into $CHCl_3$ (2x150 mL). The organic solution was dried ($Na_2SO_4$), filtered, and concentrated to yield a white amorphous solid that was carried on to the next step without further purification.

STEP C

To a cold (0°C) mixture of the solid isolated in Step B (1.8339 g, 5.00 mmol) and *N,N*-diisopropylethylamine (4.4 mL, 25.2 mmol) in DMF (12 mL) was added diphenylphosphorylazide (1.3 mL, 6.03 mmol) and cyclohexylamine (0.74 mL, 6.47 mmol). The reaction mixture was stirred for 20 h at room temperature and then was diluted with aqueous NaCl and extracted into $CHCl_3$ (2x100 mL). The organic solution was washed with $H_2O$ (5x100 mL), dried ($Na_2SO_4$), filtered, and concentrated to a residue. The residue was purified by column chromatography (silica gel, 3% MeOH in $CHCl_3$). Trituration of the resulting gum with $Et_2O$ yielded a white solid.

STEP D

A mixture of the solid isolated in Step C (1.6532 g, 3.70 mmol) and 10% Pd/C (332.4 mg) in MeOH (120 mL) was shaken under 55 psi of hydrogen gas at room temperature for 5 h. The reaction mixture was filtered through a bed of Celite®. The filter cake was rinsed with MeOH (50 mL). The filtrate was concentrated and the residue was purified by column chromatography (silica gel, 6% MeOH in $CHCl_3$) to yield a light yellow oil.

STEP E

To a solution of 5-phenoxy-2-nitrobenzaldehyde (267.0 mg, 1.10 mmol) and the oil isolated in Step D (381.0 mg, 1.22 mmol) in 1,2-dichloroethane was added sodium triacetoxyborohydride (362.1 mg, 1.71 mmol). The reaction mixture was stirred at room temperature for 18 h and then quenched with aqueous $NaHCO_3$ and extracted into $CHCl_3$ (2x40 mL). The organic solution was dried ($Na_2SO_4$), filtered, and concentrated. The residue was purified by column chromatography (silica gel, 3% MeOH in $CHCl_3$) to yield a light yellow gum.

STEP F

A mixture of the gum isolated in Step E (532.6 mg, 0.970 mmol) and 10% Pd/C (110.4 mg) in EtOH (70 mL) was shaken under 55 psi of hydrogen gas at room temperature for 4 h. The reaction mixture was filtered through a bed of Celite®. The filter cake was rinsed with MeOH (50 mL). The filtrate was concentrated and the residue was purified by column chromatography (silica gel, 2% MeOH in $CHCl_3$) to yield a flaky brown solid.

STEP G

To a solution of the solid isolated in Step F (304.1 mg, 0.596 mmol) in EtOH (10 mL) was added cyanogen bromide (83.1 mg, 0.784 mmol). The resulting mixture was stirred at room temperature for 1.5 d, refluxed for 1 h, and then concentrated to a residue. The residue was purified by preparative thin layer chromatography (5 tapered silica gel plates; 90:10:1 $CHCl_3$:MeOH:concentrated $NH_4OH$) to yield the title compound as a free base.

The free base was dissolved in $CHCl_3$ (3 mL), acidified with 1 N HCl (0.6 mL) In $Et_2O$ and further diluted with $Et_2O$ (75 mL). The HCl salt was collected by filtration and dried under vacuum at room temperature to yield the title compound as an amorphous solid, as its HCl saft.

$MH^+$ = 536

$^1H$ NMR (300 MHz, DMSO-$d_6$) δ1.10-1.80 (m, 10H), 3.30-3.60 (m, 2H), 3.65-3.85 (m, 1H), 4.25-4.35 (m, 1H), 5.05-5.15 (m, 1H), 5.90-6.10 (m, 1H), 6.90-7.10 (m, 4H), 7.15-7.30 (m, 7H), 7.50-7.60 (m, 2H), 8.35-8.50 (br s, 2H), 8.65-8.75 (m, 2H), 11.1 (s, 1H)

**Example 12**

**BACE ASSAY-1**

The following standards are reagents were used in this assay: Sodium Citrate trisodium salt dihydrate (Sigma), Citric Acid monohydrate (Merck), PEG8000 (Sigma), MCA-substrate (Bachem), ß-secretase (BACE) (Panvera), 96-well plate zwart (Costar (Elscolab)), StatVal (Bachem).

The following standard assay buffer solution was prepared and used In this assay: 0.05 M, pH 5.0 mixture of sodium citrate trisodium salt dihydrate (9.56g), citric acid monohydrate (3.68g) and PEG8000 (0.50g).

The MCA-substrate stock solution was prepared by mixing 10 mg MCA-substrate with 5 mL DMSO for a final solution concentration of 2.0 mg/mL The substrate work solution was prepared by mixing 0.1 mL substrate in 1.90 mL assay buffer for a final concentration of 0.05 mM. The β-secretase (BACE) work solution was prepared by mixing 8μL BACE in 2 mL assay buffer for a final concentration of 10μg/mL.

Test compounds were dissolved in DMSO at various concentrations in the range of $3.3x10^{-3}$ M to $1.5x10^{-6}$ M.

The screen procedure for this assay was as follows. 60 μL of assay buffer was pippeted into each well of a 96-well plate. To each well was then pipetted 1μL of test compound at the selected concentration. To each well was then added 20μL of the β-secretase work solution and 20μL of the MCA-substrate stock solution. Each well was mixed for a few second and the To measured with fluoroscan Ex320/Em405. The plates were then incubated for 1 hour at room temperature and the T60 measured with fluoroscan Ex320/Em405.

The procedure for the blank was as follows. 80 μL of assay buffer was pipetted into each well to be used as a blank control. To each well was then added 1 μL of DMSO and 20 μL of MCA-substrate solution in each well. The To was measured with fluoroscan Ex320/Em405, the plate was incubated for 1 hour at room temperature and the T60 was then measured with fluoroscan Ex320/Em405.

The procedure for the positive control was as follows. 60 μL of assay buffer was pipetted into each well to be used as a positive control. To each well was then added 1 μL of DMSO, 20 μL of BACE work solution and 20 μL of MCA-substrate stock solution. The To was measured with fluoroscan Ex320/Em405, the plate was incubated for 1 hour at room temperature and the T60 was then measured with fluoroscan Ex320/Em405.

For test compounds, measured at multiple concentrations, the measured To and $T_{60}$ values were used to calculate an $IC_{50}$ value using Graphpad Software (or PIR).

**Example 13**

**BACE ASSAY-2**

The following reagents were used in this assay: sodium acetate, PEG8000 (Sigma), DMSO, HEPES, FS 1 substrate [R(AedensE)EEVNLDAEFK-(DabcylK)R], β-secretase (BACE) (Panvera), and 96-well plate (HE microplate, Molecular Devices).

The following assay buffers were prepared and used in this assay: (1) enzyme, assay buffer (0.05 M sodium acetate, pH5, 0.1% PEG8000 (w/v)), (2) substrate assay buffer (0.05 M sodium acetate, pH5), and (3) compound vehicle (30% DMSO in 50 mM HEPES, pH 7.4).

The FS1-substrate stock solution was prepared in DMSO as a 10 mg/mL solution. The FS1-substrate working solution was prepared by diluting the 10 mg/mL stock solution with substrate assay buffer to a final concentration of 300 μg/mL The β-secretase (BACE) working solution was prepared by diluting a 0.83 mg/mL BACE stock solution with enzyme assay buffer to a final concentration of 4μg/mL.

Test compounds were dissolved in DMSO at 10mM. Compounds were further diluted in compound vehicle to various concentrations in the range of 675 μM to 13.5nM (13.5 x final compound concentration in Ki plate).

The procedure for this assay was as follows: 15 μL of BACE working solution was pipetted into each well of a 96-well plate. To each well was then pipetted 2 μL of test compound at the selected concentration. Test compound and BACE were then mixed with a pipettor and incubated for 20 min at room temperature. To each well was then added 10μL of the FS1 substrate working solution. The fluorescence for each well was then measured on a Polarstar fluorometer (Ex 390nm/Em 520 nm) for 20 min at room temperature, reading fluorescence at 1 min intervals.

The procedure for the positive control was as follows:15 μL of BACE working solution was pipetted into each well to be used as a positive control. To each well was then pipetted 2 μL of vehicle. Vehicle and BACE were then mixed with a pipettor and incubated for 20 min at room temperature. To each well was then added 10μL of the FS1 substrate working solution. The fluorescence was then measured on a Polarstar fluorometer (Ex 390nm/Em 520 nm) for 20 min at room temperature, reading fluorescence at 1 min intervals. '

For test compounds, measured at multiple concentrations, the measured To and $T_{60}$ values were used to calculate an $IC_{50}$ value using Graphpad Software (or PIR). For test compounds, $K_i$ inhibition was determined as follows: For each compound concentration and positive control, rate of cleavage of substrate ($V_i$ , where i = compound concentration in μM) was determined as Δ Fluorescence/Δ time (min). Cleavage rates ($V_i$) were plotted as a function of inhibitor concentration in μM [I]. The $K_i$ was then determined by fitting the following equation to the graph of [I] vs $V_i$

$$Y=aV_{max}/(50+24*(1+X/K_i)),$$

where 50 = substrate concentration (μM) and 24 = $K_m$ of substrate (μM).

Representative compounds of the present invention were tested according to procedures described in Example 12 and 13 above with measured $IC_{50}$ and $K_i$ values as listed in Table 15 below. The procedure used to determine a particular value is defined within the header row in parentheses (for example (158) indicates that the $IC_{50}$ values in that column were determined using the procedure described in Example 12).

**Table 15: BACE *in vitro* Assay Results**

| ID No. | $IC_{50}$ μM (158) | $IC_{50}$ μM (159) | Ki μM (159) |
|---|---|---|---|
| 62 | | 0.52 | 0.14 |
| 62 | 0.055 | 0.37 | 0.18 |
| 89 | | 0.4 | |
| 98 | 0.20 | 2.3 | 1.1 |
| | | | 0.23 |
| 110 | 0.053 | 1.239 | 0.4 |
| 136 | | | 0.5 |
| 138 | 0.023 | | 0.06 |
| 158 | | | 0.81 |

(continued)

| ID No. | $IC_{50}$ μM (158) | $IC_{50}$ μM (159) | Ki μM (159) |
|---|---|---|---|
| 159 | 0.89 | | 0.28 |
| 160 | 0.54 | | 0.12 |
| 164 | 0.11 | | 0.1 |
| 165 | 0.19 | | 0.28 |
| 167 | | | 0.03 |
| 167 | 0.014 | | 0.05 |
| 169 | 0.058 | | 0.08 |
| 170 | | | 0.77 |
| 173 | | | 0.47 |
| 174 | 0.085 | | 0.07 |
| 176 | | | 0.2 |
| 192 | | | 0.22 |
| 204 | 0.062 | | 0.23 |
| 209 | | | 0.29 |
| 211 | | | 0.69 |
| 231 | | | 3.8 |
| 259 | 0.25 | | 0.25 |
| 260 | 0.15 | | 0.21 |
| 261 | | | 0.42 |
| 262 | | | 0.66 |
| 263 | | | 0.77 |
| 264 | | | 0.78 |
| 265 | | | 1.1 |
| 268 | | | 0.65 |
| 292 | | | 1.0 |
| 293 | | | 1.1 |
| 322 | 0.14 | | 0.29 |
| 324 | | | 0.52 |
| 325 | 0.089 | | 0.1 |
| 326 | | | 0.42 |
| 328 | | | 0.33 |
| 329 | 0.012 | | 0.02 |
| 330 | 0.048 | | 0.21 |
| 331 | 0.089 | | 0.19 |
| 333 | | | 0.49 |
| 334 | | | 0.068 |
| 353 | 0.35 | | |
| 362 | 0.53 | | |

(continued)

| ID No. | $IC_{50}$ μM (158) | $IC_{50}$ μM (159) | Ki μM (159) |
|---|---|---|---|
| 376 | 0.17 | | |
| 384 | 0.13 | | |
| 396 | 0.20 | | |
| 404 | | | 0.78 |
| 418 | | | 0.035 |
| 419 | | | 0.29 |
| 424 | | | 0.40 |
| 432 | | | 0.86 |
| 476 | 0.067 | | 0.28 |
| 508 | | | 0.15 |
| 534 | | | 0.18 |
| 605 | | | 0.54 |
| 800 | 17 | | |

**Example 14**

**BACE FS1 % Inhibition Assay**

The following reagents were used in this assay: sodium acetate, PEG8000 (Sigma), DMSO, HEPES, FS1 substrate [R(AedensE)EEVNLDAEFK-(DabcylK)R], β-secretase (BACE) (Panvera), and 96-well plate (HE microplate, Molecular Devices).

The following assay buffers were prepared and used in this assay: (1) enzyme assay buffer (0.05 M sodium acetate, pH, 0.1% PEG8000 (w/v)), (2) substrate assay buffer (0.05 M sodium acetate, pH5), and (3) compound vehicle (30% DMSO in 50mM HEPES, pH 7.4).

The FS1-substrate stock solution was prepared in DMSO as a 10 mg/mL solution. The FS1-substrate working solution was prepared by diluting the 10 mg/mL stock solution with substrate assay buffer to a final concentration of 300 μg/mL. The β-secretase (BACE) working solution was prepared by diluting a 0.83 mg/mL BACE stock solution with enzyme assay buffer to a final concentration of 4μg/mL.

Test compounds were dissolved in DMSO to 10mM. Compounds were further diluted in vehicle to various concentrations in the range of 405 μM to 4.05 μM (13.5 x final compound concentration in screening plate).

The screening procedure for this assay was as follows: 15 μL of BACE working solution was pipetted into each well of a 96-well plate. To each well was then pipetted 2 μL of test compound at the selected concentration. Test compound and BACE were then mixed with a pipettor and incubated for 20 min at room temperature. To each well was then added 10μL of the FS1 substrate working solution. The plates were then incubated for 1 hour at room temperature. The fluorescence for each well was then measured on an LJL analyst (Ex 360nm /Em 530 nm).

The procedure for the blank was as follows. 15 μL of assay buffer was pipetted into each well to be used as a blank control. To each well was then added 2 μL of vehicle and 10 μL of FS1-substrate working solution. The plates were then Incubated for 1 hour at room temperature. The fluorescence was measured on an LJL analyst (Ex 360nm /Em 530 nm).

The procedure for the positive control was as follows: 15 μL of BACE working solution was pipetted into each well to be used as a positive control. To each well was then pipetted 2 μL of vehicle. Vehicle and BACE were then mixed with a pipettor and incubated for 20 min at room temperature. To each well was then added 10μL of the FS1 substrate working solution. The plates were then incubated for 1 hour at room temperature. The fluorescence (FI) was then measured on an LJL analyst (Ex 360nm /Em 530 nm).

For test compounds, % inhibition was determined at each concentration as follows:

$$\%\ \text{Inhibition} = \frac{[\text{FI(compound)} - \text{FI(negative control)}]}{[\text{FI(positive control)} - \text{FI(negative control)}]}$$

% Inhibition values of less than 30% were indistinguishable from control are listed as ≤30% in the Table below. % Inhibition values greater than 100% were Indistinguishable from 100% within the error of the measurement.

Representative compounds of the present invention were tested according to procedure described in Example 14 above with results as listed in Table 16 below.

**Table 16: % Inhibition (FS)**

| ID No. | 30 μM | 10 μM | 3 μM | 1 μM | 0.3 μM |
|---|---|---|---|---|---|
| 98 | | | 114 | 93 | 49 |
| 110 | | | 84 | 30 | <30 |
| 118 | | 91 | 48 | | |
| 120 | | 34 | <30 | | |
| 124 | | 67 | 56 | | |
| 125 | | 80 | 42 | | |
| 132 | | 118 | 54 | | |
| 136 | | 133 | 85 | | |
| 138 | | | 114 | 113 | 98 |
| 145 | | 37 | <30 | | |
| 146 | | 39 | <30 | | |
| 147 | | 71 | <30 | | |
| 158 | | | 60 | <30 | <30 |
| 158 | | | 79 | 53 | <30 |
| 159 | | | 92 | 65 | 34 |
| 160 | | | 102 | 78 | 51 |
| 161 | | | <30 | <30 | <30 |
| 162 | | | <30 | <30 | <30 |
| 163 | | | <30 | 33 | <30 |
| 164 | | | 109 | 96 | 67 |
| 165 | | | 101 | 72 | <30 |
| 166 | | | 74 | 37 | <30 |
| 167 | | | 113 | 112 | 100 |
| 167 | | | 118 | 110 | 91 |
| 168 | | | <30 | <30 | <30 |
| 169 | | | 109 | 104 | 80 |
| 170 | | | 67 | 49 | <30 |
| 171 | | | 42 | 31 | <30 |
| 172 | | | <30 | <30 | <30 |
| 173 | | | 76 | 53 | <30 |
| 174 | | | 111 | 106 | 85 |
| 175 | | | 37 | <30 | <30 |
| 176 | | | 111 | 97 | 66 |
| 187 | | | 39 | <30 | <30 |

(continued)

| ID No. | 30 μM | 10 μM | 3 μM | 1 μM | 0.3 μM |
|---|---|---|---|---|---|
| 188 | | | <30 | <30 | <30 |
| 192 | | | 110 | 92 | 38 |
| 193 | | | 67 | 34 | <30 |
| 197 | | | 32 | <30 | <30 |
| 198 | | | <30 | <30 | <30 |
| 204 | | | 107 | 66 | <30 |
| 204 | | | 115 | 97 | 54 |
| 207 | | | 108 | 90 | 48 |
| 208 | | | 67 | <30 | <30 |
| 209 | | | 103 | 58 | <30 |
| 210 | | | <30 | <30 | <30 |
| 211 | | | 68 | <30 | <30 |
| 212 | | | 32 | <30 | <30 |
| 213 | | | <30 | <30 | <30 |
| 222 | | | <30 | <30 | <30 |
| 223 | | | <30 | <30 | <30 |
| 224 | | | <30 | <30 | <30 |
| 228 | | | 40 | 49 | <30 |
| 229 | | | <30 | 35 | <30 |
| 230 | | | <30 | 33 | <30 |
| 231 | | | 75 | 71 | <30 |
| 232 | | | 31 | <30 | <30 |
| 233 | | | 36 | 32 | <30 |
| 234 | | | 43 | 57 | <30 |
| 235 | | | <30 | 46 | <30 |
| | | | | | |
| 236 | | | 49 | 37 | <30 |
| 259 | | | 97 | 66 | <30 |
| 260 | | | 112 | 86 | 53 |
| 261 | | | 110 | 87 | 51 |
| 262 | | | 95 | 63 | 39 |
| 263 | | | 92 | 60 | 20 |
| 264 | | | 92 | 63 | 26 |
| 265 | | | 92 | 55 | <30 |
| 266 | | | <30 | 34 | <30 |
| 267 | | | 38 | 39 | <30 |
| 268 | | | 71 | <30 | <30 |
| 286 | | | 36 | <30 | <30 |

(continued)

| ID No. | 30 μM | 10 μM | 3 μM | 1 μM | 0.3 μM |
|---|---|---|---|---|---|
| 290 | | | <30 | <30 | <30 |
| 292 | | | 83 | 49 | <30 |
| 293 | | | 81 | 44 | <30 |
| 306 | | | 46 | <30 | <30 |
| 307 | | | <30 | <30 | <30 |
| 310 | | | 57 | <30 | <30 |
| | | | | | |
| 315 | | | 33 | <30 | <30 |
| 316 | | | <30 | <30 | <30 |
| 320 | | | <30 | <30 | -31 |
| 321 | | | <30 | <30 | -33 |
| 322 | | | 95 | <30 | <30 |
| 323 | | | 45 | <30 | <30 |
| 324 | | | 87 | 55 | <30 |
| 325 | | | 110 | 86 | <30 |
| 326 | | | 79 | 35 | <30 |
| 327 | | | 48 | <30 | <30 |
| 328 | | | 92 | 61 | <30 |
| 329 | | | 118 | 109 | 92 |
| 330 | | | 114 | 66 | <30 |
| 331 | | | 108 | 60 | <30 |
| 332 | | | <30 | <30 | <30 |
| 333 | | | 78 | <30 | <30 |
| 334 | | | 118 | 103 | 74 |
| 395 | | | 118 | 107 | 69 |
| 400 | | | 94 | <30 | <30 |
| 401 | | | 52 | <30 | <30 |
| 402 | | | 99 | 53 | <30 |
| 403 | | | 66 | <30 | <30 |
| 404 | | | 67 | 32 | <30 |
| 405 | | | 118 | 106 | 43 |
| 406 | | | 50 | <30 | <30 |
| 407 | | | 48 | <30 | <30 |
| 408 | | | 95 | 34 | <30 |
| 409 | | | 72 | <30 | <30 |
| 410 | | | 50 | 39 | <30 |
| 411 | | | 55 | <30 | <30 |
| 412 | | | 49 | <30 | <30 |

(continued)

| ID No. | 30 μM | 10 μM | 3 μM | 1 μM | 0.3 μM |
|---|---|---|---|---|---|
| 413 | | | 53 | <30 | <30 |
| 414 | | | 27 | <30 | <30 |
| 415 | | | 63 | <30 | <30 |
| 416 | | | -3 | <30 | <30 |
| 417 | | | 78 | <30 | <30 |
| 418 | | | 119 | 89 | 54 |
| 419 | | | 109 | 64 | 38 |
| 424 | | | 94 | 50 | <30 |
| 432 | | | 84 | 41 | <30 |
| 433 | | | 88 | 33 | <30 |
| 434 | | | <30 | <30 | <30 |
| 435 | | | 85 | <30 | <30 |
| 436 | | | 50 | 37 | <30 |
| 437 | | | 38 | <30 | <30 |
| 438 | | | <30 | <30 | <30 |
| 439 | | | <30 | <30 | <30 |
| 440 | | | 72 | 35 | <30 |
| 441 | | | 48 | 29 | <30 |
| 442 | | | 101 | 58 | <30 |
| 443 | | | 67 | 39 | <30 |
| 463 | | | <30 | <30 | <30 |
| 467 | | | <30 | <30 | <30 |
| 468 | | | <30 | <30 | <30 |
| 469 | | | <30 | <30 | <30 |
| 470 | | | 31 | <30 | <30 |
| 471 | | | 38 | <30 | <30 |
| 472 | | | <30 | <30 | <30 |
| 473 | | | <30 | <30 | <30 |
| 474 | | | <30 | <30 | <30 |
| 475 | | | <30 | <30 | <30 |
| 476 | | | 102 | 66 | 19.86 |
| 477 | | | <30 | <30 | <30 |
| 478 | | | 46 | <30 | <30 |
| 479 | | | 81 | <30 | <30 |
| 480 | | | 38 | <30 | <30 |
| 481 | | | 32 | <30 | <30 |
| 482 | | | 43 | <30 | <30 |
| 483 | | | <30 | <30 | <30 |

(continued)

| ID No. | 30 μM | 10 μM | 3 μM | 1 μM | 0.3 μM |
|---|---|---|---|---|---|
| 499 | | | <30 | <30 | <30 |
| 503 | | | 90 | <30 | <30 |
| 512 | | | 51 | <30 | <30 |
| 513 | | | <30 | <30 | <30 |
| 514 | | | <30 | <30 | <30 |
| 516 | | | <30 | <30 | <30 |
| 517 | | | 40 | <30 | <30 |
| 518 | | | <30 | <30 | <30 |
| 519 | | | <30 | <30 | <30 |
| 520 | | | 33 | <30 | <30 |
| 521 | | | <30 | <30 | <30 |
| 522 | | | 79 | <30 | <30 |
| 523 | | | 58 | <30 | <30 |
| 524 | | | 44 | <30 | <30 |
| 525 | | | 70 | <30 | <30 |
| 526 | | | 98 | 36 | <30 |
| 527 | | | 51 | <30 | <30 |
| 534 | | | 126 | 103 | 54 |
| 549 | | | 71 | 30 | <30 |
| 550 | | | 67 | <30 | <30 |
| 551 | | | <30 | <30 | <30 |
| 552 | | | 36 | <30 | <30 |
| 559 | | | <30 | <30 | <30 |
| 605 | | | 111 | 72 | 47 |
| 606 | | | 56 | <30 | <30 |
| 607 | | | 123 | 116 | 94 |
| 611 | | | <30 | 36 | <30 |
| 612 | | | <30 | 34 | <30 |
| 613 | | | <30 | 31 | 36 |
| 640 | | | <30 | <30 | <30 |
| 641 | | | 48 | <30 | <30 |

Example 15

BACE % Inhibition Assay

The following reagents were used in this assay: sodium acetate, PEG8000 (Sigma), DMSO, HEPES, (Aedens)-EVNLDAEF-(Dabcyl K-amide) substrate, β-secretase (BACE) (Panvera), and 96-well plate (HE microplate, Molecular Devices).

The following assay buffers were prepared and used in this assay: (1) enzyme assay buffer (0.05 M sodium acetate, pH5, 0.1 % PEG8000 (w/v)), (2) substrate assay buffer (0.05 M sodium acetate, pH5), and (3) compound vehicle

(30% DMSO in 50mM HEPES, pH 7.4).

The substrate stock solution was prepared in DMSO as a 10 mg/mL solution. The substrate working solution was prepared by diluting the 10 mg/mL stock solution with substrate assay buffer to a final concentration of 300 μg/mL. The β-secretase (BACE) working solution was prepared by diluting a 0.83 mg/mL BACE stock solution with enzyme assay buffer to a final concentration of 4μg/mL.

Test compounds were dissolved in DMSO to 10mM. Compounds were further diluted in vehicle to various concentrations in the range of 405 μM to 4.05 μM (13.5 x final compound concentration in screening plate).

The screening procedure for this assay was as follows: 15 μL of BACE working solution was pipetted into each well of a 96-well plate. To each well was then pipetted 2 μL of test compound at the selected concentration. Test compound and BACE were then mixed with a pipettor and incubated for 20 min at room temperature. To each well was then added 10μL of the substrate working solution. The plates were then incubated for 1 hour at room temperature. The fluorescence for each well was then measured on an LJL analyst (Ex 360nm /Em 530 nm).

The procedure for the blank was as follows. 15 μL of assay buffer was pipetted into each well to be used as a blank control. To each well was then added 2 μL of vehicle and 10 μL of substrate working solution. The plates were then incubated for 1 hour at room temperature. The fluorescence was measured on an LJL analyst (Ex 360nm /Em 530 nm).

The procedure for the positive control was as follows: 15 μl of BACE working solution was pipetted into each well to be used as a positive control. To each well was then pipetted 2 μL of vehicle. Vehicle and BACE were then mixed with a pipettor and incubated for 20 min at room temperature. To each well was then added 10μL of the substrate working solution. The plates were then Incubated for 1 hour at room temperature. The fluorescence (FI) was then measured on an LJL analyst (Ex 360nm /Em 530 nm).

For test compounds, % Inhibition was determined at each concentration as follows:

$$\% \text{ Inhibition} = \frac{[\text{Fl(compound)} - \text{Fl(negative control)}]}{[\text{Fl(positive control)} - \text{Fl(negative control)}]}$$

% Inhibition values of less than 30% were indistinguishable from control are listed as ≤30% in the Table below.

Representative compounds of the present Invention were tested according to procedure described in Example 15 above with results as listed in Table 17 below. The % error in the measurements was ±10%.

**TABLE 17: % Inhibition**

| ID No. | 30 μM | 10 μM | 3 μM | 1 μM | 0.3 μM |
|---|---|---|---|---|---|
| 62 | | 82 | 62 | | |
| 62 | | 82 | 73 | | |
| 72 | | <30 | <30 | | |
| 77 | | <30 | <30 | | |
| 81 | | <30 | <30 | | |
| 87 | | <30 | <30 | | |
| 89 | | 81 | 77 | | |
| 93 | | <30 | <30 | | |
| 94 | | <30 | <30 | | |
| 97 | | 53 | <30 | | |
| 98 | | 99 | 76 | | |
| 100 | | <30 | <30 | | |
| 110 | | 96 | 75 | | |
| 336 | | | <30 | <30 | <30 |
| 337 | | | <30 | <30 | <30 |
| 338 | | | <30 | <30 | <30 |

(continued)

| ID No. | 30 μM | 10 μM | 3 μM | 1 μM | 0.3 μM |
|---|---|---|---|---|---|
| 339 | | | <30 | <30 | <30 |

**Example 16**

**BACE Assay (CEREP)**

This assay was run by CEREP (Catalog Ref. 761-B, Reffered to SOP No. 1C131; ERMOLIEFF, J., LOY, J.A., KOELSCH, G. and TANG, J., Proteolytic activation of recombinant pro-memapsin 2 (pro-β-secretase) studied with new fluorogenic substrates, Biochemistry, (2000) Vol. 39, p.12450).

More specifically the assay, run at 50μL in a 96 well plate, evaluated the effect of test compound on the activity of the human BACE-1 quantified by measuring the formation of Mca-S-E-V-N-L-$NH_2$ from Mca-S-E-V-N-L-D-A-E-F-R-K(Dnp)-R-R-$NH_2$, using a recombinant enzyme.

The test compound, reference compound or water (control) were added to a buffer containing 0.09 M sodium acetate (pH 4.5) and 0.25 μg BACE-1. Compound Interference with the fluorimetric detection method due to autofluorescence was then checked by measurements at the wavelengths defined to evaluate the enzyme activity. Thereafter, the reaction was initiated by adding 7.5 μM of the substrate Mca-S-E-V-N-L-D-A-E-F-R-K(Dnp)-R-R-$NH_2$ and the mixture was incubated for 60 min at 37°C. For control basal measurement, the substrate was omitted from the reaction mixture. Immediately after the incubation, the fluorescence intensity emitted by the reaction product Mca-S-E-V-N-L-$NH_2$ was measured at λex=320 nm and λem=405 nm using a microplate reader (Ultra, Tecan). The standard inhibitory reference compound was OM99-2, which was tested in each experiment at several concentrations to obtain an inhibition curve from which its $IC_{50}$ value was calculated.

Representative compounds of the present invention were tested according to procedure described in Example 16 above with results as listed in Table 18 below.

**TABLE 18: % Inhibition and $IC_{50}$**

| ID No. | 1 μM | 0.3 μM | $IC_{50}$ (μM) |
|---|---|---|---|
| 62 | | | 0.38 |

## Claims

**1.** A compound of formula (II)

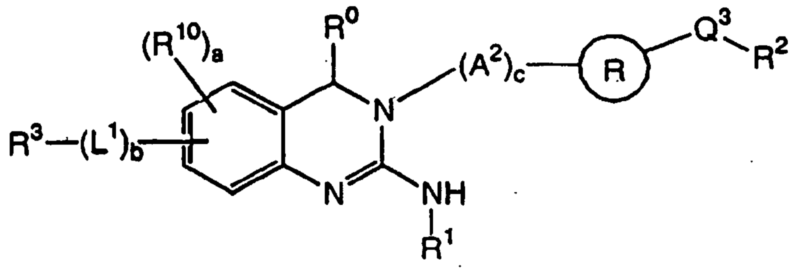

(II)

wherein

$R^0$ is selected from the group consisting of hydrogen, methyl, and $CF_3$;
$R^1$ is selected from the group consisting of hydrogen, hydroxy, methyl, ethyl, trifluoromethyl, 2,2,2-trifluoroethyl, methoxy, ethoxy and methyl-carbonyl;
c is an integer from 0 to 1;
$A^2$ is selected from the group consisting of $C_{1-4}$alkyl; wherein the $C_{1-4}$alkyl is optionally substituted with one or more $R^Y$ substituents;
wherein each $R^Y$ is independently selected from the group consisting of hydroxy, oxo, $C_{1-4}$alkyl, $C_{1-4}$alkoxy,

hydroxy substituted $C_{1-4}$alkyl, amino substituted $C_{1-6}$alkyl, cycloalkyl, cycloalkyl-$C_{1-4}$alkyl-, biphenyl, aryl, $C_{1-4}$aralkyl, heteroaryl, heteroaryl-$C_{1-4}$alkyl-, heterocycloalkyl, heterocycloalkyl-$C_{1-4}$alkyl- or spiro-heterocyclyl; wherein the cycloalkyl, aryl, heteroaryl, heterocycloalkyl or spiro-heterocyclyl group, whether alone or as part of a substituent group is optionally substituted with one or more substituents independently selected from the group consisting of fluoro, chloro, hydroxy, oxo, carboxy, $C_{1-4}$alkyl, $C_{1-4}$alkoxy, - C(O)O-($C_{1-4}$alkyl), hydroxy substituted $C_{1-4}$alkyl, fluoro substituted $C_{1-4}$alkyl, fluoro substituted $C_{1-4}$alkoxy, 5-tetrazolyl or 1-(1,4-dihydro-tetrazol-5-one);

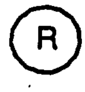

is selected from the group consisting of aryl, aryl-$C_{1-4}$alkyl, cycloalkyl, partially unsaturated carbocyclyl, heteroaryl, heterocycloalkyl and spiro-heterocyclyl;
wherein the aryl, cycloalkyl, partially unsaturated carbocyclyl, heteroaryl, heterocycloalkyl or spiro-heterocyclyl group, whether alone or as part of a substituent group, is optionally substituted with one or more substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino and di ($C_{1-4}$alkyl)amino;
provided that when c is 0, then

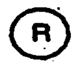

is other than aryl or heteroaryl;
$Q^3$ is selected from the group consisting of -C(O)-, -C(O)O-, -C(O)-NH-, -C(O)-N($C_{1-8}$alkyl) -, -C(O)-N(cycloalkyl) -, -NH-C(O)- and -NH-C(O)O-; wherein the cycloalkyl is optionally substituted with $C_{1-4}$alkyl;
$R^2$ is selected from the group consisting of $C_{1-10}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloalkyl-$C_{1-4}$alkyl- and spiro-heterocyclyl;
wherein the $C_{1-10}$alkyl, cycloalkyl, aryl, partially unsaturated carbocyclyl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C (O) -$C_{1-4}$alkyl, -C (O)-$C_{1-4}$aralkyl, -C(O)O-$C_{1-4}$alkyl, -C(O)O-$C_{1-4}$aralkyl, -$C_{1-4}$alkyl-C(O)O-$C_{1-4}$alkyl, -$C_{1-4}$alkyl-S-$C_{1-}$4alkyl, -C (O)-N($R^LR^M$), -$C_{1-4}$alkyl-C(O)-N($R^LR^M$), - $NR^L$-C(O)-$C_{1-4}$alkyl, -$SO_2$-N($R^L$-$R^M$), -$C_{1-4}$alkyl-$SO_2$-N- ($R^LR^M$), $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, -O-$C_{1-4}$aralkyl, -O-(tetrahydropyranyl),- NH-C(O)O-$CH_2$-(tetrahydropyranyl), -N($CH_3$)-C(O)O-$CH_2$-(tetrahydropyranyl), nitro, cyano, amino, $C_{1-4}$alkylamino, di($C_{1-4}$alkyl)amino, phenyl, 5-tetrazolyl and 1-(1,4-dihydro-tetrazol-5-one);
wherein the phenyl or tetrahydropyranyl is optionally substituted with one or more substituent independently selected from the group consisting of halogen, hydroxy, oxo, carboxy, C(O)O-$C_{1-4}$alkyl, C(O)-$C_{1-4}$alkyl, OC (O)-$C_{1-4}$alkyl, -$C_{1-4}$alkyl-OC(O)-$C_{1-4}$alkyl, -O-$C_{1-4}$aralkyl, $C_{1-4}$alkyl, fluoro substituted $C_{1-4}$alkyl, hydroxy substituted $C_{1-4}$alkyl, $C_{1-4}$alkoxy, nitro, cyano, amino, $C_{1-4}$alkylamino and di($C_{1-4}$alkyl)amino;
wherein each $R^L$ and $R^M$ is independently selected from the group consisting of hydrogen and $C_{1-4}$alkyl;
b is an integer from 0 to 1;
$L^1$ is selected from the group consisting of -O-, -S(O)$_{0-2}$-, -$NR^N$-, -C(O)-, -C(S)-, -$C_{1-4}$alkyl-, -(hydroxy substituted $C_{1-4}$alkyl)- and -($C_{2-4}$alkenyl)-; wherein $R^N$ is selected from the group consisting of hydrogen and $C_{1-4}$alkyl;
$R^3$ is selected from the group consisting of $C_{1-6}$alkyl, $C_{2-6}$alkenyl, cycloalkyl, partially unsaturated, carbocyclyl, aryl, biphenyl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;
wherein the $C_{1-6}$alkyl, cycloalkyl, partially unsaturated carbocyclyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, hydroxy substituted $C_{1-4}$alkyl, halogen substituted $C_{1-4}$alkyl, cyano substituted $C_{1-4}$alkyl, $C_{2-4}$alkenyl, $C_{2-4}$alkynyl, $C_{1-4}$alkoxy, halogen substituted $C_{1-4}$alkoxy, nitro, cyano, -$R^5$, -O-$R^5$, -S-$R^5$, -$SO_2$-$R^5$, -$SO_2$-$NR^P$-$R^5$, - $NR^P$-$SO_2$-$R^5$, -$NH_2$, -N($R^P$)-$R^5$, -C(O)-$R^5$, -C(O)-$NH_2$, -C(O)-$NR^P$-$R^5$, -$NR^P$-C(O)-$R^6$ and -$NR^P$-C(O)O-$R^5$;
wherein $R^5$ is selected from the group consisting of $C_{1-4}$alkyl, aryl, $C_{1-4}$aralkyl, partially unsaturated carbocyclyl,

cycloalkyl, cycloalkyl-$C_{1-4}$alkyl-, partially unsaturated carbocyclyl-$C_{1-4}$alkyl-, heteroaryl, heteroaryl-$C_{1-4}$alkyl-, heterocycloalkyl and heterocyclyl-$C_{1-4}$alkyl-;
wherein the aryl, partially unsaturated carbocyclyl, cycloalkyl, heteroaryl or heterocycloalkyl, whether alone or as part of a substituent group is optionally substituted with one or more substituent independently selected from the group consisting of $C_{1-4}$alkyl, $C_{1-4}$alkoxy, halogen, hydroxy, carboxy, amino, $C_{1-4}$alkylamino, di($C_{1-4}$alkyl) amino, cyano and nitro;
wherein $R^P$ is selected from the group consisting of hydrogen, $C_{1-8}$alkyl, hydroxy substituted $C_{1-4}$alkyl, $C_{1-4}$aralkyloxy substituted $C_{1-4}$alkyl, cycloalkyl, aryl, heteroaryl; heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;
wherein the cycloalkyl, aryl, heteroaryl, heterocycloalkyl or spiro-heterocyclyl, whether alone or as part of a substituents group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di($C_{1-4}$alkyl)amino,-$SO_2$-N($R^8R^T$), 5-tetrazolyl and 1-(1,4-dihydro-tetrazol-5-one);
wherein each $R^8$ and $R^T$ is independently selected from the group consisting of hydrogen and $C_{1-4}$alkyl;
a is an integer form 0 to 3;
each $R^{10}$ is Independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, -C(O)-$NR^VR^W$, -$SO_2$-$NR^VR^W$, -C(O)-$C_{1-4}$alkyl and -$SO_2$-$C_{1-4}$alkyl;
wherein each $R^V$ and $R^w$ is Independently selected from the group consisting of hydrogen and $C_{1-4}$alkyl; alternatively $R^v$ and $R^W$ are taken together with the N atom to which they are bound to form a 5 to 6 membered saturated, partially unsaturated or aromatic ring structure;
provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

**2.** A compound as in Claim 1
$R^0$ is selected from the group consisting of hydrogen and methyl;
$R^1$ is selected from the group consisting of hydrogen, methyl, trifluoromethyl, methoxy and methylcarbonyl;
c is an integer from 0 to 1;
$A^2$ is selected from the group consisting of $C_{1-4}$alkyl; wherein the $C_{1-4}$alkyl is optionally substituted with one to two $R^Y$ substituents;
wherein each $R^Y$ is independently selected from the group consisting of hydroxy, $C_{1-4}$alkyl, hydroxy substituted $C_{1-4}$alkyl, amino substituted $C_{1-6}$alkyl, cycloalkyl, cycloalkyl-$C_{1-4}$alkyl-, aryl, $C_{1-4}$aralkyl, heteroaryl, heteroaryl-$C_{1-4}$alkyl-, heterocycloalkyl and heterocycloalkyl-$C_{1-4}$alkyl-;
wherein the cycloalkyl, aryl, heteroaryl or heterocycloalkyl group, whether alone or as part of a substituent group is optionally substituted with one or more substituents independently selected from the group consisting of fluoro, chloro, hydroxy, oxo, carboxy, $C_{1-4}$alkyl, $C_{1-4}$alkoxy, .C(O)O-($C_{1-4}$alkyl), hydroxy substituted $C_{1-4}$alkyl, fluoro substituted $C_{1-4}$alkyl and fluoro substituted $C_{1-4}$alkoxy;

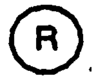

is selected from the group consisting of aryl, aryl-$C_{1-4}$alkyl-, cycloalkyl, heteroaryl and heterocycloalkyl;
wherein the aryl, cycloalkyl, heteroaryl or heterocycloalkyl group, whether alone or as part of a substituent group, is optionally substituted with one to two substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino and di($C_{1-4}$alkyl)amino;
provided that when c is 0, then

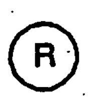

is other than aryl or heteroaryl;
$R^2$ is selected from the group consisting of $C_{1-8}$alkyl, cycloalkyl, aryl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl- and heterocycloalkyl-

$C_{1-4}$alkyl-;
wherein the $C_{1-8}$alkyl, cycloalkyl, aryl, heteroaryl, heterocycloalkyl or spiro-heterocyclyl, whether alone or as part of a substituent group is optionally substituted with one or more substituents independently selected from the group consisting of halogen, hydroxy, carboxy, -C(O)-$C_{1-4}$alkyl, -C(O)-$C_{1-4}$aralkyl, -C(O)O-$C_{1-4}$alkyl, -C(O)O-$C_{1-4}$aralkyl, -C(O)-N($R^LR^M$), -$NR^L$-C(O)-$C_{1-4}$alkyl, -$SO_2$-N($R^LR^M$) $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, nitro, cyano, amino, $C_{1-4}$alkylamino, di($C_{1-4}$alkyl)amlno and phenyl;
wherein the phenyl is optionally substituted with one to two substituent Independently selected from the group consisting of halogen, hydroxy, carboxy, -C(O)-$C_{1-4}$alkyl, $C_{1-4}$alkyl, fluoro substituted $C_{1-4}$alkyl, $C_{1-4}$alkoxy, nitro, cyano, amino, $C_{1-4}$alkylamino and di($C_{1-4}$alkyl)amino;
wherein each $R^L$ and $A^M$ is independently selected from the group consisting of hydrogen and $C_{1-4}$alkyl;
b is an integer selected from 0 to 1;
$L^1$ Is selected from the group consisting of -O-, -S(O)O-$_2^-$, -$NR^N$-, -$C_{1-4}$alkyl-, -(hydroxy substituted $C_{1-4}$alkyl)- and -($C_{2-4}$alkenyl)-;
wherein $R^N$ is selected from the group consisting of hydrogen and $C_{1-4}$alkyl;
$R^3$ is selected from the group consisting of $C_{1-6}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl- and heterocycloalkyl-$C_{1-4}$alkyl-;
wherein the $C_{1-6}$alkyl, cycloalkyl, partially unsaturated carbocyclyl, aryl, heteroaryl or heterocycloalkyl, whether alone or as part of a substituent group is optionally substituted with one or more substituents independently selected from the group consisting of halogen, hydroxy, carboxy, $C_{1-4}$alkyl, hydroxy substituted $C_{1-4}$alkyl, halogen substituted $C_{1-4}$alkyl, cyano substituted $C_{1-4}$alky, $C_{2-4}$alkenyl, $C_{2-4}$alkynyl, -$C_{1-4}$alkoxy, halogen substituted $C_{1-4}$alkoxy, nitro, cyano, -$R^6$, -O-$R^5$, -S-$R^5$, -$SO_2$-$R^5$, -$SO_2$-$NR^P$-$R^5$, -$NR^P$-$SO_2$-$R^5$, -$NH_2$, -N($R^P$)-$R^5$, - C(O)-$R^6$, -C(O)-$NH_2$, -C (O)-$NR^P$-$R^5$ and -$NR^P$-C(O)-$R^6$;
wherein $R^5$ is selected from the group consisting of $C_{1-4}$alkyl, aryl, $C_{1-4}$aralkyl, cycloalkyl, cycloalkyl-$C_{1-4}$alkyl-, partially unsaturated carbocyclyl-$C_{1-4}$alkyl-, heteroaryl, heteroaryl-$C_{1-4}$alkyl-, heterocycloalkyl and heterocyclyl-$C_{1-4}$ alkyl-;
wherein the aryl, $C_{1-4}$aralkyl, partially unsaturated carbocyclyl, cycloalkyl, heteroaryl or heterocycloalkyl, whether alone or as part of a substituent group is optionally substituted with one or more substituent independently selected from the group consisting of $C_{1-4}$alkyl, $C_{1-4}$alkoxy, halogen, hydroxy, carboxy, amino, $C_{1-4}$alkylamlno, di ($C_{1-4}$alkylamino, cyano and nitro;
wherein $R^P$ is selected from the group consisting of hydrogen, $C_{1-8}$alkyl, hydroxy substituted $C_{1-4}$alkyl, cycloalkyl, aryl, $C_{1-4}$aralkyl, heteroaryl and heterocycloalkyl;
wherein the cycloalkyl, aryl, heteroaryl or heterocycloalkyl, whether alone or as part of a substituent group is optionally substituted with one to two substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino and di($C_{1-4}$alkyl)amino;
a is an integer from 0 to 1;
$R^{10}$ is selected from the group consisting of hydroxy, halogen, $C_{1-4}$alkyl, $C_{1-4}$alkoxy, halogen substituted $C_{1-4}$alkyl, halogen substituted $C_{1-4}$alkoxy;
provided that the halogens on the halogen substituted $C_{1-4}$alkyl and the halogen substituted $C_{1-4}$alkoxy are selected from the group consisting of fluoro and chloro;

**3.** A compound as in Claim 2 wherein
$R^0$ is hydrogen;
$R^1$ Is selected from the group consisting of hydrogen, methyl and methylcarbonyl;
c Is an integer from 0 to 1;
$A^2$ is selected from the group consisting of $C_{1-4}$alkyl; wherein the $C_{1-4}$alkyl is optionally substituted with an $R^Y$ substituent;
wherein $R^Y$ is selected from the group consisting of $C_{1-4}$allkyl, aryl, $C_{1-4}$aralkyl and cycloalkyl-$C_{1-4}$alkyl-;

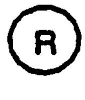

is selected from the group consisting of cycloalkyl, aryl, aryl-$C_{1-4}$alkyl-, heteroaryl, and heterocycloalkyl;
wherein the aryl, heteroaryl or heterocycloalkyl, whether alone or as part of a substituent group, is optionally substituted with one to two substituents independently selected from the group consisting of $C_{1-4}$alkyl and $C_{1-4}$alkoxy;
provided that wnen c is 0, men

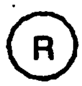

is other than aryl or heteroaryl;
$R^2$ is selected from the group consisting of $C_{1-8}$alkyl, cycloalkyl, cycloalkyl-$C_{1-4}$alkyl-, aryl, $C_{1-4}$aralkyl, heteroaryl, heteroaryl-$C_{1-4}$alkyl-, heterocycloalkyl and heterocycloalkyl-$C_{1-4}$alkyl-;
wherein the $C_{1-8}$alkyl, cycloalkyl, aryl or heterocycloalkyl, whether alone or as part of a substituent group is optionally substituted with one to three substituent Independently selected from the group consisting of halogen, $C_{1-4}$alkyl, $-SO_2-NH_2$ and phenyl;
b is an integer selected from 0 to 1;
$L^1$ is selected from the group consisting of -O-, -S-, -NH-, -$C_{1-4}$alkyl- and -$C_{2-4}$alkenyl-;
$R^3$ is selected from the group consisting of $C_{1-4}$alkyl, cycloalkyl, cycloalkyl-$C_{1-4}$alkyl-, aryl, heteroaryl- and heterocycloalkyl;
wherein the cycloalkyl or aryl, whether alone or as art of a substituent group is optionally substituted with one to two substituents independently selected from the group consisting of halogen, hydroxy, carboxy, cyano, nitro, amino, $C_{1-4}$alkylamino, di($C_{1-4}$alkylamino), $C_{1-4}$alkyl, $C_{2-4}$alkenyl, $C_{1-4}$alkoxy, hydroxy substituted $C_{1-4}$alkyl, halogen substituted $C_{1-4}$alkyl, cyano substituted $C_{1-4}$alkyl, halogen substituted $C_{1-4}$alkoxy, -O-aryl, -O-$C_{1-4}$aralkyl, -S-$C_{1-4}$alkyl, - $SO_2$-$C_{1-4}$alkyl, -C(O)-$C_{1-4}$alkyl, -C(O)-$NH_2$, -C(O)-NH($C_{1-4}$alkyl), -C(O)-N($C_{1-4}$alkyl)$_2$, -NH-C(O)-$C_{1-4}$alkyl, -C (O)-NH-$C_{1-4}$alkyl, -NH-$SO_2$-$C_{1-4}$alkyl, -NH-$SO_2$-phenyl, aryl, $C_{1-4}$aralkyl, cycloalkyl, cycloalkyl-$C_{1-4}$alkyl-, heteroaryl, heteroaryl-$C_{1-4}$alkyl-, heterocycloalkyl and heterocycloalkyl-$C_{1-4}$alkyl-;
wherein the aryl, cycloalkyl, heteroaryl or heterocycloalkyl, whether alone or as part of a substituent group, is optionally substituted with one to three substituents independently selected from the group consisting of $C_{1-4}$alkyl and $C_{1-4}$alkoxy;
a is 0;
or a pharmaceutically acceptable salt thereof.

**4.** A compound as in Claim 3 wherein
$R^0$ is hydrogen;
$R^1$ is selected from the group consisting of hydrogen, methyl and methylcarbonyl;
c is an integer from 0 to 1;
$A^2$ is selected from the group consisting of -$CH_2$-, -CH($CH_2CH_3$)-, - CH(phenyl)-, -CH(benzyl)- and -CH(cyclohexylmethyl)-;

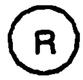

is selected from the group consisting of cyclopentyl, (S)-cyclopentyl, (R)-cyclopentyl, cyclohexyl, (R)-cyclohexyl, (S)-cyclohexyl, transcyclohexyl, phenyl, benzyl, 9-fluorenyl, 3-pyrrolidinyl, 1-indanyl, 1-(5-methoxyindanyl)-methyl, 3-piperidinyl, 4-piperidinyl, 3-azepinyl, 2-pyridyl, 4-pyridyl, 2-furyl, 2-thienyl and 5-oxazolyl;
provided that when c is 0, then 4-pyridyl, 2-furyl, 2-thienyl or 5-oxazolyl

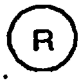
;

is other than phenyl, 2-pyridyl,
$Q^3$ is selected from the group consisting of -C(O)-, -C(O)-NH-, -C(O)-N($CH_3$)-, -(R)-C(O)-N($CH_3$), -(S)-C(O)-N($CH_3$), -C(O)-N(isopropyl)-, -C(O)-N(n-propyl)-, -C(O)-N(isobutyl)-, -C(O)-N(2-ethyl-n-hexyl)-, -C(O)-N(cyclohexyl)-, - C (O)-N(4-methyl-cyclohexyl)-, -C(O)O-, -NH-C(O)- and -NH-C(O)O-;
$R^2$ is selected from the group consisting of trifluoromethyl, methyl, ethyl, isobutyl, t-butyl, 3-n-heptyl, 4-n-heptyl, 2-ethyl-n-hexyl, cyclopentyl, cyclohexyl, 4-methyl-cyclohexyl, cyclopropyl-methyl, 4-aminosulfonyl-phenylethyl, benzhydryl, 1-adamantyl, 2-adamantyl, 2-(*R*)-adamantyl, 2-(*S*)-adamantyl, 2-decahydro-isoquinolinyl, 2-(1 -methyl-pyrrolidinyl)-ethyl, 1-piperidinyl, and 4-(1-methyl-piperidinyl);
b is an integer selected from 0 to 1;

$L^1$ is selected from the group consisting of -O-, -S-, -NH-, $-CH(CH_3)$-and -CH=CH-;
$R^3$ is selected from the group consisting of n-pentyl, isopentyl, isobutyl, isopropyl, cyclopentyl, cyclopentyl-methyl, phenyl, 2-hydroxy-phenyl, 3-carboxy-phenyl, 2-cyano-phenyl, 2-nitro-phenyl, 2-bromo-phenyl, 2-fluorophenyl, 2-chloro-phenyl, 2,6-dichloro-phenyl, 2-methyl-phenyl, 3-methyl-phenyl, 2-ethyl-phenyl, 4-isopropyl-phenyl, 3,4-dimethyl-phenyl, 3,5-dimethyl-phenyl, 2,6-dimethyl-phenyl, 2-methoxy-phenyl, 3-methoxy-phenyl, 4-methoxy-phenyl, 3,4-dimethoxy-phenyl, 2,6-dimethoxy-phenyl, 2-ethoxy-phenyl, 3-ethoxyphenyl, 2-isopropoxy-phenyl, 2-methoxy-5-methyl-phenyl, 2-methoxy-6-methylphenyl, 2-trifluoromethyl-phenyl, 2-trifluoromethoxy-phenyl, 2-methylthiophenyl, 4-methylthio-phenyl, 2-hydroxymethyl-phenyl, 2-cyanomethyl-phenyl, 2-(aminocarbonyl)-phenyl, 4-(aminocarbonyl)-phenyl, 2-(dimethylaminocarbonyl)-phenyl, 3-(dimethylamino)-phenyl, 4-(dimethylamino)-phenyl, 2-allyl-6-methylphenyl, 2-allyl-6-ethoxy-phenyl, 2-methyl-6-n-propyl-phenyl, 3-(methylcarbonylamino)-phenyl, 2-(methylaminocarbonyl)-phenyl, 2-(methylcarbonyl)-phenyl, 4-(methylcarbonylamino)-phenyl, 2-(aminocarbonylmethyl)-phenyl, 2-(methylsulfonyl)-phenyl, (3-(2-methoxy-4-methyl-phenyl)-sulfonylamino)-phenyl, 3-(2,4,6-trimethylphenylsulfonylamino)-phenyl, 3-(phenylsulfonylamino)-phenyl, 2-(t-butylaminosulfonyl)-phenyl, 2-(t-butylcarbonylamino)-5-methoxy-phenyl, 3-(phenylsulfonylamino)-phenyl, 2-phenoxy-phenyl, 3-phenoxy-phenyl, 2-benzyloxy-phenyl, 2-(2-benzthiazolyl)-5-methoxy-phenyl, 2-(2-benzthiazolyl)-phenyl, 2-(1-pyrrolyl)-phenyl, 3-(2-quinolinyl)-phenyl, 2-(1-pyrrolidinyl-methyl)-phenyl; 2-cyclopentyl-phenyl, 4-cyclohexyl-phenyl, 4-(4-morpholinyl)-phenyl, 3-methoxy-benzyl, 1-naphthyl, 2-naphthyl, 2-(5,6,7,8-tetrahydro-naphthyl), 2-biphenyl, 3-biphenyl, 2-biphenylmethyl, 3-pyridyl, 3,4-methylenedioxyphenyl, 4(3,5-dimethyl-isoxazolyl), 4-pyrazolyl, 3-thlenyl, 3-pyridyl, 4-pyridyl, 5-indolyl and 3-benzothienyl;
a is 0;
or a pharmaceutically acceptable salt thereof.

**5.** A compound as in Claim 4 wherein
$R^0$ is hydrogen;
$R^1$ is hydrogen;
c is an integer from 0 to 1;
$A^2$ is selected from the group consisting of $-CH_2-$ and $-CH(CH_2CH_3)-$;

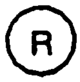

is selected from the group consisting of phenyl, 4-piperidinyl and 4-pyridyl; provided that when c is 0, then

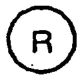

is other thanphenyl or 4-pyridyl;
$Q^3$ is selected from the group consisting of -1-C(O)O-, -3-C(O)O-, $-2-C(O)-N(CH_3)-$ and $-3-C(O)-N(CH_3)-$;
$R^2$ is selected from the group consisting of methyl, ethyl, t-butyl and cyclohexyl;
b is an integer from 0 to 1;
$L^1$ is selected from the group consisting of -O- and -S-;
$R^3$ is selected from the group consisting of phenyl, 2-bromophenyl, 2-chlorophenyl, 2,6-dichlorophenyl; 2-hydroxy-phenyl, 2-hydroxymethyl-phenyl, 2-methoxy-phenyl, 3-methoxy-phenyl, 2-ethoxy-phenyl, 2-methylthio-phenyl, 2-cyanomethyl-phenyl, 3-(phenyl-sulfonyl-amino)-phenyl, 3-(2,4,6-trimethylphenyl-sulfonyl-amino)-phenyl, (3-(2-methoxy-4-methyl-phenyl)-sulfonyl-amino)-phenyl, 2-(t-butyl-carbonyl-amino)-5-methoxy-phenyl, 1-naphthyl, 3-thienyl and 4-(3,5-dimethylisoxazolyl);
a is 0;
or a pharmaceutically acceptable salt thereof.

**6.** A compound as in Claim 5 wherein
$R^0$ is hydrogen;
$R^1$ is hydrogen;
c is an integer from 0 to 1;
$A^2$ is selected from the group consisting of $-CH_2-$ and $-CH(CH_2CH_3)-$;

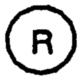

is selected from the group consisting of phenyl and 4-piperidinyl; provided that when c is 0, then

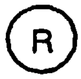

is 4-piperidinyl;
$Q^3$ is selected from the group consisting of -1-C(O)O-, -3-C(O)O- and -3-C(O)-N($CH_3$)-;
$R^2$ is selected from the group consisting of methyl, ethyl, t-butyl and cyclohexyl;
b is an Integer from 0 to 1;
$L^1$ is selected from the group consisting of -O- and -S-;
$R^3$ is selected from the group consisting of phenyl, 2-bromophenyl, 2-chlorophenyl, 2,6-dichlorophenyl, 2-hydroxyphenyl, 2-hydroxymethyl-phenyl, 2-methoxy-phenyl, 3-methoxy-phenyl, 2-ethoxy-phenyl, 2-methylthio-phenyl, 2-cyanomethyl-phenyl, 3-(phenyl-sulfonyl-amino)-phenyl, 1-naphthyl and 3-thienyl;
a is 0;
or a pharmaceutically acceptable salt thereof.

**7.** A compound as in Claim 6 where
$R^0$ is hydrogen;
$R^1$ is hydrogen;
c is 1;
$A^2$ is -$CH_2$-;

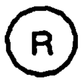

is phenyl;
$Q^3$ is -3-C(O)-N($CH_3$)-;
$R^2$ is selected from the group consisting of methyl, ethyl, t-butyl and cyclohexyl;
b is 0;
$R^3$ is selected from the group consisting of phenyl, 2-methoxy-phenyl, 2-ethoxy-phenyl and 1-naphthyl;
a is 0;
or a pharmaceutically acceptable salt thereof.

**8.** A compound as In Claim 4 wherein
$R^0$ Is hydrogen
$R^1$ is selected from the group consisting of hydrogen and methoxy; c is 1;
$A^2$ is selected from the group consisting of -$CH_2$-, -CH($CH_2CH_3$)-,-CH(phenyl)- and -CH(cyclohexyl)-;

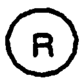

is selected from the group consisting of phenyl, 2-pyridyl and 2-furyl;
$Q^3$ Is selected from the group consisting of -3-C(O)-N($CH_3$)-, -3-C(O)-N(isopropyl)-, -3-C(O)-N(isobutyl)-,-3-C(O)-N (cyclohexyl)-, -4-C(O)-N($CH_3$)- and 5-C(O)-N($CH_3$)-;
$R^2$ is selected from the group consisting of isobutyl and cyclohexyl;
b is an integer from 0 to 1;
$L^1$ is selected from the group consisting of -O-, -S- and -NH-;
$R^3$ is selected from the group consisting of phenyl, 2-bromophenyl, 2-fluorophenyl, 2-chlorophenyl, 2,6-dichlorophenyl, 2-hydroxyphenyl, 2-hydroxymethyl-phenyl, 2-methylphenyl, 3-methylphpnyl, 2,6-dimethylphenyl, 2-methoxyphenyl, 3-methoxyphenyl, 2,6-dimethoxyphenyl, 2-ethoxyphenyl, 2-trifluoromethylphenyl, 2-trifluoromethoxyphenyl, 2-methylthio-phenyl, 2-nitrophenyl, 2-cyanophenyl, 2-cyanomethyl-phenyl, 2-phenoxy-phenyl, 2-(methyl-carbonyl-amino)-phenyl, 2-(amino-carbonyl)-phenyl, 3-(phenyl-sulfonylamino)-phenyl, 2-(t-butyl-amino-sulfonyl)-phenyl, 2-(t-butyl-carbonyl-amino)-5-methoxy-phenyl, 4-(3,5-dimethyl-soxazolyl), 1-naphthyl, 3-thienyl and 3-pyridyl;
a is 0;
or a pharmaceutically acceptable salt thereof

**9.** A compound as in Claim 8 wherein
$R^0$ is hydrogen
$R^1$ is hydrogen;
c is 1;
$A^2$ is selected from the group consisting of $-CH_2-$, $-CH(CH_2CH_3)-$ and - CH(cyclohexyl)-;

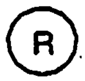.

is selected from the group consisting of phenyl and 2-pyridyl;
$Q^3$ is selected from the group consisting of -3-C(O)-N($CH_3$)-, -3-C(O)-N(isopropyl)-,-3-C(O)-N(cyclohexyl)- and -4-C(O)-N($CH_3$)-;
$R^2$ is cyclohexyl;
b is an integer from 0 to 1;
$L^1$ is-O-;
$R^3$ is selected from the group consisting of phenyl, 2-bromophenyl, 2-fluorophenyl; 2-chlorophenyl, 2-hydroxyphenyl, 2-hydroxymethyl-phenyl, 3-methylphenyl, 2-methoxyphenyl, 2-ethoxyphenyl, 2-cyanomethyl-phenyl, 2-(t-butyl-carbonyl-amino)-5-methoxy-phenyl, 1-naphthyl and 3-thienyl;
a is 0;
or a pharmaceutically acceptable salt thereof

**10.** A compound as in Claim 9 wherein
$R^0$ is hydrogen; $R^1$ is hydrogen; c is 1; $A^2$ is $-CH_2-$;

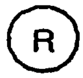

is phenyl; $Q^3$ is -3-C(O)-N($CH_3$)-; $R^2$ is cyclohexyl; b is 0; $R^3$ is selected from the group consisting of 2-methoxyphenyl and 2-ethoxyphenyl; a is 0; or a pharmaceutically acceptable salt thereof

**11.** A pharmaceutical composition comprising a pharmaceutically acceptable carrier and a compound of Claim 1.

**12.** A pharmaceutical composition made by mixing a compound of Claim 1 and a pharmaceutically acceptable carrier.

**13.** A process for masking, a pharmaceutical composition comprising mixing a compound of Claim 1 and a pharmaceutically acceptable carrier.

**14.** A compound of Claim 1 for use in treating a disorder mediated by ß-secretase,
wherein the disorder mediated by ß-secretase is selected from the group consisting of Alzheimer's disease (AD), mild cognitive impairment, senility, dementia, dementia with Lewy bodies, Down's syndrome, dementia associated with Parkinson's disease and dementia associated with beta-amyloid.

**15.** A composition of Claim 11 for use in treating a disorder mediated by ß-secretase,
wherein the disorder mediated by ß-secretase is selected from the group consisting of Alzheimer's disease (AD), mild cognitive impairment, senility, dementia, dementia with Lewy bodies, Down's syndrome, dementia associated with Parkinson's disease and dementia associated with beta-amyloid.

**16.** The use of a compound as In Claim 1 for the preparation of a medicament for treating: (a) Alzheimer's disease (AD), (b) mild cognitive Impairment, (c) senility, (d) dementia, (e) dementia with Lewy bodies, (f) Down's syndrome, (g) dementia associated with Parkinson's disease and (h) dementia associated with beta-amyloid, in a subject in need thereof.

**17.** A compound of formula (CII)

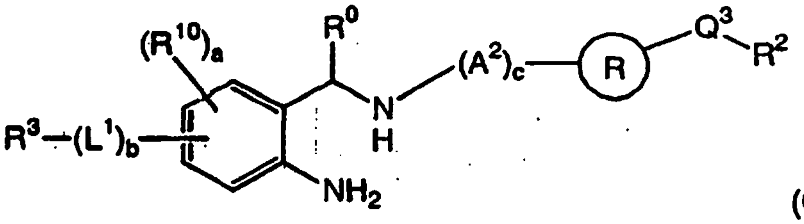

(CII)

wherein

$R^0$ is selected from the group consisting of hydrogen, methyl, and $CF_3$; c is an integer from 0 to 1;
$A^2$ is selected from the group consisting of $C_{1-4}$alkyl; wherein: the $C_{1-4}$alkyl is optionally substituted with one or more $R^Y$ substituents;
wherein each $R^Y$ is independently selected from the group consisting of hydroxy, oxo, $C_{1-4}$alkyl, $C_{1-4}$alkoxy, hydroxy substituted $C_{1-4}$alkyl, amino substituted $C_{1-6}$alkyl, cycloalkyl, cycloalkyl-$C_{1-4}$alkyl-, biphenyl, aryl, $C_{1-4}$aralkyl, heteroaryl, heteroaryl-$C_{1-4}$alkyl-, heterocycloalkyl, heterocycloalky-$C_{1-4}$alkyl- and spiro-heterocyclyl;
wherein the cycloalkyl, aryl, heteroaryl, heterocycloalkyl or spiro-heterocyclyl group, whether alone or as part of a substituent group is optionally substituted with one or more substituents independently selected from the group consisting of fluoro, chloro, hydroxy, oxo, carboxy, $C_{1-4}$alkyl, $C_{1-4}$alkoxy, - C(O)O-($C_{1-4}$alkyl), hydroxy substituted $C_{1-4}$alkyl, fluoro substituted $C_{1-4}$alkyl, fluoro substituted $C_{1-4}$alkoxy, 5-tetrazolyl and 1-(1,4-dihydro-tetrazol-5-one);

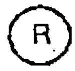

is selected from the group consisting of aryl, aryl-$C_{1-4}$alkyl, cycloalkyl, partially unsaturated carbocyclyl, heteroaryl, heterocycloalkyl and spiro-heterocyclyl;
wherein the aryl, cycloalkyl, partially unsaturated carbocyclyl, heteroaryl, heterocycloalkyl or spiro-heterocyclyl group, whether alone or as part of a substituent group, is optionally substituted with one or more substituents independently selected from the group consisting of $C_{1-4}$alkyl, $C_{1-4}$alkoxy, halogen substituted $C_{1-4}$alkyl, halogen substituted $C_{1-4}$alkoxy, hydroxy substituted $C_{1-4}$alkyl, hydroxy, carboxy, cyano, nitro, amino, $C_{1-4}$alkylamino and di($C_{1-4}$alkyl)amino;
provided that when c is 0, then

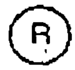

is other than aryl or heteroaryl;
$Q^3$ is selected from the group consisting of -C (O) -, -C(O)O-, -C(O)-NH-, -C(O)-N($C_{1-8}$alkyl) -, -C(O)-N(cycloalkyl)-, -NH-C(O)- and -NH-C(O)O- ; wherein the cycloalkyl is optionally substituted with $C_{1-4}$alkyl;
$R^2$ is selected from the group consisting of $C_{1-8}$alkyl, cycloalkyl, aryl, biphenyl, partially unsaturated carbocyclyl, heteroaryl, heterocycloalkyl, cycloalkyl-$C_{1-4}$alkyl-, $C_{1-4}$aralkyl, partially unsaturated carbocyclyl-$C_{1-4}$alkyl-, heteroaryl-$C_{1-4}$alkyl-, heterocycloarkyl-$C_{1-4}$alkyl- and spiro-heterocyclyl;
wherein the $C_{1-8}$alkyl, cycloalkyl, aryl, partially unsaturated carbocyclyl, heteroaryl, heterocycloalkyl or spiro-heterocyclyl, whether alone or as part of a substituent group is optionally substituted with one or more substituents independently selected from the group consisting of halogen, hydroxy, oxo, carboxy, -C(O)-$C_{1-4}$alkyl, -C (O)-$C_{1-4}$aralkyl, -C(O)O-$C_{1-4}$alkyl, -C(O)O- $C_{1-4}$aralkyl, -$C_{1-4}$alkyl-C(O)O-$C_{1-4}$alkyl, -$C_{1-4}$alkyl-S-$C_{1-4}$alkyl, -C (O)-N($R^LR^M$), -$C_{1-4}$alkyl-C(O)-N($R^LR^M$), -$NR^L$-C(O)-$C_{1-4}$alkyl, -$SO_2$-N($R^LR^M$), -$C_{1-4}$alkyl-$SO_2$-N($R^LR^M$), $C_{1-6}$alkyl, fluoro substituted $C_{1-4}$alkyl, hydroxy substituted $C_{1-4}$alkyl, carboxy substituted $C_{1-4}$alkyl, $C_{1-4}$alkoxy, -O-$C_{1-4}$aralkyl, -O-(tetrahydropyranyl), -NH-OC(O)-$CH_2$-(tetrahydropyranyl), -N($CH_3$)-OC(O)-$CH_2$-(tetrahydropyranyl), nitro, cyano, amino, $C_{1-4}$alkylamlno, di($C_{1-4}$alkyl)amino, phenyl, 5-tetrazolyl and 1-(1,4-dihydro-tetrazol-5-one);
wherein the phenyl or tetrahydropyranyl is optionally substituted with one or more substituent independently

selected from the group consisting of halogen, hydroxy, oxo, carboxy, C(O)O-$C_{1-4}$alkyl, C(O)-$C_{1-4}$alkyl, OC(O)-$C_{1-4}$alkyl; -$C_{1-4}$alkyl-OC(O)-$C_{1-4}$alkyl, -O-$C_{1-4}$aralkyl, $C_{1-4}$alkyl, fluoro substituted $C_{1-4}$alkyl, hydroxy substituted $C_{1-4}$alkyl, $C_{1-4}$alkoxy, nitro, cyano, amino, $C_{1-4}$alkylamino and di($C_{1-4}$alkyl)amino;
wherein each $R^L$ and $R^M$ is Independently selected from the group consisting of hydrogen and $C_{1-4}$alkyl;
b is an integer from 0 to 1;
$L^1$ is selected from the group consisting of -O-, -S(O)$_{0-2}$-, -N$R^N$-, -C(O)-, -C(S)-, -$C_{1-4}$alkyl-, -(hydroxy substituted $C_{1-4}$alkyl)- and -($C_{2-4}$alkenyl)-;
wherein $R^N$ is selected from the group consisting of hydrogen and $C_{1-4}$alkyl;
$R^3$ is selected from the group consisting of $C_{1-6}$alkyl, $C_{2-6}$alkenyl, cycloalkyl, partially unsaturated carbocyclyl, aryl, biphenyl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;
wherein the $C_{1-8}$alkyl, cycloalkyl, partially unsaturated carbocyclyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, hydroxy substituted $C_{1-4}$alkyl, halogen substituted $C_{1-4}$alkyl, cyano substituted $C_{1-4}$alkyl, $C_{2-4}$alkenyl, $C_{2-4}$alkynyl, $C_{1-4}$alkoxy, halogen substituted $C_{1-4}$alkoxy, nitro, cyano, -$R^5$, -O-$R^5$, -S-$R^5$, -$SO_2$-$R^5$, -$SO_2$-N$R^P$-$R^5$, - N$R^P$-$SO_2$-$R^5$, -$NH_2$, -N($R^P$-$R^5$, -C(O)-$R^5$, -C(O)-$NH_2$, -C(O)-N$R^P$-$R^5$, -N$R^P$-C(O)-$R^5$ and -N$R^P$-C(O)O-$R^5$;
wherein $R^5$ is selected from the group consisting of $C_{1-4}$alkyl, aryl, $C_{1-4}$aralkyl, partially unsaturated carbocyclyl, cycloalkyl, cycloalkyl-$C_{1-4}$alkyl-, partially unsaturated carbocyclyl-$C_{1-4}$alkyl-, heteroaryl, heteroaryl-$C_{1-4}$alkyl-, heterocycloalkyl and heterocylyl-$C_{1-4}$alkyl-;
wherein the aryl, partially unsaturated carbocyclyl, cycloalkyl, heteroaryl or heterocycloalkyl, whether alone or as part of a substituent group is optionally substituted with one or more substituent independently selected from the group consisting of $C_{1-4}$alkyl, $C_{1-4}$alkoxy, halogen, hydroxy, carboxy, amino, $C_{1-4}$alkylamino, di($C_{1-4}$alkyl)amino, cyano and nitro;
wherein $R^P$ is selected from the group consisting of hydrogen, $C_{1-8}$alkyl, hydroxy, substituted $C_{1-4}$alkyl, $C_{1-4}$aralkyloxy substituted $C_{1-4}$alkyl, cycloalkyl, aryl, heteroaryl, heterocycloalkyl, cycloalkyl-$C_{1-4}$alkyl-, $C_{1-4}$aralkyl, heteroaryl-$C_{1-4}$alkyl-, heterocycloalkyl-$C_{1-4}$alkyl- and spiro-heterocyclyl;
wherein the cycloalkyl, aryl, heteroaryl, heterocycloalkyl or spiro-heterocyclyl, whether alone or as part of a substituent group is optionally substituted with one or more substituents independently selected from the group consisting of halogen, hydroxy, oxo, carboxy, $C_{1-4}$alkyl, $C_{1-4}$alkoxy, $C_{1-4}$alkoxy-carbonyl, nitro, cyano, amino, $C_{1-4}$alkylamino, di($C_{1-4}$alkyl)amino,-$SO_2$-N($R^SR^T$), 5-tetrazolyl and 1-(1,4-dihydro-tetrazol-5-one);
wherein each $R^S$ and $R^T$ is independently selected from the group consisting of hydrogen and $C_{1-4}$alkyl; ,
a is an integer form 0 to 3;
each $R^{10}$ is independently selected from the group consisting of hydroxy, halogen, $C_{1-4}$alkyl, $C_{1-4}$alkoxy, halogen substituted $C_{1-4}$alkyl, halogen substituted $C_{1-4}$alkoxy, -C(O)-N$R^VR^W$, -$SO_2$-N$R^VR^W$, -C(O)-$C_{1-4}$alkyl and -$SO_2$-$C_{1-4}$alkyl;
wherein each $R^V$ and $R^W$ is independently selected from the group consisting of hydrogen and $C_{1-4}$alkyl; alternatively $R^V$ and $R^W$ are taken together with the N atom to which they are bound to form a 5 to 6 membered saturated, partially unsaturated or aromatic ring structure;
provided that the halogens on the halogen substituted $C_{1-4}$alkyl or the halogen substituted $C_{1-4}$alkoxy are selected from the group consisting of chloro and fluoro;
or a pharmaceutically acceptable salt thereof.

## Patentansprüche

**1.** Eine Verbindung mit der Formel (I)

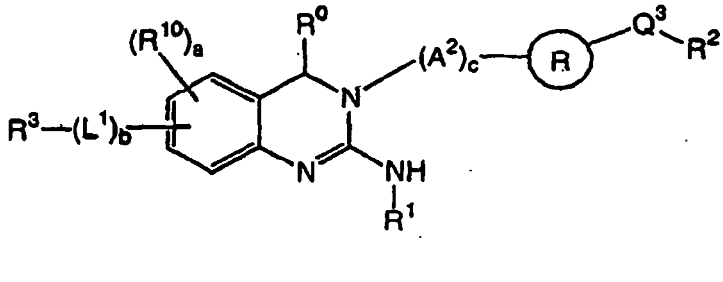

(II)

wobei

$R^{\circ}$ aus der Gruppe bestehend aus Wasserstoff, Methyl und $CF_3$ ausgewählt wird;
$R^1$ aus der Gruppe bestehend aus Wasserstoff, Hydroxy, Methyl, Ethyl, Trifluormethyl, 2,2,2-Trifluorethyl, Methoxy, Ethoxy und Methylcarbonyl ausgewählt wird;
c eine ganze Zahl von 0 bis 1 ist;
$A^2$ aus der Gruppe bestehend aus $C_{1-4}$-Alkyl ausgewählt wird; wobei $C_{1-4}$-Alkyl optional durch einen oder mehrere $R^Y$-Substituenten substituiert wird;
wobei jedes $R^Y$ unabhängig aus der Gruppe bestehend aus Folgenden ausgewählt wird, nämlich Hydroxy, Oxo, $C_{1-4}$-Alkyl, $C_{1-4}$-Alkoxy, Hydroxy-substituiertes $C_{1-4}$-Alkyl, Amino-substituiertes $C_{1-6}$-Alkyl, Cycloalkyl, Cycloalkyl- $C_{1-4}$-Alkyl-, Biphenyl, Aryl, $C_{1-4}$-Aralkyl, Heteroaryl, Heteroaryl-$C_{1-4}$-Alkyl-, Heterocycloalkyl, Heterocycloalkyl- $C_{1-4}$-Alkyl- oder Spiro-heterocyclyl;
wobei die Cycloalkyl-, Aryl-, Heteroaryl-, Heterocycloalkyl- oder Spiro-Gruppe, entweder allein oder als Teil einer Substituentengruppe optional durch einen oder mehrere Substituenten substituiert wird, der/die unabhängig aus der Gruppe bestehend aus folgenden ausgewählt wird/werden, nämlich Fluor, Chlor, Hydroxy, Oxo, Carboxy, $C_{1-4}$-Alkyl, $C_{1-4}$-Alkoxy, -C(O)O-($C_{1-4}$-Alkyl), Hydroxy-substituiertes $C_{14}$-Alkyl, Fluor-substituiertes $C_{1-4}$-Alkyl, Fluor substituiertes $C_{1-4}$-Alkoxy, 5-Tetrazolyl oder 1-(1,4-dihydrotetrazol-5-on);
® aus der Gruppe bestehend aus Aryl, Aryl- $C_{1-4}$-Alkyl, Cycloalkyl, teilweise ungesättigtes Carbocyclyl, Heteroaryl, Heterocycloalkyl oder Spiroheterocyclyl ausgewählt wird;
wobei die Aryl-, Cycloalkyl-, teilweise ungesättigte Carbocyclyl-, Heteroaryl-Heteracycloalkyl- oder Spiroheterocyclyl-Gruppe, entweder allein oder als Teil einer Substituentengruppe, optional durch einen oder mehrere Substituenten substituiert wird, der/die unabhängig aus der Gruppe bestehend aus Folgenden ausgewählt wird/werden, nämlich $C_{1-4}$-Alkyl, $C_{1-4}$-Alkoxy, Halogen-substituiertes $C_{1-4}$-Alkyl, Halogen-substituiertes $C_{1-4}$-Alkoxy, Hydroxy-substituiertes $C_{1-4}$-Alkyl, Hydroxy, Carboxy, Cyan, Nitro, Amino, $C_{1-4}$-Alkylamino und Di($C_{1-4}$-Alkyl)amino;
vorausgesetzt dass, wenn c 0 ist,

®

weder Aryl noch Heteroaryl ist;
$Q^3$ aus der Gruppe bestehend aus -C(O)-, -C(O)O-, C(O)-NH-, -C(O)-N($C_{1-8}$-Alkyl)-, - C(O)-N(Cycloalkyl)-, -NH-C(O)- und -NH-C(O)O- ausgewählt wird; wobei das Cycloalkyl optional durch $C_{1-4}$-Alkyl substituiert wird;
$R^2$ aus der Gruppe bestehend aus $C_{1-10}$Alkyl, Cycloalkyl, Aryl, Biphenyl, teilweise ungesättigtes Carbocyclyl, Heteroaryl, Heterocycloalkyl, Cycloalkyl-$C_{1-4}$-Alkyl-, $C_{1-4}$-Aralkyl, teilweise ungesättigtes Carbocyclyl- $C_{1-4}$-Alkyl, Heteroaryl- $C_{1-4}$-Alkyl, Heterocycloalkyl- $C_{1-4}$-Alkyl- oder Spiroheterocyclyl ausgewählt wird;
wobei das $C_{1-10}$-Alkyl, Cycloalkyl, Aryl, teilweise ungesättigte Carbocyclyl, Heteroaryl, Heterocycloalkyl oder Spiroheterocyclyl, entweder allein oder als Teil einer Substituentengruppe, optional durch einen oder mehrere Substituenten substituiert wird, die aus der Gruppe bestehend aus folgenden ausgewählt wird/werden, nämlich, Halogen, Hydroxy, Oxo, Carboxy, -C(O)-$C_{1-4}$-Alkyl, -C(O)-$C_{1-4}$-Aralkyl, -C(O)O-$C_{1-4}$-Alkyl, -C(O)O-$C_{1-4}$-Aralkyl, -C-$_{1-4}$-Alkyl-C(O)O-$C_{1-4}$-Alkyl, -$C_{1-4}$-Alkyl-S-$C_{1-4}$-Alkyl, -C(O)-N($R^LR^M$), -$C_{1-4}$-Alkyl-C(O)-N($R^LR^M$), -$NR^L$-C(O)-$C_{1-4}$-Alkyl, -$SO_2$-N($R^LR^M$), -$C_{1-4}$-Alkyl-$SO_2$-N($R^LR^M$), $C_{1-6}$-Alkyl, Fluor-substituiertes $C_{1-4}$-Alkyl, Hydroxy-substituiertes $C_{1-4}$-Alkyl, Carboxy-substituiertes $C_{1-4}$-Alkyl, $C_{1-4}$-Alkoxy, -O-$C_{1-4}$-Aralkyl, -O-(Tetrahydropyranyl), NH-C(O)O-$CH_2$-(Tetrahydropyranyl), -N($CH_3$)-C(O)O-$CH_2$-Tetrahydropyranyl), Nitro, Cyan, Amino, $C_{1-4}$-Alkylamino, Di($C_{1-4}$-Alkyl)amino, Phenyl, 5-Tetrazolyl und 1-(1,4-dihydrotetrazol-5-on);
wobei das Phenyl oder Tetrahydropyranyl optional durch einen oder mehrere Substituenten substituiert wird, der/die unabhängig aus der Gruppe bestehend aus Folgenden ausgewählt wird/werden, nämlich Halogen, Hydroxy, Oxo, Carboxy, -C(O)O-$C_{1-4}$-Akyl, -C(O)-$C_{1-4}$Alkyl, -OC(O)-$C_{1-4}$Alkyl, -$C_{1-4}$Alkyl-OC(O)-$C_{1-4}$-Alkyl, -O-$C_{1-4}$Aralkyl, $C_{1-4}$Alkyl, Fluor-substituiertes $C_{1-4}$-Alkyl, Hydroxy-substituiertes $C_{1-4}$-Alkyl, $C_{1-4}$-Alkoxy, Nitro, Cyan, Amino, $C_{1-4}$-Alkylamino und Di($C_{1-4}$-Alkyl)amino;
wobei $R^L$ und $R^M$ jeweils unabhängig aus der Gruppe bestehend aus Wasserstoff und $C_{1-4}$-Alkyl ausgewählt werden;
b eine ganze Zahl von 0 bis 1 ist;
$L^1$ aus der Gruppe bestehend aus -O-, -S(O)$_{0-2}$-, -$NR^N$-, -C(O)-, -C(S)-, -$C_{1-4}$-Alkyl-, - (Hydroxy-substituiertes $C_{1-4}$-Alkyl)- und -($C_{2-4}$-Alkenyl)- ausgewählt wird;
wobei $R^N$ aus der Gruppe bestehend aus Wasserstoff und $C_{1-4}$-Alkyl ausgewählt wird;
$R^3$ aus der Gruppe bestehend aus Folgenden ausgewählt wird, nämlich $C_{1-4}$-Alkyl, $C_{2-6}$-Alkenyl, Cycloalkyl,

teilweise ungesättigtes Carbocyclyl, Aryl, Biphenyl, Heteroaryl, Heterocycloalkyl, Cycloalkyl-$C_{1-4}$-Alkyl-, $C_{1-4}$-Aralkyl, Heteroaryl-$C_{1-4}$-Alkyl-, Heterocycloalkyl-$C_{1-4}$-Alkyl- und Spiroheterocyclyl;

wobei das $C_{1-6}$-Alkyl, Cycloalkyl, teilweise ungesättigte Carbocyclyl, Aryl, Heteroaryl, Heterocycloalkyl oder Spiroheterocyclyl, entweder allein oder als Teil einer Substituentengruppe optional durch einen oder mehrere Substituenten substituiert wird, der/die unabhängig aus einer Gruppe bestehend aus Folgenden ausgewählt wird/werden, nämlich Halogen, Hydroxy, Oxo, Carboxy, $C_{1-4}$-Alkyl, Hydroxy-substituiertes $C_{1-4}$-Alkyl, Halogen-substituiertes $C_{1-4}$-Alkyl, Cyan-substituiertes $C_{1-4}$-Alkyl, $C_{2-4}$-Alkenyl, $C_{2-4}$-Alkynyl, $C_{1-4}$-Alkoxy, Halogen-substituiertes $C_{1-4}$-Alkoxy, Nitro, Cyan, -$R^5$, -$OR^5$, -S-$R^5$, -$SO_2$-$R^5$, -$SO_2$-$NR^P$-$R^5$, -$NR^P$-$SO_2$-$R^5$, -$NH_2$, -N($R^P$)-$R^5$, -C(O)-$R^5$, -C(O)-$NH_2$, -C(O)-$NR^P$-$R^5$, -$NR^P$-C(O)-$R^5$ und-$NR^P$-C(O)O-$R^5$;

wobei $R^5$ aus der Gruppe bestehend aus Folgenden ausgewählt wird, nämlich $C_{1-4}$-Alkyl, Aryl, $C_{1-4}$-Aralkyl, teilweise ungesättigtes Carbocyclyl, Cycloalkyl, Cycloalkyl-$C_{1-4}$-Alkyl -, teilweise ungesättigtes Carbocyclyl-$C_{1-4}$-Alkyl-, Heteroaryl, Heteroaryl-$C_{1-4}$-Alkyl-, Heterocycloalkyl und Heterocyclyl-$C_{1-4}$-Alkyl-:

wobei das Aryl, teilweise ungesättigte Carbocyclyl, Cycloalkyl, Heteroaryl oder Heterocycloalkyl, entweder allein oder als Teil einer Substituentengruppe optional durch einen oder mehrere Substituenten substituiert wird, der/die unabhängig aus einer Gruppe bestehend aus Folgenden ausgewählt wird/werden, $C_{1-4}$-Alkyl, $C_{1-4}$-Alkoxy, Halogen, Hydroxy, Carboxy, Amino, $C_{1-4}$-Alkylamino, Di($C_{1-4}$-Alkyl)amino, Cyan und Nitro;

wobei $R^P$ aus der Gruppe bestehend aus Folgenden ausgewählt wird, nämlich Wasserstoff, $C_{1-8}$-Alkyl, Hydroxy-substituiertes $C_{1-}$-Alkyl, $C_{1-4}$-Aralkyloxy-substituiertes $C_{1-4}$-Alkyl, Cycloalkyl, Aryl, Heteroaryl, Heterocycloalkyl, Cycloalkyl-$C_{1-4}$-Alkyl-, $C_{1-4}$-Aralkyl, Heteroaryl-$C_{1-4}$-Alkyl-, Heterocycloalkyl-$C_{1-4}$-Alkyl- und Spiroheterocyclyl;

wobei das Cycloalkyl, Aryl, Heteroaryl, Heterocycloalkyl oder Spiroheterocyclyl, entweder allein oder als Teil einer Substituentengruppe optional durch einen oder mehrere Substituenten substituiert wird, der/die unabhängig aus einer Gruppe bestehend aus Folgenden ausgewählt wird/werden, nämlich Halogen, Hydroxy, Oxo, Carboxy, $C_{1-4}$-Alkyl, $C_{1-4}$-Alkoxy, $C_{1-4}$-Alkoxycarbonyl, Nitro, Cyan, Amino, $C_{1-4}$-Alkylamino, Di($C_{1-4}$-Alkyl)amino,-$SO_2$-N($R^SR^T$), 5-Tetrazolyl und 1-(1,4-Dihydrotetrazol-5-on);

wobei $R^S$ und $R^T$ jeweils unabhängig aus der Gruppe bestehend aus Wasserstoff und $C_{1-4}$-Alkyl ausgewählt werden;

a eine ganze Zahl von 0 bis 3 ist;

jedes $R^{10}$ unabhängig aus der Gruppe bestehend aus Hydroxy, Halogen, $C_{1-4}$-Alkyl, $C_{1-4}$-Alkoxy, Halogen-substituiertes $C_{1-4}$-Alkyl, Halogen-substituiertes $C_{1-4}$-Alkoxy, -C(O)-$NR^vR^w$, - $SO_2$-$NR^vR^w$, -C(O)-$C_{1-4}$-Alkyl und -$SO_2$-$C_{1-4}$-Alkyl ausgewählt wird;

wobei $R^V$ und $R^W$ jeweils unabhängig aus der Gruppe bestehend aus Wasserstoff und $C_{1-4}$-Alkyl ausgewählt werden; alternativ $R^V$ und $R^W$ zusammen mit dem N-Atom, an das sie gebunden sind, genommen werden, um eine 5- bis 6-gliedrige gesättigte, teilweise ungesättigte oder aromatische Ringstruktur zu bilden,

vorausgesetzt, dass die Halogene am Halogen-substituierten $C_{1-4}$-Alkyl oder Halogen-substituierten $C_{1-4}$-Alkoxy aus der Gruppe bestehend aus Chlor und Fluor ausgewählt werden;

oder ein pharmazeutisch verträgliches Salze dieser.

**2.** Eine Verbindung entsprechend Anspruch 1

wobei $R^o$ aus der Gruppe bestehend aus Wasserstoff und Methyl ausgewählt wird;

$R^1$ aus der Gruppe bestehend aus Wasserstoff, Hydroxy, Methyl, Trifluormethyl, Methoxy und Methylcarbonyl ausgewählt wird;

c eine ganze Zahl von 0 bis 1 ist;

$A^2$ aus der Gruppe bestehend aus $C_{1-4}$-Alkyl ausgewählt wird; wobei $C_{1-4}$-Alkyl optional durch einen oder mehrere $R^Y$-Substituenten substituiert wird;

wobei jedes $R^Y$ unabhängig aus der Gruppe bestehend aus Folgenden ausgewählt wird, nämlich Hydroxy, $C_{1-4}$-Alkyl, Hydroxy-substituiertes $C_{1-4}$-Alkyl, Amino-substituiertes $C_{1-6}$-Alkyl, Cycloalkyl, Cycloalkyl- $C_{1-4}$-Alkyl-, Aryl, $C_{1-4}$-Aralkyl, Heteroaryl, Heteroaryl-$C_{1-4}$-Alkyl-, Heterocycloalkyl und Heterocycloalkyl- $C_{1-4}$-Alkyl-;

wobei die Cycloalkyl-, Aryl-, Heteroaryl-, oder Heterocycloalkyl-Gruppe, entweder allein oder als Teil einer Substituentengruppe optional durch einen oder mehrere Substituenten substituiert wird, der/die unabhängig aus der Gruppe bestehend aus folgenden ausgewählt wird/werden, nämlich Fluor, Chlor, Hydroxy, Oxo, Carboxy, $C_{1-4}$-Alkyl, $C_{1-4}$-Alkoxy, -C(O)O-($C_{1-4}$--Alkyl), Hydroxy-substituiertes $C_{1-4}$-Alkyl, Fluor-substituiertes $C_{1-4}$-Alkyl und Fluor substituiertes $C_{1-4}$-Alkoxy;

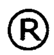

aus der Gruppe bestehend aus Aryl, Aryl-$C_{1-4}$-Alkyl, Cycloalkyl, Heteroaryl und Heterocycloalkyl ausgewählt wird;

wobei die Aryl-, Cycloalkyl-, Heteroaryl- oder Heteracycloalkyl-Gruppe, entweder allein oder als Teil einer Substituentengruppe, optional durch ein bis zwei Substituenten substituiert wird, der/die unabhängig aus der Gruppe bestehend aus Folgenden ausgewählt wird/werden, nämlich $C_{1-4}$-Alkyl, $C_{1-4}$-Alkoxy, Halogen-substituiertes $C_{1-4}$-Alkyl, Halogen-substituiertes $C_{1-4}$-Alkoxy, Hydroxy-substituiertes $C_{1-4}$-Alkyl, Hydroxy, Carboxy, Cyan, Nitro, Amino, $C_{1-4}$-Alkylamino und Di($C_{1-4}$-Alkyl)amino;
vorausgesetzt dass, wenn c 0 ist,

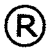

weder Aryl noch Heteroaryl ist;
$R^2$ aus der Gruppe bestehend aus $C_{1-8}$-Alkyl, Cycloalkyl, Aryl, Heteroaryl, Heterocycloalkyl, Cycloalkyl-$C_{1-4}$-Alkyl-, $C_{1-4}$-Aralkyl, teilweise ungesättigtes Carbocyclyl-$C_{1-4}$-Alkyl, Heteroaryl-$C_{1-4}$-Alkyl und Heterocycloalkyl- $C_{1-4}$-Alkyl-ausgewählt wird;
wobei das $C_{1-8}$-Alkyl, Cycloalkyl, Aryl, Heteroaryl, Heterocycloalkyl oder Spiroheterocyclyl, entweder allein oder als Teil einer Substituentengruppe, optional durch einen oder mehrere Substituenten substituiert wird, die aus der Gruppe bestehend aus folgenden ausgewählt wird/werden, nämlich, Halogen, Hydroxy, Carboxy, -C(O)-$C_{1-4}$Alkyl, - C(O)-$C_{1-4}$-Aralkyl, -C(O)O-$C_{1-4}$--Alkyl, -C(O)O-$C_{1-4}$-Aralkyl, -C(O)-N($R^LR^M$), -$NR^L$-C(O)-$C_{1-4}$-Alkyl, -$SO_2$-N($R^LR^M$) $C_{1-6}$-Alkyl, Fluor-substituiertes $C_{1-4}$-Alkyl, Hydroxy-substituiertes $C_{1-4}$-Alkyl, Carboxy-substituiertes $C_{1-4}$--Alkyl, $C_{1-4}$-Alkoxy, Nitro, Cyan, Amino, $C_{1-4}$Alkylamino, Di($C_{1-4}$-Alkyl)amino und Phenyl;
wobei das Phenyl optional durch einen oder zwei Substituenten substituiert wird, der/die unabhängig aus der Gruppe bestehend aus Folgenden ausgewählt wird/werden, nämlich Halogen, Hydroxy, Carboxy, -C(O)O-$C_{1-4}$Alkyl, $C_{1-4}$Alkyl, Fluor-substituiertes $C_{1-4}$Alkyl, $C_{1-4}$-Alkoxy, Nitro, Cyan, Amino, $C_{1-4}$-Alkylamino und Di($C_{1-4}$-Alkyl)amino;
wobei $R^L$ und $R^M$ jeweils unabhängig aus der Gruppe bestehend aus Wasserstoff und $C_{1-4}$Alkyl ausgewählt werden;
b eine ganze Zahl von 0 bis 1 ist;
$L^1$ aus der Gruppe bestehend aus -O-, -$S(O)_{0-2}$-, -$NR^N$-, -$C_{1-4}$-Alkyl,-(Hydroxy-substituiertes $C_{1-4}$Alkyl)- und -($C_{2-4}$-Alkenyl)- ausgewählt wird;
wobei $R^N$ aus der Gruppe bestehend aus Wasserstoff und $C_{1-4}$-Alkyl ausgewählt wird;
$R^3$ aus der Gruppe bestehend aus Folgenden ausgewählt wird, nämlich $C_{1-6}$-Alkyl, Cycloalkyl, Aryl, Heteroaryl, Heterocycloalkyl, Cycloalkyl-$C_{1-4}$-Alkyl-, $C_{1-4}$-Aralkyl, Heteroaryl-$C_{1-4}$-Alkyl-, und Heterocycloalkyl-$C_{1-4}$-Alkyl;
wobei das $C_{1-6}$-Alkyl, Cycloalkyl, teilweise ungesättigte Carbocyclyl, Aryl, Heteroaryl, oder Heterocycloalkyl, entweder allein oder als Teil einer Substituentengruppe optional durch einen oder mehrere Substituenten substituiert wird, der/die unabhängig aus einer Gruppe bestehend aus Folgenden ausgewählt wird/werden, nämlich Halogen, Hydroxy, Carboxy, $C_{1-4}$-Alkyl, Halogen-substituiertes $C_{1-4}$-Alkyl, Cyan-substituiertes $C_{1-4}$-Alkyl, $C_{2-4}$-Alkenyl, $C_{2-4}$-Alkynyl, $C_{1-4}$-Alkoxy, Halogen-substituiertes $C_{1-4}$-Alkoxy, Nitro, Cyan, -$R^5$, -$OR^5$, -S-$R^5$, -$SO_2$-$R^5$,- $SO_2$-$NR^P$-$R^5$, -$NR^P$-$SO_2$-$R^5$, -$NH_2$, -N($R^P$)-$R^5$, -C(O)-$R^5$, -C(O)-$NH_2$, -C(O)-$NR^P$-$R^5$ und -$NR^P$-C(O)-$R^5$;
wobei $R^5$ aus der Gruppe bestehend aus Folgenden ausgewählt wird, nämlich $C_{1-4}$-Alkyl, Aryl, $C_{1-4}$-Aralkyl, Cycloalkyl, Cycloalkyl-$C_{1-4}$-Alkyl -, teilweise ungesättigtes Carbocyclyl-$C_{1-4}$-Alkyl-, Heteroaryl, Heteroaryl-$C_{1-4}$-Alkyl-, Heterocycloalkyl und Heterocyclyl-$C_{1-4}$-Alkyl-:
wobei das Aryl, $C_{1-4}$-Aralkyl, teilweise ungesättigte Carbocyclyl, Cycloalkyl, Heteroaryl oder Heterocycloalkyl, entweder allein oder als Teil einer Substituentengruppe optional durch einen oder mehrere Substituenten substituiert wird, der/die unabhängig aus einer Gruppe bestehend aus Folgenden ausgewählt wird/werden, $C_{1-4}$-Alkyl, $C_{1-4}$-Alkoxy, Halogen, Hydroxy, Carboxy, Amino, $C_{1-4}$-Alkylamino, Di($C_{1-4}$-Alkyl)amino, Cyan und Nitro;
wobei $R^P$ aus der Gruppe bestehend aus Folgenden ausgewählt wird, nämlich Wasserstoff, $C_{1-8}$-Alkyl, Hydroxy-substituiertes $C_{1-4}$-Alkyl, Cycloalkyl, Aryl, $C_{1-4}$-Aralkyl, Heteroaryl und Heterocycloalkyl;
wobei das Cycloalkyl, Aryl, Heteroaryl oder Heterocycloalkyl, entweder allein oder als Teil einer Substituentengruppe optional durch ein bis zwei Substituenten substituiert wird, der/die unabhängig aus einer Gruppe bestehend aus Folgenden ausgewählt wird/werden, nämlich Halogen, Hydroxy, Oxo, Carboxy, $C_{1-4}$-Alkyl, $C_{1-4}$-Alkoxy, $C_{1-4}$-Alkoxycarbonyl, Nitro, Cyan, Amino, $C_{1-4}$-Alkylamino und Di($C_{1-4}$-Alkyl)amino;
a eine ganze Zahl von 0 bis 1 ist;
$R^{10}$ aus der Gruppe bestehend aus Hydroxy, Halogen, $C_{1-4}$-Alkyl, $C_{1-4}$-Alkoxy, Halogen-substituiertes $C_{1-4}$-Alkyl, Halogen-substituiertes $C_{1-4}$-Alkoxy ausgewählt wird;
vorausgesetzt, dass die Halogene am Halogen-substituierten $C_{1-4}$-Alkyl und Halogen-substituierten $C_{1-4}$-Alkoxy aus der Gruppe bestehend aus Chlor und Fluor ausgewählt werden.

**3.** Eine Verbindung entsprechend Anspruch 2,
wobei

$R^0$ Wasserstoff ist;
$R^1$ aus der Gruppe bestehend aus Wasserstoff, Hydroxy, Methyl, und Methylcarbonyl ausgewählt wird;
c eine ganze Zahl von 0 bis 1 ist;
$A^2$ aus der Gruppe bestehend aus $C_{1-4}$-Alkyl ausgewählt wird; wobei $C_{1-4}$-Alkyl optional durch einen $R^Y$-Substituenten substituiert wird;
wobei $R^Y$ aus der Gruppe bestehend aus $C_{1-4}$-Alkyl, Aryl, $C_{1-4}$-Aralkyl und Cycloalkyl-$C_{1-4}$-Alkyl- ausgewählt wird;

Ⓡ

aus der Gruppe bestehend aus Cycloalkyl, Aryl, Aryl-$_{C1-}$4-Alkyl, Heteroaryl und Heterocycloalkyl ausgewählt wird;
wobei die Aryl-,Heteroaryl- oder Heteracycloalkyl-Gruppe, entweder allein oder als Teil einer Substituentengruppe, optional durch ein bis zwei Substituenten substituiert wird, der/die unabhängig aus der Gruppe bestehend aus $C_{1-4}$-Alkyl und $C_{1-4}$-Alkoxy ausgewählt wird/werden;
vorausgesetzt dass, wenn c 0 ist,

Ⓡ

weder Aryl noch Heteroaryl ist;
$R^2$ aus der Gruppe bestehend aus $C_{1-8}$-Alkyl, Cycloalkyl, Cycloalkyl-$C_{1-4}$-Alkyl-, Aryl, $C_{1-4}$-Aralkyl, Heteroaryl, Heteroaryl-$_{C1-4}$-Alkyl, Heterocycloalkyl, und Heterocycloalkyl- $C_{1-4}$-Alkyl- ausgewählt wird;
wobei das $C_{1-8}$-Alkyl, Cycloalkyl, Aryl, oder Heterocycloalkyl, entweder allein oder als Teil einer Substituentengruppe, optional durch ein bis drei Substituenten substituiert wird, die unabhängig aus der Gruppe bestehend aus Folgenden ausgewählt wird/werden, nämlich, Halogen, $C_{1-4}$Alkyl, $-SO_2-NH_2$ und Phenyl;
b eine ganze Zahl von 0 bis 1 ist;
$L^1$ aus der Gruppe bestehend aus -O-, -S-, -NH, -$C_{1-4}$-Alkyl-und -$C_{2-4}$-Alkenyl-ausgewählt wird;
$R^3$ aus der Gruppe bestehend aus Folgenden ausgewählt wird, nämlich $C_{1-4}$-Alkyl, Cycloalkyl, Cycloalkyl-$C_{1-4}$-Alkyl-, Aryl, Heteroaryl und Heterocycloalkyl;
wobei das Cycloalkyl oder Aryl, entweder allein oder als Teil einer Substituentengruppe optional durch ein bis zwei Substituenten substituiert wird, der/die unabhängig aus einer Gruppe bestehend aus Folgenden ausgewählt wird/werden, nämlich Halogen, Hydroxy, Carboxy, Cyan, Nitro, Amino, $C_{1-4}$-Alkylamino, Di($C_{1-4}$-Alkylamino), $C_{1-4}$-Alkyl, $C_{1-4}$-Alkyl, $C_{1-4}$-Alkenyl, $C_{1-4}$-Alkoxy, Hydroxy-substituiertes $C_{1-4}$-Alkyl, Halogen-substituiertes $_{C1-}$4-Alkyl, Cyan-substituiertes $C_{1-4}$-Alkyl, Halogen-substituiertes $C_{1-4}$-Alkoxy, -O-Aryl" -O-$C_{1-4}$-Aralkyl, -S-$C_{1-4}$Alkyl, $SO_2$-$C_{1-4}$-Alkyl, C(O)- $C_{1-4}$-Alkyl, -C(O)-NH($C_{1-4}$-Alkyl), C(O)-N($C_{1-4}$-Alkyl), -NH-C(O)- $C_{1-4}$-Alkyl, -C(O)-NH- $C_{1-4}$-Alkyl, -NH-$SO_2$-$C_{1-4}$-Alkyl, -NH-$SO_2$-Phenyl, Aryl, $C_{1-4}$-Aralkyl, Cycloalkyl, Cycloalkyl-$C_{1-4}$-Alkyl, Heteroaryl, Heteroaryl- $C_{1-4}$-Alkyl, Heterocycloalkyl und Heterocycloalkyl- $C_{1-4}$-Alkyl;
wobei das Aryl, Cycloalkyl, Heteroaryl oder Heterocycloalkyl, entweder allein oder als Teil einer Substituentengruppe optional durch ein bis drei Substituenten substituiert wird, der/die unabhängig aus einer Gruppe bestehend aus Folgenden ausgewählt wird/werden, $C_{1-4}$-Alkyl und $C_{1-4}$-Alkoxy;
a 0 ist;
oder ein pharmazeutisch verträgliches Salze dieser.

**4.** Eine Verbindung entsprechend Anspruch 3,
wobei
$R^0$ Wasserstoff ist;
$R^1$ aus der Gruppe bestehend aus Wasserstoff, Hydroxy, Methyl, und Methylcarbonyl ausgewählt wird;
c eine ganze Zahl von 0 bis 1 ist;
$A^2$ aus der Gruppe bestehend aus $-CH_2-$, $-CH(CH_2CH_3)-$, CH(Phenyl)-, CH(Benzyl) und -CH(Cyclohexylmethyl ausgewählt wird;

Ⓡ

aus der Gruppe bestehend aus Cyclopentyl, (S)-Cyclopentyl, (R)-Cyclopentyl, Cyclohexyl, (R)-Cyclohexyl, (S)-Cyclohexyl, Transcyclohexyl, Phenyl, Benzyl, 9-Fluorenyl, 3-Pyrrolidinyl, 1-Indanyl, 1-(5-Methoxyindanyl)-Methyl, 3-Piperidinyl, 4-Piperidinyl, 3-Azepinyl, 2-Pyridyl, 4-Pirydil, 2-Furyl, 2-Thienyl und 5-Oxazolyl ausgewählt wird; vorausgesetzt, dass, wenn c 0 ist,

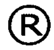

weder Phenyl, 2-Pyridyl, 4-Pirydil, 2-Furyl, 2-Thienyl noch 5-Oxayolyl ist;
$Q^3$ aus der Gruppe bestehend aus Folgenden ausgewählt wird, nämlich -C(O)-, - C(O)-NH-, -C(O)-N($CH_3$)-, -(R)-C(O)-N($CH_3$), - (S)-C(O)-N($CH_3$), -C(O)-N(Isopropyl)-, -C(O)-N(n-Propyl-, -C(O)-N(Isobutyl)-, -C(O)-N(2-Ethyl-n-Hexyl)-, -C(O)-N(Cyclohexyl)-, -C(O)-N(4-Methylcyclohexyl)-, -C(O)O-, -NH-C(O)- und -NH-C(O)O-;
$R^2$ aus der Gruppe bestehend aus Folgenden ausgewählt, nämlich Trifluormethyl, Methyl, Ethyl, Isobutyl, t-Butyl, 3-n-Heptyl, 4-n-Heptyl, 2-Ethyl-n-Hexyl, Cyclopentyl, Cyclohexyl, 4-Methylcyclohexyl, Cyclopropylmethyl, 4-Aminosulfonyl-Phenylethyl, Benzhydryl, 1-Adamantyl, 2-Adamantyl, 2-(R)-Adamantyl, 2-(S)-Adamantyl, 2-Decahydroxyquinolinyl, 2-(1-Methylpyrrolidinyl)-Ethyl, 1-Piperidinyl und 4-(1-Methylpiperidinyl);
b eine ganze Zahl von 0 bis 1 ist;
$L^1$ aus der Gruppe bestehend aus -O-, -S-, --CH($CH_3$)- und -CH=CH ausgewählt wird;
$R^3$ aus der Gruppe bestehend aus Folgenden ausgewählt wird, nämlich n-Pentyl, Isopentyl, Isobutyl, Isopropyl, Cyclopentyl, Cyclopentylmethyl, Phenyl, 2-Hydroxyphenyl, 3-Carboxyphenyl, 2-Cyanphenyl, 2-Nitrophenyl, 2-Bromphenyl, 2-Fluorphenyl, 2-Chlorphenyl, 2,6-Dichlorphenyl, 2-Methylphenyl, 3-Methylphenyl, 2-Ethylphenyl, 4-Isopropylphenyl, 3,4-Dimethylphenyl, 3,5-Dimethylphenyl, 2,6-Dimethylphenyl, 2-Methoxyphenyl, 3-Methoxyphenyl, 4-Methoxyphenyl, 3,4-Dimethoxyphenyl, 2,6-Dimethoxyphenyl, 2-Ethoxyphenyl, 3-Ethoxyphenyl, 2-Isopropoxyphenyl, 2-Methoxy-5-Methylphenyl, 2-Methoxy-6-Methylphenyl, 2-Trifluormethylphenyl, 2-Trifluormethoxyphenyl, 2-Methylthiophenyl, 4-Methylthiophenyl, 2-Hydroxymethylphenyl, 2-Cyanmethylphenyl, 2-(Aminocarbonyl)-Phenyl, 4-(Aminocarbonyl)-Phenyl, 2-(Dimethylaminocarbonyl)-Phenyl, 3-(Dimethylamino)-Phenyl, 4-(Dimethylamino)-Phenyl, 2-Allyl-6-Methylphenyl, 2-Allyl-6-Ethoxyphenyl, 2-Methyl-6-n-Propylphenyl, 3-(Methylcarbonylamino)-Phenyl, 2-(Methylaminocarbonyl)-Phenyl, 2-(Methylcarbonyl)-Phenyl, 4-(Methylcarbonylamino)-Phenyl, 2-(Aminocarbonylmethyl)-Phenyl, 2-(Methylsulfonyl)-Phenyl, (3-(2-Methoxy-4-Methylphenyl)-Sulfonylamino)-Phenyl, 3-(2,4,6-Trimethylphenylsulfonylamino)-Phenyl, 3-(Phenylsulfonylamino)-Phenyl, 2-(t-Butylaminosulfonyl)-Phenyl, 2-(t-Butylcarbonylamino)-5-Methoxyphenyl, 3-(Phenylsulfonylamino)-Phenyl, 2-Phenoxyphenyl, 3-Phenoxyphenyl, 2-Benzyloxyphenyl, 2-(2-Benzthiazolyl)-5-Methoxyphenyl, 2-(2-Benzthiazolyl)-Phenyl, 2-(1-Pyrrolyl)-Phenyl, 3-(2-Quinolinyl)-Phenyl, 2-(1-Pyrrolidinylmethyl)-Phenyl, 2-Cyclopentylphenyl, 4-Cyclohexylphenyl, 4-(4-Morpholinyl)-Phenyl, 3-Methoxybenzyl, 1-Naphthyl, 2-Naphthyl, 2-(5,6,7,8-Tetrahydronaphthyl), 2-Biphenyl, 3-Biphenyl, 2-Biphenylmethyl, 3-Pyridyl, 3,4-Methylendioxyphenyl, 4(3,5-Dimethyisoxazolyl), 4-Pyrazolyl, 3-Thienyl, 3-Pyridyl, 4-Pyridyl, 5-Indolyl und 3-Benzothienyl;
a 0 ist;
oder ein pharmazeutisch verträgliches Salz dieser.

**5.** Eine Verbindung entsprechend Anspruch 4,
wobei
$R^0$ Wasserstoff ist;
$R^1$ Wasserstoff ist;
c eine ganze Zahl von 0 bis 1 ist;
$A^2$ aus der Gruppe bestehend aus -$CH_2$- und -CH($CH_2CH_3$)- ausgewählt wird;

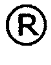

aus der Gruppe bestehend aus Phenyl, 4-Piperidinyl und 4-Pyridyl ausgewählt wird, vorausgesetzt dass, wenn c 0 ist,

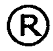

weder Phenyl noch 4-Pyridyl ist;
$Q^3$ aus der Gruppe bestehend aus Folgenden ausgewählt wird, nämlich -1-C(O)O-, - 3-C(O)O-, 2-C(O)-N($CH_3$)- und -3-C(O)-N($CH_3$);

$R^2$ aus der Gruppe bestehend aus Methyl, Ethyl, t-Butyl und Cyclohexyl ausgewählt wird;
b eine ganze Zahl von 0 bis 1 ist;
$L^1$ aus der Gruppe bestehend aus -O- und -S- ausgewählt wird,
$R^3$ aus der Gruppe bestehend aus Folgenden ausgewählt, nämlich Phenyl, 2-Bromphenyl, 2-Chlorphenyl, 2,6-Dichlorphenyl, 2-Hydroxyphenyl, 2-Hydroxymethylphenyl, 2-Methoxyphenyl, 3-Methoxyphenyl, 2-Ethoxyphenyl, 2-Methylthiophenyl, 2-Cyanmethylphenyl, 3-(Phenylsulfonylamino)-Phenyl, 3-(2,4,6-Trimethylphenylsulfonylamino)-Phenyl, (3-(2-Methoxy-4-Methylphenyl)-Sulfonylamino)-Phenyl, 2-(t-Butylcarbonylamino)-5-Methoxyphenyl, 1-Naphthyl, 3-Thienyl und 4-(3,5-Dimethylisoxazolyl);
a 0 ist;
oder ein pharmazeutisch verträgliches Salz dieser.

**6.** Eine Verbindung entsprechend Anspruch 5,
wobei
$R^0$ Wasserstoff ist;
$R^1$ Wasserstoff ist;
c eine ganze Zahl von 0 bis 1 ist;
$A^2$ aus der Gruppe bestehend aus $-CH_2-$ und $-CH(CH_2CH_3)-$ ausgewählt wird;

®

aus der Gruppe bestehend aus Phenyl, 4-Piperidinyl und 4-Pyridyl ausgewählt wird, vorausgesetzt dass, wenn c 0 ist,

®

4-Pyridyl ist;
$Q^3$ aus der Gruppe bestehend aus Folgenden ausgewählt wird, nämlich -1-C(O)O-, - 3-C(O)O- und $-3-C(O)-N(CH_3)$;
$R^2$ aus der Gruppe bestehend aus Methyl,, Ethyl, t-Butyl und Cyclohexyl ausgewählt wird;
b eine ganze Zahl von 0 bis 1 ist;
$L^1$ aus der Gruppe bestehend aus -O- und -S- ausgewählt wird,
$R^3$ aus der Gruppe bestehend aus Folgenden ausgewählt, nämlich Phenyl, 2-Bromphenyl, 2-Chlorphenyl, 2,6-Dichlorphenyl, 2-Hydroxyphenyl, 2-Hydroxymethylphenyl, 2-Methoxyphenyl, 3-Methoxyphenyl, 2-Ethoxyphenyl, 2-Methylthiophenyl, 2-Cyanmethylphenyl, 3-(Phenylsulfonylamino)-Phenyl, 1-Naphthyl und 3-Thienyl;
a 0 ist;
oder ein pharmazeutisch verträgliches Salz dieser.

**7.** Eine Verbindung entsprechend Anspruch 6,
wobei
$R^0$ Wasserstoff ist;
$R^1$ Wasserstoff ist;
c 1 ist;
$A^2$ $-CH_2-$ ist;

®

Phenyl ist;
$Q^3$ $-3-C(O)-N(CH_3)$ ist;
$R^2$ aus der Gruppe bestehend aus Methyl, Ethyl, t-Butyl und Cyclohexyl ausgewählt wird;
b 0 ist;
$R^3$ aus der Gruppe bestehend aus Folgenden ausgewählt, nämlich Phenyl, 2-Methoxyphenyl, 2-Ethoxyphenyl und 1-Naphthyl;
a 0 ist;
oder ein pharmazeutisch verträgliches Salz dieser.

8. Eine Verbindung entsprechend Anspruch 4,
wobei
$R^0$ Wasserstoff ist;
$R^1$ aus der Gruppe bestehend aus Wasserstoff und Methoxy ausgewählt wird;
c 1 ist;
$A^2$ aus der Gruppe bestehend aus $-CH_2-$, $-CH(CH_2CH_3)-$, CH(Phenyl)- und -CH-(Cyclohexyl)- ausgewählt wird;

Ⓡ

aus der Gruppe bestehend aus Phenyl, 2-Pyridyl und 2-Furyl ausgewählt wird;
$Q^3$ aus der Gruppe bestehend aus Folgenden ausgewählt wird, nämlich -3-C(O)-N($CH_3$)-, -3-C(O)-N(Isobutyl)-, -3-C(O)-N(Cyclohexyl)-, -4-C(O)-N($CH_3$)- und -5-C(O)-N($CH_3$);
$R^2$ aus der Gruppe bestehend aus Isobutyl und Cyclohexyl ausgewählt wird;
b eine ganze Zahl von 0 bis 1 ist;
$L^1$ aus der Gruppe bestehend aus -O-, -S- und -NH- ausgewählt wird;
$R^3$ aus der Gruppe bestehend aus Folgenden ausgewählt wird, nämlich Phenyl, 2-Bromphenyl, 2-Fluorphenyl, 2-Chlorphenyl, 2,6-Dichlorphenyl, 2-Hydroxyphenyl, 2-Hydroxymethylphenyl, 2 Methylphenyl, 3-Methylphenyl, 2,6-Dimethylphenyl, 2-Methoxyphenyl, 3-Methoxyphenyl, 2,6-Dimethoxyphenyl, 2-Ethoxyphenyl, 2-Trifluormethylphenyl, 2-Trifluormethoxyphenyl, 2-Methylthiophenyl, 2-Nitrophenyl, 2-Cyanphenyl, 2-Cyanmethylphenyl, 2-Phenoxyphenyl, 2-(Methylcarbonylamino)-Phenyl, 2-(Aminocarbonyl)-Phenyl, 2-(Methylcarbonyl)-Phenyl, 4-(Methylcarbonylamino)-Phenyl, 2-(Aminocarbonylmethyl)-Phenyl, 3-(Phenylsulfonylamino)-Phenyl, 2-(t-Butylaminosulfonyl)-Phenyl, 2-(t-Butylcarbonylamino)-5-Methoxyphenyl, 4(3,5-Dimethyisoxazolyl), 1-Naphthyl, 3-Thienyl und 3-Pyridyl;
a 0 ist;
oder ein pharmazeutisch verträgliches Salz dieser.

9. Eine Verbindung entsprechend Anspruch 8,
wobei
$R^0$ Wasserstoff ist;
$R^1$ Wasserstoff ist;
c 1 ist;
$A^2$ aus der Gruppe bestehend aus $-CH_2-$, $-CH(CH_2CH_3)-$ und -CH-(Cyclohexyl)-ausgewählt wird;

Ⓡ

aus der Gruppe bestehend aus Phenyl und 2-Pyridyl ausgewählt wird;
$Q^3$ aus der Gruppe bestehend aus Folgenden ausgewählt wird, nämlich -3-C(O)-N($CH_3$)-, -3-C(O)-N(Isopropyl)-, -3-C(O)-N(Cyclohexyl)- und -4-C(O)-N($CH_3$)- ;
$R^2$ Cyclohexyl ist;
b eine ganze Zahl von 0 bis 1 ist;
$L^1$ -O- ist;
$R^3$ aus der Gruppe bestehend aus Folgenden ausgewählt wird, nämlich Phenyl, 2-Bromphenyl, 2-Fluorphenyl, 2-Chlorphenyl, 2-Hydroxyphenyl, 2-Hydroxymethylphenyl, 3-Methylphenyl, 2-Methoxyphenyl, 2-Ethoxyphenyl, 2-Cyanmethylphenyl, 2-(t-Butylcarbonylamino)-5-Methoxyphenyl, 2-Naphthyl und 3-Thienyl;
a 0 ist;
oder ein pharmazeutisch verträgliches Salz dieser.

10. Eine Verbindung entsprechend Anspruch 9, wobei
$R^0$ Wasserstoff ist; $R^1$ Wasserstoff ist; c 1 ist; $A^2$ $-CH_2-$ ist,

Ⓡ

Phenyl ist; $Q^3$ -3-C(O)-N($CH_3$)- ist; $R^2$ Cyclohexyl ist; b 0 ist; $R^3$ aus der Gruppe bestehend aus 2-Methoxyphenyl und 2-Ethoxyphenyl ausgewählt wird; a 0 ist; oder ein pharmazeutisch verträgliches Salz dieser.

**11.** Eine pharmazeutische Zusammensetzung bestehend aus einem pharmazeutisch verträglichen Trägerstoff und einer Verbindung gemäß Anspruch 1.

**12.** Eine pharmazeutische Zusammensetzung, die durch Mischen einer Verbindung gemäß Anspruch 1 und einem pharmazeutische verträglichen Trägerstoff erstellt wird.

**13.** Ein Prozess zur Erstellung einer pharmazeutischen Zusammensetzung bestehend aus Mischen einer Verbindung gemäß Anspruch 1 und einem pharmazeutisch verträglichen Trägerstoff.

**14.** Eine Verbindung gemäß Anspruch 1 zur Verwendung bei der Behandlung einer durch β-Sekretase vermittelten Krankheit,
wobei die durch β-Sekretase vermittelte Krankheit aus der Gruppe bestehend aus Folgenden ausgewählt wird, nämlich Alzheimer-Krankheit (AD), milde kognitive Störung, Senilität, Demenz, Lewy-Körper-Demenz, Down-Syndrom, mit Parkinson-Krankheit assoziierte Demenz und mit Beta-Amyloid assoziierte Demenz.

**15.** Eine Verbindung gemäß Anspruch 11 zur Verwendung bei der Behandlung einer durch β-Sekretase vermittelten Krankheit,
wobei die durch β-Sekretase vermittelte Krankheit aus der Gruppe bestehend aus Folgenden ausgewählt wird, nämlich Alzheimer-Krankheit (AD), milde kognitive Störung, Senilität, Demenz, Lewy-Körper-Demenz, Down-Syndrom, mit Parkinson-Krankheit assoziierte Demenz und mit Beta-Amyloid assoziierte Demenz.

**16.** Die Verwendung einer Verbindung entsprechend Anspruch 1 zur Vorbereitung eines Medikaments zur Behandlung von (a) Alzheimer-Krankheit (AD), (b) milder kognitive Störung, (c) Senilität, (d) Demenz, (e) Lewy-Körper-Demenz, (f) Down-Syndrom, (g) mit Parkinson-Krankheit assoziierte Demenz und (h) mit Beta-Amyloid assoziierte Demenz bei einem Patienten, der dessen bedarf:

**17.** Eine Verbindung mit der Formel (II)

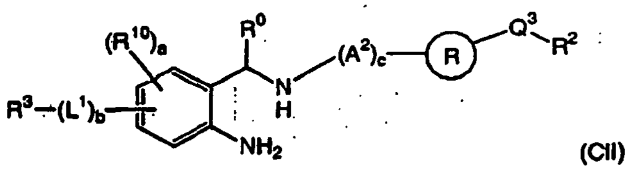

(CII)

wobei

$R^0$ aus der Gruppe bestehend aus Wasserstoff, Methyl und $CF_3$ ausgewählt wird;
c eine ganze Zahl von 0 bis 1 ist;
$A^2$ aus der Gruppe bestehend aus $C_{1-4}$-Alkyl ausgewählt wird; wobei $C_{1-4}$-Alkyl optional durch einen oder mehrere $R^Y$-Substituenten substituiert wird;
wobei jedes $R^Y$ unabhängig aus der Gruppe bestehend aus Folgenden ausgewählt wird, nämlich Hydroxy, Oxo, $C_{1-4}$-Alkyl, $C_{1-4}$-Alkoxy, Hydroxy-substituiertes $C_{1-4}$-Alkyl, Amino-substituiertes $C_{1-6}$-Alkyl, Cycloalkyl, Cycloalkyl- $C_{1-4}$-Alkyl-, Biphenyl, Aryl, $C_{1-4}$-Aralkyl, Heteroaryl, Heteroaryl-$C_{1-4}$-Alkyl-, Heterocycloalkyl, Heterocycloalkyl- $C_{1-4}$-Alkyl- oder Spiro-heterocyclyl;
wobei die Cycloalkyl-, Aryl-, Heteroaryl-, Heterocycloalkyl- oder Spiro-Gruppe, entweder allein oder als Teil einer Substituentengruppe optional durch einen oder mehrere Substituenten substituiert wird, der/die unabhängig aus der Gruppe bestehend aus folgenden ausgewählt wird/werden, nämlich Fluor, Chlor, Hydroxy, Oxo, Carboxy, $C_{1-4}$-Alkyl, $C_{1-4}$-Alkoxy, -C(O)O-($C_{1-4}$-Alkyl), Hydroxy-substituiertes $C_{1-4}$-Alkyl, Fluor-substituiertes $C_{1-4}$-Alkyl, Fluor substituiertes $C_{-1-4}$-Alkoxy, 5-Tetrazolyl oder 1-(1,4-dihydrotetrazol-5-on);

®

aus der Gruppe bestehend aus Aryl, Aryl- $C_{1-4}$-Alkyl, Cycloalkyl, teilweise ungesättigtes Carbocyclyl, Heteroaryl, Heterocycloalkyl oder Spiroheterocyclyl ausgewählt wird;

wobei die Aryl-, Cycloalkyl-, teilweise ungesättigte Carbocyclyl-, Heteroaryl-Heteracycloalkyl- oder Spiroheterocyclyl-Gruppe, entweder allein oder als Teil einer Substituentengruppe, optional durch einen oder mehrere Substituenten substituiert wird, der/die unabhängig aus der Gruppe bestehend aus Folgenden ausgewählt wird/werden, nämlich $C_{1-4}$-Alkyl, $C_{1-4}$-Alkoxy, Halogen-substituiertes $C_{1-4}$-Alkyl, Halogen-substituiertes $C_{1-4}$-Alkoxy, Hydroxy-substituiertes $C_{1-4}$-Alkyl, Hydroxy, Carboxy, Cyan, Nitro, Amino, $C_{1-4}$-Alkylamino und Di($C_{1-4}$-Alkyl) amino;

vorausgesetzt dass, wenn c 0 ist,

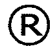

weder Aryl noch Heteroaryl ist;

$Q^3$ aus der Gruppe bestehend aus -C(O)-, -C(O)O-, C(O)-NH-, -C(O)-N($C_{1-8}$-Alkyl)-, - C(O)-N(Cycloalkyl)-, -NH-C(O)- und -NH-C(O)O- ausgewählt wird; wobei das Cycloalkyl optional durch $C_{1-4}$-Alkyl substituiert wird;

$R^2$ aus der Gruppe bestehend aus $C_{1-8}$-Alkyl, Cycloalkyl, Aryl, Biphenyl, teilweise ungesättigtes Carbocyclyl, Heteroaryl, Heterocycloalkyl, Cycloalkyl-$C_{1-4}$-Alkyl-, $C_{1-4}$-Aralkyl, teilweise ungesättigtes Carbocyclyl-$C_{1-4}$-Alkyl, Heteroaryl- $C_{1-4}$-Alkyl, Heterocycloalkyl- $C_{1-4}$-Alkyl- oder Spiroheterocyclyl ausgewählt wird;

wobei das $C_{1-10}$-Alkyl, Cycloalkyl, Aryl, teilweise ungesättigte Carbocyclyl, Heteroaryl, Heterocycloalkyl oder Spiroheterocyclyl, entweder allein oder als Teil einer Substituentengruppe, optional durch einen oder mehrere Substituenten substituiert wird, die aus der Gruppe bestehend aus folgenden ausgewählt wird/werden, nämlich, Halogen, Hydroxy, Oxo, Carboxy, -C(O)-$C_{1-4}$Alkyl, -C(O)-$C_{1-4}$-Aralkyl, -C(O)O-$C_{1-4}$-Alkyl, -C(O)O-$C_{1-4}$-Aralkyl, -C-$_{1-4}$-Alkyl-C(O)O-$C_{1-4}$-Alkyl, -$C_{1-4}$-Alkyl-S-$C_{1-4}$-Alkyl, -C(O)-N($R^LR^M$), -$C_{1-4}$-Alkyl-C(O)-N($R^LR^M$), -$NR^L$-C (O)-$C_{1-4}$-Alkyl, -$SO_2$-N($R^LR^M$), -$C_{1-4}$-Alkyl-$SO_2$-N($R^LR^M$), $C_{1-6}$-Alkyl, Fluor-substituiertes $C_{1-4}$-Alkyl, Hydroxy-substituiertes $C_{1-4}$-Alkyl, Carboxy-substituiertes $C_{1-4}$-Alkyl, $C_{1-4}$-Alkoxy, -O-$C_{1-4}$-Aralkyl, -O-(Tetrahydropyranyl), NH-C(O)O-$CH_2$-(Tetrahydropyranyl), -N($CH_3$)-C(O)O-$CH_2$-Tetrahydropyranyl), Nitro, Cyan, Amino, $C_{1-4}$-Alkylamino, Di($C_{1-4}$-Alkyl)amino, Phenyl, 5-Tetrazolyl und 1-(1,4-dihydrotetrazol-5-on);

wobei das Phenyl oder Tetrahydropyranyl optional durch einen oder mehrere Substituenten substituiert wird, der/die unabhängig aus der Gruppe bestehend aus Folgenden ausgewählt wird/werden, nämlich Halogen, Hydroxy, Oxo, Carboxy, -C(O)O-$C_{1-4}$Alkyl, -C(O)-$C_{1-4}$Alkyl, -OC(O)-$C_{1-4}$Alkyl, -$C_{1-4}$Alkyl-OC(O)-$C_{1-4}$-Alkyl, -O-$C_{1-4}$Aralkyl, $C_{1-4}$Alkyl, Fluor-substituiertes $C_{1-4}$-Alkyl, Hydroxy-substituiertes $C_{1-4}$-Alkyl, $C_{1-4}$-Alkoxy, Nitro, Cyan, Amino, $C_{1-4}$-Alkylamino und Di($C_{1-4}$-Alkyl)amino;

wobei $R^L$ und $R^M$ jeweils unabhängig aus der Gruppe bestehend aus Wasserstoff und $C_{1-4}$Alkyl ausgewählt werden;

b eine ganze Zahl von 0 bis 1 ist;

$L^1$ aus der Gruppe bestehend aus -O-, -S(O)$_{0-2}$-, -$NR^N$-, -C(O)-, -C(S)-, -$C_{1-4}$-Alkyl-, - (Hydroxy-substituiertes $C_{1-4}$Alkyl)- und -($C_{2-4}$-Alkenyl)- ausgewählt wird;

wobei $R^N$ aus der Gruppe bestehend aus Wasserstoff und $C_{1-4}$-Alkyl ausgewählt wird;

$R^3$ aus der Gruppe bestehend aus Folgenden ausgewählt wird, nämlich $C_{1-4}$-Alkyl, $C_{2-6}$-Alkenyl, Cycloalkyl, teilweise ungesättigtes Carbocyclyl, Aryl, Biphenyl, Heteroaryl, Heterocycloalkyl, Cycloalkyl-$C_{1-4}$-Alkyl-, $C_{1-4}$-Aralkyl, Heteroaryl-$C_{1-4}$-Alkyl-, Heterocycloalkyl-$C_{1-4}$-Alkyl- und Spiroheterocyclyl;

wobei das $C_{1-6}$-Alkyl, Cycloalkyl, teilweise ungesättigte Carbocyclyl, Aryl, Heteroaryl, Heterocycloalkyl oder Spiroheterocyclyl, entweder allein oder als Teil einer Substituentengruppe optional durch einen oder mehrere Substituenten substituiert wird, der/die unabhängig aus einer Gruppe bestehend aus Folgenden ausgewählt wird/werden, nämlich Halogen, Hydroxy, Oxo, Carboxy, $C_{1-4}$-Alkyl, Hydroxy-substituiertes $C_{1-4}$-Alkyl, Halogen-substituiertes $C_{1-4}$-Alkyl, Cyan-substituiertes $C_{1-4}$-Alkyl, $C_{2-4}$-Alkenyl, $C_{2-4}$-Alkynyl, $C_{1-4}$-Alkoxy, Halogen-substituiertes $C_{1-4}$-Alkoxy, Nitro, Cyan, -$R^5$, -$OR^5$, -S-$R^5$, -$SO_2$-$R^5$, -$SO_2$-$NR^P$-$R^5$, -$NR^P$-$SO_2$-$R^5$, -$NH_2$, -N($R^P$)-$R^5$, -C(O)-$R^5$, -C(O)-$NH_2$, -C(O)-$NR^P$-$R^5$, -$NR^P$-C(O)-$R^5$ und-$NR^P$-C(O)O-$R^5$;

wobei $R^5$ aus der Gruppe bestehend aus Folgenden ausgewählt wird, nämlich $C_{1-4}$-Alkyl, Aryl, $C_{1-4}$-Aralkyl, teilweise ungesättigtes Carbocyclyl, Cycloalkyl, Cycloalkyl-$C_{1-4}$-Alkyl -, teilweise ungesättigtes Carbocyclyl-$C_{1-4}$-Alkyl-, Heteroaryl, Heteroaryl-$C_{1-4}$-Alkyl-, Heterocycloalkyl und Heterocyclyl-$C_{1-4}$-Alkyl-:

wobei das Aryl, teilweise ungesättigte Carbocyclyl, Cycloalkyl, Heteroaryl oder Heterocycloalkyl, entweder allein oder als Teil einer Substituentengruppe optional durch einen oder mehrere Substituenten substituiert wird, der/die unabhängig aus einer Gruppe bestehend aus Folgenden ausgewählt wird/werden, $C_{1-4}$-Alkyl, $C_{1-4}$-Alkoxy, Halogen, Hydroxy, Carboxy, Amino, $C_{1-4}$-Alkylamino, Di($C_{1-4}$-Alkyl)amino, Cyan und Nitro;

wobei $R^P$ aus der Gruppe bestehend aus Folgenden ausgewählt wird, nämlich Wasserstoff, $C_{1-8}$-Alkyl, Hydroxy-

substituiertes $C_{1-4}$-Alkyl, $C_{1-4}$-Aralkyloxy-substituiertes $C_{1-4}$-Alkyl, Cycloalkyl, Aryl, Heteroaryl, Heterocycloalkyl, Cycloalkyl-$C_{1-4}$-Alkyl-, $C_{1-4}$-Aralkyl, Heteroaryl-$C_{1-4}$-Alkyl-, Heterocycloalkyl-$C_{1-4}$-Alkyl- und Spiroheterocyclyl; wobei das Cycloalkyl, Aryl, Heteroaryl, Heterocycloalkyl oder Spiroheterocyclyl, entweder allein oder als Teil einer Substituentengruppe optional durch einen oder mehrere Substituenten substituiert wird, der/die unabhängig aus einer Gruppe bestehend aus Folgenden ausgewählt wird/werden, nämlich Halogen, Hydroxy, Oxo, Carboxy, $C_{1-4}$-Alkyl, $C_{1-4}$-Alkoxy, $C_{1-4}$-Alkoxycarbonyl, Nitro, Cyan, Amino, $C_{1-4}$-Alkylamino, Di($C_{1-4}$-Alkyl)amino,-$SO_2$-N($R^SR^T$), 5-Tetrazolyl und 1-(1,4-Dihydrotetrazol-5-on);
wobei $R^S$ und $R^T$ jeweils unabhängig aus der Gruppe bestehend aus Wasserstoff und $C_{1-4}$-Alkyl ausgewählt werden;
a eine ganze Zahl von 0 bis 3 ist;
jedes $R^{10}$ unabhängig aus der Gruppe bestehend aus Hydroxy, Halogen, $C_{1-4}$-Alkyl, $C_{1-4}$-Alkoxy, Halogen-substituiertes $C_{1-4}$-Alkyl, Halogen-substituiertes $C_{1-4}$-Alkoxy, -C(O)-$NR^VR^W$, - $SO_2$-$NR^VR^W$, -C(O)-$C_{1-4}$-Alkyl und -$SO_2$-$C_{1-4}$-Alkyl ausgewählt wird;
wobei $R^V$ und $R^W$ jeweils unabhängig aus der Gruppe bestehend aus Wasserstoff und $C_{1-4}$-Alkyl ausgewählt werden; alternativ $R^v$ und $R^W$ zusammen mit dem N-Atom, an das sie gebunden sind, genommen werden, um eine 5- bis 6-gliedrige gesättigte, teilweise ungesättigte oder aromatische Ringstruktur zu bilden,
vorausgesetzt, dass die Halogene am Halogen-substituierten $C_{1-4}$-Alkyl oder Halogen-substituierten $C_{1-4}$-Alkoxy aus der Gruppe bestehend aus Chlor und Fluor ausgewählt werden;
oder ein pharmazeutisch verträgliches Salze dieser.

## Revendications

**1.** Un composé de la formule (II)

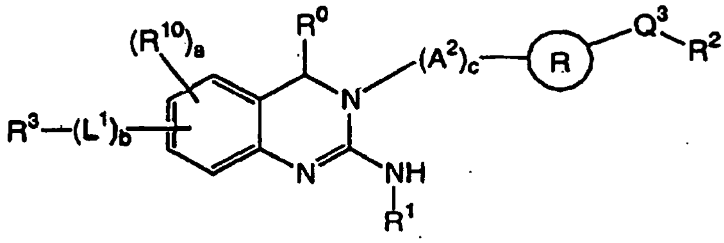

(II)

où

$R^0$ est sélectionné dans le groupe se composant d'hydrogène, méthyle et $CF_3$,
$R^1$ est sélectionné dans le groupe se composant d'hydrogène, hydroxy, méthyle, éthyle, trifluorométhyle, 2,2,2-trifluoroéthyle, méthoxy, éthoxy et méthyle-carbonyle,
c est un entier de 0 à 1,
$A^2$ est sélectionné dans le groupe se composant de $C_{1-4}$alkyle, où le $C_{1-4}$alkyle est éventuellement substitué par un ou plusieurs substituants $R^Y$,
où chaque $R^Y$ est sélectionné indépendamment dans le groupe se composant d'hydroxy, oxo, $C_{1-4}$alkyle, $C_{1-4}$alkoxy, $C_{1-4}$alkyle substitué par hydroxy, $C_{1-6}$alkyle substitué par amino, cycloalkyle, cycloalkyle-$C_{1-4}$alkyle-, biphényle, aryle, $C_{1-4}$aralkyle, hétéroaryle, hétéroaryle-$C_{1-4}$alkyle-, hétérocycloalkyle, hétérocycloalkyle-$C_{1-4}$alkyle ou spiro-hétérocyclyle,
où le groupe cycloalkyle, aryle, hétéroaryle, hétérocycloalkyle ou spiro- hétérocyclyle, soit seul ou en tant que partie d'un groupe substituant est éventuellement substitué par un ou plusieurs substituants sélectionnés indépendamment dans le groupe se composant de fluoro, chloro, hydroxy, oxo, carboxy, $C_{1-4}$alkyle, $C_{1-4}$alkoxy, -C(O)O-($C_{1-4}$alkyle), $C_{1-4}$alkyle substitué par hydroxy, $C_{1-4}$alkyle substitué par fluoro, $C_{1-4}$alkoxy substitué par fluoro, 5-tétrazolyle ou 1-(1,4-dihydro-tétrazole-5-one),

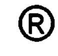

est sélectionné dans le groupe se composant d'aryle, aryle-$C_{1-4}$alkyle, cycloalkyle, carbocyclyle partiellement non saturé, hétéroaryle, hétérocycloalkyle et spiro-hétérocyclyle,
où le groupe aryle, cycloalkyle, carbocyclyle partiellement non saturé, hétéroaryle, hétérocycloalkyle ou spiro-

hétérocyclyle, soit seul ou en tant que partie d'un groupe substituant, est éventuellement substitué par un ou plusieurs substituants sélectionnés indépendamment dans le groupe se composant de $C_{1-4}$alkyle, $C_{1-4}$alkoxy, $C_{1-4}$alkyle substitué par halogène, $C_{1-4}$alkoxy substitué par halogène, $C_{1-4}$alkyle substitué par hydroxy, hydroxy, carboxy, cyano, nitro, amino, $C_{1-4}$alkylamino et di($C_{1-4}$alkyle)amino ;

à condition que, lorsque c est 0, alors

®

est autre qu'aryle ou hétéroaryle,

$Q^3$ est sélectionné dans le groupe se composant de -C(O)-, -C(O)O-, -C(O)-NH-, - C(O)-N($C_{1-8}$,alkyle)-, -C (O)-N(cycloalkyle)-, -NH-C(O)- et -NH-C(O)O-, où le cycloalkyle est éventuellement substitué par $C_{1-4}$alkyle,

$R^2$ est sélectionné dans le groupe se composant de $C_{1-10}$alkyle, cycloalkyle, aryle, biphényle, carbocyclyle partiellement non saturé, hétéroaryle, hétérocycloalkyle, cycloalkyle-$C_{1-4}$alkyle-, $C_{1-4}$aralkyle, carbocyclyle-$C_{1-4}$alkyle- partiellement non saturé, hétéroaryle-$C_{1-4}$alkyle-, hétérocycloalkyle-$C_{1-4}$alkyle- et spiro-hétérocyclyle,

où le $C_{1-10}$alkyle, cycloalkyle, aryle, carbocyclyle partiellement non saturé, hétéroaryle, hétérocycloalkyle ou spiro-hétérocyclyle, soit seul ou en tant que partie d'un groupe substituant est éventuellement substitué par un ou plusieurs substituants indépendamment sélectionnés dans le groupe se composant d'halogène, hydroxy, oxo, carboxy, -C(O)-$C_{1-4}$alkyle, -C(O)-$C_{1-4}$aralkyle, -C(O)O-$C_{1-4}$alkyle, -C(O)O-$C_{1-4}$aralkyle, -$C_{1-4}$alkyle-C(O) O-$C_{1-4}$alkyle, -$C_{1-4}$alkyle-S-$C_{1-4}$alkyle, -C(O)-N($R^LR^M$), -$C_{1-4}$alkyle-C(O)-N($R^LR^M$), - $NR^L$-C(O)-$C_{1-4}$alkyle, -$SO_2$-N($R^LR^M$), -$C_{1-4}$alkyle-$SO_2$-N($R^LR^M$), $C_{1-4}$alkyle, $C_{1-4}$alkyle substitué par fluoro, $C_{1-4}$alkyle substitué par hydroxy, $C_{1-4}$alkyle substitué par carboxy, $C_{1-4}$alkoxy, -O$C_{1-4}$aralkyle, -O-(tétrahydropyranyle), -NH-C(O)O-$CH_2$(tétrahydropyranyle), -N($CH_3$)-C(O)O-$CH_2$-(tétrahydropyranyle), nitro, cyano, amino, $C_{1-4}$alkylamino, di ($C_{1-4}$alkyle)amino, phényle, 5-tétrazolyle et 1-(1,4-dihydro-tétrazole-5-one),

où le phényle ou le tétrahydropyranyle est éventuellement substitué par un ou plusieurs substituants sélectionnés indépendamment dans le groupe se composant d'halogène, hydroxy, oxo, carboxy, C-(O)O-$C_{1-4}$alkyle, C (O)-$C_{1-4}$alkyle, OC(O)-$C_{1-4}$alkyle, -$C_{1-4}$alkyle-OC(O)-$C_{1-4}$alkyle, -O-$C_{1-4}$aralkyle, $C_{1-4}$alkyle, $C_{1-4}$alkyle substitué par fluoro, $C_{1-4}$alkyle substitué par hydroxy, $C_{1-4}$alkoxy, nitro, cyano, amino, $C_{1-4}$alkylamino et di($C_{1-4}$alkyle) amino,

où chaque $R^L$ et $R^M$ est sélectionné indépendamment dans le groupe se composant d'hydrogène et $C_{1-4}$alkyle,

b est un entier de 0 à 1,

$L^1$ est sélectionné dans le groupe se composant de -O-, -S(O)$_{0-2}$-, -$NR^N$, -C(O)-, - C(S)-, -$C_{1-4}$alkyle-, -($C_{1-4}$alkyle substitué par hydroxy)- et -($C_{2-4}$alcényle)-,

où $R^N$ est sélectionné dans le groupe se composant d'hydrogène et $C_{1-4}$alkyle,

$R^3$ est sélectionné dans le groupe se composant de $C_{1-6}$alkyle, $C_{2-6}$alcényle, cycloalkyle, carbocyclyle partiellement non saturé, aryle, biphényle, hétéroaryle, hétérocycloalkyle, cycloalkyle $C_{1-4}$alkyle-, $C_{1-4}$aralkyle, hétéroaryle-$C_{1-4}$alkyle-, hétérocycloalkyle-$C_{1-4}$alkyle- et spiro-hétérocyclyle,

où le $C_{1-4}$alkyle, cycloalkyle, carbocyclyle partiellement non saturé, aryle, hétéroaryle, hétérocycloalkyle ou spiro-hétérocyclyle, soit seul ou en tant que partie d'un groupe substituant est éventuellement substitué par un ou plusieurs substituants sélectionnés indépendamment dans le groupe se composant d'halogène, hydroxy, oxo, carboxy, $C_{1-4}$alkyle, $C_{1-4}$alkyle substitué par hydroxy, $C_{1-4}$alkyle substitué par halogène, $C_{1-4}$alkyle substitué par cyano, $C_{2-4}$alcényle, $C_{2-4}$alcynyle, $C_{1-4}$alkoxy, $C_{1-4}$alkoxy substitué par halogène, nitro, cyano -$R^5$, -O-$R^5$, -S-$R^5$, -$SO_2$-$R^5$, -$SO_2$,-$NR^P$-$R^5$, $NR^P$-$SO_2$-$R^5$, $NH_2$, -N($R^P$)-$R^5$, -C(O)-$R^5$, -C(O)-$NH_2$, -C(O)-$NR^P$-$R^5$, -$NR^P$-C (O)-$R^5$ et -$NR^P$-C(O)O-$R^5$,

où $R^5$ est sélectionné dans le groupe se composant de $C_{1-4}$alkyle, aryle, $C_{1-4}$aralkyle, carbocyclyle partiellement non saturé, cycloalkyle, cycloalkyle-$C_{1-4}$alkyle-, carbocyclyle$C_{1-4}$alkyle- partiellement non saturé, hétéroaryle, hétéroaryle-$C_{1-4}$alkyle-, hétérocycloalkyle et hétérocyclyle-$C_{1-4}$alkyle,

où l'aryle, carbocyclyle partiellement non saturé, cycloalkyle, hétéroaryle ou hétérocycloalkyle, soit seul ou en tant que partie d'un groupe substituant est éventuellement substitué par un ou plusieurs substituants indépendamment sélectionnés dans le groupe se composant de $C_{1-4}$alkyle, $C_{1-4}$alkoxy, halogène, hydroxy, carboxy, amino, $C_{1-4}$alkylamino, di($C_{1-4}$alkyle)amino, cyano et nitro,

où $R^P$ est sélectionné dans le groupe se composant d'hydrogène, $C_{1-8}$alkyle, $C_{1-4}$alkyle substitué par hydroxy, $C_{1-4}$alkyle substitué par $C_{1-4}$aralkyloxy, cycloalkyle, aryle, hétéroaryle, hétérocycloalkyle, cycloalkyle-$C_{1-4}$alkyle-, $C_{1-4}$aralkyle, hétéroaryle-$C_{1-4}$alkyle-, hétérocycloalkyle $C_{1-4}$alkyle- et spiro-hétérocyclyle,

où le cycloalkyle, aryle, hétéroaryle, hétérocycloalkyle ou spiro-hétérocyclyle, soit seul ou en tant que partie d'un groupe substituant est éventuellement substitué par un ou plusieurs substituants indépendamment sélectionnés dans le groupe se composant d'halogène, hydroxy, oxo, carboxy, $C_{1-4}$alkyle, $C_{1-4}$alkoxy, $C_{1-4}$alkoxy-

carbonyle, nitro, cyano, amino, $C_{1-4}$alkylamino, di($C_{1-4}$alkyle)amino, -$SO_2$-N($R^SR^T$), 5-tétrazolyle et 1-(1,4-dihydrotétrazole-5-one),
où chaque $R^S$ et $R^T$ est indépendamment sélectionné dans le groupe se composant d'hydrogène et $C_{1-4}$alkyle,
a est un entier de 0 à 3,
chaque $R^{10}$ est sélectionné indépendamment dans le groupe se composant d'hydroxy, halogène, $C_{1-4}$alkyle, $C_{1-4}$alkoxy, $C_{1-4}$alkyle substitué par halogène, $C_{1-4}$alkoxy substitué par halogène, -C(O)-$NR^VR^W$, -$SO_2$-$NR^VR^W$, -C(O)-$C_{1-4}$alkyle et -$SO_2$-$C_{1-4}$alkyle,
où chaque $R^V$ et $R^W$ est sélectionné indépendamment dans le groupe se composant d'hydrogène et $C_{1-4}$alkyle, dans une variante, $R^V$ et $R^W$ sont pris conjointement avec l'atome N auquel ils sont liés de façon à former une structure en anneau aromatique, saturée ou partiellement non saturée de 5 à 6 chaînons,
à condition que les halogènes sur le $C_{1-4}$alkyle substitué par halogène ou le $C_{1-4}$alkoxy substitué par halogène soient sélectionnés dans le groupe se composant de chloro et fluoro,
ou un sel pharmaceutiquement acceptable de ceux-ci.

**2.** Un composé selon la Revendication 1 où
$R^0$ est sélectionné dans le groupe se composant d'hydrogène et méthyle,
$R^1$ est sélectionné dans le groupe se composant d'hydrogène, méthyle, trifluorométhyle, méthoxy et méthylecarbonyle,
c est un entier de 0 à 1,
$A^2$ est sélectionné dans le groupe se composant de $C_{1-4}$alkyle, où le $C_{1-4}$alkyle est éventuellement substitué par un à deux substituants $R^Y$,
où chaque $R^Y$ est sélectionné indépendamment dans le groupe se composant d'hydroxy, $C_{1-4}$alkyle, $C_{1-4}$alkyle substitué par hydroxy, $C_{1-4}$alkyle substitué par amino, cycloalkyle, cycloalkyle-$C_{1-4}$alkyle-, aryle, $C_{1-4}$aralkyle, hétéroaryle, hétéroaryle-$C_{1-4}$alkyle-, hétérocycloalkyle et hétérocycloalkyle-$C_{1-4}$alkyle,
où le groupe cycloalkyle, aryle, hétéroaryle ou hétérocycloalkyle, soit seul ou en tant que partie d'un groupe substituant est éventuellement substitué par un ou plusieurs substituants sélectionnés indépendamment dans le groupe se composant de fluoro, chloro, hydroxy, oxo, carboxy, $C_{1-4}$alkyle, $C_{1-4}$alkoxy, -C(O)O-($C_{1-4}$alkyle), $C_{1-4}$alkyle substitué par hydroxy, $C_{1-4}$alkyle substitué par fluoro et $C_{1-4}$alkoxy substitué par fluoro,

®

est sélectionné dans le groupe se composant d'aryle, aryle-$C_{1-4}$alkyle-, cycloalkyle, hétéroaryle et hétérocycloalkyle, où le groupe aryle, cycloalkyle, hétéroaryle ou hétérocycloalkyle, soit seul ou en tant que partie d'un groupe substituant, est éventuellement substitué par un à deux substituants sélectionnés indépendamment dans le groupe se composant de $C_{1-4}$alkyle, $C_{1-4}$alkoxy, $C_{1-4}$alkyle substitué par halogène, $C_{1-4}$alkoxy substitué par halogène, $C_{1-4}$alkyle substitué par hydroxy, hydroxy, carboxy, cyano, nitro, amino, $C_{1-4}$alkylamino et di($C_{1-4}$alkyle)amino,
à condition que, lorsque c est 0, alors

®

est autre qu'aryle ou hétéroaryle,
$R^2$ est sélectionné dans le groupe se composant de $C_{1-8}$alkyle, cycloalkyle, aryle, hétéroaryle, hétérocycloalkyle, cycloalkyle-$C_{1-4}$alkyle-, $C_{1-4}$aralkyle, carbocyclyle-$C_{1-4}$alkyle-partiellement non saturé, hétéroaryle-$C_{1-4}$alkyle- et hétérocycloalkyle-$C_{1-4}$alkyle-,
où le $C_{1-4}$alkyle, cycloalkyle, aryle, hétéroaryle, hétérocycloalkyle ou spiro-hétérocyclyle, soit seul ou en tant que partie d'un groupe substituant est éventuellement substitué par un ou plusieurs substituants sélectionnés indépendamment dans le groupe se composant d'halogène, hydroxy, carboxy, -C(O)-$C_{1-4}$alkyle, -C(O)-$C_{1-4}$aralkyle, -C(O)O-$C_{1-4}$alkyle, -C(O)O-$C_{1-4}$aralkyle, -C(O)-N($R^LR^M$), -$NR^L$-C(O)-$C_{1-4}$alkyle, -$SO_2$-N($R^LR^M$) $C_{1-6}$alkyle, $C_{1-4}$alkyle substitué par fluoro, $C_{1-4}$alkyle substitué par hydroxy, $C_{1-4}$alkyle substitué par carboxy, $C_{1-4}$alkoxy, nitro, cyano, amino, $C_{1-4}$alkylamino, di($C_{1-4}$alkyle)amino et phényle,
où le phényle est éventuellement substitué par un à deux substituants sélectionnés indépendamment dans le groupe se composant d'halogène, hydroxy, carboxy, -C(O)-$C_{1-4}$alkyle, $C_{1-4}$alkyle, $C_{1-4}$alkyle substitué par fluoro, $C_{1-4}$alkoxy, nitro, cyano, amino, $C_{1-4}$alkylamino et di($C_{1-4}$alkyle)amino,
où chaque $R^L$ et $R^M$ est sélectionné indépendamment dans le groupe se composant d'hydrogène et $C_{1-4}$alkyle,
b est un entier sélectionné de 0 à 1,
$L^1$ est sélectionné dans le groupe se composant de-O-, -$S(O)_{0-2}$-, -$NR^N$-, $C_{1-4}$alkyle-, - ($C_{1-4}$alkyle substitué par

hydroxy)- et -($C_{2-4}$alcényle),
où $R^N$ est sélectionné dans le groupe se composant d'hydrogène et $C_{1-4}$alkyle,
$R^3$ est sélectionné dans le groupe se composant de $C_{1-6}$alkyle, cycloalkyle, aryle, hétéroaryle, hétérocycloalkyle, cycloalkyle-$C_{1-4}$alkyle-, $C_{1-4}$aralkyle, hétéroaryle-$C_{1-4}$alkyle- et hétérocycloalkyle-$C_{1-4}$alkyle-,
où le $C_{1-6}$alkyle, cycloalkyle, carbocyclyle partiellement non saturé, aryle, hétéroaryle ou hétérocycloalkyle, soit seul ou en tant que partie d'un groupe substituant est éventuellement substitué par un ou plusieurs substituants sélectionnés indépendamment dans le groupe se composant d'halogène, hydroxy, carboxy, $C_{1-4}$alkyle, $C_{1-4}$alkyle substitué par hydroxy, $C_{1-4}$alkyle substitué par halogène, $C_{1-4}$alkyle substitué par cyano, $C_{2-4}$alcényle, $C_{2-4}$alcynyle, $C_{1-4}$alkoxy, $C_{1-4}$alkoxy substitué par halogène, nitro, cyano, $-R^5$, $-O-R^5$, $-S-R^5$, - $SO_2-R^5$, $-SO_2-NR^P-R^5$, $-NR^P-SO_2-R^5$, $-NH_2$, $-N(R^P)-R^5$, $-C(O)R^5$, $-C(O)-NH_2$, $-C(O)-NR^P-R^5$ et $NR^P-C(O)-R^5$,
où $R^5$ est sélectionné dans le groupe se composant de $C_{1-4}$alkyle, aryle, $C_{1-4}$aralkyle, cycloalkyle, cycloalkyle$C_{1-4}$alkyle-, carbocyclyle-$C_{1-4}$alkyle partiellement non saturé, hétéroaryle, hétéroaryle-$C_{1-4}$alkyle-, hétérocycloalkyle et hétérocyclyle-$C_{1-4}$alkyle-,
où l'aryle, $C_{1-4}$aralkyle, carbocyclyle partiellement non saturé, cycloalkyle, hétéroaryle ou hétérocycloalkyle, soit seul ou en tant que partie d'un groupe substituant est éventuellement substitué par un ou plusieurs substituants sélectionnés indépendamment dans le groupe se composant de $C_{1-4}$alkyle, $C_{1-4}$alkoxy, halogène, hydroxy, carboxy, amino, $C_{1-4}$alkylamino, di($C_{1-4}$alkyle)amino, cyano et nitro,
où $R^P$ est sélectionné dans le groupe se composant d'hydrogène, $C_{1-8}$alkyle, $C_{1-4}$alkyle substitué par hydroxy, cycloalkyle, aryle, $C_{1-4}$aralkyle, hétéroaryle et hétérocycloalkyle,
où le cycloalkyle, aryle, hétéroaryle ou hétérocycloalkyle, soit seul ou en tant que partie d'un groupe substituant est éventuellement substitué par un à deux substituants sélectionnés indépendamment dans le groupe se composant d'halogène, hydroxy, oxo, carboxy, $C_{1-4}$alkyle, $C_{1-4}$alkoxy, $C_{1-4}$alkoxy-carbonyle, nitro, cyano, amino, $C_{1-4}$alkylamino et di($C_{1-4}$alkyle)amino,
a est un entier de 0 à 1,
$R^{10}$ est sélectionné dans le groupe se composant d'hydroxy, halogène, $C_{1-4}$alkyle, $C_{1-4}$alkoxy, $C_{1-4}$alkyle substitué par halogène, $C_{1-4}$alkoxy substitué par halogène,
à condition que les halogènes sur le $C_{1-4}$alkyle substitué par halogène et le $C_{1-4}$alkoxy substitué par halogène soient sélectionnés dans le groupe se composant de fluoro et chloro.

**3.** Un composé selon la Revendication 2 où
$R^0$ est hydrogène,
$R^1$ est sélectionné dans le groupe se composant d'hydrogène, méthyle et méthylecarbonyle,
c est un entier de 0 à 1,
$A^2$ est sélectionné dans le groupe se composant de $C_{1-4}$alkyle, où le $C_{1-4}$alkyle est éventuellement substitué par un substituant $R^Y$,
où $R^Y$ est sélectionné dans le groupe se composant de $C_{1-4}$alkyle, aryle, $C_{1-4}$aralkyle et cycloalkyle-$C_{1-4}$alkyle-

®

est sélectionné dans le groupe se composant de cycloalkyle, aryle, aryle-$C_{1-4}$alkyle, hétéroaryle et hétérocycloalkyle, où le groupe aryle, hétéroaryle ou hétérocycloalkyle, soit seul ou en tant que partie d'un groupe substituant, est éventuellement substitué par un à deux substituants indépendamment sélectionnés dans le groupe se composant de $C_{1-4}$alkyle et $C_{1-4}$alkoxy,
à condition que, lorsque c est 0, alors

®

est autre qu'aryle ou hétéroaryle,
$R^2$ est sélectionné dans le groupe se composant de $C_{1-8}$alkyle, cycloalkyle, cycloalkyle-$C_{1-4}$alkyle, aryle, $C_{1-4}$aralkyle, hétéroaryle, hétéroaryle-$C_{1-4}$alkyle-, hétérocycloalkyle et hétérocycloalkyle-$C_{1-4}$alkyle-,
où le $C_{1-8}$alkyle, cycloalkyle, aryle ou hétérocycloalkyle, soit seul ou en tant que partie d'un groupe substituant est éventuellement substitué par un à trois substituants sélectionnés indépendamment dans le groupe se composant d'halogène, $C_{1-4}$alkyle, $-SO_2-NH_2$ et phényle,
b est un entier sélectionné de 0 à 1,
$L^1$ est sélectionné dans le groupe se composant de -O-, -S-, -NH-, -$C_{1-4}$alkyle-et -$C_{2-4}$alcényle-,
$R^3$ est sélectionné dans le groupe se composant de $C_{1-4}$alkyle, cycloalkyle, cycloalkyle-$C_{1-4}$alkyle-, aryle, hétéroaryle

et hétérocycloalkyle,
où le cycloalkyle ou aryle, soit seul ou en tant que partie d'un groupe substituant est éventuellement substitué par un à deux substituants sélectionnés indépendamment dans le groupe se composant d'halogène, hydroxy, carboxy, cyano, nitro, amino, $C_{1-4}$alkylamino, di($C_{1-4}$alkylamino), $C_{1-4}$alkyle, $C_{2-4}$alcényle, $C_{1-4}$alkoxy, $C_{1-4}$alkyle substitué par hydroxy, $C_{1-4}$alkyle substitué par halogène, $C_{1-4}$alkyle substitué par cyano, $C_{1-4}$alkoxy substitué par halogène, -O-aryle, -O-$C_{1-4}$aralkyle, -S-$C_{1-4}$alkyle, -$SO_2$-$C_{1-4}$alkyle, -C(O)$C_{1-4}$alkyle, -C(O)-$NH_2$, -C(O)-NH($C_{1-4}$alkyle), C(O)-N ($C_{1-4}$alkyle)$_2$, -NH-C(O)-$C_{1-4}$alkyle, C(O)-NH-$C_{1-4}$alkyle, - NH-$SO_2$-$C_{1-4}$alkyle, -NH-$SO_2$-phényle, aryle, $C_{1-4}$aralkyle, cycloalkyle, cycloalkyle-$C_{1-4}$alkyle-, hétéroaryle, hétéroaryle-$C_{1-4}$alkyle-, hétérocycloalkyle et hétérocycloalkyle-$C_{1-4}$alkyle-,
où l'aryle, cycloalkyle, hétéroaryle ou hétérocycloalkyle, soit seul ou en tant que partie d'un groupe substituant, est éventuellement substitué par un à trois substituants sélectionnés indépendamment dans le groupe se composant de $C_{1-4}$alkyle et $C_{1-4}$alkoxy,
où a est 0,
ou un sel pharmaceutiquement acceptable de ceux-ci.

**4.** Un composé selon la Revendication 3 où
$R^0$ est hydrogène,
$R^1$ est sélectionné dans le groupe se composant d'hydrogène, méthyle et méthylecarbonyle,
c est un entier de 0 à 1,
$A^2$ est sélectionné dans le groupe se composant de -$CH_2$-, -CH($CH_2CH_3$)-, - CH(phényle)-, -CH(benzyle)- et -CH (cyclohexyleméthyle)-,

®

est sélectionné dans le groupe se composant de cyclopentyle, (S)-cyclopentyle, (R)-cyclopentyle, cyclohexyle, (R)-cyclohexyle, (S)-cyclohexyle, trans-cyclohexyle, phényle, benzyle, 9-fluorényle, 3-pyrrolidinyle, 1-indanyle, 1-(5-méthoxy-indanyle)-méthyle, 3-pipéridinyle, 4-pipéridinyle, 3-azépinyle, 2-pyridyle, 4-pyridyle, 2-furyle, 2-thiényle et 5-oxazolyle,
à condition que, lorsque c est 0, alors

®

est autre que phényle, 2-pyridyle, 4-pyridyle, 2-furyle, 2-thiényle ou 5-oxazolyle,
$Q^3$ est sélectionné dans le groupe se composant de -C(O)-, -C(O)-NH-, -C(O)-N($CH_3$)-, -(R)-C(O)-N($CH_3$), -(S)-C (O)-N($CH_3$), -C(O)-N(isopropyle)-, -C(O)-N(n-propyle)-, - C(O)-N(isobutyle)-, -C(O)-N(2-éthyle-n-hexyle)-, -C(O)-N (cyclohexyle)-, C(O)-N(4-méthylecyclohexyle)-, -C(O)O-, -NH-C(O)- et -NH-C(O)O-,
$R^2$ est sélectionné dans le groupe se composant de trifluorométhyle, méthyle, éthyle, isobutyle, t-butyle, 3-n-heptyle, 4-n-heptyle, 2-éthyle-n-hexyle, cyclopentyle, cyclohexyle, 4-méthyle-cyclohexyle, cyclopropyle-méthyle, 4-aminosulfonyle-phényléthyle, benzhydryle, 1-adamentyle, 2-adamantyle, 2-(R)-adamantyle, 2-(S)-adamantyle, 2-décahydro-isoquinolinyle, 2-(1-méthyle-pyrrolidinyle)-éthyle, 1-pipéridinyle et 4-(1-méthyle-pipéridinyle),
b est un entier sélectionné de 0 à 1,
$L^1$ est sélectionné dans le groupe se composant de -O-, -S-, -NH-, -CH($CH_3$)- et - CH=CH-,
$R^3$ est sélectionné dans le groupe se composant de n-pentyle, isopentyle, isobutyle, isopropyle, cyclopentyle, cyclopentyle-méthyle, phényle, 2-hydroxy-phényle, 3-carboxyphényle, 2-cyano-phényle, 2-nitro-phényle, 2-bromo-phényle, 2-fluoro-phényle, 2-chlorophényle, 2,6-dichloro-phényle, 2-méthyle-phényle, 3-méthyle-phényle, 2-éthyle-phényle, 4-isopropyle-phényle, 3,4-diméthyle-phényle, 3,5-diméthyle-phényle, 2,6-diméthyle-phényle, 2-méthoxy-phényle, 3-méthoxy-phényle, 4-méthoxy-phényle, 3,4-diméthoxy-phényle, 2,6-diméthoxy-phényle, 2-éthoxy-phényle, 3-éthoxy-phényle, 2-isopropoxy-phényle, 2-méthoxy-5-méthyle-phényle, 2-méthoxy-6-méthyle-phényle, 2-trifluorométhyle-phényle, 2-trifluorométhoxy-phényle, 2-méthylethio-phényle, 4-méthylethio-phényle, 2-hydroxyméthyle-phényle, 2-cyanométhyle-phényle, 2-(aminocarbonyle)-phényle, 4-(aminocarbonyle)-phényle, 2-(diméthylaminocarbonyle)-phényle, 3-(diméthylamino)-phényle, 4-(diméthylamino)-phényle, 2-allyle-6-méthyle-phényle, 2-allyle-6-éthoxy-phényle, 2-méthyle-6-n-propyle-phényle, 3-(méthylecarbonylamino)-phényle, 2-(méthylaminocarbonyle)-phényle, 2-(méthylecarbonyle)-phényle, 4-(méthylecarbonylamino)-phényle, 2-(aminocarbonylméthyle)-phényle, 2-(méthylesulfonyle)-phényle, (3-(2-méthoxy-4-méthylephényle)-sulfonylamino)-phényle, 3-(2,4,6-triméthylephénylesulfonylamino)-phényle, 3-(phénylesulfonylamino)-phényle, 2-(t-butylaminosulfonyle)-phényle, 2-(t-butylecarbonylamino)-5-méthoxy-phényle, 3-(phénylesulfonylamino)-phényle, 2-phénoxy-phényle, 3-phénoxy-phényle,

2-benzyloxy-phényle, 2-(2-benzthiazolyle)-5-méthoxy-phényle, 2-(2-benzthiazolyle)-phényle, 2-(1-pyrrolyle)-phényle, 3-(2-quinolinyle)-phényle, 2-(1-pyrrolidinyle-méthyle)-phényle, 2-cyclopentyle-phényle, 4-cyclohexyle-phényle, 4-(4-morpholinyle)-phényle, 3-méthoxy-benzyle, 1-naphtyle, 2-naphtyle, 2-(5,6,7,8-tétrahydronaphtyle), 2-biphényle, 3-biphényle, 2-biphényle-méthyle, 3-pyridyle, 3,4-méthylènedioxyphényle, 4(3,5-diméthyle-isoxazolyle), 4-pyrazolyle, 3-thiényle, 3-pyridyle, 4-pyridyle, 5-indolyle et 3-benzothiényle,
a est 0,
ou un sel pharmaceutiquement acceptable de ceux-ci.

**5.** Un composé selon la Revendication 4 où
$R^0$ est hydrogène,
$R^1$ est hydrogène,
c est un entier de 0 à 1,
$A^2$ est sélectionné dans le groupe se composant de $-CH_2-$ et $-CH(CH_2CH_3)$,

®

est sélectionné dans le groupe se composant de phényle, 4-pipéridinyle et 4-pyridyle, à condition que, lorsque c est 0, alors

®,

est autre que phényle ou 4-pyridyle,
$Q^3$ est sélectionné dans le groupe se composant de -1-C(O)O-, -3-C(O)O-, -2-C(O)-N($CH_3$)- et -3-C(O)-N($CH_3$)-,
$R^2$ est sélectionné dans le groupe se composant de méthyle, éthyle, t-butyle et cyclohexyle,
b est un entier de 0 à 1,
$L^1$ est sélectionné dans le groupe se composant de -O- et -S-,
$R^3$ est sélectionné dans le groupe se composant de phényle, 2-bromophényle, 2-chlorophényle, 2,6-dichlorophényle, 2-hydroxy-phényle, 2-hydroxyméthyle-phényle, 2-méthoxy-phényle, 3-méthoxy-phényle, 2-éthoxy-phényle, 2-méthylethio-phényle, 2-cyanométhyle-phényle, 3-(phényle-sulfonyle-amino)-phényle, 3-(2,4,6-triméthylephényle-sulfonyle-amino)-phényle, (3-(2-méthoxy-4-méthyle-phényle)-sulfonyle-amino)-phényle, 2-(t-butyle-carbonyle-amino)-5-méthoxy-phényle, 1-naphtyle, 3-thiényle et 4-(3,5-diméthylisoxazolyle),
a est 0,
ou un sel pharmaceutiquement acceptable de ceux-ci.

**6.** Un composé selon la Revendication 5 où
$R^0$ est hydrogène,
$R^1$ est hydrogène,
c est un entier de 0 à 1,
$A^2$ est sélectionné dans le groupe se composant de $-CH_2-$ et $-CH(CH_2CH_3)-$,

®,

est sélectionné dans le groupe se composant de phényle et 4-pipéridinyle,
à condition que, lorsque c est 0, alors

®

est 4-pipéridinyle,
$Q^3$ est sélectionné dans le groupe se composant de -1-C(O)O-, -3-C(O)O- et -3-C(O)-N($CH_3$)-,
$R^2$ est sélectionné dans le groupe se composant de méthyle, éthyle, t-butyle et cyclohexyle,
b est un entier de 0 à 1,
$L^1$ est sélectionné dans le groupe se composant de -O- et -S-,
$R^3$ est sélectionné dans le groupe se composant de phényle, 2-bromophényle, 2-chlorophényle, 2,6-dichlorophényle, 2-hydroxy-phényle, 2-hydroxyméthyle-phényle, 2-méthoxy-phényle, 3-méthoxy-phényle, 2-éthoxy-phényle, 2-méthylethio-phényle, 2-cyanométhyle-phényle, 3-(phényle-sulfonyle-amino)-phényle, 1-naphtyle et 3-thiényle,
a est 0,
ou un sel pharmaceutiquement acceptable de ceux-ci.

**7.** Un composé selon la Revendication 6 où
$R^0$ est hydrogène,
$R^1$ est hydrogène,
c est 1,
$A^2$ est $-CH_2-$,

®

est phényle,
$Q^3$ est $-3-C(O)-N(CH_3)-$,
$R^2$ est sélectionné dans le groupe se composant de méthyle, éthyle, t-butyle et cyclohexyle,
b est 0,
$R^3$ est sélectionné dans le groupe se composant de phényle, 2-méthoxy-phényle, 2-éthoxy-phényle et 1-naphtyle,
a est 0,
ou un sel pharmaceutiquement acceptable de ceux-ci.

**8.** Un composé selon la Revendication 4 où
$R^0$ est hydrogène,
$R^1$ est sélectionné dans le groupe se composant d'hydrogène et méthoxy,
c est 1,
$A^2$ est sélectionné dans le groupe se composant de $-CH_2-$, $-CH(CH_2CH_3)-$, - CH(phényle)- et -CH(cyclohexyle)-,

®,

est sélectionné dans le groupe se composant de phényle, 2-pyridyle et 2-furyle,
$Q^3$ est sélectionné dans le groupe se composant de $-3-C(O)-N(CH_3)$, -3-C(O)-N(isopropyle)-, -3-C(O)-N(isobutyle)-, -3-C(O)-N(cyclohexyle)-, $-4-C(O)-N(CH_3)-$ et $5-C(O)-N(CH_3)-$,
$R^2$ est sélectionné dans le groupe se composant d'isobutyle et cyclohexyle,
b est un entier de 0 à 1,
$L^1$ est sélectionné dans le groupe se composant de -O-, -S- et -NH-,
$R^3$ est sélectionné dans le groupe se composant de phényle, 2-bromophényle, 2-fluorophényle, 2-chlorophényle, 2,6-dichlorophényle, 2-hydroxyphényle, 2-hydroxyméthyle-phényle, 2-méthylephényle, 3-méthylephényle, 2,6-diméthylephényle, 2-méthoxyphényle, 3-méthoxyphényle, 2,6-diméthoxyphényle, 2-éthoxyphényle, 2-trifluorométhylephényle, 2-trifluorométhoxyphényle, 2-méthylethio-phényle, 2-nitrophényle, 2-cyanophényle, 2-cyanométhylephényle, 2-phénoxy-phényle, 2-(méthyle-carbonyle-amino)-phényle, 2-(amino-carbonyle)-phényle, 3-(phényle-sulfonyle-amino)-phényle, 2-(t-butyle-aminosulfonyle)-phényle, 2-(t-butyle-carbonyle-amino)-5-méthoxy-phényle, 4-(3,5-diméthylesoxazolyle), 1-naphtyle, 3-thiényle et 3-pyridyle,
a est 0,
ou un sel pharmaceutiquement acceptable de ceux-ci.

**9.** Un composé selon la Revendication 8 où
$R^0$ est hydrogène,
$R^1$ est hydrogène,
c est 1,
$A^2$ est sélectionné dans le groupe se composant de $-CH_2-$, $-CH(CH_2CH_3)-$ et - CH(cyclohexyle)-,

®

est sélectionné dans le groupe se composant de phényle et 2-pyridyle,
$Q^3$ est sélectionné dans le groupe se composant de $-3-C(O)-N(CH_3)-$, -3-C(O)-N(isopropyle)-, -3-C(O)-N(cyclohexyle)- et $-4-C(O)-N(CH_3)-$,

$R^2$ est cyclohexyle,
b est un entier de 0 à 1,
$L^1$ est -O-,
$R^3$ est sélectionné dans le groupe se composant de phényle, 2-bromophényle, 2-fluorophényle, 2-chlorophényle, 2-hydroxyphényle, 2-hydroxyméthyle-phényle, 3-méthylephényle, 2-méthoxyphényle, 2-éthoxyphényle, 2-cyanométhyle-phényle, 2-(t-butylecarbonyle-amino)-5-méthoxy-phényle, 1-naphtyle et 3-thiényle,
a est 0,
ou un sel pharmaceutiquement acceptable de ceux-ci.

**10.** Un composé selon la Revendication 9 où
$R^0$ est hydrogène, $R^1$ est hydrogène, c est 1, $A^2$ est $-CH_2-$,

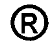

est phényle, $Q^3$ est $-3-C(O)-N(CH_3)$, $R^2$ est cyclohexyle, b est 0, $R^3$ est sélectionné dans le groupe se composant de 2-méthoxyphényle et 2-éthoxyphényle, a est 0, ou un sel pharmaceutiquement acceptable de ceux-ci.

**11.** Une composition pharmaceutique contenant un excipient pharmaceutiquement acceptable et un composé selon la Revendication 1.

**12.** Une composition pharmaceutique fabriquée par un mélange d'un composé selon la Revendication 1 et d'un excipient pharmaceutiquement acceptable.

**13.** Un processus de fabrication d'une composition pharmaceutique comprenant le mélange d'un composé selon la Revendication 1 et d'un excipient pharmaceutiquement acceptable.

**14.** Un composé selon la Revendication 1 destiné à une utilisation dans le traitement d'un trouble médié par β-sécrétase, où le trouble médié par β-sécrétase est sélectionné dans le groupe se composant de maladie d'Alzheimer (AD), déficience cognitive légère, sénilité, démence, démence avec corps de Lewy, syndrome de Down, démence associée à la maladie de Parkinson et démence associée à bêta-amyloïde.

**15.** Une composition selon la Revendication 11 destinée à une utilisation dans le traitement d'un trouble médié par β-sécrétase,
où le trouble médié par β-sécrétase est sélectionné dans le groupe se composant de maladie d'Alzheimer (AD), déficience cognitive légère, sénilité, démence, démence avec corps de Lewy, syndrome de Down, démence associée à la maladie de Parkinson et démence associée à bêta-amyloïde.

**16.** L'utilisation d'un composé selon la Revendication 1 pour la préparation d'un médicament destiné au traitement de : (a) maladie d'Alzheimer (AD), (b) déficience cognitive légère, (c) sénilité, (d) démence, (e) démence avec corps de Lewy, (f) syndrome de Down, (g) démence associée à la maladie de Parkinson et (h) démence associée à bêta-amyloïde, chez un sujet en ayant besoin.

**17.** Un composé de la formule (CII)

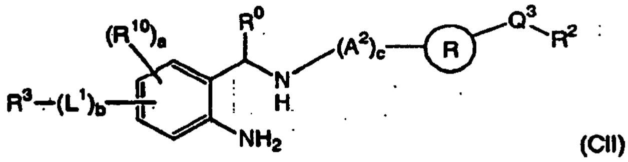

(CII)

où

$R^0$ est sélectionné dans le groupe se composant d'hydrogène, méthyle, et $CF_3$,
c est un entier de 0 à 1,
$A^2$ est sélectionné dans le groupe se composant de $C_{1-4}$alkyle, où le $C_{1-4}$alkyle est éventuellement substitué

par un ou plusieurs substituants $R^Y$,
où chaque $R^Y$ est sélectionné indépendamment dans le groupe se composant d'hydroxy, oxo, $C_{1-4}$alkyle, $C_{1-4}$alkoxy, $C_{1-4}$alkyle substitué par hydroxy, $C_{1-4}$alkyle substitué par amino, cycloalkyle, cycloalkyle-$C_{1-4}$alkyle-, biphényle, aryle, $C_{1-4}$aralkyle, hétéroaryle, hétéroaryle-$C_{1-4}$alkyle-, hétérocycloalkyle, hétérocycloalkyle-$C_{1-4}$alkyle- et spiro-hétérocyclyle,
où le groupe cycloalkyle, aryle, hétéroaryle, hétérocycloalkyle ou spiro-hétérocyclyle, soit seul ou en tant que partie d'un groupe substituant est éventuellement substitué par un ou plusieurs substituants sélectionnés indépendamment dans le groupe se composant de fluoro, chloro, hydroxy, oxo, carboxy, $C_{1-4}$alkyle, C1-4alkoxy, -C(O)O-($C_{1-4}$alkyle), $C_{1-4}$alkyle substitué par hydroxy, $C_{1-4}$alkyle substitué par fluoro, $C_{1-4}$alkoxy substitué par fluoro, 5-tétrazolyle et 1-(1,4-dihydro-tétrazole-5-one),

®

est sélectionné dans le groupe se composant d'aryle, aryle-$C_{1-4}$alkyle, cycloalkyle, carbocyclyle partiellement non saturé, hétéroaryle, hétérocycloalkyle et spiro-hétérocyclyle,
où le groupe aryle, cycloalkyle, carbocyclyle partiellement non saturé, hétéroaryle, hétérocycloalkyle ou spiro-hétérocyclyle, soit seul ou en tant que partie d'un groupe substituant, est éventuellement substitué par un ou plusieurs substituants sélectionnés indépendamment dans le groupe se composant de $C_{1-4}$alkyle, $C_{1-4}$alkoxy, $C_{1-4}$alkyle substitué par halogène, $C_{1-4}$alkoxy substitué par halogène, $C_{1-4}$alkyle substitué par hydroxy, hydroxy, carboxy, cyano, nitro, amino, $C_{1-4}$alkylamino et di($C_{1-4}$alkyle)amino,
à condition que, lorsque c est 0, alors

®

est autre qu'aryle ou hétéroaryle,
$Q^3$ est sélectionné dans le groupe se composant de -C(O)-, -C(O)O-, -C(O)-NH-, - C(O)-N($C_{1-4}$alkyle)-, -C(O)-N (cycloalkyle)-, -NH-C(O)- et -NH-C(O)O-, -où le cycloalkyle est éventuellement substitué par $C_{1-4}$alkyle,
$R^2$ est sélectionné dans le groupe se composant de $C_{1-8}$alkyle, cycloalkyle, aryle, biphényle, carbocyclyle partiellement non saturé, hétéroaryle, hétérocycloalkyle, cycloalkyle-$C_{1-4}$alkyle-, $C_{1-4}$aralkyle, carbocyclyle-$C_{1-4}$alkyle- partiellement non saturé, hétéroaryle-$C_{1-4}$alkyle-, hétérocycloalkyle-$C_{1-4}$alkyle- et spiro-hétérocyclyle,
où le $C_{1-8}$alkyle, cycloalkyle, aryle, carbocyclyle partiellement non saturé, hétéroaryle, hétérocycloalkyle ou spiro-hétérocyclyle, soit seul ou en tant que partie d'un groupe substituant est éventuellement substitué par un ou plusieurs substituants sélectionnés indépendamment dans le groupe se composant d'halogène, hydroxy, oxo, carboxy, -C(O)-$C_{1-4}$alkyle, -C(O)-$C_{1-4}$aralkyle, -C(O)O-$C_{1-4}$alkyle, -C(O)O-$C_{1-4}$aralkyle, -$C_{1-4}$alkyle-C(O) O-$C_{1-4}$alkyle, -$C_{1-4}$alkyle-S-$C_{1-4}$alkyle, -C(O)-N($R^LR^M$), -$C_{1-4}$alkyle-C(O)-N($R^LR^M$), -$NR^L$-C(O)-$C_{1-4}$alkyle, -$SO_2$-N($R^LR^M$), -$C_{1-4}$alkyle-$SO_2$-N($R^LR^M$), $C_{1-6}$alkyle, $C_{1-4}$alkyle substitué par fluoro, $C_{1-4}$alkyle substitué par hydroxy, $C_{1-4}$alkyle substitué par carboxy,$C_{1-4}$alkoxy, -O-$C_{1-4}$aralkyle, -O-(tétrahydropyranyle), -NH-OC (O)-$CH_2$-(tétrahydropyranyle), -N($CH_3$)-OC(O)-$CH_2$-(tétrahydropyranyle), nitro, cyano, amino, $C_{1-4}$alkylamino, di($C_{1-4}$alkyle)amino, phényle, 5-tétrazolyle et 1-(1,4-dihydro-tétrazole-5-one),
où le phényle ou le tétrahydropyranyle est éventuellement substitué par un ou plusieurs substituants sélectionnés indépendamment dans le groupe se composant d'halogène, hydroxy, oxo, carboxy, C(O)O-$C_{1-4}$alkyle, C (O)-$C_{1-4}$alkyle, OC(O)-$C_{1-4}$alkyle, -$C_{1-4}$alkyle-OC(O)-$C_{1-4}$alkyle, -O-$C_{1-4}$aralkyle, $C_{1-4}$alkyle, $C_{1-4}$alkyle substitué par fluoro, $C_{1-4}$alkyle substitué par hydroxy, $C_{1-4}$alkoxy, nitro, cyano, amino, $C_{1-4}$alkylamino et di($C_{1-4}$alkyle) amino,
où chaque $R^L$ et $R^M$ est sélectionné indépendamment dans le groupe se composant d'hydrogène et $C_{1-4}$alkyle,
b est un entier de 0 à 1,
$L^1$ est sélectionné dans le groupe se composant de -O-, -$S(O)_{o-2}$-, -$NR^N$-, -C(O)-, - C(S)-, -$C_{1-4}$alkyle-, -($C_{1-4}$alkyle substitué par hydroxy)- et -($C_{2-4}$alcényle)-,
où $R^N$ est sélectionné dans le groupe se composant d'hydrogène et $C_{1-4}$alkyle,
$R^3$ est sélectionné dans le groupe se composant de-$C_{1-6}$alkyle, $C_{2-6}$alcényle, cycloalkyle, carbocyclyle partiellement non saturé, aryle, biphényle, hétéroaryle, hétérocycloalkyle, cycloalkyle-$C_{1-4}$alkyle-, $C_{1-4}$aralkyle, hétéroaryle-$C_{1-4}$alkyle-, hétérocycloalkyle-$C_{1-4}$alkyle- et spiro-hétérocyclyle,
où le $C_{1-6}$alkyle, cycloalkyle, carbocyclyle partiellement non saturé, aryle, hétéroaryle, hétérocycloalkyle ou spiro-hétérocyclyle, soit seul ou en tant que partie d'un groupe substituant est éventuellement substitué par un ou plusieurs substituants sélectionnés indépendamment dans le groupe se composant d'halogène, hydroxy, oxo, carboxy, $C_{1-4}$alkyle, $C_{1-4}$alkyle substitué par hydroxy, $C_{1-4}$alkyle substitué par halogène, $C_{1-4}$alkyle subs-

titué par cyano, $C_{2-4}$alcényle, $C_{2-4}$alcynyle,$C_{1-4}$alkoxy, $C_{1-4}$alkoxy substitué par halogène, nitro, cyano, $R^5$, -O-$R^5$, -S-$R^5$, -$SO_2$-$R^5$, -$SO_2$-$NR^P$-$R^5$, -$NR^P$-$SO_2$-$R^5$, -$NH_2$, -N($R^P$)-$R^5$, -C(O)-$R^5$, -C(O)$NH_2$, -C(O)-$NR^P$-$R^5$, -$NR^P$-C(O)-$R^5$ et -$NR^P$-C(O)O-$R^5$,

où $R^5$ est sélectionné dans le groupe se composant de $C_{1-4}$alkyle, aryle, $C_{1-4}$aralkyle, carbocyclyle partiellement non saturé, cycloalkyle, cycloalkyle-$C_{1-4}$alkyle-, carbocyclyle-$C_{1-4}$alkyle- partiellement non saturé, hétéroaryle, hétéroaryle-$C_{1-4}$alkyle-, hétérocycloalkyle et hétérocyclyle-$C_{1-4}$alkyle-,

où l'aryle, carbocyclyle partiellement non saturé, cycloalkyle, hétéroaryle ou hétérocycloalkyle, soit seul ou en tant que partie d'un groupe substituant est éventuellement substitué par un ou plusieurs substituants sélectionnés indépendamment dans le groupe se composant de $C_{1-4}$alkyle, $C_{1-4}$alkoxy, halogène, hydroxy, carboxy, amino, $C_{1-4}$alkylamino, di($C_{1-4}$alkyle)amino, cyano et nitro,

où $R^P$ est sélectionné dans le groupe se composant d'hydrogène, $C_{1-8}$alkyle, $C_{1-4}$alkyle substitué par hydroxy, $C_{1-4}$alkyle substitué par $C_{1-4}$aralkyloxy, cycloalkyle, aryle, hétéroaryle, hétérocycloalkyle, cycloalkyle-$C_{1-4}$alkyle-, $C_{1-4}$aralkyle, hétéroaryle-$C_{1-4}$alkyle-, hétérocycloalkyle-$C_{1-4}$alkyle- et spiro-hétérocyclyle,

où le cycloalkyle, aryle, hétéroaryle, hétérocycloalkyle ou spiro-hétérocyclyle, soit seul ou en tant que partie d'un groupe substituant est éventuellement substitué par un ou plusieurs substituants sélectionnés indépendamment dans le groupe se composant d'halogène, hydroxy, oxo, carboxy, $C_{1-4}$alkyle, $C_{1-4}$alkoxy, $C_{1-4}$alkoxy-carbonyle, nitro, cyano, amino, $C_{1-4}$alkylamino, di($C_{1-4}$alkyle)amino, -$SO_2$-N($R^SR^T$), 5-tétrazolyle et 1-(1,4-dihydrotétrazole-5-one),

où chaque $R^S$ et $R^T$ est sélectionné indépendamment dans le groupe se composant d'hydrogène et $C_{1-4}$alkyle,

a est un entier de 0 à 3,

chaque R'° est sélectionné indépendamment dans le groupe se composant d'hydroxy, halogène, $C_{1-4}$alkyle, $C_{1-4}$alkoxy, $C_{1-4}$alkyle substitué par halogène, $C_{1-4}$alkoxy substitué par halogène, -C(O)-$NR^VR^W$, -$SO_2$-$R^VR^W$, -C(O)-$C_{1-4}$alkyle et -$SO_2$-$C_{1-4}$alkyke,

où chaque $R^V$ et $R^W$ est indépendamment sélectionné dans le groupe se composant d'hydrogène et $C_{1-4}$alkyle,

dans une variante $R^V$ et $R^W$ sont pris conjointement avec l'atome N auquel ils sont liés de façon à former une structure en anneau aromatique, saturée ou partiellement non saturée de 5 à 6 chaînons,

à condition que les halogènes sur le $C_{1-4}$alkyle substitué par halogène ou le $C_{1-4}$alkoxy substitué par halogène soient sélectionnés dans le groupe se composant de chloro et fluoro,

ou un sel pharmaceutiquement acceptable de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1407774 A
- WO 0138315 A
- EP 0371564 B1


### Non-patent literature cited in the description


- **KIENZLE, FRANK et al.** *European Journal of Medicinal Chemistry,* vol. 17 (6), 547-56
- **WEBB, THOMAS H ; WILCOX.** *Journal of Organic Chemistry,* 1990, vol. 55 (1), 363-365
- **T.W. GREENE ; P.G.M. WUTS.** Protective Groups in Organic Synthesis. John Wiley & Sons, 1999
- **T.W. GREENE ; P.G.M. WUTS.** Protective Groups in Organic Synthesis. John Wiley & Sons, 1991
- Protective Groups in Organic Chemistry. Plenum Press, 1973
- **KATRITZKY, A.R. ; CHASSAING, C. ; TOADER, D. ; GILL, K.** *J. Chem. Research,* 1999, 504-505
- **KATRITZKY, A.R. ; LANG, H. ; WANG, Z. ; ZHANG, Z. ; SONG, H.** *J. Org. Chem.,* 1990, vol. 60, 7619-7624
- **VETELINO, M.G. ; COE, J.W.** *Tetrahedron Lett.,* 1994, vol. 35 (2), 219-22
- **MIYAURA, N. ; SUZUKI, A.** *Chem. Rev.,* 1995, vol. 95, 2457
- **SUZUKI, A.** *J. Organomet. Chem.,* 1999, vol. 576, 147
- **MIYAURA, N. ; SUZUKI, A.** *Chem. Rev.,* 1995, vol. 95, 2457
- **LHERMITTE, F. ; CARBONI, B.** *SYNLETT,* 1996, 377
- **MATSUBARA, S. ; OTAKE, Y. ; HASHIMOTO, Y. ; UTIMOTO, K.** *Chem. Lett.,* 1999, 747
- **TAKAI, K. ; SHINOMIYA, N. ; KAIHARA, H. ; YOSHIDA, N. ; MORIWAKE, T.** *SYNLETT,* 1995, 963
- **DELOUX, L. ; SREBNIK, M.** *J. Org. Chem.,* 1994, vol. 59, 6871
- **YANG, D. ; YIP, Y.-C. ; JIAO, G.-S. ; WONG, M.-K.** *Org. Synth.,* 2000, vol. 78, 225
- **JUNG, M. E. ; LAM, P. Y.-S. ; MANSURI, M. M. ; SPELTZ, L. M.** *J. Org. Chem.,* 1985, vol. 50, 1087
- **BAKKE, J. M. ; LORENTZEN, G. B.** *Acta Chem. Scand. B,* 1974, vol. 28, 650
- **BAUMGARTH, M. ; BEIER, N. ; GERICKE, R.** *J. Med. Chem.,* 1998, vol. 41, 3736
- **JUNG, M.E. ; DANSEREAU, S.M.K.** *Heterocycles,* 1994, vol. 39, 767
- **HU, Y.-Z. ; ZHANG, G. ; THUMMEL, R.P.** *Org. Lett.,* 2003, vol. 5, 2251
- **BURK, M. J. ; GROSS, M. F. ; MARTINEZ, J. P.** *J. Am. Chem. Soc.,* 1995, vol. 117, 9375
- **SMRCINA, M. ; MAJER, P. ; MAJEROVÁ, E. ; GUERASSINA, T. A. ; EISSENSTAT, M. A.** *Tetrahedron,* 1997, vol. 53, 12867
- **HINTERMANN, T. ; GADEMANN, K. ; JAUN, B. ; SEEBACH, D.** *Helv. Chim. Acta,* 1998, vol. 81, 983
- *J. Med. Chem.,* 1985, vol. 28, 164
- **ERMOLIEFF, J. ; LOY, J.A. ; KOELSCH, G. ; TANG, J.** Proteolytic activation of recombinant pro-memapsin 2 (pro-β-secretase) studied with new fluorogenic substrates. *Biochemistry,* 2000, vol. 39, 12450